# EUROPEAN PATENT SPECIFICATION

(11) **EP 3 354 653 B1**
(45) Date of publication and mention of the grant of the patent: **04.09.2019**
(21) Application number: 16827250.8
(22) Date of filing: 21.07.2016
(51) Int. Cl.: C07D 495/04, C07D 471/04, C07D 405/14, C07D 401/14, A61P 9/10, A61P 35/00, A61P 35/02, A61P 37/06, A61K 31/519

(54) **FUSED RING PYRIMIDINE COMPOUND, INTERMEDIATE, AND PREPARATION METHOD, COMPOSITION AND USE THEREOF**
KONDENSIERTE RINGPYRIMIDINVERBINDUNG, ZWISCHENPRODUKT UND HERSTELLUNGSVERFAHREN, ZUSAMMENSETZUNG UND VERWENDUNG DAVON
COMPOSÉ PYRIMIDINE À CYCLES CONDENSÉS, INTERMÉDIAIRE, ET PROCÉDÉ DE PRÉPARATION, COMPOSITION ET UTILISATION ASSOCIÉE

(30) Priority: 21.07.2015 CN 201510430641
(43) Date of publication of application: 01.08.2018
(73) Proprietor: Guangzhou Maxinovel Pharmaceuticals Co. Ltd., Guangzhou, Guangdong 510555 (CN)
(72) Inventor: XU, Zusheng, Shanghai 201203 (CN); ZHANG, Nong, Shanghai 201203 (CN); WANG, Tinghan, Shanghai 201203 (CN); SUN, Qingrui, Shanghai 201203 (CN); WANG, Yuguang, Shanghai 201203 (CN)
(74) Representative: Rentsch Partner AG
(86) International application number: PCT/CN2016/090798
(87) International publication number: WO 2017/012559

(56) References cited:
- WO-A1-2009/062258
- WO-A1-2011/079231
- WO-A1-2012/030894
- WO-A1-2014/023385
- WO-A1-2014/037750
- WO-A1-2016/133935
- WO-A2-2011/049332
- WO-A2-2015/027222
- US-A1- 2015 175 601

## Description

The present application claims the priority of Chinese Patent Application CN201510430641.5 filed on July 21, 2015.

### Field of invention

The present invention relates to a fused ring pyrimidine compound, an intermediate, a preparation method, a composition and a use thereof.

### Prior arts

JAK-STAT (Janus kinase-signal transducer and activator of transcription) signal pathway is a cytokine-stimulated signal transduction pathway found in recent years and is involved in many important biological processes such as cell proliferation, differentiation, apoptosis and immune regulation (Aaronson, DS et al. Science 2002, 296, 1653-1655; O 'Shea, JJ et al. Nat. Rev. Drug Discovery 2004, 3, 555-564). Compared with other signal pathways, this signal pathway is relatively simple. It mainly consists of three components which areatyrosine kinase related receptor, atyrosine kinase JAK and atranscription factor STAT. JAK (Janus Kinase), a class of molecules in the cells, is rapidly raised on the receptor and activated, after receiving signals from the upstream receptor molecules. The activated JAK catalyzes tyrosine phosphorylation of the receptor, and phosphorylated tyrosine on the receptor molecules is the recognition and binding site of STAT SH2, a class of signal molecules. Tyrosine phosphorylation also occurs after STAT binds to the receptor. Tyrosine phosphorylated STAT forms dimer and enters the nucleus. As an active transcription factor, dimeric STAT molecules directly affect the expression of related genes, thereby changing the proliferation or differentiation of target cells.

The JAK-STAT pathway widely presents in various tissue cells *in vivo,* and plays an important role in differentiation, proliferation and anti-infection of lymphocyte lines and is involved in the interaction and signal transduction of various inflammatory factors (Kiesseleva T. et al. J. Gene, 2002, 285, 1-24). Abnormal activation of this pathway is closely related to many diseases. To find and screen JAK inhibitors can help further study the regulation mechanism of JAK-STAT and provide new drugs and methods for the prevention and treatment of related diseases.

The formation, growth, invasion and metastasis of tumors are related to JAK-STAT signal transduction pathway. The activation of STATs in normal signal transduction is rapid and transient, and the persistent activation of STATs is closely related to the malignant transformation process of cells (Buettner R. et al. Clin. Cancer Res. 2002, 8(4), 945-954). STAT3 is the focal point of many oncogenic tyrosine kinase signal pathways such as EGFR, IL-6/JAK and Src etc. and is activated in many tumor cells and tissues such as breast cancer, ovarian cancer, head and neck squamous cell carcinoma cancer, prostate cancer, malignant melanoma, multiple myeloma, lymphoma, brain tumor, non-small cell lung cancer and various leukemias (Niu G. et al. Oncogene 2002, 21(13), 2000-2008). JAK-STAT pathway inhibitor belongs to PTK inhibitors, and the enzyme is a member of the oncogene protein and proto-oncoprotein family and plays an important role in the normal and abnormal proliferation of cells. The development and growth of tumors cannot be separated from PTK, therefore, JAK-STAT pathway inhibitor inhibits tumor growth by antagonizing PTK and has obvious anti-tumor effect (Mora L.B. et al. J. Cancer Res. 2002, 62(22), 6659-6666).

In addition, recent studies have shown that organ transplant rejection, psoriasis, tissue and organ fibrosis, bronchial asthma, ischemic cardiomyopathy, heart failure, myocardial infarction, hematological and immune system diseases are all closely related to JAK-STAT signal transduction pathway. This signal pathway is not only important for maintaining the normal physiological function of cells, but also plays an important regulatory role in the occurrence and development of the disease.

The family of fibroblast growth factor receptors belongs to a new family of receptor kinases, and includes four receptor subtypes encoded by four closely related genes (FGFR-1, 2, 3 and 4) and some isomeric molecules which participate in regulating physiological processes in living organisms through forming ternary complexes with fibroblast growth factor (FGF) and heparan sulfate and then triggering a series of signal transduction pathways. FGFR has a wide range of physiological and pathological functionsin the body: (1) Embryonic development. Studies have shown that during the process of embryonic development, FGFR signal transduction is crucial for most organ development and embryonic pattern formation. (2) Cell division, migration and differentiation. FGFR, which stimulates cell proliferation and is involved in the regulation of cell transformation during pathological process, has many parallel pathways that enable FGFR-mediated signal transduction of cell division as evidenced by many studies (J.K. Wang et al., Oncogene1997, 14, 1767-1778.). (3) Bone disease. Bone growth and differentiation are also regulated by the FGF family, and mutations in FGFR can lead to skeletal deformities (R.Shang et al., Cell1994, 78, 335-342.). (4) Tumor development. FGFR promotes the migration, proliferation and differentiation of endothelial cells and plays an important role in the regulation of vascularization and angiogenesis. Uncontrolled angiogenesis may lead to the development of tumors and the growth of metastases (J. Folkman. Nat. Med. 1995, 1, 27-31.).

FMS-like tyrosine kinase 3 (FLT3) is a family member of receptor tyrosine kinase III (RTK III), and is composed of three parts, extracellular region, intracellular region and transmembrane region. It is first expressed in human hematopoietic stem cells, where FLT3 interacts with its ligand FL to stimulate or act on stem cells, which is of great importance for the growth and differentiation of stem cell. FLT3 kinase has wild type FLT3-WT and its major activating mutations FLT3-ITD and FLT3-D835Y. FLT3 is mainly expressed in the precursors of normal myeloid cells, but its abnormal expression is also found in a large part of acute myeloid leukemia (AML). In recent years, many large sample studies have confirmed that activating mutations of FLT3 play a very important pathological role in the pathogenesis and progression of acute myeloid leukemia. FLT3 has become an important target for the treatment of acute myeloid leukemia.

Src family kinase (SFK) is a family of non-receptor tyrosine kinases, including c-Src, LYN, FYN, LCK, HCK, FGR, BLK, YES and YRK, among which LYN kinase has two subtypes of LYNα and LYNβ, and LYN kinase and its two subtypes can cause similar intracellular tyrosine phosphorylation. According to the amino acid sequence, SFK can be divided into two subfamilies: a subfamily of c-Src, FYN, YES and FGR, widely expressed in different tissues; the other subfamily of LCK, BLK, LYN and HCK, closely related to hematopoietic cells. SFK is linked to multiple *in vivo* signal transduction pathways and is activated by growth factors, cytokines and immune cell receptors, G-protein coupled receptors, and integrins and other cell adhesion molecules, and then activating the corresponding signal transduction pathway, causing a variety of physiological effects of the cell. The activity of SFK mainly includes the regulation of cell morphology, cell motility, cell proliferation and survival. Abnormal activation and expression of these kinases lead to the development and progression of a wide range of diseases, such as a large number of solid tumors, a variety of hematological malignancies and some neuronal pathologies. Therefore, finding SFK inhibitors is a promising research topic in the field of medicinal chemistry.

WO2011049332A2 discloses thieno[3,2-d]pyrimidines which are substituted at the 3-position by a phenyl or heteroaryl which may be lined by amino, and at the 5-position by heteroaryloamino. The heteroaryl group represented by A may be pyrazolyl (see paragraph [0050]. However, no examples in which A is pyrazolyl are disclosed.

WO2015027222A2 discloses quinazolines which are substituted by aryl or heteroaryl at the 8-position and by heteroarylamino at the 2-position (see page 2, formula 1). The heteroaryl group represented by A may be pyrazolyl (see paragraph [006]). No examples in which A is pyrazolyl have been prepared.

WO2009062258A1 discloses thieno[3,2-d]pyrimidines substituted by phenyl at the 3-position and by phenylamino or pyridylamino at the 5-position (see table). Compounds in which the 5-position is substituted by pyrazolylamino are not disclosed.

WO2011079231A1 discloses some bicyclic heteroaryl compounds, including thieno[2,3-d]pyrimidines substituted by phenyl at the 3-position and by phenylamino at the 5-position (see tables and example 3).

WO2014023385A1 discloses pyrido[4,3-d]pyrimidines substituted at the 2-position by cyclohexyl or piperidin-3-ylamiethylamino, and at the 8-position by indol-3-yl (see examples).

WO2014037750A1 discloses pyrido[3,4-d]pyrimidines substituted at the 8-position by groups including pyrazol-4-yl and at the 2-position by phenylamino (see examples 91 and 95-98).

US2015175601A1 discloses 2-chloro-8-(3-aminophenyl)-quinazoline (example 43 intermediate on page 89, paragraph [2059]). The products of examples 44 to 129 were also prepared according to the process of example 43. Consequently, intermediates 2-chloro-8-(3-aminophenyl)-qujnazoline in which the phenyl is also substituted by 2-fluoro, 2-chloro-, 2-methoxycarbonyl, 2-methoxy, or 6-fluoro are implicitly disclosed, as well as 2-chloro-8-pyridinyl-quinazoline.

WO2016133935A1 discloses 2-chloro-8-phenyl-quinazoline (example 1, paragraph [0292]). The compounds of examples 4, 5, and 7-12 were prepared according to the same process as example i. Consequently, 2-chloro-8- phenylquinazoline in which the phenyl is substituted by 3-chloro-3-fluoro, 2-chloro, 2-fluoro-, as well as 2-chloro-8-(pyridin--3-yl or pyridin-4-yl)-quinazoline are implicitly disclosed.

WO2012030894A1 discloses thieno[2,3-d]pyrimidines which are substituted at the 4-position by pyrazol-4-ylamino, and at the 2-position by phenylmethyl or phenylcarbonyl. These compounds are also JAK kinsae inhihitors.

### Content of the present invention

The technical problem to be solved in the present invention is to provide a fused ring pyrimidine compound, an intermediate, a preparation method, a composition and a use thereof. The compound has a strong inhibitory effect on Janus kinase (JAK), FGFR kinase, FLT3 kinase and Src family kinase.

The present invention provides a fused ring pyrimidine compound of fomula I, a tautomer, an enantiomer, a diastereoisomer or a pharmaceutically acceptable salt thereof; 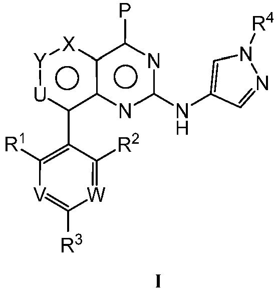
wherein, P is selected from a hydrogen atom or a deuterium atom;
X is selected from CH or S;
Y is selected from N or CR⁵;
U is selected from a chemical bond or CH;
V is selected from N or CH;
W is selected from N or CR⁶;
each of R¹, R², R³ and R⁶ isindependently selected from the group consisting of a hydrogen, a deuterium, a halogen, a substituted or unsubstituted alkyl, 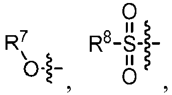 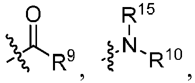 a cycloalkyl or a heterocycloalkyl; each of R⁷, R⁸, R⁹, R¹⁰ and R¹⁵ is independently selected from the group consisting of a hydrogen, a deuterium, a halogen, a hydroxyl, an amino, a substituted or unsubstituted alkyl, an alkoxy, 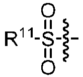 or a heterocycloalkyl; R¹¹ is a hydrogen, a deuterium or an alkyl(preferably a C₁₋₄ alkyl, such as a methyl); or, R⁶, R² and the two atoms on the ring to which they are attached form a "substituted or unsubstituted 5- to 7-membered carbon heterocycle"; or, R⁶, R³ and the two atoms on the ring to which they are attached form a "substituted or unsubstituted 5- to 7-membered carbon heterocycle"; the heteroatom contained in the "substituted or unsubstituted 5- to 7-membered carbon heterocycle" is selected from the group consisting of nitrogen, oxygen and sulfur;
R⁴ is a hydrogen, a deuterium, a substituted or unsubstituted alkyl, an alkoxy, a cycloalkyl, or a substituted or unsubstituted heterocycloalkyl;
R⁵ is a hydrogen, a deuterium, a halogen, or an alkyl;
in the definitions of R¹, R², R³ and R⁶, the "substituted" in "a substituted or unsubstituted alkyl" means to be substituted with the substituents selected from the group consisting of a halogen(preferably fluorine), a hydroxyl, an amino, an alkyl(preferably a C₁₋₁₀ alkyl, more preferably a C₁₋₄ alkyl, such as a methyl), an alkoxy(preferably a C₁₋₁₀ alkoxy, more preferably a C₁₋₄ alkoxy, such as a methoxy), 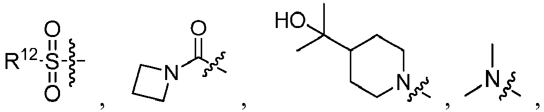 and a heterocycloalkyl (the heterocycloalkyl may be linked to other groups via a carbon atom or a heteroatom thereof; preferably, "a heterocycloalkyl with 1-4 heteroatoms and 3-8 carbon atoms in which the heteroatom is oxygen and/or nitrogen"; more preferably,"a heterocycloalkyl with 1-4 (for example 1 or 2) heteroatoms and 3-6 carbon atoms in which the heteroatom is oxygen and/or nitrogen"; most preferably, 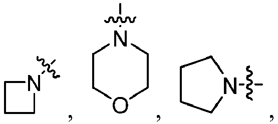 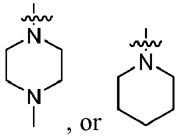 ), in the case when multiple substituents are present, the substituents are the same or different; R¹² is a hydrogen, a deuterium, or an alkyl(preferably a C₁₋₄ alkyl, such as a methyl);
in the definitions of R⁷, R⁸, R⁹, R¹⁰ and R¹⁵, the "substituted" in "a substituted or unsubstituted alkyl" means to be substituted with the substituents selected from the group consisting of a deuterium, a halogen(preferably fluorine), a hydroxyl, an amino, an alkyl(preferably a C₁₋₁₀ alkyl, more preferably a C₁₋₄ alkyl, such as a methyl), an alkoxy(preferably a C₁₋₁₀ alkoxy, more preferably a C₁₋₄ alkoxy, such as a methoxy), 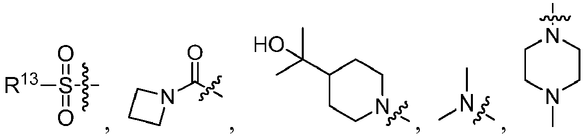 and a heterocycloalkyl(the heterocycloalkyl may be linked to other groups via a carbon atom or a heteroatom thereof; preferably, "a heterocycloalkyl with 1-4 heteroatoms and 3-8 carbon atoms in which the heteroatom is oxygen and/or nitrogen"; more preferably,"a heterocycloalkyl with 1-4 (for example 1 or 2) heteroatoms and 3-6 carbon atoms in which the heteroatom is oxygen and/or nitrogen"; most preferably, 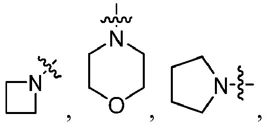 or 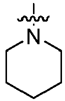 ), in the case when multiple substituents are present, the substituents are the same or different; R¹³ is a hydrogen or an alkyl(preferably a C₁₋₄ alkyl, such as a methyl);
in the definition of R⁴, the "substituted" in "a substituted or unsubstituted alkyl" and "a substituted or unsubstituted heterocycloalkyl" means to be substituted with the substituents selected from the group consisting of a hydroxyl, an alkyl(preferably a C₁₋₄ alkyl, such as a methyl), 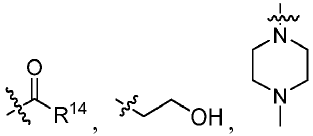 or a heterocycloalkyl(the heterocycloalkyl may be linked to other groups via a carbon atom or a heteroatom thereof; preferably, "a heterocycloalkyl with 1-4 heteroatoms and 3-6 carbon atoms in which the heteroatom is oxygen and/or nitrogen"; more preferably, 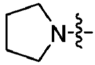 ), in the case where multiple substituents are present, the substituents are the same or different; R¹⁴ is a hydrogen, an alkyl(preferably a C₁₋₄ alkyl, more preferably a methyl), a hydroxymethyl or an alkoxy(preferably a C₁₋₄ methoxy, more preferably a tert-butoxy or an ethoxy);
the "substituted" in "substituted or unsubstituted 5- to 7-membered carbon heterocycle" means to be substituted with one or more than one alkyl(preferably a C₁₋₄ alkyl, such as a methyl, an ethyl, a propyl and the like).

In the definitions of R¹, R², R³ and R⁶, the halogen is fluorine or chlorine;the alkyl in "substituted or unsubstituted alkyl" is preferably a C₁₋₄ alkyl, more preferably a methyl; the heterocycloalkyl may be linked to other groups via a carbon atom or a heteroatom thereof; the heterocycloalkyl is preferably "a heterocycloalkyl with 1-4 heteroatoms and 3-8 carbon atoms in which the heteroatom is oxygen and/or nitrogen", more preferably "a heterocycloalkyl with 1-4 (for example 1 or 2) heteroatoms and 3-6 carbon atoms in which the heteroatom is oxygen and/or nitrogen", most preferably

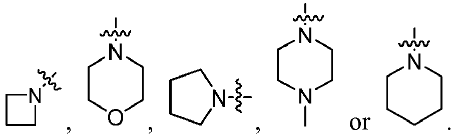

In the definitions of R⁷, R⁸, R⁹, R¹⁰ and R¹⁵, the halogen is preferably fluorine;the alkyl in "substituted or unsubstituted alkyl" is preferably a C₁₋₁₀ alkyl, more preferably a C₁₋₄ alkyl, most preferably a methyl, atrideuteromethyl, an ethyl, a propyl or an isopropyl; the alkoxy is preferably a C₁₋₁₀ alkoxy, more preferably a C₁₋₄ alkoxy, most preferably a methoxy; the heterocycloalkyl may be linked to other groups via a carbon atom or a heteroatom thereof; the heterocycloalkyl is preferably "a heterocycloalkyl with 1-4 heteroatoms and 3-8 carbon atoms in which the heteroatom is oxygen and/or nitrogen", more preferably "a heterocycloalkyl with 1-4 (for example 1 or 2) heteroatoms and 3-6 carbon atoms in which the heteroatom is oxygen and/or nitrogen", most preferably

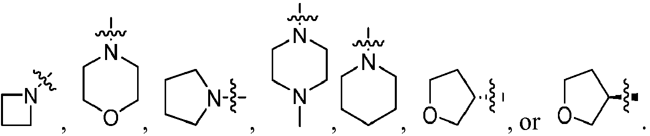

In the definition of R⁴, the alkyl in "substituted or unsubstituted alkyl" is preferably a C₁₋₄ alkyl, more preferably a methyl, an ethyl, a propyl or an isopropyl; the alkoxy is preferably a C₁₋₄ alkoxy; the heterocycloalkyl in "substituted or unsubstituted heterocycloalkyl"may be linked to other groups via a carbon atom or a heteroatom thereof; the heterocycloalkyl in "substituted or unsubstituted heterocycloalkyl" is preferably "a heterocycloalkyl with 1-4 heteroatoms and 3-8 carbon atoms in which the heteroatom is oxygen and/or nitrogen", more preferably "a heterocycloalkyl with 1-2 heteroatoms and 3-6 carbon atoms in which the heteroatom is oxygen or nitrogen", such as

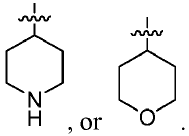

In the definition of R⁵, the halogen is preferably fluorine; the alkyl is preferably a C₁₋₄ alkyl, more preferably a methyl.

The "5- to 7-membered carbon heterocycle" in "substituted or unsubstituted 5- to 7-membered carbon heterocycle" is preferably "a 5- to 7-membered carbon heterocycle with 1-4 heteroatoms and 2-6 carbon atoms in which the heteroatom is oxygen and/or nitrogen", more preferably

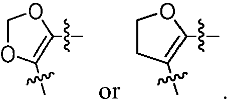

The compound I is preferably of a structure shown as formula I-1 or 1-2, 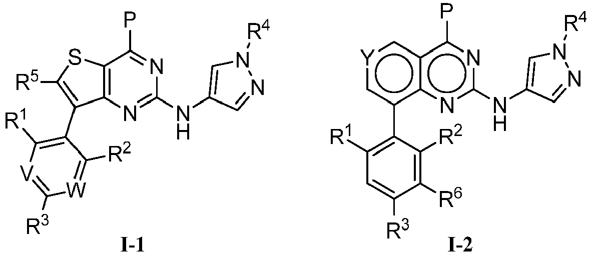 wherein, each of R¹,R²,R³,R⁴,R⁵,R⁶,Y, V, W and P is independently defined as above.

The compound I-1 is preferably of a structure shown as I-1-1 or 1-1-2, 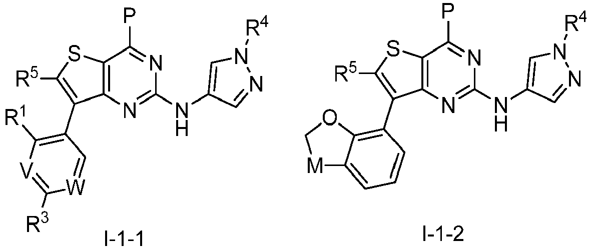 wherein, M is CH₂ or O; each of R¹,R³,R⁴,R⁵, P, V, and W is independently defined as above.

The compound 1-2 is preferably of a structure shown as 1-2-1 or 1-2-2, 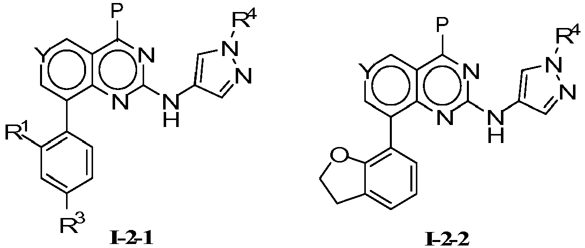 wherein, each of R¹,R³,R⁴,Y and P is independently defined as above.

In the definition of compound I, Y is preferably CR⁵.

In the definition of compound I, R⁵ is preferably a hydrogen or an alkyl.

In the definition of compound I, W is preferably CR⁶.

In the definition of compound I, R⁶ is preferably a hydrogen.

In the definition of compound I, preferably, R⁶ and R² together with two atoms on the ring to which they are attached form "a substituted or unsubstituted 5- to 7-membered carbon heterocycle".

In the definition of compound I, preferably, R¹ and R² are independently a hydrogen or

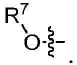

In the definition of compound I, preferably, R¹ or R² is a hydrogen.

In the definition of compound I, R³ is preferably a hydrogen, 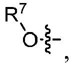 a halogen, or

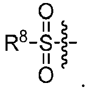

In the definition of compound I, R⁴ is preferably "a substituted or unsubstituted alkyl", or "a substituted or unsubstituted heterocycloalkyl".

For the target of JAK1, each of the substituents mentioned above is preferably as follows:

In the definition of compound I, Y is preferably CR⁵.

In the definition of compound I, R⁵ is preferably a hydrogen or an alkyl.

In the definition of compound I, W is preferably CR⁶.

In the definition of compound I, R⁶ is preferably a hydrogen.

In the definition of compound I, preferably, R⁶ and R² together with two atoms on the ring to which they are attached form "a substituted or unsubstituted 5- to 7-membered carbon heterocycle".

In the definition of compound I, preferably, each of R¹ and R² is independently a hydrogen or

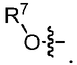

In the definition of compound I, preferably, R¹ or R² is a hydrogen.

In the definition of compound I, R³ is preferably a hydrogen, 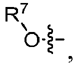 a halogen, or

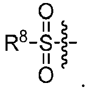

In the definition of compound I, R⁴ is preferably "a substituted or unsubstituted alkyl", or "a substituted or unsubstituted heterocycloalkyl".

For the target of JAK2, each of the substituents mentioned above is preferably as follows:

In the definition of compound I, X is preferably S.

In the definition of compound I, Y is preferably CR⁵.

In the definition of compound I, R⁵ is preferably an alkyl.

In the definition of compound I, U is preferably a chemical bond.

In the definition of compound I, W is preferably CR⁶.

In the definition of compound I, R⁶ is preferably a hydrogen.

In the definition of compound I, preferably, R⁶ and R² together with two atoms on the ring to which they are attached form "a substituted or unsubstituted 5- to 7-membered carbon heterocycle".

In the definition of compound I, preferably, each of R¹ and R² is independently a hydrogen or

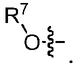

In the definition of compound I, preferably, R¹ or R² is a hydrogen.

In the definition of compound I, R³ is preferably a hydrogen, 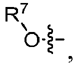 a halogen, or

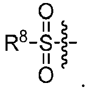

In the definition of compound I, R⁴ is preferably "a substituted or unsubstituted alkyl", or "a substituted or unsubstituted heterocycloalkyl".

For the target of JAK3, each of the substituents mentioned above is preferably as follows:

In the definition of compound I, X is preferably S.

In the definition of compound I, Y is preferably CR⁵.

In the definition of compound I, R⁵ is preferably an alkyl.

In the definition of compound I, W is preferably CR⁶.

In the definition of compound I, R⁶ is preferably a hydrogen.

In the definition of compound I, preferably, R⁶ and R² together with two atoms on the ring to which they are attached form "a substituted or unsubstituted 5- to 7-membered carbon heterocycle".

In the definition of compound I, preferably, each of R¹ and R² is independently a hydrogen or

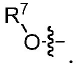

In the definition of compound I, preferably, R¹ or R² is a hydrogen.

In the definition of compound I, R³ is preferably a hydrogen or

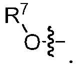

In the definition of compound I, R⁴ is preferably "a substituted or unsubstituted alkyl", or "a substituted or unsubstituted heterocycloalkyl". Preferably, the compound I of the present invention is selected from the group consisting of

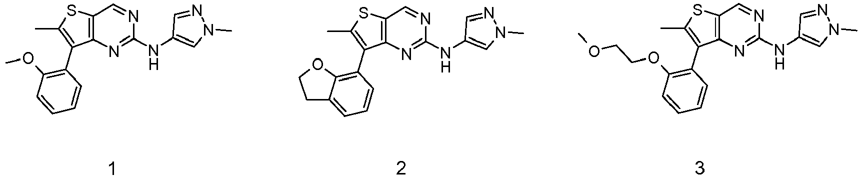

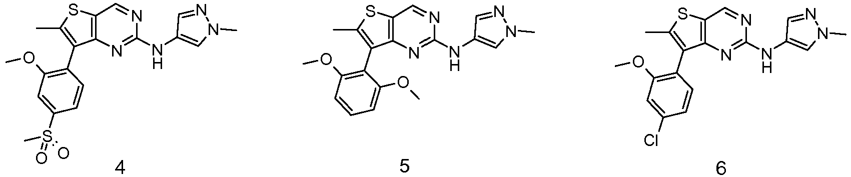

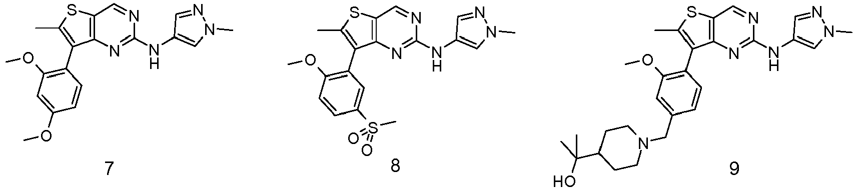

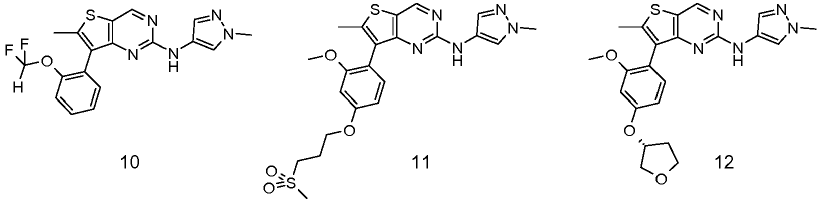

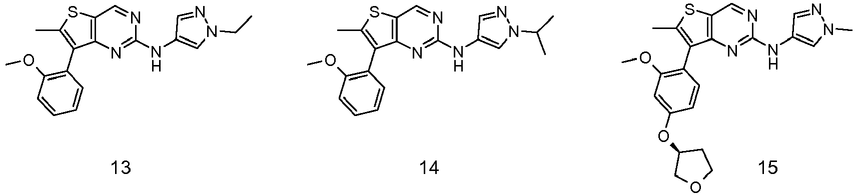

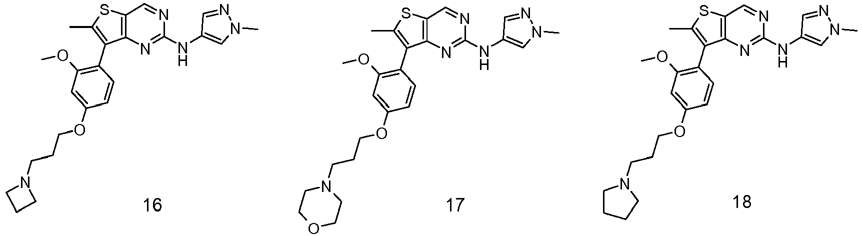

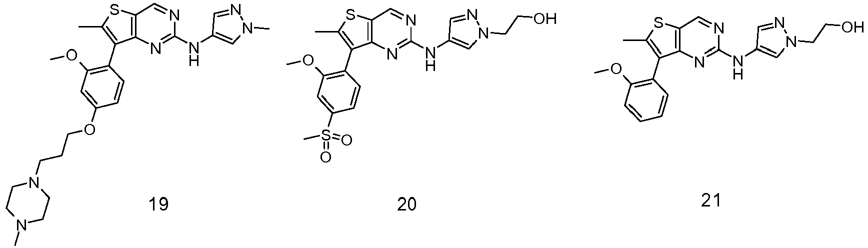

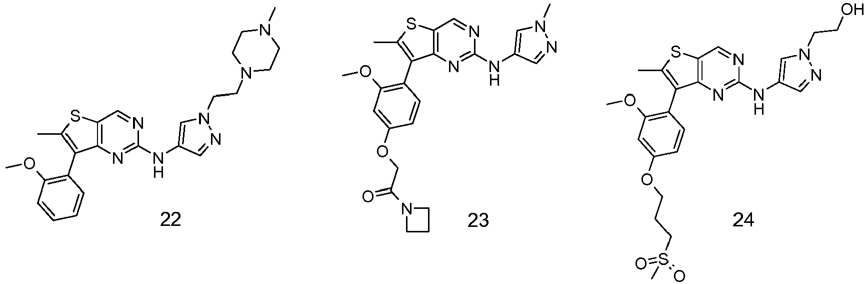

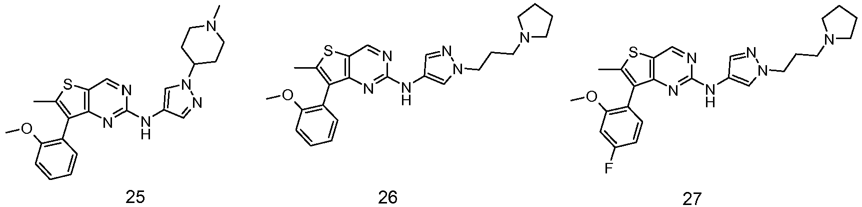

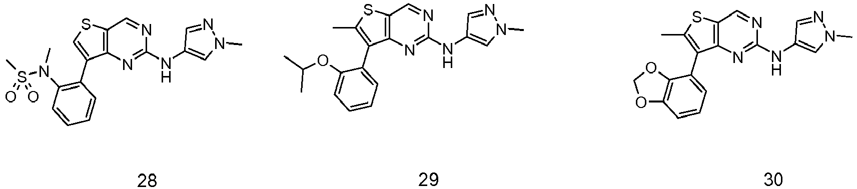

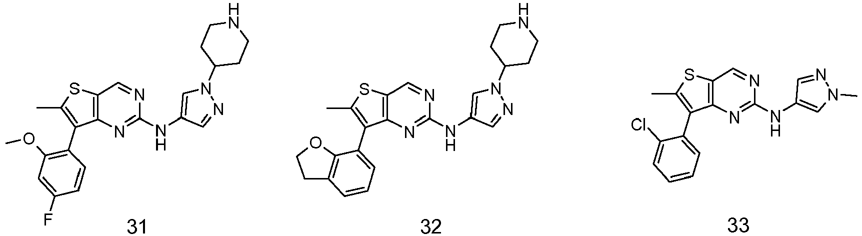

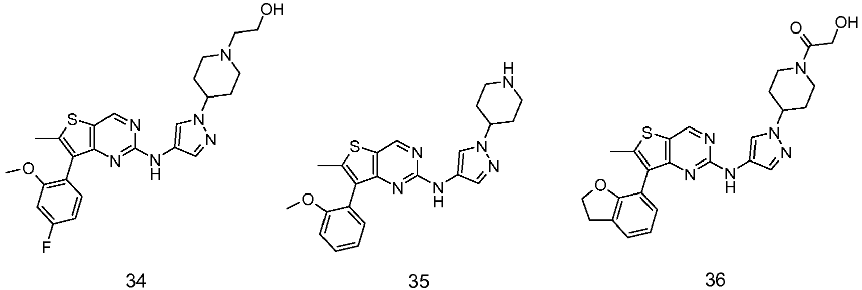

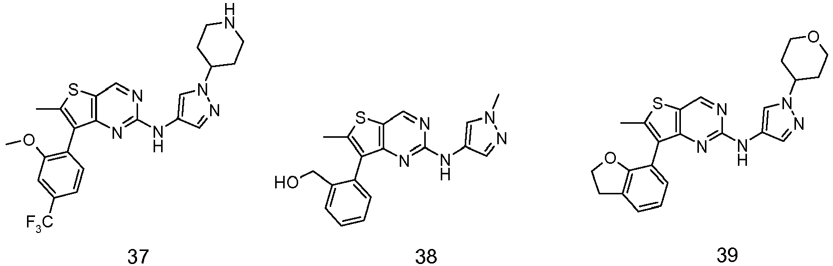

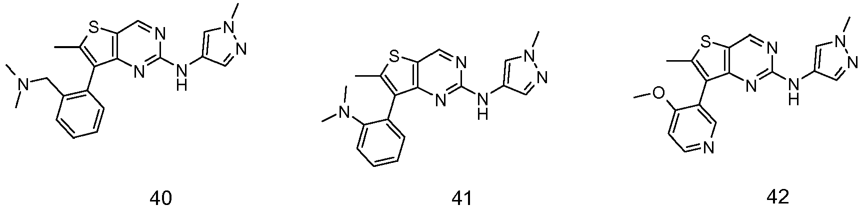

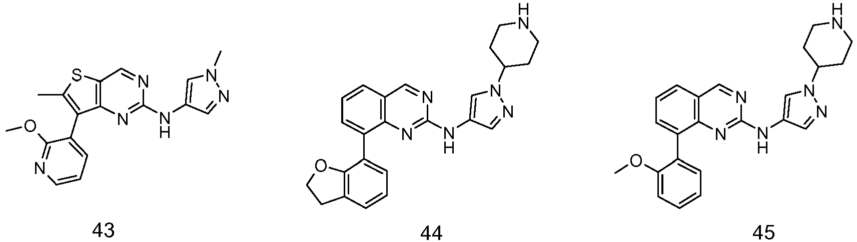

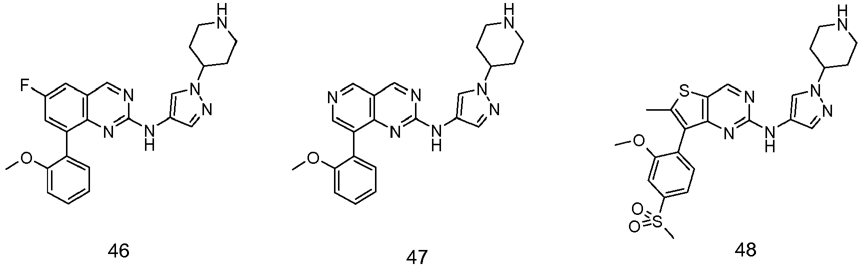

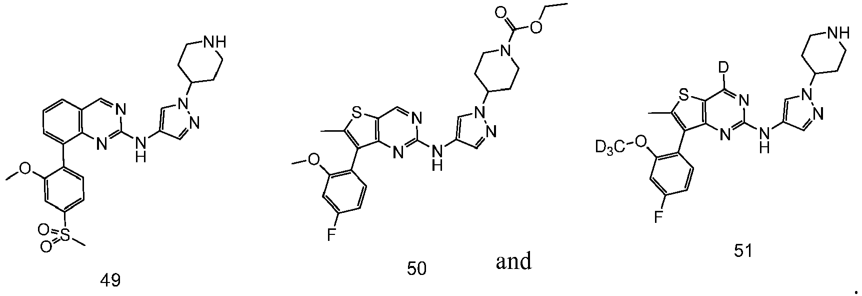

The compound I of the present invention may exhibit tautomerism, structural isomerism and stereoisomerism. The present invention includes any tautomeric or structural or stereoisomeric forms thereof and mixtures thereof that have the ability to modulate kinase activity and this ability is not limited to any one of the isomeric forms or mixtures thereof; the kinases are preferably JAK, FGFR kinase, FLT3 kinase and Src family kinase.

In the present invention, the isotopes of the atoms contained in the fused ring pyrimidine compound of formula I, the tautomer, the enantiomer, the diastereoisomer or the pharmaceutically acceptable salt thereof usually present according to the distribution of each isotopic abundance in nature. The isotopic abundance, also known as the relative isotopic abundance, refers to relative contents(in atomic percent) of various isotopes of an element existing in nature, for example, the isotopic abundance of hydrogen atom: ¹H=99.985%,D=0.015%; the isotopic abundance of oxygen atom: ¹⁶O=99.76%,¹⁷O=0.04%,¹⁸O=0.20%.

In the present invention, one or more than one isotopes of the atoms contained in the fused ring pyrimidine compound of formula I, the tautomer, the enantiomer, the diastereoisomer or the pharmaceutically acceptable salt thereof may be arbitrarily replaced, for example, ¹H is replaced by D, and the isotope-replaced compound can be prepared according to the preparation method of the pre-replacement compound and has the same biological activity as the pre-replacement compound. In the present invention, the isotopes may be those existing in nature or those artificially produced.

The present invention also provides a process for preparing the compound of formula I, which is any one of processes 1-13,
process 1 comprising the steps of carrying out a substitution reaction with compound **1-a** and a methylation reagent in an organic solvent(preferably acetone)and in the presence of a base(preferably potassium carbonate) to give the compound of formula I; the conditions for the substitution reaction may be conventional conditions for this type of reaction in the art; 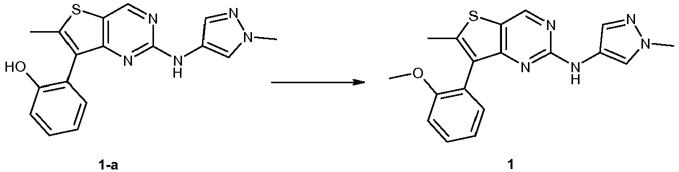
process 2 comprising the steps of carrying out a substitution reaction with compound II and compound VI in an organic solvent(preferably *n*-butanol and/or N, N-dimethylformamide)and in the presence of a catalyst(preferably selected from the group consisting of *p*-toluenesulfonic acid, *p*-toluenesulfonic acid monohydrate and tris(dibenzylidene-indan-acetone)dipalladium)) to give the compound of formula I; the conditions for the substitution reaction may be conventionally used in the art; when the catalyst is tris(dibenzylidene-indanacetone)dipalladium, preferably, the reaction further includes a base(preferably potassium carbonate) and a ligand (preferably 2-dicyclohexylphosphino-2',6'-diisopropoxy-1,1'-biphenyl), and is carried out under inert gas atmosphere; 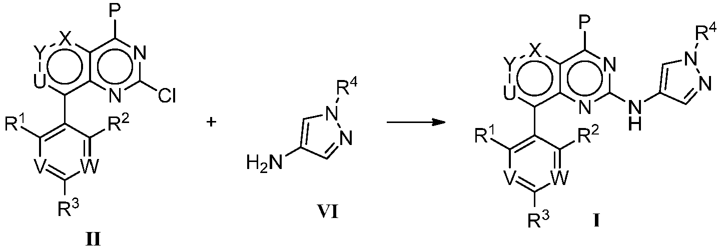
process 3 comprising the steps of under inert gas atmosphere, carrying out a coupling reaction with compound III and compound VII in an organic solvent(preferably selected from the group consisting of 1,4-dioxane, toluene and N, N-dimethylformamide) and in the presence of a base(preferably selected from the group consisting of sodium carbonate, potassium phosphate and potassium carbonate) and a palladium catalyst(preferably selected from the group consisting of [1,1'-bis(diphenylphosphino)ferrocene]dichloropalladium • dichloromethane, palladium acetate, [1,1'-bis(diphenylphosphino)ferrocene]dichloropalladium and tetrakis(triphenylphosphine)palladium) to give the compound of formula I; wherein, A is Br or I; the conditions for the coupling reaction may be conventionally used in the art; when the organic solvent is 1,4-dioxane, preferably, the reaction system may further comprise water; when the palladium catalyst is palladium acetate, preferably, the reaction system may further comprise 2-dicyclohexylphosphine-2,4,6-triisopropylbiphenyl; 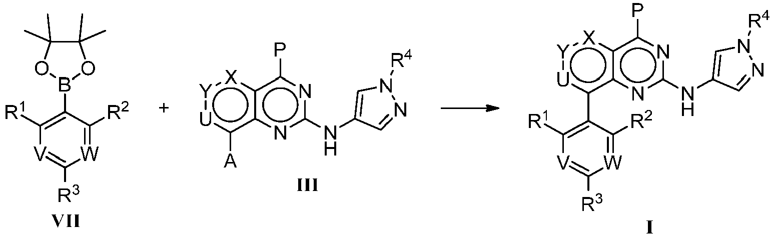
process 4 comprising the steps of carrying out a substitution reaction with compound **9-a** and 2-(4-piperidyl)-2-propanol in an organic solvent(preferably dichloromethane)and in the presence of a base(preferably diisopropylethylamine) to give compound **9;** the conditions for the substitution reaction may be conventionally used in the art; 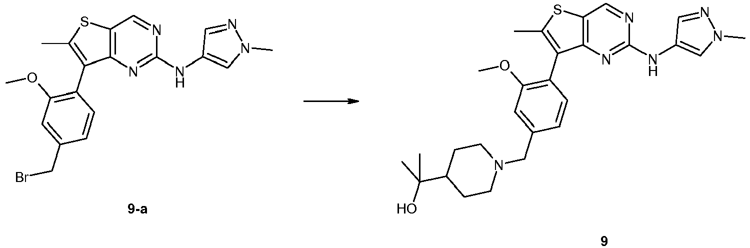
process 5 comprising the steps of carrying out a substitution reaction with compound **17-a** and morpholine in an organic solvent(preferably acetonitrile)and in the presence of a base(preferably potassium carbonate) to give compound **17;** the conditions for the substitution reaction may be conventionally used in the art; 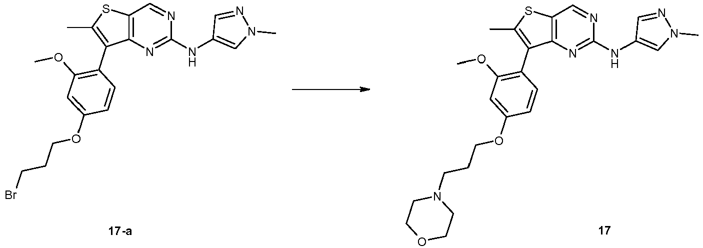
process 6 comprising the steps of carrying out a substitution reaction with compound **17-a** and pyrrolidine in an organic solvent(preferably acetonitrile) and in the presence of a base(preferably potassium carbonate) to give compound **18;** the conditions for the substitution reaction may be conventionally used in the art; 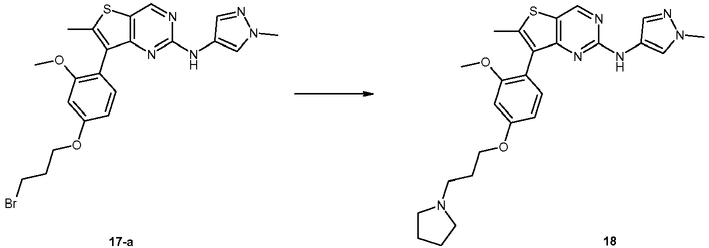
process 7 comprising the steps of carrying out a substitution reaction with compound **17-a** and N-methylpiperazine in an organic solvent(preferably acetonitrile)and in the presence of a base(preferably potassium carbonate) to give compound **19;** the conditions for the substitution reaction may be conventionally used in the art; 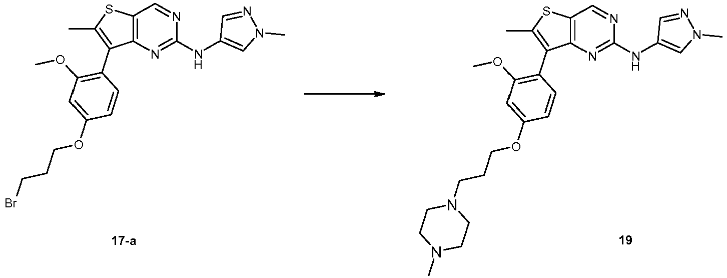
process 8 comprising the steps of carrying out a condensation reaction with compound **23-b** and azetidine in an organic solvent(preferably dichloromethane) and in the presence of a base(preferably N,N-diisopropylethylamine), N-hydroxybenzotriazole and 1 -ethyl-(3-dimethylaminopropyl)carbodiimide hydrochloride to give compound **23;** the conditions for the condensation reaction may be conventionally used in the art; 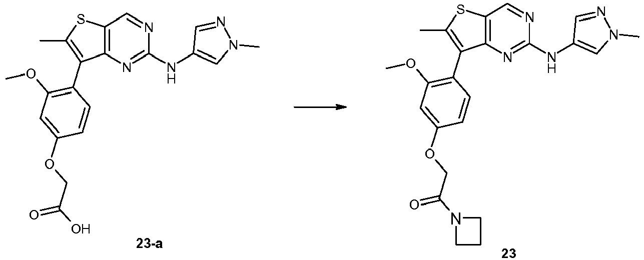
process 9 comprising the steps of deprotecting compound IV in an organic solvent(preferably dichloromethane) and in the presence of an acid(preferably trifluoroacetic acid) to give compound I; wherein R⁴ is 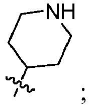 the conditions for the deprotection reaction may be conventionally used in the art; 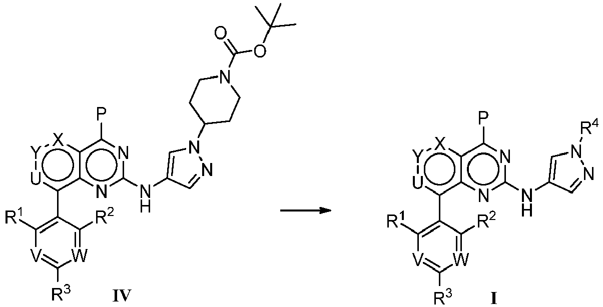
process 10 comprising the steps of carrying out a substitution reaction with compound **31** and 2-haloethanol in an organic solvent(preferably N,N-dimethylformamide) and in the presence of a base(preferably potassium carbonate) to give compound **34;** the conditions for the substitution reaction may be conventionally used in the art; 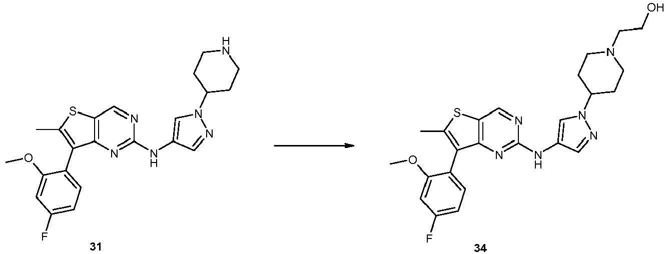
process 11 comprising the steps of carrying out a condensation reaction with compound **32** and 2-hydroxyacetic acid in an organic solvent(preferably dichloromethane) and in the presence of a base(preferably diisopropylethylamine),1-hydroxybenzotriazole and 1-ethyl-(3-dimethylaminopropyl)carbodiimide hydrochloride to give compound **36**; the conditions for the condensation reaction may be conventionally used in the art; 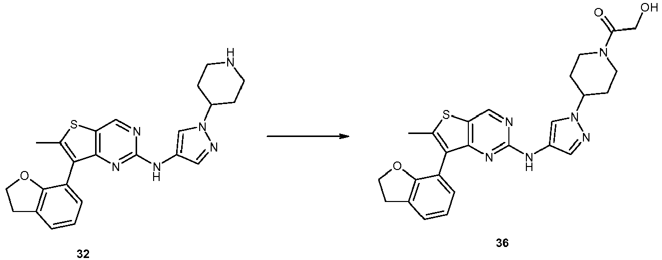
process 12 comprising the steps of carrying out a reductive amination reaction with compound **40-a,** dimethylamine and sodium triacetoxyborohydride in an organic solvent(preferably dichloromethane)and in the presence of an acid(preferably acetic acid) to give compound **40;** the conditions for the reductive amination reaction may be conventionally used in the art; 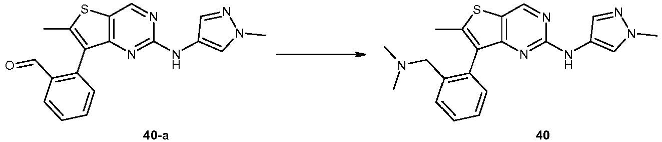
process 13 comprising the steps of carrying out a condensation reaction with compound **31** and ethyl chloroformate in an organic solvent(preferably dichloromethane)and in the presence of a base(preferably triethylamine) to give compound **50;** the conditions for the condensation reaction may be conventionally used in the art;

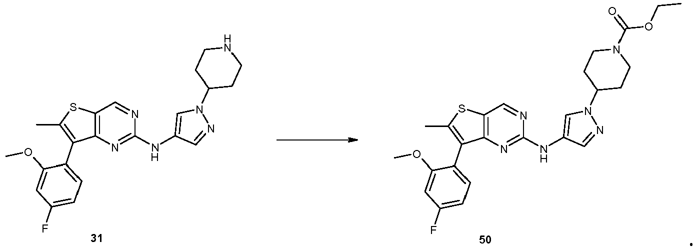

The present invention also provides a compound,
which is selected from the group consisting of

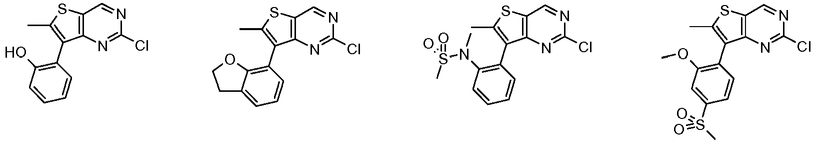

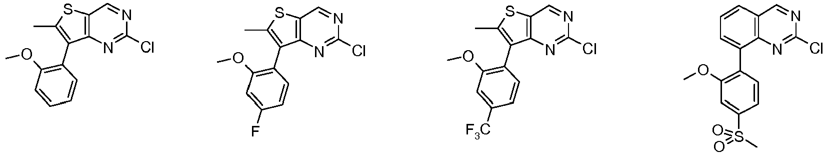

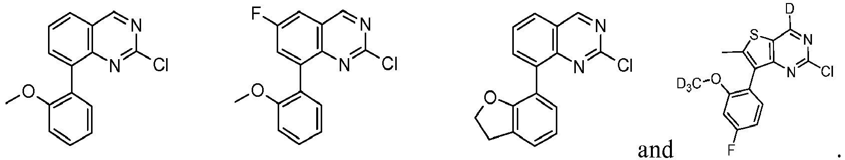

The present invention also provides a compound of formula III, 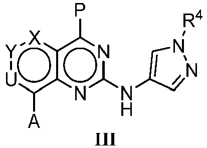 wherein, A is Br or I; each of R⁴, X, Y, U and P is as defined above. Preferably, the compound of formula III is selected from the group consisting of

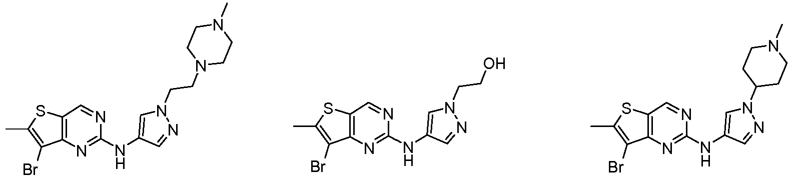

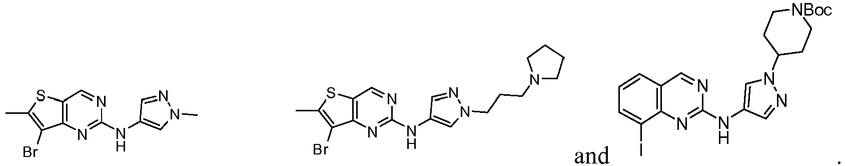

The present invention also provides a compound of formula IV, 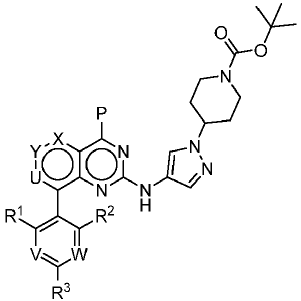 wherein, each of R¹,R²,R³,X,Y,U,V, W and P is as defined above. Preferably, the compound of formula IV is selected from the group consisting of 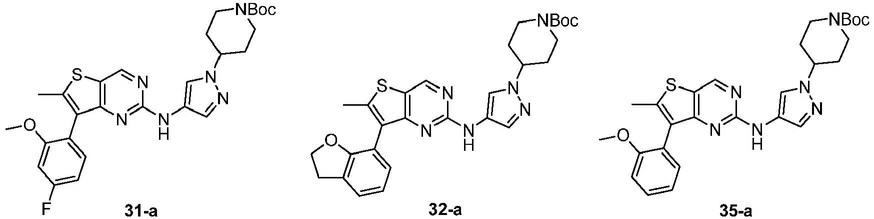 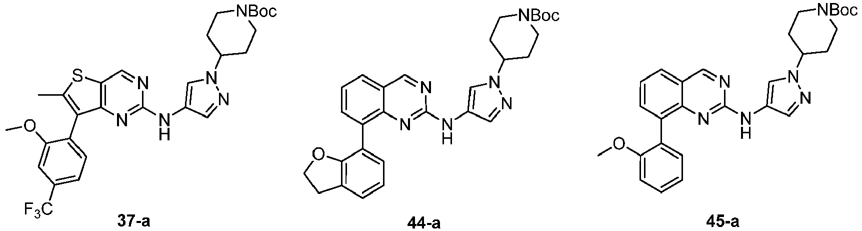 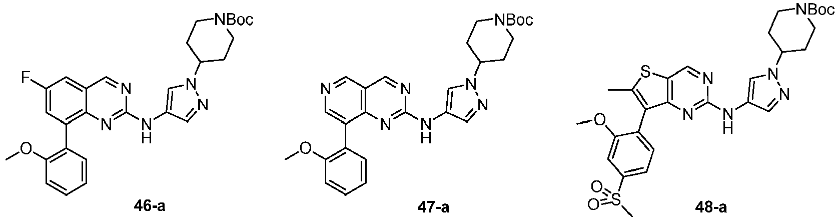 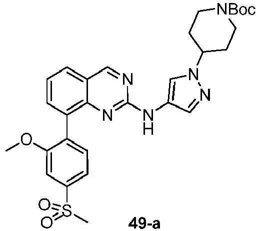 and

The present invention also provides a compound V, which is selected from the group consisting of

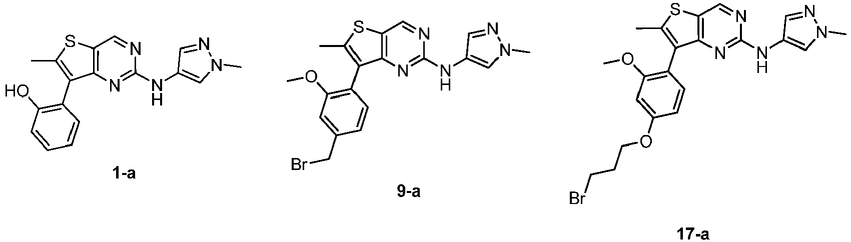

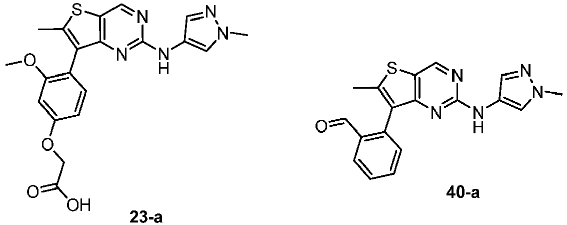

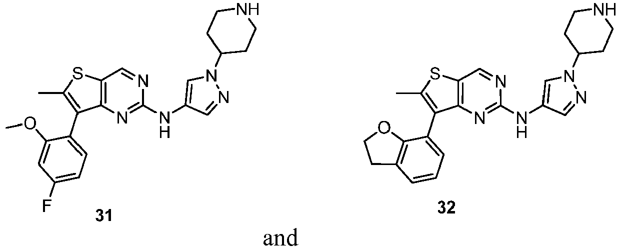

The present invention further relates to the fused ring pyrimidine compound, the tautomer, the enantiomer, the diastereoisomer or the pharmaceutically acceptable salt thereof for use in the prevention, alleviation or treatment of a disease selected from the group consisting of immune system disease, autoimmune disease, cell proliferative disease, allergic disorder and cardiovascular disease; one example of the immune system disease is organ transplant rejection; examples of the autoimmune disease are rheumatoid arthritis, psoriasis, Crohn's disease, multiple sclerosis and the like; examples of the cell proliferative disease are myelofibrosis, hematological tumor (such as leukemia, lymphoma etc.) and solid tumor(such as renal cancer, liver cancer, stomach cancer, lung cancer, breast cancer, prostate cancer, pancreatic cancer, thyroid cancer, ovarian cancer, glioblastoma, skin cancer and melanoma etc.); one example of the allergic disorder is bronchial asthma; examples of the cardiovascular disease are ischemic cardiomyopathy, heart failure, myocardial infarction and the like.

The present invention further relates to the fused ring pyrimidine compound, the tautomer, the enantiomer, the diastereoisomer or the pharmaceutically acceptable salt thereof in manufacturing drug for use in inhibiting Janus kinase, FGFR kinase, FLT3 kinase and Src family kinase; the Janus kinase is preferably selected from the group consisting of JAK1, JAK2 and JAK3;the FGFR kinase is preferably selected from the group consisting of FGFR1, FGFR2 and FGFR3; the FLT3 kinase is preferably selected from FLT3-WT, FLT3-ITD and FLT3-D835Y; the Src family kinase is preferably selected from c-Src, Lyn, Fyn, Lck, Hck, Fgr, Blk, Yes and Yrk; inhibiting Janus kinase, FGFR kinase, FLT3 kinase and/or Src family kinase can prevent, alleviate or treat the disease selected from the group consisting of immune system disease, autoimmune disease, cell proliferative disease, allergic disorder and cardiovascular disease; one example of the immune system disease is organ transplant rejection; examples of the autoimmune disease are rheumatoid arthritis, psoriasis, Crohn's disease, multiple sclerosis and the like; examples of the cell proliferative disease are myelofibrosis, hematological tumor (such as leukemia, lymphoma etc.) and solid tumor(such as renal cancer, liver cancer, stomach cancer, lung cancer, breast cancer, prostate cancer, pancreatic cancer, thyroid cancer, ovarian cancer, glioblastoma, skin cancer and melanoma); one example of the allergic disorder is bronchial asthma; examples of the cardiovascular disease are ischemic cardiomyopathy, heart failure, myocardial infarction and the like.

The present invention relates to a pharmaceutical composition, which comprises the fused ring pyrimidine compound, the tautomer, the enantiomer, the diastereoisomer or the pharmaceutically acceptable salt thereof, and one or more than one pharmaceutically acceptable carrier(s) and/or diluent(s); preferably, the dose of the fused ring pyrimidine compound, the tautomer, the enantiomer, the diastereoisomer or the pharmaceutically acceptable salt thereof is a therapeutically effective amount.

The pharmaceutical composition of the present invention may be in a form suitable for oral use or in the form of a sterile injectable aqueous solution. Oral or injectable compositions may be prepared according to any method known in the art for preparing pharmaceutical compositions.

The pharmaceutical composition of the present invention may be used in combination with one or more than one clinically used chemotherapeutic agents in any suitable ratio to produce a single dosage form, in particular a liposomal dosage form, according to conventional methods in the art, to treat various oncological diseases.

Unless otherwise indicated, the following terms when used in the description and the claims of the present invention have the following meanings:

The term "alkyl"(used alone or included in other groups) refers to branched and straight-chain saturated aliphatic hydrocarbon groups comprising 1 to 20 carbon atoms, preferably 1 to 10 carbon atoms, more preferably 1 to 8 carbon atoms, such as a methyl, an ethyl, a *n*-propyl, an isopropyl, a *n*-butyl, a *t*-butyl, an isobutyl, a pentyl, a hexyl, a heptyl, an octyl, a nonyl, a decyl, 4,4-dimethylpentyl, 2,2,4-trimethylpentyl, an undecyl, a dodecyl and various isomers thereof.

The term "alicyclic" or "cycloalkyl" (used alone or included in other groups) refers to saturated or partially unsaturated (containing 1 or 2 double bonds, but none of the rings has a completely conjugated π electron system) cyclic hydrocarbon groups comprising 1 to 3 rings, including monocycloalkyl, bicycloalkyl and tricycloalkyl groups, containing 3 to 20 carbons enabling to form a ring, preferably 3 to 10 carbons, for example, cyclopropyl, cyclobutyl, cyclopentyl, cyclohexyl, cycloheptyl, cyclooctyl, cyclodecane, cyclododecyl, cyclohexenyl and the like.

The term "heterocycloalkyl" (used alone or included in other groups) refers to 4-12 membered monocyclic or polycyclic groups containing 1-4 heteroatoms (such as selected from the group consisting of nitrogen, oxygen and sulfur), wherein each ring may contain one or more than one double bonds, but none of the rings has a completely conjugated π electron system. Heterocycloalkyl within the scope of this definition include, but is not limited to oxazoline, oxycyclobutyl, pyranyl, tetrahydropyranyl, azetidinyl, 1,4-dioxanyl, hexahydroazepinyl, piperazinyl, piperidinyl, pyrrolidinyl, morpholinyl, thiomorpholinyl, dihydrofuryl, dihydroimidazolyl, indolinyl, dihydroisoxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydrotetrazolyl, dihydrothiadiazolyl, dihydrothiazolyl dihydrothiophenyl, dihydrotriazolyl, dihydroazetidinyl, tetrahydrofuryl and tetrahydrothiophenyl and N-oxides thereof. Heterocycloalkyl may be linked to other groups via carbon atoms or heteroatoms thereof. In addition, any heterocycloalkyl ring can be fused to a cycloalkyl, an aryl, a heteroaryl or a heterocycloalkyl ring to form a fused, a bridged, or a spiro ring.

The term "alkoxy" (used alone or included in other groups)refers to a cyclic or acyclic alkyl having indicated number of carbon atoms attached through an oxygen bridge.Thus, "alkoxy" embraces the definitions of alkyl and cycloalkyl.

The term "aryl" (used alone or included in other groups) refers to any stable monocyclic or bicyclic carbocyclic rings with up to 7 atoms in each ring, at least one of which is an aromatic ring. Examples of the aryl include phenyl, naphthyl, tetrahydronaphthyl, indanyl, biphenylyl, phenanthryl, anthryl or acenaphthyl.It is to be understood that where the aryl is bicyclic and one of the rings is a non-aromatic ring, the attachment is made through an aromatic ring.

The term "aryl hetero" or "heteroaryl" (used alone or included in other groups) refers to stable monocyclic or bicyclic rings with up to 7 atoms in each ring, at least one of which is an aromatic ring and contains 1-4 heteroatoms selected from O, N and S. The heteroaryl within the scope of the definition includes, but is not limited to acridinyl, carbazolyl, cinnolinyl, quinoxalinyl, pyrazolyl, indolyl, benzotriazolyl, furanyl, thienyl, benzothienyl, benzofuranyl, quinolinyl isoquinolyl, oxazolyl, isoxazolyl, indolyl, pyrazinyl, pyridazinyl, pyridinyl, pyrimidinyl, pyrrolyl, tetrahydroquinoline.As the definition of "heterocycloalkyl", "heteroaryl" should also be understood to include N-oxide derivatives of any nitrogen-containing heteroaryl.In the case where the heteroaryl is a bicyclic substituent and one ring is non-aromatic or contains no heteroatoms, it is understood that the attachment is made through the aromatic ring or through the heteroatomon the ring, respectively.

The term "halogen" refers to fluorine, chlorine, bromine, iodine or astatine.

The term "hydroxyl" refers to -OH.

The term "amino" refers to -NH₂.

The term "cyano" refers to -CN.

The term "sulfonyl" refers to 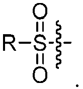 R- may include the definitions of the terms above.

The term "acyl" refers to 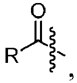 i.e. the remaining monovalent atomic group after removing the hydroxyl of an organic or inorganic oxo acid. R- may contain the definitions of the terms above.

The term "-BOC" refers to

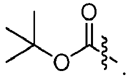

In the present invention, "pharmaceutically acceptable salts" refer to conventional acid addition salts or base addition salts which retain the biological effectiveness and properties of compound A, which are formed from suitable non-toxic organic or inorganic acids, or organic or inorganic bases. Examples of acid addition salts include those derived from inorganic acids such as hydrochloric acid, hydrobromic acid, hydroiodic acid, sulfuric acid, sulfamic acid, phosphoric acid and nitric acid, and those derived from organic acids such as *p*-toluenesulfonic acid, salicylic acid, methanesulfonic acid, oxalic acid, succinic acid, citric acid, maleic acid, lactic acid, fumaric acid and the like. Examples of base addition salts include those derived from ammonium, potassium, sodium and quaternary ammonium hydroxides, such as tetramethylammonium hydroxide. Chemical modification of pharmaceutical compounds (i.e. drugs) into salts is well-known technique for pharmacists to obtain the compounds with improved physical and chemical stability, hygroscopicity, flowability and solubility.

In the present invention, "pharmaceutically acceptable" in "one or more than one pharmaceutically acceptable carrier(s) and/or diluent(s)" means to be pharmaceutically acceptable and substantially non-toxicto the administered subject for a particular compound.

The above preferred conditions of the present invention may be arbitrarily combined without departing from the general knowledge in the art to obtain the preferred examples of the present invention.

The reagents and raw materials used in the present invention are commercially available.

The advantages of the present invention lie in that this compound has a strong inhibitory effect on Janus kinase (JAK), FGFR kinase, FLT3 kinase and Src family kinase.

### Detailed description of the preferred embodiment

The following examples further illustrate the present invention, but the present invention is not limited thereto. In the following examples, experimental methods for which specific conditions are not specified are selected according to conventional methods and conditions, or according to the product specification.

The structure of the compound was confirmed by nuclear magnetic resonance (NMR) or mass spectrometry (MS). The nuclear magnetic resonance spectrum was obtained by a Bruker Avance-500 instrument with deuterated dimethylsulfoxide, deuterated chloroform and deuterated methanol etc. as solvents and silane (TMS) as internal standard. Mass spectra was obtained using a Liquid Chromatography-Mass Spectrometry (LC-MS) instrument Agilent Technologies 6110 with ESI source.

The microwave reaction was carried out in the Explorer automatic microwave synthesizer manufactured by CEM Company of the United States. The magnetron frequency was 2450 MHz and the continuous microwave output power was 300W.

The instrument used for high performance liquid preparation was Gilson 281 and the preparative column used was Shimadazu Shim-Pack, PRC-ODS, 20 × 250 mm, 15 µm.

### Example 1

### N- [7-(2-methoxyphenyl)-6-methylthieno [3,2-d]pyrimidinyl-2-yl] -1 -methyl-1H-pyrazol-4-amine (Compound 1)

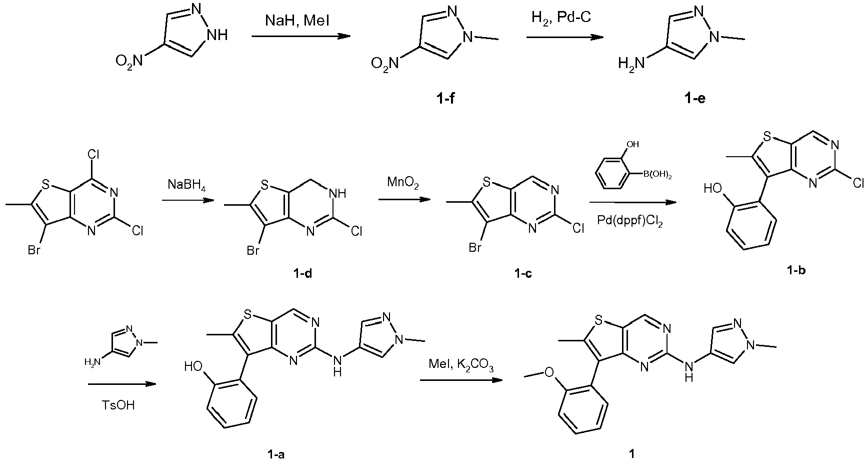

### Synthesis of compound 1-f

Sodium hydride (1.3g, 32.1mmol) was added to a solution of 4-nitropyrazole (3.3g, 29.2mmol) in dry tetrahydrofuran (30mL) at 0°C. After stirring for 1 hour, methyl iodide (20mL) was added and the mixture was stirred for another 2 hours at room temperature. The mixture was poured into ice water (100mL) and extracted with ethyl acetate (50mLx3). The organic phase was dried over anhydrous sodium sulfate, filtered and the filtrate was concentrated under reduced pressure. The residue was added to a mixed solvent (20mL) of petroleum ether and ethyl acetate (20:1), and stirred, solid was precipitated out. The solid was filtered out and dried in vacuo for 8 hours to give **1-f** as a white solid (2.6g, yield70%). The product was directly used in the next reaction without further purification. LC-MS (ESI): m/z = 128 [M + H]⁺.

### Synthesis of compound 1-e

Palladium 10% on carbon (0.2g) was added to a solution of compound 1-f (1.0g, 7.87mmol) in ethanol (15mL) under hydrogen atmosphere (latm). The mixture was reacted at 25°C for 18 hours, filtered and the filtrate was concentrated under reduced pressure. The residue was purified by silica gel column chromatography (petroleum ether: ethyl acetate = 1:1) to give **1-e** as a red oil (700mg, yield 92%). The product was used without further purification.

### Synthesis of compound 1-d

7-Bromo-2,4-dichloro-6-methylthieno[3,2-d]pyrimidine (5.0g, 16.89mmol) was dissolved in tetrahydrofuran (50mL) and ethanol(50mL). The reaction solution was cooled to 0°C and sodium borohydride (3.19g, 84.5mmol) was added in portions. The reaction solution was warmed to room temperature and further stirred for 3 hours, then added with water (500mL) and extracted with dichloromethane (300mLx3). The combined organic phases were dried over anhydrous sodium sulfate, filtered and the filtrate was concentrated under reduced pressure to give **1-d** as a yellow liquid (4g, yield 90%) which was used without further purification. LC-MS (ESI): m/z = 265 [M + H]⁺.

### Synthesis of compound 1-c

Compound **1-d** (4.0g, 15.15mmol) was dissolved in dichloromethane (100mL), activated manganese dioxide (6.6g, 75.8mmol) was added and the mixture was stirred at room temperature for 16 hours. The reaction solution was filtered through celite and the filter cake was washed with dichloromethane (50mLx5). The combined filtrate was concentrated under reduced pressure to give **1-c** as a yellow solid (3.8g, yield 96%) which was used without further purification. LC-MS (ESI): m/z = 263 [M + H]⁺.

### Synthesis of compound 1-b

Compound **1-c** (500mg, 1.91mmol), 2-hydroxybenzeneboronic acid (267mg, 1.91mmol) and sodium carbonate (619mg, 5.73mmol) were suspended in dioxane/water (5mL/5mL), and [1,1'-bis(diphenylphosphino)ferrocene]dichloropalladium •dichloromethane (163mg, 0.2mmol) was added. The reaction solution was purged with nitrogen gas for three times and heated to 80°C to react overnight. After removing the solvent by rotary evaporation, the mixture was partitioned with dichloromethane (150mL) and water (150mL). The organic phase was separated, dried over anhydrous sodium sulfate, filtered, concentrated and purified by silica gel column chromatography (methylene chloride:methanol = 100:1) to give **1-b** as a pale brown solid (610mg). LC-MS (ESI): m/z = 277 [M + H]⁺.

### Synthesis of compound 1-a

Compound **1-b** (610mg, 2.21mmol) and 1-methyl-4-aminopyrazole (643mg, 6.63mmol) were dissolved in n-butanol (15mL) and *p*-toluenesulfonic acid monohydrate(1.3g, 6.63mmol) was added. The mixture was heated to 110°C to react overnight, then concentrated to remove the solvent, and partitioned between dichloromethane (150mL) and saturated sodium carbonate (150mL). The organic phase was separated and dried, filtered, concentrated and purified by silica gel columnchromatography (dichloromethane:methanol = 50:1) to give **1-a** as a yellow solid (250mg, yield 39%). LC-MS (ESI): m/z = 338 [M + H]⁺.

¹H-NMR (400MHz, CDCl₃) δ: 8.78 (s, 1H), 8.20 (br, 1H), 7.77 (s, 1H), 7.42 (s, 1H), 7.39 (t, *J*=8Hz, 1H), 7.28 (d, *J*=8Hz, 1H), 7.18 (d, *J*=8Hz, 1H), 7.08 (t, *J*=8Hz, 1H), 6.99 (br, 1H), 3.85 (s, 3H), 2.69 (s, 3H) ppm

### Synthesis of compound 1

Compound **1-a** (120mg, 0.36mmol) was dissolved in acetone (2mL), and anhydrous potassium carbonate (74mg, 0.54mmol) was added, then methyl iodide (77mg, 0.54mmol) was added slowly and the mixture was stirred at room temperature overnight. The mixture was filtered and washed with acetone (20mL). The combined filtrate was concentrated under reduced pressure and purified by prep-HPLC (mobile phase: 0.05% aqueous trifluoroacetic acid: acetonitrile = 30% to 62%) to give **1** as a pale yellow solid (40mg, yield 32%). LC-MS (ESI): m/z = 352 [M + H]⁺.

¹H-NMR (400MHz, CDCl₃) δ: 8.73 (s, 1H), 7.79 (s, 1H), 7.42 (m, 2H), 7.37 (s, 1H), 7.05-7.14 (m, 3H), 3.77 (s, 1H), 3.76 (s, 3H), 2.49 (s, 3H) ppm

### Example 2

### N- [7- (2,3-dihydro-1-benzofuran-7-yl)-6-methylthieno[3,2-d]pyrimidinyl-2-yl]-1-methyl-1H-pyrazol-4-amine (Compound 2)

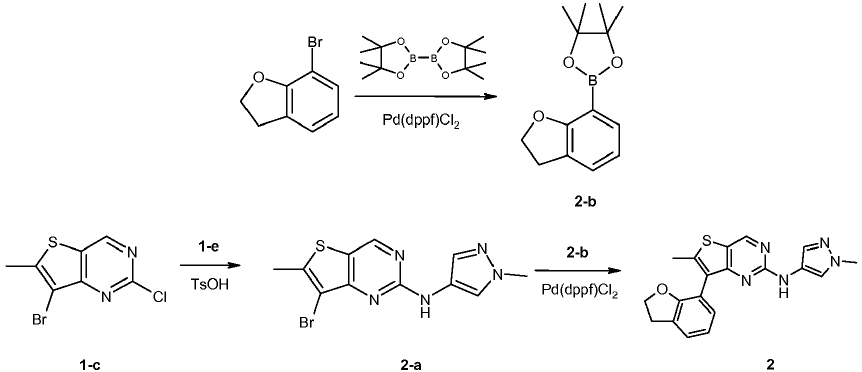

### Synthesis of compound 2-b

The compound 7-bromobenzodihydrofuran (0.4g, 2mmol), bis(pinacolato)diboron (0.78g, 3mmol) and anhydrous potassium acetate (0.4g, 4mmol)were suspended in dimethyl sulfoxide (5mL), and [1,1'-bis(diphenylphosphino)ferrocene]dichloropalladium (0.16g, 0.2mmol) was added. The reaction solution was purged with nitrogen gas for three times to remove the oxygen contained in the system and heated at 80°C for 8 hours. The reaction was cooled to room temperature, diluted with ice water (100mL) and extracted with ethyl acetate (50mL x 3). The combined organic phase was washed with water (50mL x 3) and brine (50mL) successively, dried over anhydrous sodium sulfate, filtered and the filtrate was concentrated under reduced pressure. The residue was purified by silica gel column chromatography (petroleum ether: ethyl acetate = 30: 1) to give compound **2-b** (0.29g, yield 56%).

¹H-NMR (400MHz, CDC13) δ: 7.53 (d, *J*=8Hz, 1H), 7.27 (d,J=8Hz, 1H), 6.83 (t, *J*=8Hz, 1H), 4.63 (t, *J*=8.8Hz, 1H), 3.16 (t, *J*=8.8Hz, 1H), 1.36 (s, 12H) ppm

### Synthesis of compound 2-a

4-Amino-1-methylpyrazole **1-e** (0.9g, 9mmol), *p*-toluenesulfonic acid (2.26g, 12mmol) and compound **1-c** (1.5g, 6mmol) were added to *n*-butanol(10mL). The solution was heated to 108°C and stirred for 6 hours. The reaction solution was concentrated, quenched with saturated aqueous sodium bicarbonate (80mL), extracted with dichloromethane (100mL × 5), dried over anhydrous sodium sulfate and filtered and the filtrate was concentrated under reduced pressure. The residue was purified by silica gel TLC preparative plate (petroleum ether: ethyl acetate = 1: 1) to give **2-a** as a yellow solid (1660mg, yield 86.7%). LC-MS (ESI): m/z = 324 [M + H]⁺.

### Synthesis of compound 2

Compound **2-a** (180mg, 0.75mmol), compound **2-b** (164mg, 0.5mmol), [1,1'-bis(diphenylphosphino)ferrocene]dichloropalladium (36mg, 0.05mmol) and sodium carbonate (106mg, 1mmol) were dissolved in 1,4-dioxane (8mL) and water (2mL). The reaction solution was purged with nitrogen gas for three times to remove the oxygen contained in the system and heated at 90°C for 8 hours. The reaction solution was cooled to room temperature, diluted with ice water (10mL) and extracted with dichloromethane (50mL x 3). The combined organic phase was washed with water (20mL x 3) and brine (20mL) successively, dried over anhydrous sodium sulfate, filtered and the filtrate was concentrated under reduced pressure. The residue was purified by silica gel TLC preparative plate (petroleum ether: ethyl acetate = 10: 1) to give **2** as a yellow solid (41mg, yield 22.6%). LC-MS (ESI): m/z = 364 [M + H]⁺.

¹H-NMR (400MHz, MeOD) δ: 8.79 (s, 1H), 7.91 (s, 1H), 7.60 (d, *J*=8Hz, 1H), 7.52 (s, 1H), 7.36 (d, *J*=2Hz, 1H), 7.06 (t, *J*=8Hz, 1H), 4.88 (t, *J*=8Hz, 2H), 4.58 (t, *J*=8Hz, 2H), 3.77 (s, 3H), 2.55 (s, 3H) ppm

### Example 3

### N-[7-[2-(2-methoxyethoxy)phenyl]-6-methylthieno[3,2-d]pyrimidinyl-2-yl]-1-methyl-1H-pyrazole -4-amine (Compound 3)

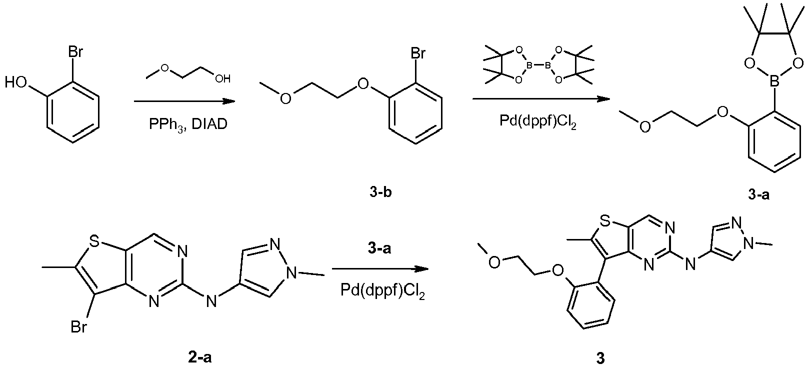

### Synthesis of compound 3-b

2-Bromophenol (5g, 29.07mmol), ethylene glycol monomethyl ether (3.3g, 43.61mmol) and triphenylphosphine (11.4g, 43.61mmol) were dissolved in anhydrous tetrahydrofuran(100mL). The solution was cooled to 0 °C and diisopropylazodicarboxylate (8.9g, 43.61mmol) was slowly added dropwise. After the addition, the mixture was stirred at room temperature for 3 hours. After concentration, a mixed solvent (100mL) of petroleum ether and ethyl acetate (10: 1) was added and the mixture was stirred for 30 minutes, filtered and the filtrate was concentrated under reduced pressure. The residue was purified by silica gel column chromatography (petroleum ether: ethyl acetate = 10: 1) to give **3-b** as a pale yellow oil (5g, yield 75%).

### Synthesis of compound 3-a

Compound **3-b** (1g, 4.44mmol) and bis(pinacol)borate (1.7g, 6.67mmol) were dissolved in dioxane (10mL) and anhydrous potassium acetate (1.1g,13.32mmol) and [1,1'-bis(diphenylphosphino)ferrocene]dichloropalladium (370mg, 0.45mmol) were added. The reaction solution was heated to 80°C to react overnight under nitrogen gas atmosphere, and then concentrated under reduced pressure, the residue was purified by silica gel column chromatography (petroleum ether: ethyl acetate = 20: 1) to give **3-a** as a yellow oil (630mg, yield 51%).

### Synthesis of compound 3

Compound **3-a** (51mg, 0.06mmol), compound **2-b** (30mg, 0.03mmol) and sodium carbonate (42mg, 0.39mmol) were suspended in dioxane (0.5mL) and water (0.5mL), and [1,1'-bis(diphenylphosphino)ferrocene]dichloropalladium •dichloromethane (13mg, 0.016mmol) was added. The mixture was purged with nitrogen gas for three times, and heated to 90°C with microwave and reacted for 40 minutes. After the reaction solution was cooled to room temperature, the solvent was distilled off under reduced pressure. The residue was purified by silica gel TLC preparative plate (ethyl acetate) to give **3** as a yellow solid (10mg, yield 27%). LC-MS (ESI): m/z = 396 [M + H]⁺.

¹H-NMR (400MHz, CDC13) δ: 8.72 (s, 2H), 7.79 (s, 1H), 7.41-7.744 (m, 2H), 7.38 (s, 1H), 7.13 (t, J=8Hz, 1H), 7.06 (d, J=8Hz, 1H), 6.94 (br, 1H), 4.13 (m, 1H), 4.01 (m, 1H), 3.77 (s, 3H), 3.54 (m, 2H), 3.23 (s, 3H), 2.53 (s, 3H) ppm

### Example 4

### N-[7-(4-methylsulfanyl-2-methoxyphenyl)-6-methylthieno[3,2-d]pyrimidinyl-2-yl]-1-methyl-1H-pyrazol-4-amine (Compound 4)

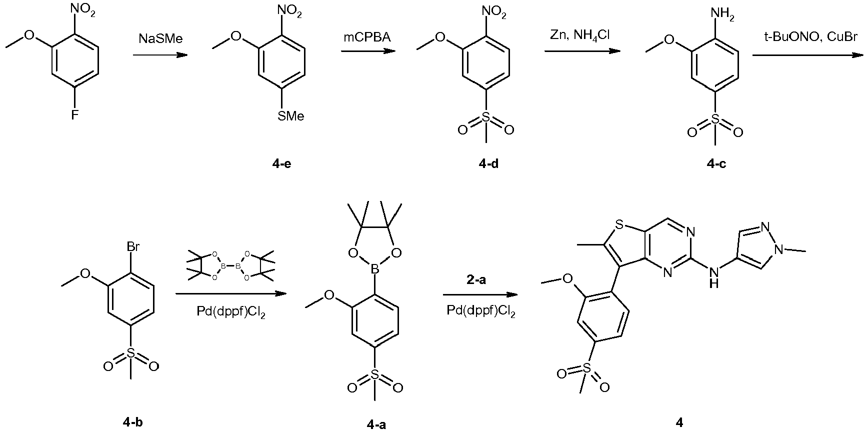

### Synthesis of compound 4-e

2-Methoxy-4-fluoronitrobenzene (5g, 29.24mmol) was dissolved in N,N-dimethylformamide (35mL), 50% sodium methanethiolate (6.1g, 43.86mmol) was added and the mixture was stirred overnight at room temperature. The mixture was poured into water (200mL) and extracted with ethyl acetate (200mL). The separated organic phase was dried over anhydrous sodium sulfate and filtered. The filtrate was concentrated under reduced pressure and the residue was washed with a mixed solvent of petroleum ether and ethyl acetate (10: 1, 50mL) to give **4-e** as a yellow solid (2.8g, yield 48%).

¹H-NMR (400MHz, CDCl₃) δ: 7.89 (d, *J*=9Hz, 1H), 6.86 (s, 1H), 6.83 (d, J=9Hz, 1H), 3.97 (s, 3H), 2.54 (s, 3H) ppm

### Synthesis of compound 4-d

Compound **4-e** (3g, 15.09mmol) was dissolved in dichloromethane (10mL), m-chloroperbenzoic acid (7.8g, 37.74mmol) was added and the reaction was stirred at room temperature for 16 hours. After cooling to 0°C, the reaction mixture was filtered and the filter cake was washed with cold dichloromethane. The combined filtrate was concentrated under reduced pressure and the residue was purified by silica gel column chromatography (petroleum ether: ethyl acetate = 1: 2) to give **4-d** as a yellow solid (1.7g, yield 49%). LC-MS (ESI): m/z = 232 [M + H]⁺.

### Synthesis of compound 4-c

Compound **4-d** (1.7g, 7.36mmol) was dissolved in ethanol (20mL) and water (20mL) and ammonium chloride (2g, 36.79mmol) and zinc dust (2.4g, 36.79mmol) were added. The mixture was heated to 80°Cto react for 2 hours. After cooling to room temperature, the mixture was concentrated under reduced pressure. The residue was partitioned between ethyl acetate (200mL) and water (200mL). The organic phase was separated, dried over anhydrous sodium sulfate, filtered, and the filtrate was concentrated under reduced pressure to give **4-c** as a brown oil (1g, yield 68%), which was used without further purification. LC-MS (ESI): m/z = 202 [M + H]⁺.

### Synthesis of compound 4-b

Compound **4-c** (1g, 4.98mmol) was dissolved in acetonitrile (10mL) and copper bromide (1.9g, 7.50mmol) was added, followed by slowly adding tert-butyl nitrite (0.73mL). The mixture was heated to 80°C and reacted for 1 hour, cooled to room temperature and then concentrated under reduced pressure. The residue was diluted with ethyl acetate (100mL) and water (50mL) and filtered through celite. The combined organic phase was dried over anhydrous sodium sulfate, filtered and the filtrate was concentrated under reduced pressure. The residue was purified by silica gel column chromatography (petroleum ether: ethyl acetate = 1: 1) to give **4-b** as a pale yellow solid (540mg, yield 41%). LC-MS (ESI): m/z = 265 [M + H]⁺.

### Synthesis of compound 4-a

Compound **4-b** (300mg, 1.14mmol) and bis(pinacolato)diboron(433mg, 1.71mmol) were dissolved in dioxane (5mL) and anhydrous potassiumacetate (281mg, 3.42mmol) and [1,1'-bis(diphenylphosphino)ferrocene]dichloropalladium • dichloromethane (98mg, 0.15mmol) were added. Under nitrogen gas atmosphere, the mixture was heated to 85°C and reacted for 16 hours. After cooling to room temperature, the mixture was diluted with ethyl acetate (20mL) and filtered through celite. The filtrate was concentrated to dryness to give **4-a** as a black oil (350mg) which was used in the next reaction without further purification.

### Synthesis of compound 4

Compound **4-a** (72mg, 0.23mmol), compound **2-a** (50mg, 0.16mmol) and sodium carbonate (50mg, 0.47mmol) were suspended in dioxane (0.5mL) and water (0.5mL), [1,1'-bis(diphenylphosphino)ferrocene]dichloropalladium • dichloromethane (14mg, 0.02mmol) was added. The mixture was purged with nitrogen gas for three times, and heated to 90°C with microwave, reacted for 40 minutes. After the solvent was evaporated under reduced pressure, the residue was partitioned between dichloromethane (50mL) and water (50mL). The organic phase was dried over anhydrous sodium sulfate, filtered and the filtrate was concentrated under reduced pressure. The residue was purified by silica gel TLCpreparative plate (ethyl acetate) to give **4** as a pale yellow solid (15mg, yield 23%). LC-MS (ESI): m/z = 430 [M + H]⁺.

¹H-NMR (400MHz, CDCl₃) δ: 8.76 (s, 1H), 7.68-7.69 (m, 2H), 7.65 (d, *J*=8Hz, 1H), 7.59 (d, *J*=2Hz, 1H), 7.36 (s, 1H), 7.26 (br, 1H), 3.87 (s, 3H), 3.79 (s, 3H), 3.16 (s, 3H), 2.50 (s, 4H) ppm

### Example 5

### N-[7-(2,6-dimethoxyphenyl)-6-methylthieno[3,2-d]pyrimidinyl-2-yl]-1-methyl-1H-pyrazol-4-amine (Compound 5)

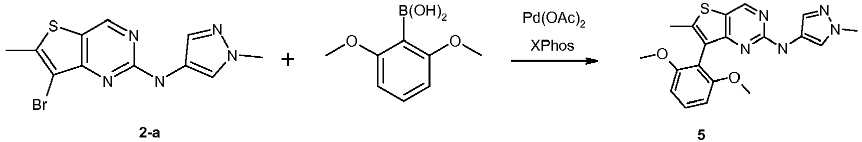

The compound 2,6-dimethoxyphenylboronic acid (136mg, 0.75mmol), compound **2-a** (164mg, 0.5mmol), 2-dicyclohexylphosphino-2,4,6-triisopropylbiphenyl (36mg, 0.05mmol) and palladium acetate (0.112g, 0.5mmol) and potassium phosphate (0.422g, 2mmol) were dissolved in toluene (2mL). The reaction mixture was purged with nitrogen gas for three times to remove the oxygen contained in the system, and then the mixture was heated at 90°C for 8 hours. The reaction was cooled to room temperature, diluted with ice water (10mL) and extracted with dichloromethane (50mL x 3). The combined organic phase was washed with water (20mL x 3) and brine (20mL) successively, dried over anhydrous sodium sulfate, filtered and the filtrate was concentrated under reduced pressure. The residue was purified by silica gel TLC preparative plate (petroleum ether: ethyl acetate = 5: 1) to give **5** as a yellow solid (53mg, yield 27.8 %). LC-MS (ESI): m/z = 382 [M + H]⁺.

¹H-NMR (400MHz, MeOD) δ: 8.75 (s, 1H), 7.72 (s, 1H), 7.49 (d, *J*=8Hz, 1H), 7.46 (s, 1H), 6.84 (d, *J*=2Hz, 1H), 3.93 (s, 3H), 3.74 (s, 6H), 2.41 (s, 3H) ppm

### Example 6

### N-[7-(4-chloro-2-dimethoxyphenyl)-6-methylthieno[3,2-d]pyrimidinyl-2-yl]-1-methyl-1H-pyrazol-4-amine (Compound 6)

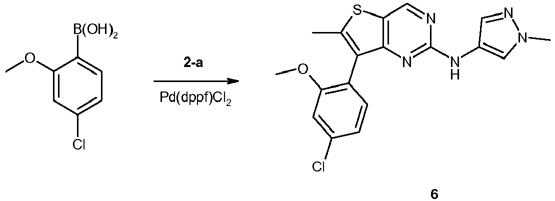

### Synthesis of compound 6

4-chloro-2-methoxybenzeneboronic acid (52mg, 0.27mmol), compound **2-a** (75mg, 0.23mmol) and sodium carbonate (73mg, 0.69mmol) were suspended in dioxane (1.2mL) and water (0.3mL), and [1,1'-bis(diphenylphosphino)ferrocene]dichloropalladium • dichloromethane (17mg, 0.02mmol) was added. The mixture was purged with nitrogen gas for three times and heated at 90°C under microwave for 1 hour. After cooling to room temperature, the reaction solution was added with water (30mL) and extracted with dichloromethane (50mL × 2). The combined organic phase was dried over anhydrous sodium sulfate, filtered and the filtrate was concentrated under reduced pressure. The residue was purified by silica gel TLC preparative plate (ethyl acetate) to give **6** as a pale yellow solid (30mg, yield 23%). LC-MS (ESI): m/z = 385 [M + H]⁺.

¹H NMR (400 MHz, DMSO-d₆) δ: 9.43 (s, 1H), 8.95 (s, 1H), 7.657 (s, 1H), 7.19-7.38 (m, 4H), 3.76 (s, 3H), 3.70 (s, 3H), 2.42 (s, 3H) ppm

### Example 7

### N-[7-(2,4-dimethoxyphenyl)-6-methylthieno[3,2-d]pyrimidinyl-2-yl]-1-methyl-1H-pyrazol-4-amine (Compound 7)

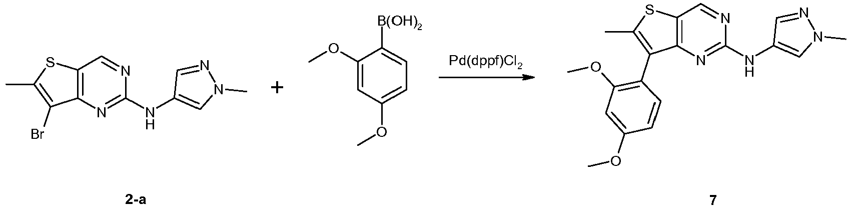

### Synthesis of compound 7

2,4-Dimethoxyphenylboronic acid (43mg, 0.23mmol), compound **2-a** (50mg, 0.16mmol) and sodium carbonate (51mg, 0.47mmol) were suspended in dioxane (0.5mL) and water (0.5mL), and [1,1'-bis(diphenylphosphino)ferrocene] dichloropalladium • dichloromethane (13mg, 0.02mmol) was added. The reaction solution was purged with nitrogen gas for three times and heated at 90°C under microwave for 40 minutes. After cooling to room temperature, the solvent was evaporated under reduced pressure. The residue was partitioned between dichloromethane (50mL) and water (50 mL). The organic phase was dried over anhydrous sodium sulfate, filtered and the filtrate was concentrated under reduced pressure. The residue was purified by silica gel TLC preparative plate (ethyl acetate) to give **7** as a pale yellow solid (15mg, yield 25%). LC-MS (ESI): m/z = 381 [M + H]⁺.

¹H-NMR (400MHz, CDCl₃) δ: 8.72 (s, 1H), 7.83 (s, 1H), 7.39 (s, 1H), 7.31 (d, J=8Hz, 1H), 7.07 (br, 1H), 6.63-6.68 (m, 2H), 3.89 (s, 3H), 3.79 (s, 3H), 3.76 (s, 3H), 2.49 (s, 3H) ppm

### Example 8

### N-[7-(5-methylsulfonyl-2-dimethoxyphenyl)-6-methylthieno[3,2-d]pyrimidinyl-2-yl]-1-methyl-1H-pyrazol-4-amine (Compound 8)

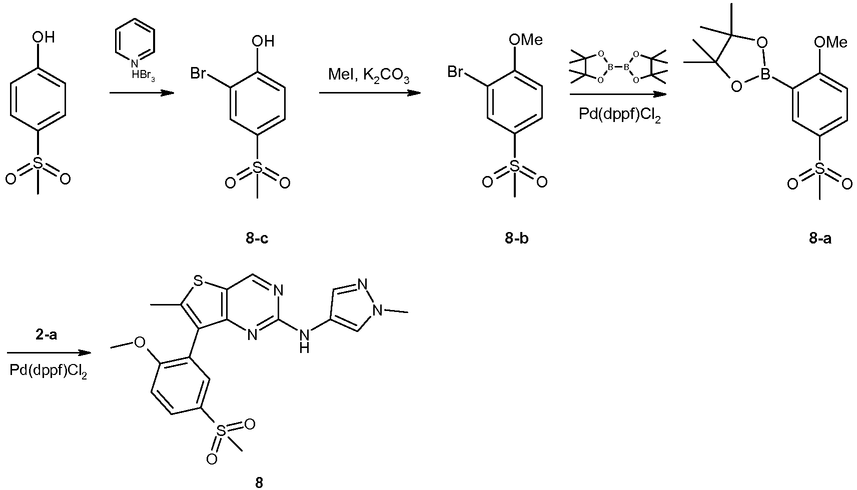

### Synthesis of compound 8-c

4-Hydroxyphenyl methyl sulfone (4.5g, 26.16mmol) was dissolved in dichloromethane (50mL) and methanol (50mL), pyridinium tribromide (8.3g, 26.16mmol) was added at room temperature and the mixture was stirred at room temperature for 2 days. The reaction solution was concentrated under reduced pressure. The residue was partitioned between dichloromethane (100mL) and water (100mL). The organic phase was dried over anhydrous sodium sulfate, filtered and the filtrate was concentrated under reduced pressure. The residue was added to a mixed solvent (50mL) of petroleum ether and ethyl acetate (3: 1), solid was precipitated out, and filtered to obtain **8-c** as a white solid (1.2g, yield 19%).

### Synthesis of compound 8-b

Compound **8-c** (150mg, 0.57mmol) and potassium carbonate (236mg, 1.71mmol) were suspended in acetone (10mL) and methyl iodide (809mg, 5.71mmol) was added. The reaction mixture was stirred at room temperature for 16 hours, filtered and the filter cake was washed with ethyl acetate (10mL). The filtrate was concentrated under reduced pressure and the residue was washed with ethyl acetate (50mL) and water (50mL). The organic phase was dried over anhydrous sodium sulfate, filtered, and the filtrate was concentrated under reduced pressure to give **8-b** as a pale yellow solid (150mg, yield 95%).

### Synthesis of compound 8-a

Compound **8-b** (150mg, 0.57mmol) and bis(pinacolato)diboron (160mg, 0.63mmol) were dissolved in dioxane (3mL) and anhydrous potassium acetate (141mg, 1.71mmol) and [1,1'-bis(diphenylphosphino)ferrocene]dichloropalladium • dichloromethane (130mg, 0.17mmol) were added. Under nitrogengas atmosphere, the mixture was heated to 85 °C and reacted overnight. After cooling to room temperature, the mixture was diluted with ethyl acetate (20mL) and filtered through celite. The filtrate was concentrated under reduced pressure to give **8-a** as a black oil (185mg), which was used directly in the next reaction without further purification.

### Synthesis of compound 8

Compound **8-a** (150mg, 0.46mmol), compound **2-a** (100mg, 0.31mmol) and sodium carbonate (100mg, 0.93mmol) were suspended in dioxane (0.5mL) and water (0.5mL), [1,1'-bis(diphenylphosphino)ferrocene]dichloropalladium • dichloromethane (26mg, 0.03 mmol) was added. The reaction solution was purged with nitrogen gas for three times,heated to 80°C and reacted for 18 hours. After cooling to room temperature, the solvent was removed by rotary evaporation. The residue was partitioned between dichloromethane (50mL) and water (50 mL). The organic phase was dried over anhydrous sodium sulfate, filtered and the filtrate was concentrated under reduced pressure. The residue was purified by silica gel TLC preparative plate (ethyl acetate) to give **8** as a white solid (25mg, yield 19%). LC-MS (ESI): m/z = 430 [M + H]⁺.

¹H-NMR (400MHz, CDCl₃) δ: 8.73 (s, 1H), 8.04 (s, 1H), 8.02 (d, *J*=9Hz, 1H), 7.71 (s, 1H), 7.39 (s, 1H), 7.19 (d, *J*=9Hz, 1H), 6.85 (br, 1H), 3.88 (s, 3H), 3.81 (s, 3H), 3.10 (s, 3H), 2.49 (s, 3H) ppm

### Example 9

### 2-{1-[(3-methoxy-4-{6-methyl-2-[-methyl-1H-pyrazol-4-yl)amino]thieno[3,2-d]pyrimidinyl-7-yl}phenyl)methyl]piperidinyl-4-yl}propan-2-ol (Compound 9)

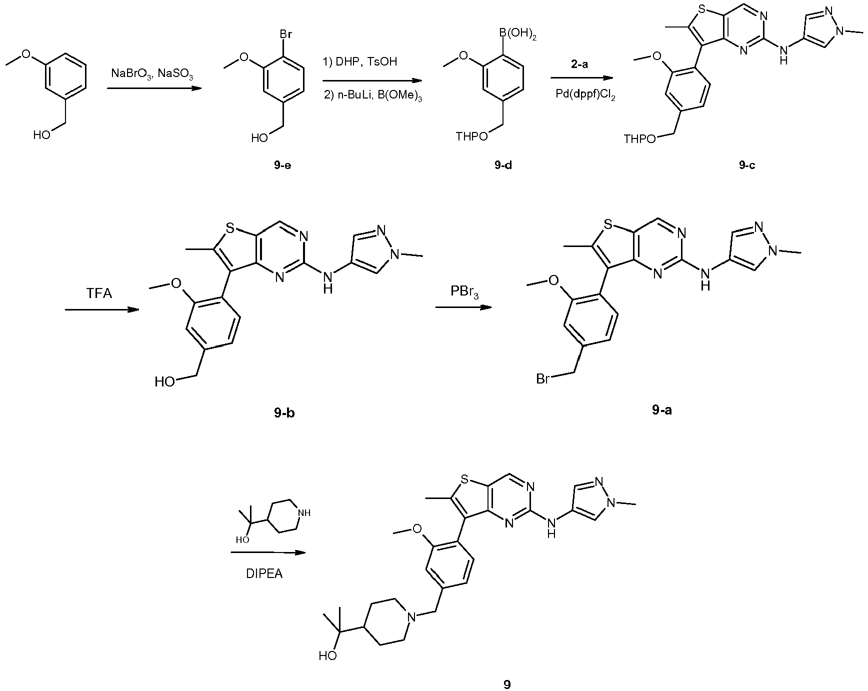

### Synthesis of compound 9-e

The compound 3-methoxybenzyl alcohol (10g, 72.4mmol) was dissolved in a mixture of acetonitrile (250mL) and water (250mL) and then sodium bromate (19.1g, 127mmol) and sodium bisulfite (13.2g, 127mmol) were added. The reaction solution was stirred at room temperature for 1.5 hours, quenched with a saturated aqueous solution of sodium thiosulfate (250mL) and then extracted with dichloromethane (200mL × 3). The combined organic phase was washed with water (200mL x 3) and brine (20mL)sequentially, dried over anhydrous sodium sulfate and filtered. The filtrate was concentrated under reduced pressure, the residue was purified by silica gel TLC preparative plate (petroleum ether: ethyl acetate = 3: 1) to give **9-e** as a yellow solid (1.39g, yield 88%).

¹H-NMR (400MHz, MeOD) δ: 7.41 (d, *J*=12Hz, 1H), 7.06 (d, *J*=4Hz, 1H), 6.71 (dd, *J*=4Hz, *J*=8Hz, 1H), 4.70 (d, *J*=8Hz, 2H), 3.81(s, 3H) ppm

### Synthesis of compound 9-d

Compound **9-e** (2.16g, 10mmol) and *p*-toluenesulfonic acid (1.72g, 1mmol) were added to dichloromethane (50mL), followed by slow addition of 3,4-dihydropyran (1.64g, 20mmol) and the resultant was stirred at room temperature for 3 hours. The reaction solution was concentrated under reduced pressure, quenched with saturated aqueous sodium bicarbonate solution (50mL) and the mixture was extracted with dichloromethane (50mL x 3). The combined organic phase was dried over sodium sulfate, filtered and the filtrate was concentrated under reduced pressure. The residue was added directly to anhydrous tetrahydrofuran (10mL) pre-cooled to -78°C, followed by the dropwise addition of n-butyllithium in n-hexane (5mL, 12.5mmol). After stirring for 2 hours, trimethyl borate (1.3g, 12.5mmol) was added to the reaction solution. The reaction mixture was slowly warmed to room temperature and further stirred for 2 hours. The reaction was quenched with water (50mL) and sodium hydroxide (0.8g, 20mmol) and extracted with ethyl acetate (50mL x 3). The aqueous phase is adjusted to pH = 7 with 1M aqueous hydrochloric acid solution and then extracted with ethyl acetate (50mL x 3). The organic phase was concentrated under reduced pressure to give **9-d** as a yellow solid (2.1g, yield 72.7%). LC-MS (ESI): m/z = 289 [M + H]⁺.

### Synthesis of compound 9-c

Compound **9-d** (486mg, 10mmol), compound **2-a** (480mg, 15mmol), [1,1'-bis(diphenylphosphino)ferrocene]dichloropalladium (36mg, 0.05mmol) and 2M aqueous sodium carbonate solution (8mL, 16mmol) were dissolved in 1,4-dioxane (13mL). The reaction solution was purged with nitrogen gas for three times to remove the oxygen contained in the system and then heated at 90°C for 6 hours. The reaction was cooled to room temperature, diluted with ice water (100mL) and extracted with dichloromethane (100mL x 3). The combined organic phase was washed with water (50mL x 3) and brine (50mL) successively, dried over anhydrous sodium sulfate, filtered and the filtrate was concentrated under reduced pressure. The residue was purified by silica gel TCL preparative plate (petroleum ether: ethyl acetate = 1: 1) to give **9-c** as a yellow solid (610mg, yield 87%). LC-MS (ESI): m/z = 466 [M + H]⁺.

### Synthesis of compound 9-b

Compound **9-c** (468mg, 1mmol) was dissolved in dichloromethane (5mL). The reaction solution was cooled to 0°C,trifluoroacetic acid (1mL) was added and the mixture was stirred at room temperature for 2 hours. The reaction solution was concentrated under reduced pressure and the residue was dissolved in dichloromethane (50mL) and diluted with saturated sodium carbonate solution (50mL). The separated organic phase was dried over anhydrous sodium sulfate, filtered and the filtrate was concentrated under reduced pressure. The residue was purified by preparative HPLC (mobile phase: 10mM aqueous ammonium bicarbonate solution: acetonitrile = 40%-50%) to give **9-b** (310mg, yield 81%) as a yellow solid. LC-MS (ESI): m/z = 382 [M + H]⁺.

### Synthesis of compound 9-a

Phosphorus tribromide (1mL) was slowly added dropwise to a solution of compound **9-b** (310mg, 0.81mmol) in dichloromethane (5mL),the mixture was stirred at room temperature for 3 hours and then quenched with saturated aqueous sodium bicarbonate solution (10mL). The mixture was extracted with dichloromethane (50mL x 3). The combined organic phase was dried over sodium sulfate, filtered and the filtrate was concentrated under reduced pressure to give compound **9-a** (280mg, yield 78%), which was used without further purification. LC-MS (ESI): m/z = 444 [M + H]⁺.

### Synthesis of compound 9

Compound **9-a** (140mg, 0.316mmol), 2-(4-piperidinyl)-2-propanol (54mg, 0.38mmol) and diisopropylethylamine (0.082g, 0.632mmol) were added to dichloromethane (5mL), and the reaction solution was stirred at room temperature for 3 hours, and then concentrated under reduced pressure, the residue was purified by silica gel TLC preparative plate (dichloromethane: methanol = 10: 1) to give **9** as a yellow solid (100mg, yield 62.5%). LC-MS (ESI): m/z = 507 [M + H]⁺.

¹H-NMR (400MHz, DMSO-*d6*) δ: 9.37(s, 1H), 8.94 (s, 1H), 7.33 (s, 1H), 7.13 (s, 1H), 7.11 (m, 1H), 6.95 (m, 1H), 3.95(s, 1H), 3.83(s, 3H), 3.65(s, 3H), 3.17(s, 2H), 2.60 (t, *J*=8Hz, 2H), 2.41 (t, *J*=8Hz, 2H), 2.38(s, 3H), 1.56 (t, *J*=8Hz, 2H), 1.41 (t, J=8Hz, 2H), 0.91 (s, 6H) ppm

### Example 10

### N-{7-[2-(difluoromethoxy)phenyl]-6-methylthieno[3,2-d]pyrimidinyl-2-yl]-1-methyl-1H-pyrazol-4-amine (Compound 10)

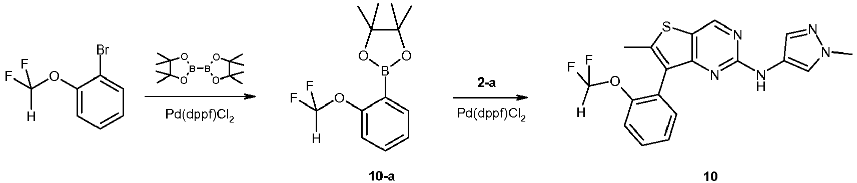

### Synthesis of compound 10-a

2-Bromodifluoromethylphenylether (1g, 4.5 mmol) and bis(pinacolato)diboron (1.71g, 6.75mmol) were dissolved in dioxane (10mL), and anhydrous potassium acetate (1.1g, 13.53mmol) and [1,1'-bis(diphenylphosphino)ferrocene]dichloropalladium • dichloromethane (369mg, 0.45mmol) were added. Under nitrogen gas atmosphere, the mixture was heated to 85°C and stirred for 16 hours. After cooling to room temperature, the reaction was concentrated under reduced pressure. The residue was purified by silica gel column chromatography (petroleum ether: ethyl acetate = 10: 1) to give **10-a** as a pale yellow oil (1.1g, yield 90.2%).

### Synthesis of compound 10

Compound **10-a** (126mg, 0.465mmol), compound **2-a** (100mg, 0.31mmol) and sodium carbonate (99mg, 0.93mmol) were suspended in dioxane (0.5mL) and water (0.5mL), [1,1'-bis(diphenylphosphino)ferrocene]dichloropalladium • dichloromethane (25mg, 0.03mmol) was added. The reaction solution was purged with nitrogen gas for three times and heated at 90°C under microwave for 50 minutes. After cooling to room temperature, the reaction mixture was concentrated under reduced pressure. The residue was partitioned between dichloromethane (50mL) and water (50mL). The organic phase was dried over anhydrous sodium sulfate, filtered and the filtrate was concentrated under reduced pressure. The residue was purified by silica gel TLC preparative plate (ethyl acetate) to give **10** as a pale yellow solid (35mg, yield 30%). LC-MS (ESI): m/z = 389 [M + H]⁺.

¹H-NMR (400MHz, CDCl₃) δ: 8.75 (s, 1H), 7.68 (s, 1H), 7.47-7.51 (m, 2H), 7.37-7.41 (m, 1H), 7.32-7.34 (m, 1H), 6.81(br, 1H), 6.13-6.50 (t, *J_{H-F}*=74Hz, 1H), 3.77 (s, 3H), 2.53 (s, 3H) ppm

### Example 11

### N-{7-[4-(3-methylsulfonylpropoxy)-2-methoxyphenyl]-6-methylthieno[3,2-d]pyrimidinyl-2-yl]-1-methyl-1H-pyrazol-4-amine (Compound 11)

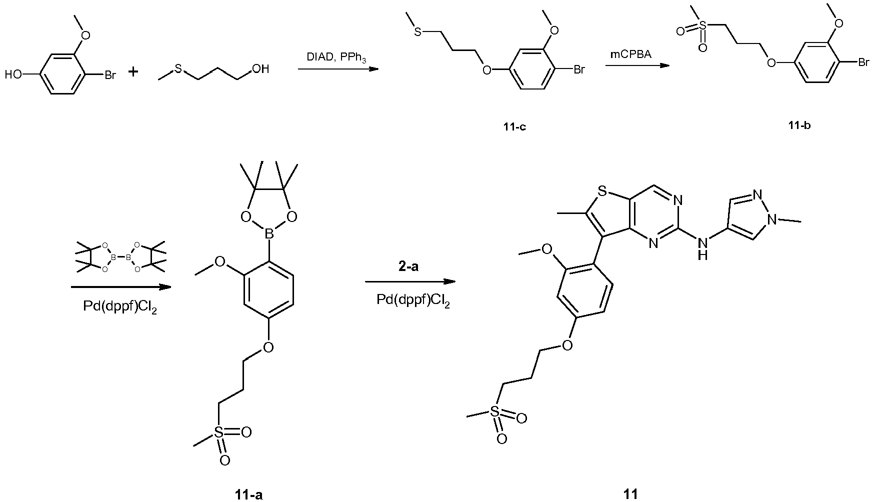

### Synthesis of compound 11-c

3-Methylthiopropanol (830mg, 7.83mmol) and 3-methoxy-4-bromophenol (1.44g, 7.13mmol) were dissolved in anhydrous tetrahydrofuran (50mL) and triphenylphosphine (2.8g, 10.68mmol). The reaction solution was cooled to 0°C and diisopropyl azodicarboxylate (2.24g, 7.13mmol) was added dropwise. After the addition was complete, the temperature was raised to room temperature and stirring continued for 18 hours. The reaction mixture was concentrated under reduced pressure and the residue was purified by silica gel column chromatography (petroleum ether: ethyl acetate = 10: 1) to give **11-c** as a yellow oil (250mg, yield 12%).

### Synthesis of compound 11-b

Compound **11-c** (610mg, 2.10mmol) was dissolved in dichloromethane (15mL), *m*-chloroperbenzoic acid (907mg, 5.26mmol) was added and the mixture was stirred at room temperature for 16 hours. The reaction mixture was cooled to 0°C, filtered, the filter cake was washed with cold dichloromethane and the filtrate was concentrated under reduced pressure. The residue was purified by silica gel column chromatography (petroleum ether: ethyl acetate = 1: 1) to give **11-b** as a white solid (210mg, yield 31%).

¹H-NMR (400MHz, CDCl₃) δ: 7.40 (d, *J*=9Hz, 1H), 6.47 (d, *J*=2Hz, 1H), 6.38 (dd, *J*=9Hz, *J*=2Hz, 1H), 4.11 (t, *J*=6Hz, 2H), 3.87 (s, 3H), 3.26 (t, *J*=6Hz, 2H), 2.96 (s, 3H), 2.35 (m, 2H) ppm

### Synthesis of compound 11-a

Compound **11-b** (100mg, 0.31mmol) and bis(pinacolato)diboron (120mg, 0.46mmol) were dissolved in dioxane (5mL) and anhydrous potassium acetate (77mg,0.93mmol) and [1,1'-bis(diphenylphosphino)ferrocene]dichloro-palladium•dichloromethane (25mg, 0.03mmol) were added. Under nitrogen gas atmosphere, the reaction solution was heated to 85°C and stirred for 16 hours. After cooling to room temperature, the reaction mixture was diluted with ethyl acetate (20mL), filtered through celite and the filtrate was concentrated under reduced pressure. The residue was purified by silica gel TLC preparative plate(petroleum ether: ethyl acetate = 1: 1) to give **11-a** as a pale yellow solid (45mg, yield 39%).

### Synthesis of compound 11

Compound **11-a** (40mg, 0.11mmol), compound **2-a** (35mg, 0.11mmol) and sodium carbonate (35mg, 0.33mmol) were suspended in dioxane (0.5mL) and water (0.5mL), and [1,1'-bis(diphenylphosphino)ferrocene]dichloropalladium•dichloromethane (16mg, 0.02mmol) was added. The reaction solution was purged with nitrogen gas for three times and was heated at 90°C under microwave for 40 minutes. After cooling to room temperature, the reaction solution was concentrated under reduced pressure to remove the solvent. The residue was partitioned between dichloromethane (50mL) and water (50mL). The organic phase was dried over anhydrous sodium sulfate, filtered and the filtrate was concentrated under reduced pressure. The residue was purified by silica gel TLC preparative plate (dichloromethane: methanol = 10: 1) to give **11** as a pale yellow solid (15mg, yield 29%). LC-MS (ESI): m/z = 488 [M + H]⁺.

¹H-NMR (400MHz, CDCl₃) δ: 8.72 (s, 1H), 7.77 (s, 1H), 7.44 (s, 1H), 7.31 (d, J=8Hz, 1H), 6.90 (br, 1H), 6.61-6.64 (m, 2H), 4.20 (t, *J*=6Hz, 1H), 3.81 (s, 3H), 3.76 (s, 3H), 3.31 (t, *J*=6Hz, 2H), 2.99 (s, 3H), 2.47 (s, 3H), 2.38-2.42 (m, 2H) ppm

### Example 12

### N-(7-{2-methoxy-4-[(3R)-3-tetrahydrofuranoxyl]phenyl]-6-methylthieno[3,2-d]pyrimidinyl-2-yl]-1-methyl-1H-pyrazol-4-amine (Compound 12)

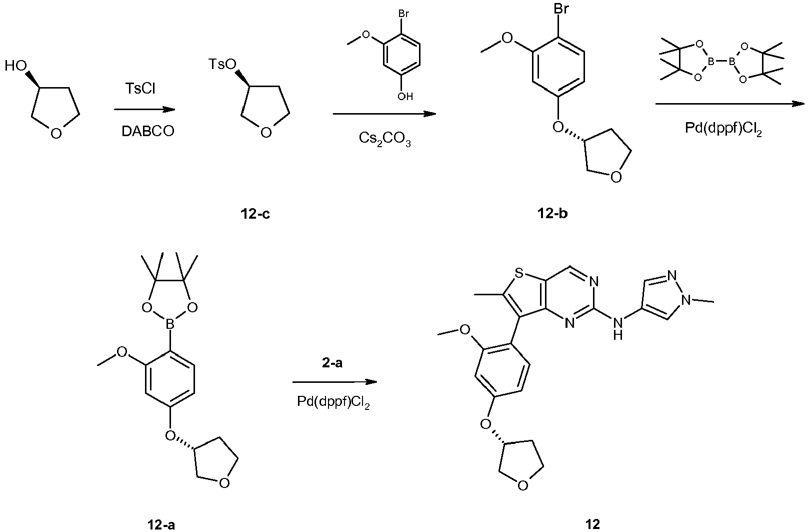

### Synthesis of compound 12-c

In an ice bath, 1,4-diazabicyclo[2.2.2]octane (4.76g, 42.43mmol) and p-toluenesulfonyl chloride (3.09g, 16.2mmol) were added into a solution of (S)-tetrahydrofuran-3-methanol (1mL, 12.48mmol) in dichloromethane (10mL) respectively. After the addition, the reaction solution was warmed to room temperature and stirred for 1 hour. Additional *p*-toluenesulfonyl chloride (1g, 5.25mmol) was added and the reaction solution was stirred at 28°C for another 16 hours. The reaction solution was diluted with dichloromethane (30mL) and washed with water (30mL). The organic phase was dried over anhydrous sodium sulfate and the filtrate was concentrated under reduced pressure. The residue was purified by silica gel column chromatography (petroleum ether: ethyl acetate = 30: 1) to give **12-c** as an oil (2.1g, yield 70%).

### Synthesis of compound 12-b

At room temperature, 3-methoxy-4-bromophenol (0.4g, 1.97mmol) and cesium carbonate (0.96g, 2.96mmol) were added into a solution of compound **12-c** (0.57g, 2.36mmol) in N,N-dimethylformamide (5mL) respectively. After the addition, the reaction solution was stirred at 75°C for 16 hours. The reaction solution was diluted with ethyl acetate (10mL), washed sequentially with water (10mL × 3) and brine (10mL × 3). The organic phase was dried over anhydrous sodium sulfate and the filtrate was concentrated under reduced pressure. The residue was purified by silica gel column chromatography (petroleum ether: ethyl acetate = 10: 1) to give compound **12-b** (0.55mg, yield 86%).

### Synthesis of compound 12-a

Compound **12-b** (615mg, 2.25mmol) and bis(pinacolato)diboron (860mg, 3.38mmol) were dissolved in dioxane (10mL), and anhydrous potassium acetate (662mg, 6.75mmol) and [1,1'-bis(diphenylphosphino)ferrocene]dichloropalladium•dichloromethane (183mg, 0.23mmol) were added. Under nitrogen gas atmosphere, the reaction was heated to 100°C and stirred for 16 hours. After cooling to room temperature, the reaction mixture was diluted with ethyl acetate (20mL), filtered through celite and the filtrate was concentrated under reduced pressure. The residue was purified by silica gel column chromatography (petroleum ether: ethyl acetate =10:1) to give compound **12-a** (550mg, yield 53%). LC-MS (ESI): m/z = 321 [M + H]⁺.

### Synthesis of compound 12

Compound **12-a** (70mg, 0.22mmol), compound **2-a** (72mg, 0.22mmol) and sodium carbonate (70mg, 0.66mmol) were suspended in dioxane (3mL) and water (3mL), [1,1'-bis(diphenylphosphino)ferrocene]dichloropalladium•dichloromethane (20mg, 0.025mmol) was added. The reaction solution was purged replaced with nitrogen gas for three times, and stirred at 80°C for 16 hours. After cooling to room temperature, the reaction mixture was filtered through celite, the filter cake was washed with ethyl acetate (20mL), the filtrate was washed with brine (20mL), the organic phase was dried over anhydrous sodium sulfate, filtered and the filtrate was concentrated under reduced pressure, and the residue was purified by silica gel TLC preparative plate (dichloromethane: methanol = 10: 1) to give compound **12** (10mg, yield 10%). LC-MS (ESI): m/z = 438 [M + H]⁺.

¹H-NMR (400MHz, CDCl₃) δ: 8.73 (s, 1H), 7.80 (s, 1H), 7.40 (s, 1H), 7.30 (d, *J* = 8.4 Hz, 1H), 7.27 (brs, 1H), 6.61 (d, *J* =2.4 Hz, 1H), 6.58 (dd, *J* = 2.4, 5.4 Hz, 1H), 4.96-5.07 (m, 1H), 3.99-4.11 (m, 3H), 3.88-3.99 (m, 1H), 3.80 (s, 3H), 3.74 (s, 3H), 2.48 (s, 3H), 2.17-2.33 (m, 2H) ppm

### Example 13

### N-[7-(2-methoxyphenyl)-6-methylthieno[3,2-d]pyrimidinyl-2-yl]-1-ethyl-1H-pyrazol-4-amine (Compound 13)

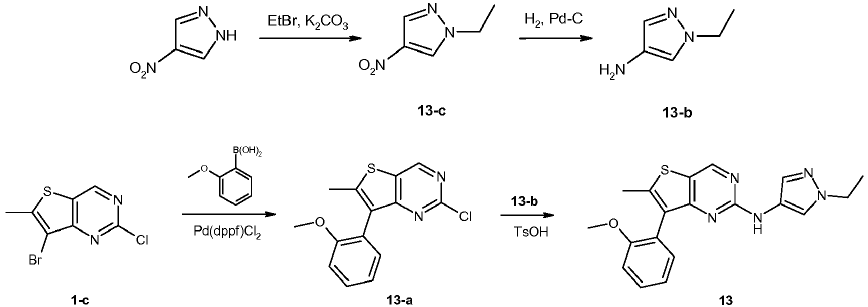

### Synthesis of compound 13-c

Bromoethane (1.1g, 10mmol) and potassium carbonate (2.76g, 20mmol) were added to a solution of 4-nitropyrazole (1.13g, 10mmol) in N,N-dimethylformamide (15mL), the mixture was heated to 90°C and stirred for 12 hours. After cooling to room temperature, the reaction solution was added with water (60mL) and extracted with ethyl acetate (20mL x 3). The organic phases were combined, dried over anhydrous sodium sulfate, filtered and the filtrate was concentrated under reduced pressure. The residue was purified by silica gel column chromatography (petroleum ether: ethyl acetate = 5: 1) to give compound **13-c** (1.2g, yield 85%). LC-MS (ESI): m/z = 142 [M + H]⁺.

### Synthesis of compound 13-b

Palladium 10% on carbon (0.1g) was added to a solution of compound **13-c** (1.0g, 7.1mmol) in methanol (10mL) under hydrogen gas atmosphere (latm). The mixture was reacted at 25°C for 12 hours, then filtered and the filtrate was concentrated under reduced pressure to give compound **13-b** (760mg, yield 96%), which was directly used for the next step without purification. LC-MS (ESI): m/z = 112 [M + H]⁺.

### Synthesis of compound 13-a

2-Methoxyphenylboronic acid (150mg, 1mmol), compound **1-c** (380mg, 1.5mmol), [1,1'-bis(diphenylphosphino)ferrocene]dichloropalladium (36mg, 0.05mmol) and 2M aqueous sodium carbonate solution (2mL, 4mmol) were dissolved in 1,4-dioxane (8mL). The reaction solution was purged with nitrogen gas for three times to remove oxygen contained in the system, and then heated at 110°C for 6 hours. The reaction was cooled to room temperature, diluted with ice water (100mL) and extracted with dichloromethane (100mL x 3). The combined organic phase was washed with water (50mL x 3) and brine (50mL) successively, dried over anhydrous sodium sulfate, filtered and the filtrate was concentrated under reduced pressure. The residue was purified by silica gel TLCpreparative plate (petroleum ether: ethyl acetate = 10: 1) to give **13-a** as a yellow solid (239mg, yield 87%). LC-MS (ESI): m/z = 291 [M + H]⁺.

### Synthesis of compound 13

Compound **13-b** (83mg, 0.75mmol), *p*-toluenesulfonic acid (150mg, 0.75mmol) and compound **13-a** (145mg, 0.5 mmol) were added to n-butanol (10mL), the mixture was heated to 108 °C and stirred for 6 hours. After cooling to room temperature, the reaction solution was concentrated, the residue was added toa saturated aqueous sodium bicarbonate solution (80mL) and extracted with dichloromethane (100mL x 3). The combined organic phase was dried over anhydrous sodium sulfate, filtered and the filtrate was concentrated under reduced pressure. The residue was purified by silica gel TLCpreparative plate (petroleum ether: ethyl acetate = 1: 1) to give **13** as a yellow solid (67mg, yield 38%). LC-MS (ESI): m/z =366 [M + H]⁺.

¹H-NMR (400MHz, MeOD) δ: 8.67(s, 1H), 7.72 (s, 1H), 7.40 (t, *J*=8Hz, 1H), 7.34 (s, 1H), 7.26 (d, *J*=8Hz, 1H), 7.08 (d, *J*=8Hz, 1H), 7.03 (t, *J*=8Hz, 1H), 3.90 (q, *J*=8Hz, 2H), 3.67 (s, 3H), 2.37 (s, 3H), 1.23 (t, *J*=8Hz, 3H) ppm

### Example 14

### N-[7-(2-methoxyphenyl)-6-methylthieno[3,2-d]pyrimidinyl-2-yl]-1-isopropyl-1H-pyrazol-4-amine (Compound 14)

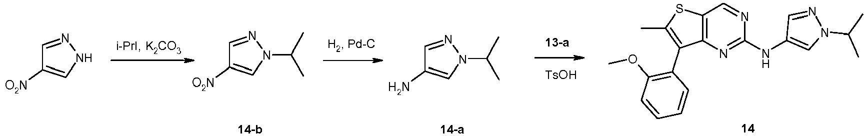

### Synthesis of compound 14-b

2-Iodopropane (2.3g, 13.27mmol) and potassium carbonate (1.81g, 13.27mmol) were sequentially added to a solution of 4-nitropyrazole (1.0g, 8.85mmol) in N,N-dimethylformamide (10mL) and the mixture was heated to 60°C for 3 hours. The mixture was poured into ice water (100mL) and extracted with ethyl acetate (100mL x 3). The organic phases were combined, dried over anhydrous sodium sulfate, filtered and the filtrate was concentrated under reduced pressure to give **14-b** as a yellow oil (1.1g, yield 81%), which was directly used for the next reaction without purification.

### Synthesis of compound 14-a

Palladium 10% on carbon (0.2g) was added to a solution of compound **14-b** (1.0g, 8.8mmol) in ethanol (20mL) under hydrogen gas atmosphere (latm). The mixture was reacted at 25°C for 12hours, filtered and the filtrate was concentrated under reduced pressure to give compound **14-a** (830mg, yield 94%), which was directly used for the next reaction without purification. LC-MS (ESI): m/z =126 [M + H]⁺.

### Synthesis of compound 14

Compound **14-a** (94mg, 0.75mmol), *p*-toluenesulfonic acid (150mg, 0.75mmol) and compound **13-a** (145mg, 0.5 mmol) were added to n-butanol (10mL), the mixture was heated to 108 °C and stirred for 6 hours. After cooling to room temperature, the reaction solution was concentrated, the residue was added toa saturated aqueous sodium bicarbonate solution (80mL) and extracted with dichloromethane (100mL x 3). The combined organic phase wasdried over anhydrous sodium sulfate, filtered and the filtrate was concentrated under reduced pressure. The residue was purified by silica gel TLCpreparative plate (petroleum ether: ethyl acetate = 1: 1) to give **14** as a yellow solid (87mg, yield 46%). LC-MS (ESI): m/z =380 [M + H]⁺.

¹H-NMR (400MHz, DMSO-d6) δ: 8.67 (s, 1H), 7.74 (s, 1H), 7.36 (t, *J*=8Hz, 1H), 7.32 (s, 1H), 7.25 (d, *J*=8Hz, 1H), 7.08 (d, *J*=8Hz, 1H), 7.03 (t, *J*=8Hz, 1H), 4.20 (m, 1 H), 3.67 (s, 3H), 2.35 (s, 3H), 1.25 (d, *J*=8Hz, 6H) ppm

### Example 15

### N-(7-{2-methoxy-4-[(3S)-3-tetrahydrofuranoxy]phenyl}-6-methylthieno[3,2-d]pyrimidinyl-2-yl]-1-methyl-1H-pyrazol-4-amine (Compound 15)

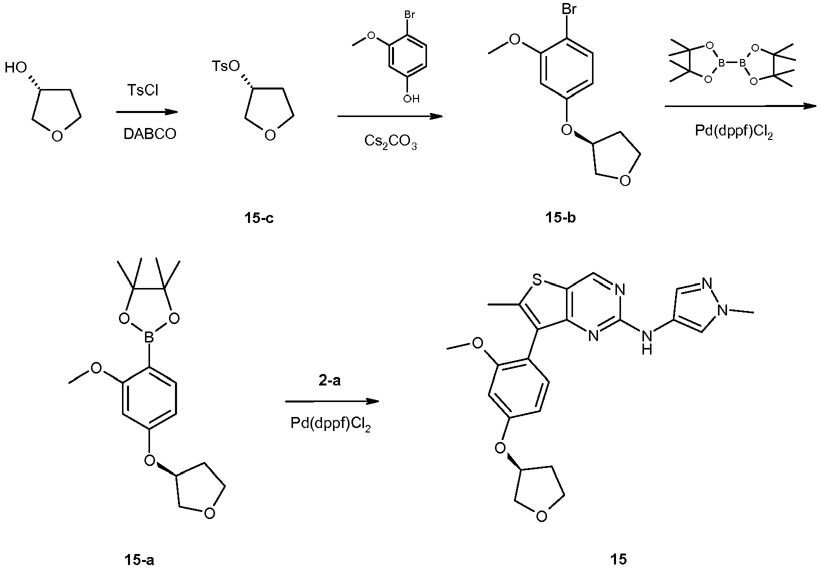

### Synthesis of compound 15-c

In an ice bath, 1,4-diazabicyclo[2.2.2]octane (2.52g, 22.47mmol) and p-toluenesulfonyl chloride (4.28g, 22.45mmol) were added to a solution of (R)-tetrahydrofuran-3-methanol (0.9mL, 11.24mmol) in dichloromethane (10mL) respectively. After the addition, the reaction solution was warmed to room temperature and stirred for 1 hour. The reaction solution was diluted with dichloromethane (30mL) and washed with water (30mL). The organic phase was dried over anhydrous sodium sulfate and the filtrate was concentrated under reduced pressure. The residue was purified by silica gel column chromatography (petroleum ether: ethyl acetate = 30: 1) to give **15-c** (2.17g, yield 80%).

### Synthesis of compound 15-b

At room temperature, 3-methoxy-4-bromophenol (0.4g, 1.97mmol) and cesium carbonate (0.96g, 2.96mmol) were added to a solution of compound **15-c** (0.43g, 1.78mmol) in N,N-dimethylformamide (4mL) respectively. After the addition, the reaction solution was stirred at 80°C for 16 hours. The reaction solution was diluted with ethyl acetate (10mL), washed sequentially with water (10mL × 3) and brine (10mL × 3). The organic phase was dried over anhydrous sodium sulfate and the filtrate was concentrated under reduced pressure. The residue was purified by silica gel column chromatography (petroleum ether: ethyl acetate = 10: 1) to give compound **15-b** (0.31mg, yield 64%).

¹H-NMR (400MHz, CDCl₃) δ: 7.40 (d, *J* = 8.4 Hz, 1H), 6.48 (d, *J* = 2.8 Hz, 1H), 6.33 (dd, *J* = 2.8, 8.8 Hz, 1H), 4.85-4.94 (m, 1H), 3.94-4.05 (m, 3H), 3.87-3.94 (m, 1H), 3.86 (s, 3H), 2.07-2.29 (m, 2H) ppm

### Synthesis of compound 15-a

Compound **15-b** (309mg, 1.13mmol) and bis(pinacolato)diboron (430mg, 1.7mmol) were dissolved in dioxane (10mL), and anhydrous potassium acetate (333mg, 3.39mmol) and [1,1'-bis(diphenylphosphino)ferrocene]dichloropalladium•dichloromethane (90mg, 0. 1mmol) were added. Under nitrogen gas atmosphere, the reaction solution was heated to 100°C and stirred for 16 hours. After cooling to room temperature, the reaction mixture was diluted with ethyl acetate (20mL), filtered through celite and the filtrate was concentrated under reduced pressure. The residue was purified by silica gel column chromatography (petroleum ether: ethyl acetate = 10:1) to give compound **15-a** (170mg, yield 47%). LC-MS (ESI): m/z =321 [M + H]⁺.

### Synthesis of compound 15

Compound **15-a** (92mg, 0.28mmol), compound **2-a** (100mg, 0.31mmol) and sodium carbonate (92mg, 0.86mmol) were suspended in dioxane (2.5mL) and water (2.5mL), [1,1'-bis(diphenylphosphino)ferrocene]dichloropalladium•dichloromethane (15mg, 0.018mmol) was added. The reaction solution was purged with nitrogen gas for three times, and stirred at 80°C for 16 hours. After cooling to room temperature, the reaction mixture was filtered through celite, the filter cake was washed with ethyl acetate (20mL), the filtrate was washed with brine (20mL), the organic phase was dried over anhydrous sodium sulfate, filtered and the filtrate was concentrated under reduced pressure, and the residue was purified by silica gel TLC preparative plate (dichloromethane: methanol = 10: 1) to give compound **15** (16mg, yield 13%). LC-MS (ESI): m/z =438 [M + H]⁺.

¹H-NMR (400MHz, CDCl₃) δ: 8.73 (s, 1H), 7.80 (s, 1H), 7.40 (s, 1H), 7.30 (d, *J* = 8.4 Hz, 1H), 7.17 (brs, 1H), 6.52 - 6.66 (m, 2H), 4.96-5.07 (m, 1H), 3.99-4.11 (m, 3H), 3.88-3.99 (m, 1H), 3.80 (s, 3H), 3.74 (s, 3H), 2.48 (s, 3H), 2.17-2.31 (m, 2H) ppm

### Example 16

### N-(7-{4-[3-(1-azetidinyl)propoxy-2-methoxyphenyl}-6-methylthieno[3,2-d]pyrimidinyl-2-yl)-1-methyl-1H-pyrazol-4-amine (Compound 16)

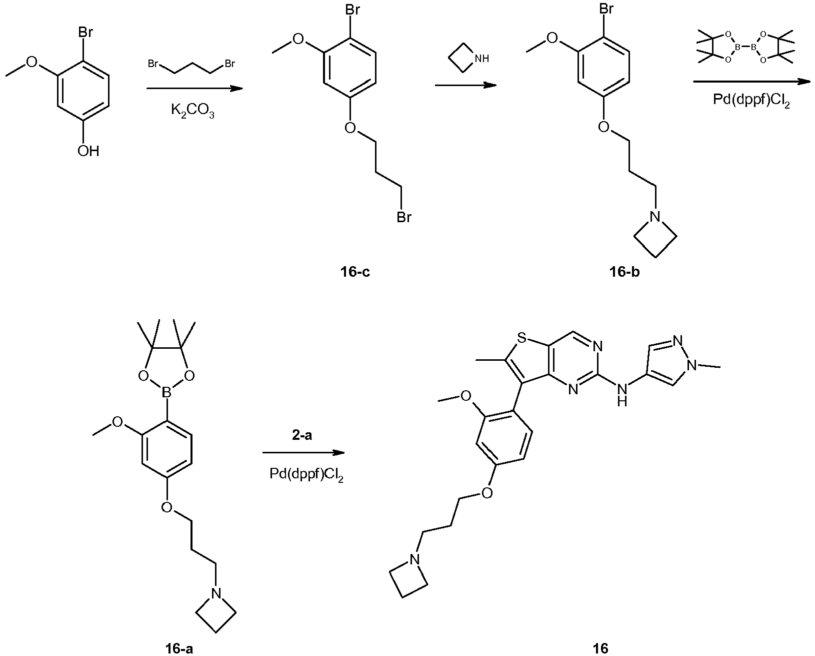

### Synthesis of compound 16-c

3-Methoxy-4-bromophenol (350mg, 1.73mmol) and potassium carbonate (716mg, 5.19mmol) were suspended in acetonitrile (10mL), 1,3-dibromopropane (700mg, 3.46mmol) was added. The reaction solution was heated to 80°C for 6 hours. After cooling to room temperature, the reaction solution was filtered, the filter cake was washed with ethyl acetate (50mL), and the filtrate was concentrated under reduced pressure. The residue was partitioned between ethyl acetate (50mL) and water (50mL). The organic phase was dried over anhydrous sodium sulfate, filtered and the filtrate was concentrated under reduced pressure. The residue was purified by silica gel column chromatography (petroleum ether: ethyl acetate = 10: 1) to give **16-c** as a colorless oil (310mg, yield 56%).

¹H-NMR (400MHz, CDCl₃) δ: 7.40 (d, *J*=9Hz, 1H), 6.49 (d, *J*=3Hz, 1H), 6.40 (dd, *J*=9Hz, *J*=3Hz, 1H), 4.11 (t, *J*=6Hz, 2H), 3.87 (s, 3H), 3.61 (t, *J*=6Hz, 2H), 2.31 (m, 2H) ppm

### Synthesis of compound 16-b

Compound **16-c** (150mg, 0.47mmol) was dissolved in N,N-dimethylacetamide (1mL), and azetidine (0.5mL) was added. The reaction solution was heated to 80°C and reacted for 2 hours, then diluted with water (20mL), followed by extraction with ethyl acetate (20mL x 2). The combined organic phase was dried over anhydrous sodium sulfate, filtered and the filtrate was concentrated under reduced pressure to give **16-b** as a pale yellow oil (310mg), which was directly used for the next step without purification. LC-MS (ESI): m/z =300 [M + H]⁺.

### Synthesis of compound 16-a

Compound **16-b** (310mg, 1.03mmol) and bis(pinacolato)diboron(177mg, 0.69mmol) were dissolved in dioxane (5mL), anhydrous potassiumacetate (114mg, 1.39mmol) and [1,1'-bis(diphenylphosphino)ferrocene]dichloropalladium • dichloromethane (41mg, 0.05mmol) were added. Under nitrogen gas atmosphere, the mixture was heated to 85°C and stirred for 16 hours. After cooling to room temperature, the mixture was diluted with ethyl acetate (20mL) and filtered through celite. The filtrate was concentrated to dryness to give **16-a** as a black oil (190mg), which was directly used for the next step without purification. LC-MS (ESI): m/z =348 [M + H]⁺.

### Synthesis of compound 16

Compound **16-a** (190mg, 0.54mmol), compound **2-a** (90mg, 0.28mmol) and sodium carbonate (88mg, 0.84mmol) were suspended in dioxane (0.5mL) and water (0.5mL), [1,1'-bis(diphenylphosphino)ferrocene]dichloropalladium • dichloromethane (25mg, 0.03mmol) was added. The mixture was purged with nitrogen gas for three times, and heated to 90°C undermicrowave to react for 40 minutes. After cooling to the room temperature, the reaction was concentrated under reduced pressure to remove solvent, the residue was partitioned between dichloromethane (50mL) and water (50mL). The combined organic phase was dried over anhydrous sodium sulfate, filtered and the filtrate was concentrated under reduced pressure. The residue was purified by preparative HPLC (mobile phase: 10 mM aqueous ammonium bicarbonate solution: acetonitrile = 35%-45%) to give **16** as awhite solid (12mg, yield 7%). LC-MS (ESI): m/z =465 [M + H]⁺.

¹H-NMR (400MHz, CDCl₃) δ: 8.72 (s, 2H), 7.82 (s, 1H), 7.39 (s, 1H), 7.28-7.39 (m, 1H), 6.94 (br, 1H), 6.62-6.65 (m, 2H), 4.08 (t, *J*=6Hz, 2H), 3.86 (s, 3H), 3.80 (s, 3H), 3.20-3.27 (m, 4H), 2.60-2.64 (m, 2H), 2.48 (s, 3H), 2.11 (m, 2H), 1.84 (m, 2H) ppm

### Example 17

### N-(7-{2-methoxy-4-[3-(4-morpholinyl)propoxy]phenyl}-6-methylthieno[3,2-d]pyrimidinyl-2-yl)-1-methyl-1H-pyrazol-4-amine (Compound 17)

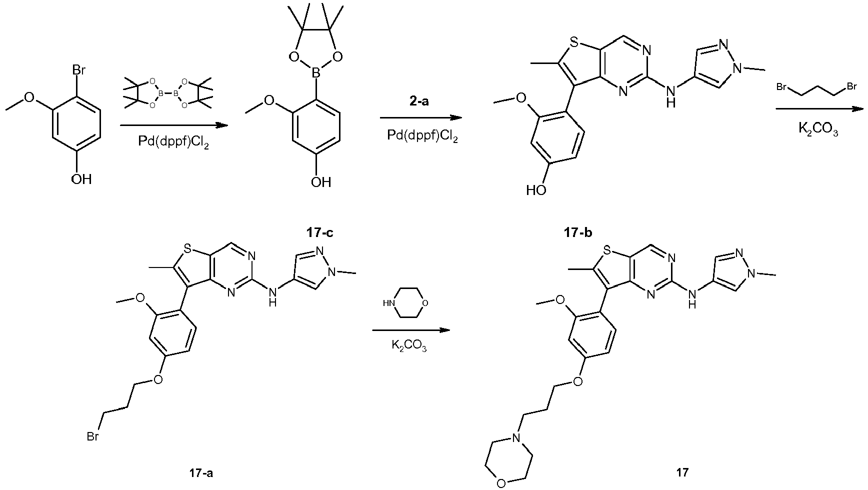

### Synthesis of compound 17-c

3-Methoxy-4-bromophenol (5g, 24.75mmol) and bis(pinacolato)diboron(9.43g, 37.13mmol) were dissolved in dioxane (50mL), anhydrous potassiumacetate (6.1g, 74.25mmol) and [1,1'-bis(diphenylphosphino)ferrocene]dichloro-palladium •dichloromethane (2g, 2.47mmol) were added. Under nitrogen gas atmosphere, the mixture was heated to 80°C and stirred for 16 hours. After cooling to room temperature, the mixture was diluted with ethyl acetate (50mL) and filtered through celite, the filtrate was concentrated under reduced pressure. The residue was purified by silica gel column chromatography (petroleum ether: ethyl acetate = 1: 1) to give **17-c** as a pale yellow solid (2.8g, yield 45%). LC-MS (ESI): m/z =251 [M + H]⁺.

### Synthesis of compound 17-b

Compound **17-c** (900mg,3.6mmol), compound **2-a** (969mg, 3.0mmol) and sodium carbonate (980mg, 9.0mmol) were suspended in dioxane (5mL) and water (5mL), [1,1'-bis(diphenylphosphino)ferrocene]dichloropalladium•dichloromethane (245mg, 0.3mmol) was added. The reaction solution was purged with nitrogen gas for three times and heated to 90°C under microwave for 40 minutes. After cooling to room temperature, the reaction solution was concentrated under reduced pressure to remove the solvent. The residue was partitioned between ethyl acetate (50mL) and water (50mL). The organic phase was dried over anhydrous sodium sulfate, filtered and the filtrate was concentrated under reduced pressure. The residue was purified by silica gel column chromatography (dichloromethane: methanol = 20: 1) to give **17-b** as a pale yellow solid (350mg, yield 32%). LC-MS (ESI): m/z =368 [M + H]⁺.

### Synthesis of compound 17-a

Compound **17-b** (90mg, 0.25mmol) and potassium carbonate (51mg, 0.37mmol) were suspended in acetonitrile (10mL), and 1,3-dibromopropane (72mg, 0.36mmol) was added. The reaction mixture was heated to 60°C and stirred for 3 hours. After cooling to room temperature, the mixturewas filtered and the filter cake was washed with ethyl acetate (50mL). The filtrate was concentrated under reduced pressure and the residue was partitioned between ethyl acetate (50mL) and water (50mL). The organic phase was dried over anhydrous sodium sulfate, filtered, and the filtrate was concentrated under reduced pressure to give **17-a** as a pale yellow oil(120mg), which was directly used for the next step without purification.

### Synthesis of compound 17

Compound **17-a** (35mg, 0.072mmol) and potassium carbonate (30mg, 0.22mmol) were suspended in acetonitrile (2mL), and morpholine (19mg, 0.22mmol) was added. The reaction mixture was heated to 60°C and stirred for 3 hours. After cooling to room temperature, the mixture was filtered and the filter cake was washed with acetonitrile (10mL). The filtrate was concentrated under reduced pressure and the residue was purified by preparative HPLC (mobile phase: 0.05% aqueous trifluoroacetic acid: acetonitrile = 20% to 50%) to give **17** as a pale yellow solid(20mg, yield 56%). LC-MS (ESI): m/z =495 [M + H]⁺.

¹H-NMR (400MHz, CDCl₃) δ: 8.72 (s, 1H), 7.81 (s, 1H), 7.41 (s, 1H), 7.29 (d, *J*=8Hz, 1H), 6.87 (br, 1H), 6.61-6.67 (m, 2H), 4.10 (t, *J*=6Hz, 2H), 3.93 (s, 3H), 3.75 (s, 3H), 3.74 (br, 4H), 2.59 (t, *J*=6Hz, 2H), 2.51 (br, 4H), 2.48 (s, 3H), 2.03 (m, 2H) ppm

### Example 18

### N-(7-{2-methoxy-4-[3-(1-pyrrolidinyl)propoxy]phenyl}-6-methylthieno[3,2-d]pyrimidinyl-2-yl)-1-methyl-1H-pyrazol-4-amine (Compound 18)

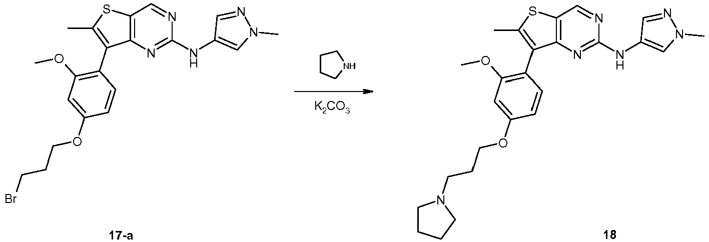

### Synthesis of compound 18

Compound **17-a** (35mg, 0.072mmol) and potassium carbonate (15mg, 0.11mmol) were suspended in acetonitrile (2mL), and pyrrolidine (8mg, 0.11mmol) was added. The reaction mixture was heated to 80°C and stirred for 3 hours. After cooling to room temperature, the mixture was filtered and the filter cake was washed with acetonitrile (10mL). The filtrate was concentrated under reduced pressure and the residue was purified by preparative HPLC (mobile phase: 0.05% aqueous ammonium bicarbonate solution: acetonitrile = 45% to 75%) to give **18** as a pale yellow solid(8mg, yield 28%). LC-MS (ESI): m/z =408 [M + H]⁺.

¹H-NMR (400MHz, CDCl₃) δ: 8.72 (s, 1H), 7.82 (s, 1H), 7.39 (s, 1H), 7.29 (d, *J*=8Hz, 1H), 7.13 (br, 1H), 6.62-6.66 (m, 2H), 4.10 (t, *J*=6Hz, 2H), 3.79 (s, 3H), 3.75 (s, 3H), 2.69 (t, *J*=6Hz, 2H), 2.56 (m, 4H), 2.48 (s, 3H), 2.07 (m, 2H), 1.81 (m, 4H) ppm

### Example 19

### N-(7-{2-methoxy-4-[3-(4-methylpiperazin-1-yl)propoxy]phenyl}-6-methylthieno[3,2-d]pyrimidinyl-2-yl)-1-methyl-1H-pyrazol-4-amine (Compound 19)

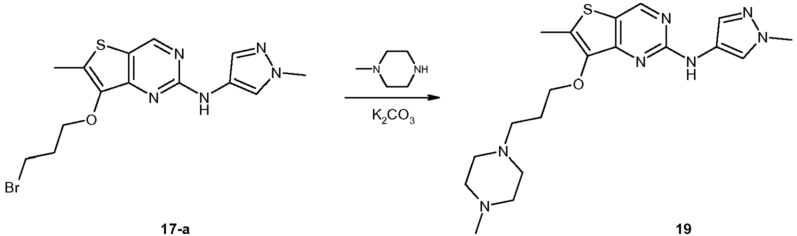

### Synthesis of compound 19

Compound **17-a** (35mg, 0.072mmol) and potassium carbonate (15mg, 0.11mmol) were suspended in acetonitrile (2mL), and N-methylpiperazine (11mg, 0.11mmol) was added. The reaction mixture was heated to 80°C and stirred for 3 hours. After cooling to room temperature, the mixturewas filtered and the filter cake was washed with acetonitrile (10mL). The filtrate was concentrated under reduced pressure and the residue was purified by preparative HPLC (mobile phase: 0.05% aqueous ammonium bicarbonate solution: acetonitrile = 20% to 50%) to give **19** as a pale yellow solid(8mg, yield 28%). LC-MS (ESI): m/z =508 [M + H]⁺.

¹H-NMR (400MHz, CDCl₃) δ: 8.72 (s, 1H), 7.82 (s, 1H), 7.39 (s, 1H), 7.29 (d, *J*=8Hz, 1H), 6.83 (br, 1H), 6.61-6.66 (m, 2H), 4.10 (t, *J*=6Hz, 2H), 3.79 (s, 3H), 3.75 (s, 3H), 2.59 (t, *J*=6Hz, 2H), 2.52 (br, 4H), 2.47 (s, 3H), 2.32 (s, 3H), 2.07 (m, 2H), 1.67 (br, 4H) ppm

### Example 20

### 2-(4-{[7-(4-methylsulfonyl-2-methoxyphenyl)-6-methylthieno[3,2-d]pyrimidinyl-2-yl]amino}-1H-pyrazol-1-yl)-1-ethanol (Compound 20)

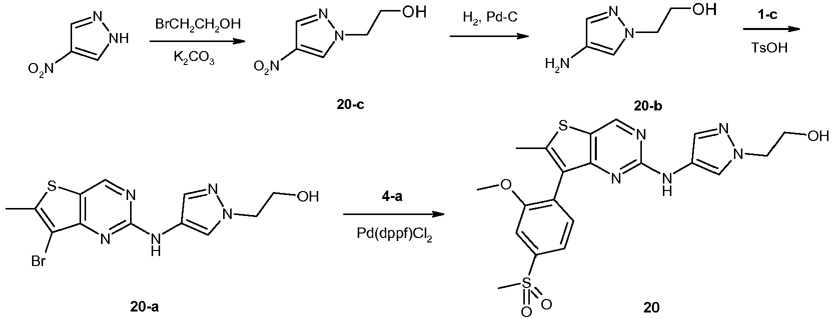

### Synthesis of compound 20-c

Bromoethanol (1.9g, 15.57mmol) and potassium carbonate (2.9g, 21.12mmol) were sequentially added to a solution of 4-nitropyrazole (1.6g, 14.16mmol) in acetonitrile (20mL). The suspension was stirred at 60°C for 16 hours. After cooling to room temperature, the mixturewas filtered and the filter cake was washed with acetonitrile (10mL). The filtrate was concentrated under reduced pressure to give **20-c** as a yellow oil (1.1 g, yield 49.5%), which was directly used for the next step without purification.

### Synthesis of compound 20-b

Palladium 10% on carbon (0.2g) was added to a solution of compound **20-c** (1.1g, 7mmol) in ethanol (20mL) under hydrogen gas atmosphere (latm). The mixture was reacted at 25°C for 16 hours, filtered and the filtrate was concentrated under reduced pressure to give **20-b** as a red oil (740mg, yield 83%), which was directly used for the next step without purification. LC-MS (ESI): m/z =128 [M + H]⁺.

### Synthesis of compound 20-a

Compound **1-c** (1g, 3.82mmol) and compound **20-b** (1.45g, 11.45mmol) were dissolved in n-butanol (15mL), and *p*-toluenesulfonic acid monohydrate(2.17g, 11.45mmol) was added. The mixture was heated to 110°C and stirred for 16 hours, then cooled to 0°C, filtered and the filter cake was washed with saturated sodium bicarbonate solution (50mL) to give **20-a** as a pale yellow solid (950mg, yield 71%), which was used without purification. LC-MS (ESI): m/z =354 [M + H]⁺.

### Synthesis of compound 20

Compound **20-a** (102mg, 0.29mmol), compound **4-a** (136mg, 0.43mmol) and sodium carbonate (93mg, 0.86mmol) were suspended in dioxane (2mL) and water (2mL), and [1,1'-bis(diphenylphosphino)ferrocene]dichloropalladium • dichloromethane (25mg, 0.03mmol) was added. The system was purged with nitrogen gas for three times, and the reaction solution was heated to 90°C under microwave for 40 minutes. After the reaction solution was cooled to room temperature, the reaction solution was concentrated under reduced pressure, the residue was partitioned between dichloromethane (50mL) and water (50mL). The organic phase was dried over anhydrous sodium sulfate, filtered and the filtrate was concentrated under reduced pressure. The residue was purified by silica gel TLCpreparative plate (ethyl acetate) to give **20** as a pale yellow solid (10mg, yield 8%). LC-MS (ESI): m/z =460 [M + H]⁺.

¹H-NMR (400MHz, CDCl₃) δ: 8.76 (s, 2H), 7.94 (s, 1H), 7.82 (s, 1H), 7.49 (d, J=8Hz, 1H), 7.44 (s, 1H), 7.36 (d, *J*=8Hz, 1H), 6.98 (br, 1H), 4.19 (t, *J*=6Hz, 1H), 3.94 (s, 5H), 3.13 (s, 4H), 2.67 (s, 3H) ppm

### Example 21

### 2-(4-{[7-(2-methoxyphenyl)-6-methylthieno[3,2-d]pyrimidinyl-2-yl]amino}-1H-pyrazol-1-yl)-1-ethanol (Compound 21)

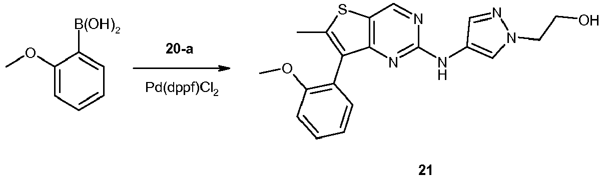

### Synthesis of compound 21

Compound **20-a** (100mg, 0.28mmol), 2-methoxyphenylboronic acid (65mg, 0.42mmol) and sodium carbonate (89mg, 0.84mmol) were suspended in dioxane (2mL) and water (2mL), and [1,1'-bis(diphenylphosphino)ferrocene]dichloropalladium • dichloromethane (25mg, 0.03mmol) was added. The system was purged with nitrogen gas for three times, and the reaction solution was heated to 90 °C under microwave for 40 minutes. After the reaction solution was cooled to room temperature, the reaction solution was concentrated under reduced pressure, the residue was partitioned between dichloromethane (50mL) and water (50mL). The organic phase was dried over anhydrous sodium sulfate, filtered and the filtrate was concentrated under reduced pressure. The residue was purified by silica gel TLCpreparative plate (ethyl acetate) to give **21** as a pale yellow solid (25mg, yield 23%). LC-MS (ESI): m/z =382 [M + H]⁺.

¹H-NMR (400MHz, CDCl₃) δ: 8.73 (s, 1H), 7.86 (s, 1H), 7.38-7.74 (m, 3H), 7.13 (t, *J*=8Hz, 1H), 7.06 (d, *J*=8Hz, 2H), 6.88 (br, 1H), 4.08 (t, *J*=4Hz, 1H), 3.95 (t, *J*=4Hz, 4H), 3.09 (br, 1H), 2.49 (s, 3H) ppm

### Example 22

### 4-[2-(4-{[7-(2-methoxyphenyl)-6-methylthieno[3,2-d]pyrimidinyl-2-yl]amino}-1H-pyrazol-1-yl)ethyl]piperazin-1-amine (Compound 22)

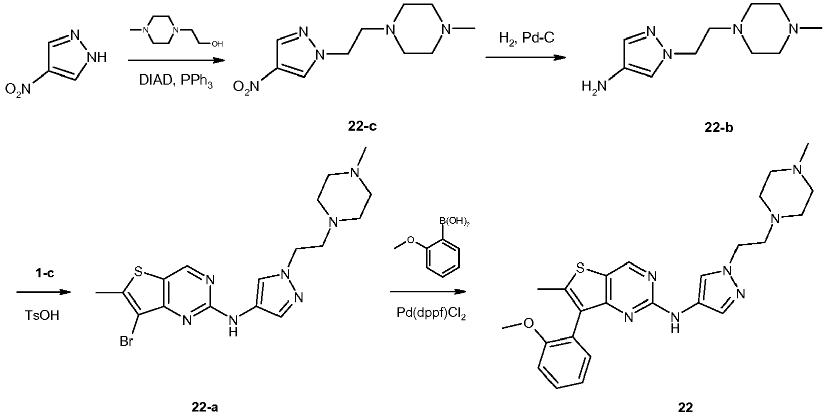

### Synthesis of compound 22-c

4-Nitropyrazole (2.81g, 13.88mmol) and 1-hydroxyethyl-4-methylpiperazine (1.0g, 6.94mmol) were dissolved in anhydrous tetrahydrofuran (50mL),and a solution of triphenylphosphine (3.64g, 13.88mmol) and diisopropyl azodicarboxylate (2.81g, 13.88mmol) in anhydrous tetrahydrofuran (6mL) was added dropwise under nitrogengas atmosphere. The reaction solution was stirred at room temperature for 1 hour, and then IN hydrochloric acid (30mL) and water (50mL) was added. The aqueous phase was extracted with ethyl acetate (50mL × 2). The combined organic phase was dried over anhydrous sodium sulfate, filtered and the filtrate was concentrated. The residue was purified by silica gel column chromatography (dichloromethane: methanol = 10: 1) to give compound **22-c** (1.0g, yield 60.2%). LC-MS (ESI): m/z =240.2 [M + H]⁺.

### Synthesis of compound 22-b

Palladium 10% on carbon (0.2g) was slowly added to a solution of compound **22-c** (1.0g, 4.18mmol) in methanol (20mL) under hydrogen gas atmosphere (latm). The mixture was reacted at 25 °C for 16 hours, and dichloromethane(50mL) was added to the reaction solution. The reaction solution was filtered through celite to remove palladium-carbon, and the filtrate was concentrated under reduced pressure to give **20-b** (680mg, yield 77.8%), which was directly used for the next step without purification.

### Synthesis of compound 22-a

Compound **1-c** (200mg, 0.76mmol) and compound **22-b** (319mg, 1.53mmol) were dissolved in *iso*-butanol (5mL), and *p*-toluenesulfonic acid monohydrate (435mg, 2.29mmol) was added. The mixture was heated to 108°C and stirred for 16 hours. After the mixture was cooled to room temperature, a saturated sodium bicarbonate solution (50mL) was slowly added thereto. The aqueous phase was extracted with a mixed solvent (50mL × 2) of tetrahydrofuran and ethyl acetate (1: 1). The combined organic layer was dried over anhydrous sodium sulfate, filtered and the filtrate was concentrated under reduced pressure. The residue was purified by silica gel column chromatography (dichloromethane: methanol: aqueous ammonia = 64: 8: 1) to give compound **22-a** (150mg, yield 45%). LC-MS (ESI): m/z =436 [M + H]⁺.

### Synthesis of compound 22

Compound **22-a** (60mg, 0.138mmol), 2-methoxyphenylboronic acid (42mg, 0.275mmol) and sodium carbonate (44mg, 0.414mmol) were suspended in dioxane (1.6mL) and water (0.4mL), and [1,1'-bis(diphenylphosphino)ferrocene]dichloropalladium • dichloromethane (10mg, 0.0138mmol) was added. The reaction solution was purged with nitrogen gas for three times, and heated to 90 °C under microwave for 40 minutes. After cooling to room temperature, the reaction solution was concentrated under reduced pressure, the residue was dissolved in dichloromethane (50mL), filtered through celite and the filtrate was concentrated under reduced pressure. The residue was purified by preparative HPLC (mobile phase: 10mM aqueous ammonium bicarbonate: acetonitrile = 40% - 50%) to give **22** as a pale yellow solid (38mg, yield 59.6%). LC-MS (ESI): m/z =464 [M + H]⁺.

¹H NMR (400 MHz, CDCl₃)δ : 8.73 (s, 1H), 7.82 (s, 1H), 7.39-7.45 (m, 3H), 7.05-7.14 (m, 2H), 6.85 (s, 1H), 4.10 (t, *J* = 7.2Hz, 2H), 3.78 (s, 3H), 2.74 (t, *J* = 7.2Hz, 2H), 2.39-2.56 (m, 11H), 2.29 (s, 3H) ppm

### Example 23

### 1-(azetidinyl)-2-(3-methoxy-4-{6-methyl-2-[(1-methyl-1H-pyrazol-4-yl)amino]thieno[3,2-d]pyrimidinyl-7-yl}phenoxy)-1-ethanol (Compound 23)

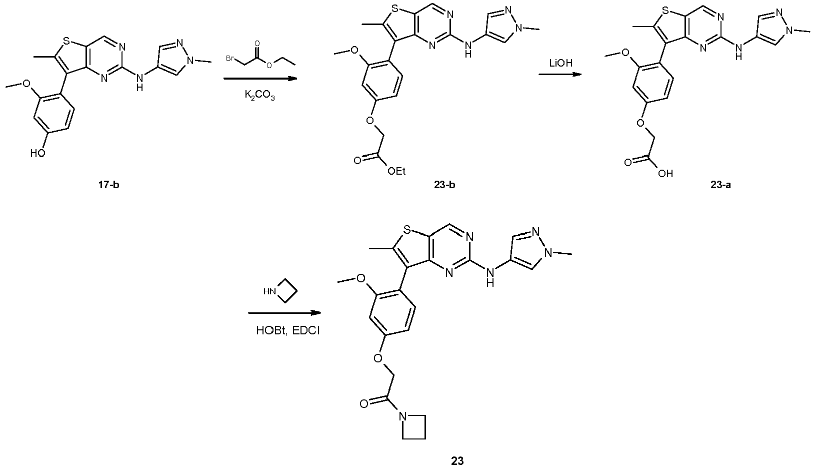

### Synthesis of compound 23-b

Compound **17-b** (120mg, 0.33mmol) and ethyl bromoacetate (82mg, 0.49mmol) were dissolved in acetonitrile (2mL) and potassium carbonate (69mg, 0.49mmol) was added and the reaction mixture was stirred at room temperature for 16 hours, then filtered. The filter cake was washed with dichloromethane (50mL) and the combined filtrate was concentrated to dryness to give **23-b** as a pale yellow syrup (110mg, yield 74%), which was used directly for the next step without further purification. LC-MS (ESI): m/z =454 [M + H]⁺.

### Synthesis of compound 23-a

Compound **23-b** (110mg, 0.24mmol) was dissolved in tetrahydrofuran (3mL) and water (0.5mL), and lithium hydroxide monohydrate (20mg, 0.49mmol) was added and the mixture was stirred overnight at room temperature. The reaction solution was concentrated and the residue was adjusted to pH = 3 with 2N hydrochloric acid, then filtered and the filter cake was dried in vacuo to give **23-b** as a pale yellow solid (108mg), which was used directly for the next step without further purification.

### Synthesis of compound 23

**23-b** (108mg, 0.25mmol) was dissolved in dichloromethane (5mL), and N, N-diisopropylethylamine (0.5mL), N-hydroxybenzotriazole (7mg, 0.05mmol), azetidine (28mg, 0.49mmol) and 1-ethyl-(3-dimethylaminopropyl)carbodiimide hydrochloride (139mg, 0.73mmol) were added. The reaction solution was stirred at room temperature for 16 hours, diluted with dichloromethane (50mL), washed sequentially with water (20mL), 0.1N hydrochloric acid (20mL) and water (20mL). The organic phase was separated and dried over anhydrous sodium sulfate, then filtered and the filtrate was concentrated under reduced pressure, the residue was purified by preparative HPLC (mobile phase: 10mM aqueous ammonium bicarbonate solution: acetonitrile = 35% to 45%) to give **23** as a colorless solid (25mg, yield 23%). LC-MS (ESI): m/z =465 [M + H]⁺.

¹H-NMR (400MHz, CDCl₃) δ: 8.73 (s, 1H), 7.80 (s, 1H), 7.41 (s, 1H), 7.32 (d, *J*=8Hz, 1H), 6.81 (br, 1H), 6.62-6.65 (m, 2H), 4.64 (s, 2H), 4.41 (t, *J*=8Hz, 2H), 4.14 (t, *J*=8Hz, 4H), 3.81 (s, 3H), 3.76 (s, 3H), 2.47 (s, 3H), 2.36 (m, 2H) ppm

### Example 24

### 2-(4-{[7-(4-(3-methylsulfonylpropoxy)-2-methoxyphenyl]-6-methylthieno[3,2-d]pyrimidinyl-2-yl}amino)-1H-pyrazol-1-yl]-1-ethanol (Compound 24)

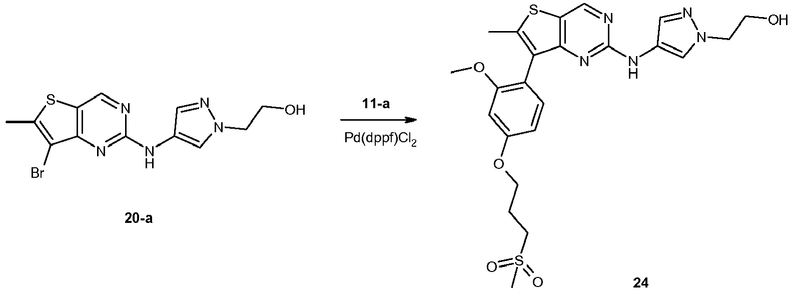

### Synthesis of compound 24

Compound **20-a** (100mg, 0.28mmol), compound **11-a** (125mg, 0.34mmol) and sodium carbonate (89mg, 0.84mmol) were suspended in dioxane (10mL) and water (1mL), and [1,1'-bis(diphenylphosphino)ferrocene]dichloropalladium • dichloromethane (41mg, 0.056mmol) was added. The reaction solution was purged with nitrogen gas for three times, thenheated to 90 °C and stirred for 16hours. After cooling to room temperature, the reaction solution was separated and concentrated under reduced pressure, the residue was purified by preparative HPLC (mobile phase: 10mM aqueous ammonium bicarbonate solution: acetonitrile = 35% to 45%) to give **19** as a pale yellow solid (41mg, yield 28%). LC-MS (ESI): m/z =518 [M + H]⁺.

¹H NMR (400 MHz, CDCl₃) δ: 8.71 (s, 1H), 7.85 (s, 1H), 7.45 (s, 1H), 7.30 (d, *J=* 8.1 Hz, 1H), 6.97 (s, 1H), 6.66 - 6.57 (m, 2H), 4.22 (t, *J* = 5.5 Hz, 2H), 4.11 (t, *J* = 4.9 Hz, 2H), 3.95 (s, 2H), 3.76 (s, 3H), 3.37 - 3.28 (m, 2H), 2.99 (s, 3H), 2.47 (s, 3H), 2.41 (dd, *J* = 13.7, 6.9 Hz, 2H) ppm

### Example 25

### N-[7-(2-methoxyphenyl)-6-methylthieno[3,2-d]pyrimidinyl-2-yl]-1-(1-methylpiperidin-4-yl)-1H-pyrazol-4-amine (Compound 25)

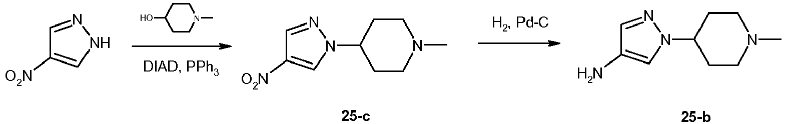

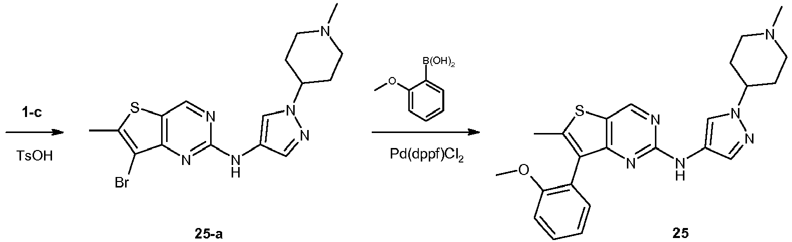

### Synthesis of compound 25-c

4-Nitropyrazole (2g, 17.69mmol), triphenylphosphine (6.95g, 26.54mmol) and N-methyl-4-hydroxypiperidine (2.4g, 21.23mmol) were dissolved in anhydrous tetrahydrofuran (50mL). The solution was cooled to 0 °C and diisopropylazodicarboxylate (5.4g, 26.54mmol) was slowly added dropwise. After the addition, the mixturewas warmed to room temperature and stirred for 16 hours. The mixture was concentrated under reduced pressure and the residue was diluted with ethyl acetate (50mL) and 3N aqueous hydrochloric acid solution (50mL) was added. The aqueous phase was adjusted to pH=9 with saturated potassium carbonate solution and then extracted with ethyl acetate (50mL x 2). The combined organic phase was dried over anhydrous sodium sulfate, filtered and the filtrate was concentrated under reduced pressure to give **25-c** as a yellow oil (2.1g, yield: 57%).

### Synthesis of compound 25-b

Palladium 10% on carbon (0.1g) was added to a solution of compound **25-c** (1.0g, 4.18mmol) in ethanol (10mL) under hydrogen gas atmosphere (latm). The mixture was reacted at 25 °C for 16 hours, then filtered through celite to remove palladium-carbon and the filtrate was concentrated under reduced pressure to give **25-b** as a reddish brown oil (420mg, yield 98%), which was used directly for the next step without further purification. LC-MS (ESI): m/z =181 [M + H]⁺.

### Synthesis of compound 25-a

Compound **1-c** (200mg, 3.09mmol) and compound **25-b** (334mg, 9.28mmol) were dissolved in *n*-butanol (2mL), and *p*-toluenesulfonic acid monohydrate(588mg, 15.48mmol) was added. The reaction solution was heated to 110°C and stirred for 16 hours. After cooling to room temperature, the reaction solution was concentrated under reduced pressure, and the residue was partitioned between dichloromethane (20mL) and saturated sodium carbonate (20mL). The organic phase was dried over anhydrous sodium sulfate, filtered and the filtrate was concentrated under reduced pressure, and the residue was purified by TLC preparative plate (dichloromethane: methanol = 10: 1) to give **25-a** as a pale yellow solid (50mg, yield 20%). LC-MS (ESI): m/z =407 [M + H]⁺.

### Synthesis of compound 25

Compound **25-a** (50mg, 0.12mmol), 2-methoxyphenylboronic acid (28mg, 0.19mmol) and sodium carbonate (40mg, 0.37mmol) were suspended in dioxane (0.5mL) and water (0.5mL), and [1,1'-bis(diphenylphosphino)ferrocene]dichloropalladium •dichloromethane (11mg, 0.02mmol) was added. The reaction solution was purged with nitrogen gas for three times, thenheated to 80°C under microwave for40 minutes. After cooling to room temperature, the reaction solution was concentrated under reduced pressure to remove solvent, and the residue was partitioned between dichloromethane (50mL) and water (50mL). The organic phase was dried over anhydrous sodium sulfate, filtered and the filtrate was concentrated under reduced pressure, and the residue was purified by silica gel TLC preparative plate (dichloromethane: methanol = 10:1) to give **25** as a pale yellow solid (21mg, yield 40%). LC-MS (ESI): m/z =435 [M + H]⁺.

¹H-NMR (400MHz, CDCl₃) δ: 8.71 (s, 1H), 7.86 (s, 1H), 7.36-7.45 (m, 3H), 7.04-7.13 (m, 3H), 3.94 (m, 1H), 3.77 (s, 3H), 2.97 (m, 2H), 2.47 (s, 3H), 2.36 (s, 3H), 2.14 (m, 2H), 2.05 (m, 2H), 1.91 (m, 2H) ppm

### Example 26

### N-[7-(2-methoxyphenyl)-6-methylthieno[3,2-d]pyrimidinyl-2-y1]-1-[3-(1-pyrrolidinyl)propyl]-1H-pyrazol-4-amine (Compound 26)

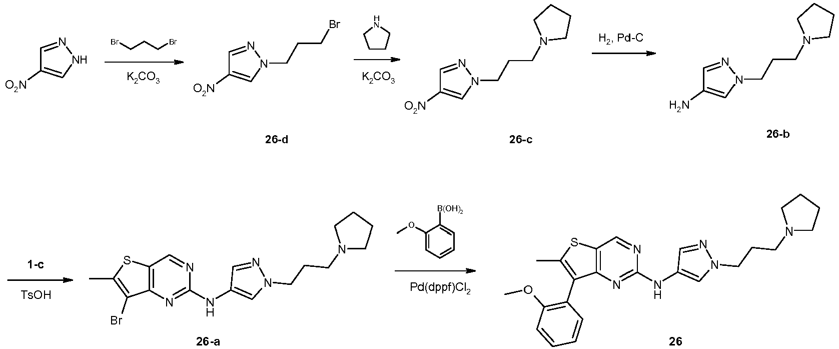

### Synthesis of compound 26-d

1,3-Dibromopropane (8.92g, 44.2mmol) and potassium carbonate (6.10g, 44.2mmol) were sequentially added to a solution of 4-nitropyrazole (2.5g, 22.12mmol) in acetonitrile (50mL). The suspension was stirred at 60°C for 8 hours. After cooling to room temperature, the mixture was added with dichloromethane(200mL), then filtered and the filtrate was concentrated under reduced pressure. The residue was washed with petroleum ether (30mL × 2)to give **26-c** (3.6 g, yield 69.6%), which was directly used for the next step without purification.

### Synthesis of compound 26-c

Pyrrolidine (910mg, 12.82mmol) and potassium carbonate (1.77g, 12.82mmol) were added successively to a solution of compound **26-d** (1.5g, 6.41mmol) in acetonitrile (25mL). The suspension was stirred at 60°C for 16 hours. After cooling to room temperature, the mixture was added with dichloromethane(50mL), then filtered and the filtrate was concentrated under reduced pressure. The residue was purified by silica gel column chromatography (dichloromethane: methanol: aqueous ammonia = 10: 1: 0 to 80: 8: 1) to give compound **26-c** (750mg, yield 52.2%). LC-MS (ESI): m/z =225 [M + H]⁺.

### Synthesis of compound 26-b

Palladium 10% on carbon (0.2g) was slowly added to a solution of compound **26-c** (750mg, 3.35mmol) in methanol (15mL) under hydrogen gas atmosphere (latm). The mixture was reacted at 25 °C for 16 hours, and dichloromethane(50mL) was added to the reaction solution. The reaction solution was filtered through celite to remove palladium-carbon, and the filtrate was concentrated under reduced pressure to give **26-b** (609mg, yield 93.8%), which was used directly for the next step without further purification.

### Synthesis of compound 26-a

Compound **1-c** (311mg, 1.18mmol) and compound **26-b** (458mg, 2.36mmol) were dissolved in *iso*-butanol (10mL), and *p*-toluenesulfonic acid monohydrate (448mg, 2.36mmol) was added. The mixture was heated to 108°C and stirred for 16 hours. After cooling to room temperature, a saturated sodium bicarbonate solution (50mL) was slowly added thereto. The aqueous phase was extracted with dichloromethane(50mL × 2). The combined organic layer was dried over anhydrous sodium sulfate, filtered and the filtrate was concentrated under reduced pressure. The residue was purified by silica gel column chromatography (dichloromethane: methanol: aqueous ammonia = 80: 8: 1) to give compound **26-a** (175mg, yield 35.2%). LC-MS (ESI): m/z =421 [M + H]⁺.

### Synthesis of compound 26

Compound **26-a** (60mg, 0.142mmol), 2-methoxyphenylboronic acid (43mg, 0.285mmol) and sodium carbonate (45mg, 0.427mmol) were suspended in dioxane (1.6mL) and water (0.4mL), and [1,1'-bis(diphenylphosphino)ferrocene]dichloropalladium·dichloromethane (10mg, 0.014mmol) was added. The reaction solution was purged with nitrogen gas for three times, and heated to 90 °C under microwave for 40 minutes. After cooling to room temperature, the reaction solution was concentrated under reduced pressure, the residue was dissolved in dichloromethane (40mL), filtered through celite and the filtrate was concentrated under reduced pressure. The residue was purified by silica gel column chromatography(dichloromethane: methanol: aqueous ammonia = 80: 8: 1) to give **26** (25mg, yield 39.2%). LC-MS (ESI): m/z =449 [M + H]⁺.

¹H-NMR (400 MHz, CDC13) δ: 8.73 (s, 1H), 7.81 (s, 1H), 7.38-7.46 (m, 3H), 7.05-7.14 (m, 2H), 4.03 (t, *J* = 6.8Hz, 2H), 3.77(s, 3H), 2.40-2.49 (m, 9H), 1.96-2.02(m, 2H), 1.76-1.79 (m, 4H) ppm

### Example 27

### N-[7-(4-fluoro-2-methoxyphenyl)-6-methylthieno[3,2-d]pyrimidinyl-2-yl]-1-[3-(1-pyrrolidinyl)propyl]-1H-pyrazol-4-amine (Compound 27)

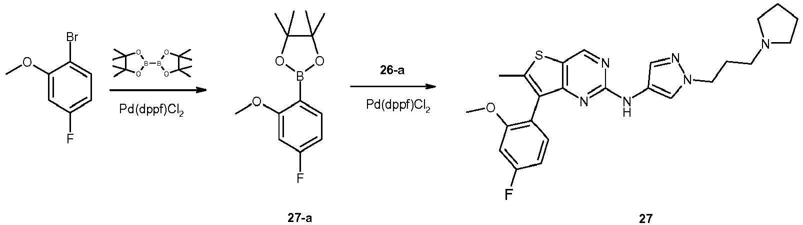

### Synthesis of compound 27-a

1-Bromo-4-fluoro-3-methoxybenzene (773mg, 3.77mmol) and bis(pinacolato)diboron(1.24mg, 4.90mmol) were dissolved in dioxane (10mL), and anhydrous potassiumacetate (1.11mg, 11.31mmol) and [1,1'-bis(diphenylphosphino)ferrocene]dichloropalladium • dichloromethane (276mg, 0.377mmol) were added. Under nitrogen gas atmosphere, the mixture was heated to 80 °C and stirred for 16 hours. After cooling to room temperature, the mixture was filtered through celite, and the filtrate was concentrated under reduced pressure. The residue was purified by silica gel TLC preparative plate (petroleum ether: ethyl acetate = 1: 1) to give compound **27-a** (600mg, yield 63%).

¹H-NMR (400 MHz, CDCl₃) δ: 7.65 (t, *J* = 8.0Hz, 1H), 6.55-6.66 (m, 2H), 3.82 (s, 3H), 1.34 (s, 12H) ppm

### Synthesis of compound 27

Compound **27-a** (84mg, 0.33mmol), compound **26-a** (70mg, 0.16mmol) and sodium carbonate (53mg, 0.5mmol) were suspended in dioxane (1.6mL) and water (0.4mL), [1,1'-bis(diphenylphosphino)ferrocene]dichloropalladium • dichloromethane (12mg, 0.016mmol) was added. The reaction solution was purged with nitrogen gas for three times, heated to 80 °C and stirred for 16 hours. After cooling to the room temperature, the reaction was concentrated under reduced pressure, the residue was dissolved in dichloromethane (40mL), then filtered through celite, and the filtrate was concentrated under reduced pressure. The residue was purified by preparative HPLC (mobile phase: 0.05% aqueous ammonium bicarbonate solution: acetonitrile = 40%-70%) to give **27** (15mg, yield 19.3%). LC-MS (ESI): m/z =467 [M + H]⁺.

¹H-NMR (400 MHz, CDC13) δ: 8.73(s, 1H), 7.79 (s, 1H), 7.42 (s, 1H), 7.34-7.40(m, 1H), 7.12 (s, 1H), 6.77-6.86 (m, 2H), 4.06(t, *J* = 6.8Hz, 2H),3.76 (s, 3H), 2.43-2.51(m, 9H),1.98-2.05 (m, 2H), 1.75-1.82 (m, 4H) ppm

### Example 28

### N-methyl-N-(2-{2-[(1-methyl-1H-pyrazol-4-yl)amino]thieno[3,2-d]pyrimidinyl-7-yl}phenyl)methanesulfonamide (Compound 28)

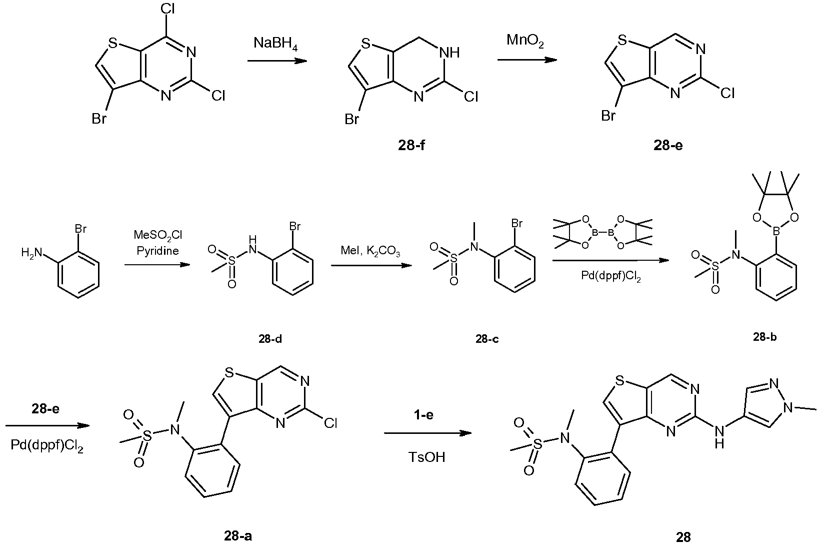

### Synthesis of compound 28-f

7-Bromo-2,4-dichlorothieno[3,2-d]pyrimidine (4.0g, 14.18mmol) was dissolved in tetrahydrofuran (60mL) and ethanol(60mL). The solution was cooled to 0°C and sodium borohydride (2.7g, 71.05mmol) was added in portions. The reaction solution was warmed to room temperature and stirred for 1 hour, then dichloromethane (500mL) and water (500mL) were added. The separated organic phase was dried over anhydrous sodium sulfate, filtered and the filtrate was concentrated under reduced pressure to give **28-f** as a yellow solid (2.5g, yield 71%) which was used without further purification. LC-MS (ESI): m/z =251 [M + H]⁺.

### Synthesis of compound 28-e

Compound **28-f** (500mg, 2.02mmol) was dissolved in dichloromethane (5mL), active manganese dioxide (270mg, 3.04mmol) was added and the mixture was stirred at room temperature for 3 hours. The reaction solution was filtered through celite and the filter cake was washed with dichloromethane (5mL × 4). The combined filtrate was concentrated under reduced pressure to give **28-e** as a white solid (430mg, yield 86%) which was used without further purification. LC-MS (ESI): m/z =249 [M + H]⁺.

### Synthesis of compound 28-d

2-Bromoaniline (10.0g, 58.5mmol) was dissolved in pyridine (50mL) and acetonitrile (50mL), and the reaction solution was cooled to 0°C and methanesulfonyl chloride (10.0g, 87.7mmol) was added dropwise. The reaction solution was warmed to room temperature and stirred for 30 minutes, then concentrated under reduced pressure. The residue was dissolved in ethyl acetate (250mL) and diluted with water (250mL). The separated organic phase was adjusted to pH=7 with 1M aqueous hydrochloric acid. The organic phase was dried over anhydrous sodium sulfate, filtered and the filtrate was concentrated under reduced pressure to give **28-d** as a yellow solid (14g, yield 96%) which was used without further purification. LC-MS (ESI): m/z =250 [M + H]⁺.

### Synthesis of compound 28-c

Compound **28-d** (5.0g, 20.08mmol) was dissolved in acetone (100mL), anhydrous potassium carbonate (4.2g, 30.12mmol) was added and then iodomethane (4.3g, 30.12mmol) was added slowly. The reaction was stirred at room temperature for 16 hours, then filtered, and the filter cake was washed with acetone (100mL), the combined filtrate was concentrated under reduced pressure. The residue was dissolved in ethyl acetate (150mL) and diluted with water (100mL). The separated organic phase was dried over anhydrous sodium sulfate, filtered and the filtrate was concentrated under reduced pressure to give **28-c** as a pale yellow solid (3.1g, yield 59%), which was used without further purification. LC-MS (ESI): m/z =264 [M + H]⁺.

### Synthesis of compound 28-b

Compound **28-c** (4.0g, 15.21mmol), bis(pinacolato)diboron (5.6g, 22.05mmol) and anhydrous potassium acetate (4.5g, 45.9mmol) were suspended in dioxane (60mL), and [1,1'-bis(diphenylphosphino)ferrocene]dichloropalladium (1.2g, 1.52mmol) was added. The mixture was purged with nitrogen gas for three times to remove oxygen contained in the system, thenheated at 80 °C for 16 hours. The reaction was cooled to room temperature, diluted with ice water (100mL) and extracted with ethyl acetate (50mL x 3). The combined organic phase was washed with water (50mL x 3) and brine (50mL) successively, dried over anhydrous sodium sulfate, filtered and the filtrate was concentrated under reduced pressure. The residue was purified by silica gel column chromatography (petroleum ether: ethyl acetate = 5: 1)to give **28-b** as apale yellow oil (3.4g, yield 72%). LC-MS (ESI): m/z =312 [M + H]⁺.

### Synthesis of compound 28-a

Compound **28-b** (1.05g, 3.38mmol), compound **28-e** (840mg, 3.38mmol), [1,1'-bis(diphenylphosphino)ferrocene]dichloropalladium (316mg, 0.38mmol) and sodium carbonate (1.06g, 9.92mmol) were dissolved in 1,4-dioxane (11mL) and water (11mL). The reaction solution was purged with nitrogen gas for three times to remove the oxygen contained in the system, then heated at 90°C for 30 minutes. The reaction was cooled to room temperature, diluted with ice water (10mL) and extracted with dichloromethane (50mL x 3). The combined organic phase was washed with water (20mL x 3) and brine (20mL) successively, dried over anhydrous sodium sulfate, filtered and the filtrate was concentrated under reduced pressure. The residue was purified by silica gel column chromatography (dichloromethane: methanol = 100: 1)to give **28-a** as a pale brown solid (610mg, yield 51%). LC-MS (ESI): m/z =354 [M + H]⁺.

### Synthesis of compound 28

Compound **28-a** (100mg, 0.28mmol) and compound **1-e** (83mg, 0.85mmol) were dissolved in *n*-butanol (2mL), and *p*-toluenesulfonic acid monohydrate(161mg, 0.85mmol) was added. The mixture was heated to 110°C and stirred for 16 hours. After cooling to room temperature, the reaction mixture was concentrated under reduced pressure, and the residue was partitioned between dichloromethane (50mL) and saturated sodium carbonate (50mL). The organic phase was dried over anhydrous sodium sulfate, filtered and the filtrate was concentrated under reduced pressure, the residue was purified by preparative HPLC (mobile phase: 0.05% aqueous trifluoroacetic acid solution: acetonitrile = 25% to 50%) to give **28** as a yellow solid (14mg, yield 12%). LC-MS (ESI): m/z =415 [M + H]⁺.

¹H-NMR (400MHz, CDCl₃) δ: 8.92 (s, 1H), 8.18 (s, 1H), 7.74 (s, 1H), 7.45-7.53 (m, 3H), 7.44 (s, 2H), 6.94 (br, 1H), 3.80 (s, 3H), 3.06 (s, 3H), 2.82 (s, 3H) ppm

### Example 29

### 1-Methyl-N-{6-methyl-7-[2-(isopropoxy)phenyl]thieno[3,2-d]pyrimidinyl-2-yl]-1H-pyrazol-4-amine (Compound 29)

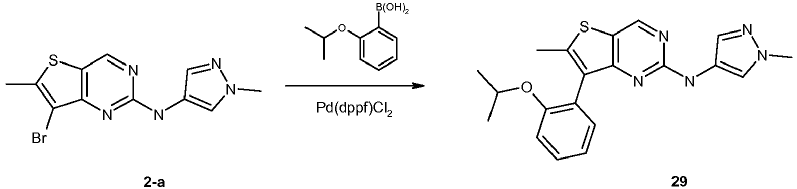

### Synthesis of compound 29

Compound **2-a** (50mg, 0.15mmol), 2-isopropoxybenzeneboronic acid (42mg, 0.23mmol), [1,1'-bis(diphenylphosphino)ferrocene]dichloropalladium (13mg, 0.12mmol) and sodium carbonate (66mg, 0.62mmol) were dissolved in 1,4-dioxane (2mL) and water (0.2mL). The reaction solution was purged with nitrogen gas for three times to remove oxygen contained in the system, and heated at 80°C under microwave for 1 hour. The reaction was cooled to room temperature, diluted with ice water (10mL) and extracted with dichloromethane (20mL x 3). The combined organic phase was washed with water (10mL x 3) and brine (10mL) successively, dried over anhydrous sodium sulfate, filtered and the filtrate was concentrated under reduced pressure. The residue was purified by preparative HPLC(mobile phase: 10mM aqueous ammonium bicarbonate solution: acetonitrile = 50% to 80%) to give **29** as a white solid (14mg, yield 24%). LC-MS (ESI): m/z =380 [M + H]⁺.

¹H-NMR (400MHz, CDCl₃) δ: 8.73 (s, 1H), 7.83 (s, 1H), 7.45-7.37 (m, 3H), 7.12-7.04 (m, 2H), 6.89 (s, 1H), 4.39-4.35 (m, 1H), 3.77 (s, 3H), 2.51 (s, 3H), 1.25 (d, *J*=6Hz, 3H), 1.06 (d, *J*=6Hz, 3H) ppm

### Example 30

### N-[7-(2H-1,3-benzodioxol-4-yl)-6-methylthieno[3,2-d]pyrimidinyl-2-yl]-1-methyl-1H-pyrazol-4-amine (Compound 30)

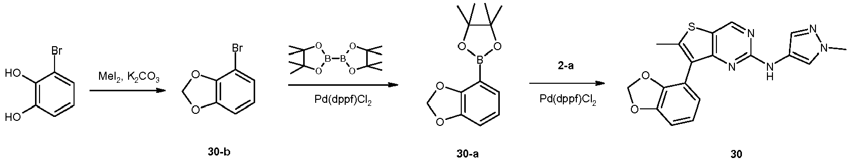

### Synthesis of compound 30-b

3-Bromoeatechol (1.88g, 10mmol) was added to a reaction solution of N,N-dimethylformamide (10mL) and potassium carbonate (2.76mL, 20mmol), and then diiodomethane (5.4g, 20mmol) was added to the mixture and stirred at 60 °C for 3 hours. The reaction was quenched with water (50mL) and extracted with ethyl acetate (100mL x 5). The reaction mixture was concentrated under reduced pressure and the residue was purified by silica gel TLC preparative plate (petroleum ether) to give **30-b** as a solid (1460mg, yield 73%).

¹H-NMR (400MHz, CDCl₃) δ: 6.97 (d, *J*=8Hz, 1H), 6.77 (d, *J*=8Hz, 1H), 6.71 (d, *J*=8Hz, 1H), 6.03(s, 2H) ppm

### Synthesis of compound 30-a

Compound **30-b** (1g, 5mmol) was added to anhydrous tetrahydrofuran (20mL) at -78 °C, and *n*-butyllithium (3mL, 7.5mmol) was then slowly added dropwise and stirred for 2 hours. Trimethylborate (1g, 10mmol) was added to the reaction mixture and stirred for 2 hours. After warming to room temperature, the reaction was quenched by the addition of IN hydrochloric acid (10mL, 10mmol) and the mixture was extracted with dichloromethane (100mL x 5). The organic phase was concentrated under reduced pressure to give compound **30-a** (530mg, yield 64 %), which was used without further purification.

### Synthesis of compound 30

Compound **30-a** (125mg, 0.75mmol), **2-a** (160mg, 0.5mmol), [1,1'-bis(diphenylphosphino)ferrocene]dichloropalladium (36mg, 0.05mmol) and 2M aqueous sodium carbonate solution (2mL, 4mmol) weredissolved in 1,4-dioxane (13mL). The reaction solution was purged with nitrogen gas for three times to remove oxygen contained in the system, and heated at 90°C for 6 hours. The reaction was cooled to room temperature, diluted with ice water (100mL) and extracted with dichloromethane (100mL x 3). The combined organic phase was washed with water (50mL x 3) and brine (50mL) successively, dried over anhydrous sodium sulfate, filtered and the filtrate was concentrated under reduced pressure. The residue was purified by silica gel TLC preparative plate (petroleum ether: ethyl acetate = 1: 1)to give **30** as a yellow solid (71mg, yield 39%). LC-MS (ESI): m/z =366 [M + H]⁺.

¹H-NMR (400MHz, DMSO-*d6*) δ: 9.48 (s, 1H), 8.98 (s, 1H), 7.83 (s, 1H), 7.43 (s, 1H), 7.05(m, 2H), 6.03 (s, 2H), 3.71 (s, 3H), 2.51 (s, 3H) ppm

### Example 31

### N-[7-(4-fluoro-2-methoxyphenyl)-6-methylthieno[3,2-d]pyrimidinyl-2-yl]-1-(piperidinyl-4-yl)-1H-pyrazol-4-amine (Compound 31)

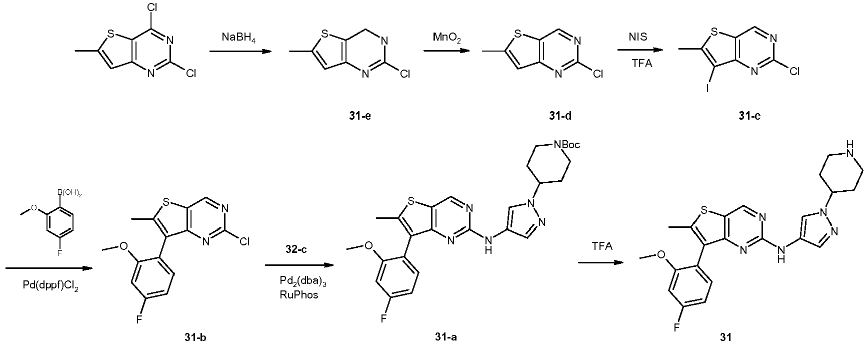

### Synthesis of compound 31-e

2,4-Dichloro-6-methylthieno[3,2-d]pyrimidine (10g, 45.6mmol) was dissolved in tetrahydrofuran (100mL) and ethanol(100mL). The reaction solution was cooled to 0°C and sodium borohydride (12.5g, 198mmol) was added in portions. The reaction solution was warmed to room temperature and stirred for further 16 hours, then diluted with water (500mL) and adjusted to pH = 7 with IN aqueous hydrochloric acid solution. The aqueous phase was extracted with ethyl acetate (150mL x 3). The organic phase was washed with water (100mL x 3) and brine (100mL) successively, dried over anhydrous sodium sulfate, filtered and the filtrate was concentrated under reduced pressure to give **31-e** as a white solid (7.5g, yield 88%) which was used without further purification. LC-MS (ESI): m/z =187 [M + H]⁺.

### Synthesis of compound 31-d

Compound **31-e** (7.5g, 40mmol) was dissolved in dichloromethane (300mL)at 0°C, and activated manganese dioxide (35g, 400mmol) was added. The reaction solution was warmed to room temperature and stirred at room temperature for 16 hours. The reaction solution was filtered through celite and the filter cake was washed with chloroform (100mL x 3). The combined filtrates were concentrated under reduced pressure to give **31-d** as a white solid (6.6g, yield 89%) which was used without further purification. LC-MS (ESI): m/z =185 [M + H]⁺.

### Synthesis of compound 31-c

Compound **31-d** (3.1g, 16.8mmol) was dissolved in trifluoroacetic acid (30mL) at 0°C and N-iodosuccinimide (5.7g, 25.3mmol) was added in portions. The reaction solution was warmed to room temperature and stirred for 1 hour. The reaction solution was quenched by the addition of water (50mL) and extracted with dichloromethane (50mL x 3). The organic phase was washed with water (50mL x 3) and brine (50mL) sequentially, dried over anhydrous sodium sulfate and filtered. The filtrate was concentrated under reduced pressure to give **31-c** as a white solid (4.9g, yield94%), which was used without further purification. LC-MS (ESI): m/z =311 [M + H]⁺.

### Synthesis of compound 31-b

Compound **31-c** (615mg, 1.98mmol), 2-methoxy-4-fluorobenzeneboronic acid (405mg, 2.38mmol) and sodium carbonate (630mg, 5.94mmol) were suspended in dioxane (5mL) and water (5mL), [1,1'-bis(diphenylphosphino)ferrocene]dichloropalladium •dichloromethane (163mg, 0.2mmol) was added. The reaction solution was purged with nitrogen gas for three times and heated to 80 °C to react for 16 hours. After cooling to room temperature, the reaction mixture was concentrated under reduced pressure. The residue was partitioned between dichloromethane (50mL) and water (50mL). The organic phase was dried over anhydrous sodium sulfate, filtered and the filtrate was concentrated under reduced pressure. The residue was purified by silica gel column chromatography (petroleum ether: dichloromethane = 1: 1) to give **31-b** as a white solid (240mg, yield 39%). LC-MS (ESI): m/z =309 [M + H]⁺.

### Synthesis of compound 31-a

Compound **31-b** (240mg, 0.78mmol) and compound **32-c** (208mg, 0.78mmol) were dissolved in N,N-dimethylformamide (3mL), potassium carbonate (323mg, 2.34mmol), 2-dicyclohexylphosphino-2',6'-diisopropoxy-1,1'-biphenyl (112mg, 0.24mmol) and tris(dibenzylideneacetone)dipalladium(0) (134mg, 0.24mmol) were added. Under nitrogen gas atmosphere, the mixture was heated to 110 °C and reacted for 16 hours. After cooling to room temperature, the reaction solution was partitioned between dichloromethane (50mL) and water (50mL). The organic phase was dried over anhydrous sodium sulfate, filtered and the filtrate was concentrated under reduced pressure. The residue was purified by silica gel TLC preparative plate (petroleum ether: ethyl acetate = 1: 1) to give **31-a** as a yellow viscous oil (190mg, yield 45%). LC-MS (ESI): m/z =539 [M + H]⁺.

### Synthesis of compound 31

**31-a** (190mg, 0.35mmol) was dissolved in dichloromethane (3mL), trifluoroacetic acid (3mL) was added and the mixture was stirred at room temperature for 3 hours. The reaction mixture was concentrated under reduced pressure and the residue was partitioned between ethyl acetate (50mL) and IN aqueous hydrochloric acid solution (50mL). The aqueous phase was adjusted to pH=10 with saturated aqueous potassium carbonate solution and solid was precipitated out. The solid was filtered out and the filter cake was washed with water (20mL x 3). The solid was dried under vacuum to give **31** as a pale yellow solid (22mg, yield 14%). LC-MS (ESI): m/z =439 [M + H]⁺.

¹H-NMR (400MHz, MeOD) δ: 8.78 (d, *J*=5Hz, 1H), 7.87 (s, 1H), 7.48 (s, 1H), 7.35 (m, 1H), 7.05 (dd, *J*=11Hz, *J*=2Hz, 1H), 6.91 (m, 1H), 4.10 (m, 1H), 3.79 (s, 3H), 3.22 (m, 2H), 2.77 (m, 2H), 2.47 (s, 3H), 2.03 (m, 2H), 1.73 (m, 2H) ppm

### Example 32

### N-[7-(2,3-dihyro-1-benzofuran-7-yl)-6-methylthieno[3,2-d]pyrimidinyl-2-yl]-1-(piperidinyl-4-yl)-1H-pyrazol-4-amine (Compound 32)

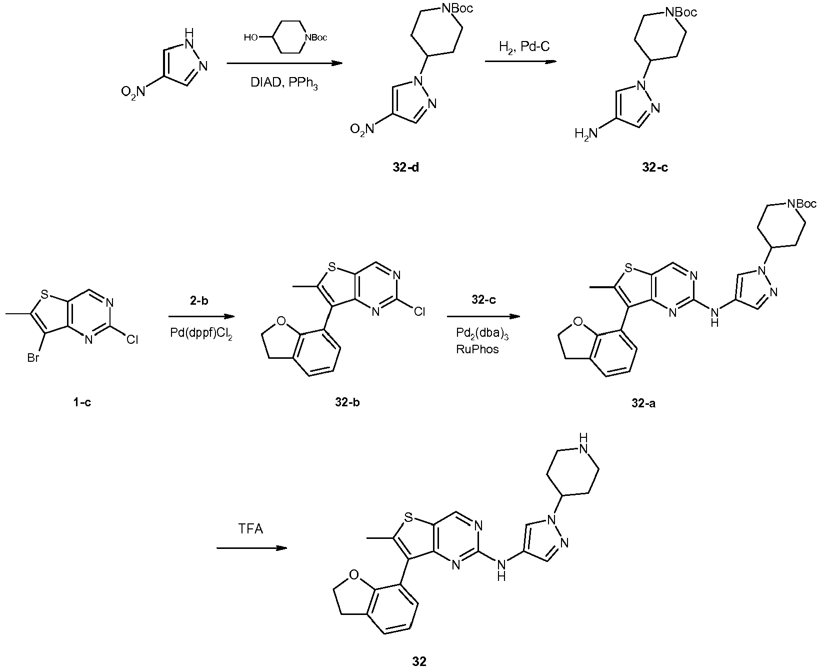

### Synthesis of compound 32-d

4-Nitropyrazole (1.14g, 10mmol), N-Boc-4-hydroxypiperidine (2.01g, 10mmol), diisopropyl azodicarboxylate (3g, 15mmol) and triphenylphosphine (3.9g, 15mmol) were added to tetrahydrofuran (50mL), and the reaction solution was stirred at room temperature for 6 hours. The reaction solution was concentrated under reduced pressure and the residue was purified by silica gel TLC preparative plate (petroleum ether: ethyl acetate = 1: 1-1: 2) to give **32-d** as a yellow solid (1460mg, yield 50%). LC-MS (ESI): m/z =241 [M+H-t-Bu]+.

### Synthesis of compound 32-c

Compound **32-d** (614mg, 2mmol) and palladium-carbon (0.1g) were added to methanol (10mL) under hydrogen gas atmosphere (latm). The reaction solution was heated to 40 °C and stirred for 3 hours. After cooling to room temperature, the reaction solution was filtered and the filtrate was concentrated under reduced pressure to give **32-c** as a purplesolid (500mg, yield 94%), which was used without further purification. LC-MS (ESI): m/z =267 [M + H]⁺.

### Synthesis of compound 32-b

Compound **1-c** (1.23g, 5mmol), compound **2-b** (1.5g, 5mmol), [1,1'-bis(diphenylphosphino)ferrocene]dichloropalladium (36mg, 0.05mmol) and sodium carbonate (1.06g,10mmol) were dissolved in dioxane (8mL) and water (2mL). The mixture was purged with nitrogen gas for three times to remove the oxygen contained in the system, thenstirred at 90°C for 8 hours. The reaction solution was cooled to room temperature, diluted with ice water (10mL) and extracted with dichloromethane (50mL x 3). The combined organic phases were washed with water (20mL x 3) and brine (20mL) successively, dried over anhydrous sodium sulfate, filtered and the filtrate was concentrated under reduced pressure. The residue was purified by silica gel TLC preparative plate (petroleum ether: ethyl acetate = 10: 1) to give **32-b** as a yellow solid (860mg, yield 57%). LC-MS (ESI): m/z =303 [M + H]⁺.

### Synthesis of compound 32-a

Compound **32-b** (1.35g, 5mmol), compound **32-c** (1.5g, 5mmol), potassium carbonate (1.38g, 10mmol), tris(dibenzylidene indenone)dipalladium (140mg, 0.1mmol) and 2-dicyclohexylphosphine-2',6'-diisopropoxy-1,1'-biphenyl (150mg, 0.2mmol) were dissolved in N,N-dimethylformamide (150mL) and the reaction solution was purged with nitrogen gas for three times to remove oxygen contained in the system and then heated at 110°C for 16 hours. The reaction was cooled to room temperature, and concentrated under reduced pressure. The residue was purified by silica gel column chromatography (dichloromethane: methanol = 40: 1) to give compound **32-a** (1g, yield 38%). LC-MS (ESI): m/z =533 [M + H]⁺.

### Synthesis of compound 32

Compound **32-a** (1.0g, 1.9mmol) was dissolved in dichloromethane (6mL). The reaction solution was cooled to 0 °C, trifluoroacetic acid (2mL) was added and the mixture was stirred at room temperature for 1 hour. The reaction solution was adjusted to pH=8-9 with saturated aqueous sodium carbonate solution and then extracted with dichloromethane (15mL × 3). The combined organic phases were dried over anhydrous sodium sulphate, filtered and the filtrate was concentrated under reduced pressure. The residue was purified by preparative HPLC (mobile phase: 10mM aqueous ammonium bicarbonate solution: acetonitrile = 38% to 46%) to give compound **32**(100mg, yield 12.3%). LC-MS (ESI): m/z =433 [M + H]⁺.

¹H-NMR (400 MHz, MeOD) δ: 8.78 (s, 1H), 7.98 (s, 1H), 7.53 (s, 1H), 7.36 (d, *J* = 8.0 Hz, 1H), 7.27 (d, *J* = 8.0 Hz, 1H), 7.05 (t, *J*=8Hz, 1H), 4.56 (t, *J*=8Hz, 2H), 4.10 (m, 1H), 3.36 (d, *J* = 12 Hz, 2H), 3.20 (d, *J* = 12 Hz, 2H), 2.75 (t, *J*=8Hz, 2H), 2.02 (d, *J* = 12 Hz, 2H), 1.78 (d, *J* = 12Hz, 2H) ppm

### Example 33

### N-[7-(2-chlorophenyl)-6-methylthieno[3,2-d]pyrimidinyl-2-yl]-1-methyl-1H-pyrazol-4-amine (Compound 33)

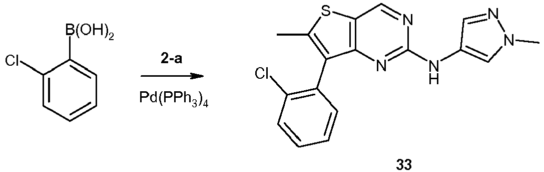

### Synthesis of compound 33

2-Chlorobenzeneboronic acid (100mg, 0.31mmol), compound **2-a** (59mg, 0.37mmol), tetrakis(triphenylphosphine)palladium (17mg, 0.016mmol) and potassium carbonate (86mg, 0.62mmol)) were dissolved in 1,4-dioxane (4mL) and water (1mL). The reaction was purged with nitrogen gas for three times to remove the oxygen contained in the system, and then stirred at 80 °C for 16 hours. The reaction solution was cooled to room temperature and concentrated under reduced pressure. The residue was added with water (10mL) and solid was precipitated out. The solid was filtered out and washed with a mixed solvent (20mL) of petroleum ether, ethyl acetate and methanol (1: 1: 1) to give **33** as a white solid (40mg, yield 37%). LC-MS (ESI): m/z =356 [M + H]⁺.

¹H NMR (400 MHz, CDCl₃) δ: 8.75 (s, 1H), 7.68 (s, 1H), 7.58 (t, *J* = 3.6 Hz, 1H), 7.42-7.37 (m, 4H), 7.22 (s, 1H), 3.74 (s, 3H), 2.51 (s, 3H) ppm

### Example 34

### 2-[4-(4-{[7-(4-fluoro-2-methoxyphenyl)-6-methylthieno[3,2-d]pyrimidinyl-2-yl]amino}-1H-pyrazol-1-yl)-piperidinyl-1-yl]-1-ethanol (Compound 34)

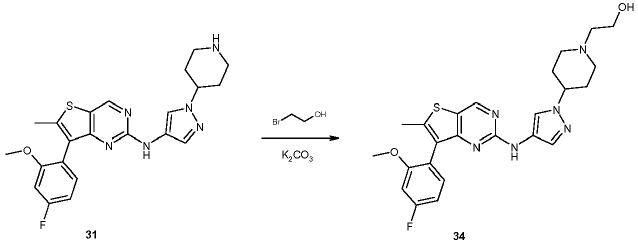

### Synthesis of compound 34

Compound **31** (300mg, 0.68mmol), bromoethanol (129mg, 1.03mmol) and potassium carbonate (282mg, 2.04mmol) were added to N,N-dimethylformamide (10mL), and the mixture was heated to 70°C and stirred for 16 hours. After cooling to room temperature, water (30mL) was added and the mixture was extracted with ethyl acetate (40mL). The organic phase was washed with brine (40mL), dried over anhydrous sodium sulfate, filtered and the filtrate was concentrated under reduced pressure. The residue was purified by silica gel column chromatography (dichloromethane: methanol = 20: 1 to 15: 1) to give **34** as a yellow solid (125mg, yield 38%). LC-MS (ESI): m/z =483 [M + H]⁺.

¹H NMR (400 MHz, DMSO) δ: 9.43 (s, 1H), 8.94 (s, 1H), 7.78 (s, 1H), 7.35 (m, 2H), 7.11 (dd, *J* = 11.5, 2.2 Hz, 1H), 6.96 (m, *J* = 7.5 Hz, 1H), 4.39 (t, 1H), 3.88 (m, 1H), 3.74 (s, 3H), 3.55 (dd, *J* = 11.8, 6.1 Hz, 2H), 2.95 (d, *J* = 11.5 Hz, 2H), 2.44 (t, *J* = 6.3 Hz, 2H), 2.40 (s, 3H), 2.11 (t, *J* = 11.3 Hz, 2H), 1.87 (m, *J* = 10.9 Hz, 2H), 1.72 (m, 2H) ppm

### Example 35

### N-[7-(2-methoxyphenyl)-6-methylthieno[3,2-d]pyrimidinyl-2-yl]-1-(piperidinyl-4-yl)-1H-pyrazol-4-amine (Compound 35)

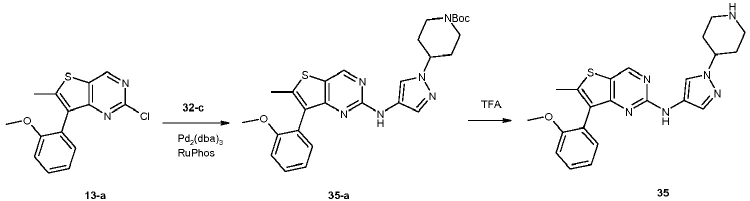

### Synthesis of compound 35-a

Compound **13-a** (200mg, 0.67mmol) and compound **32-c** (178mg, 0.67mmol) were dissolved in N,N-dimethylformamide (2mL), potassium carbonate (290mg, 2.7mmol), 2-dicyclohexylphosphino-2',6'-diisopropoxy-1,1'-biphenyl (98mg, 0.21mmol) and tris(dibenzylideneacetone)dipalladium (115mg, 0.21mmol) were added. Under nitrogengas atmosphere, the mixture was heated to 110°C and reacted for 16 hours. After cooling to room temperature, the reaction solution was partitioned between dichloromethane (50mL) and water (50mL). The organic phase was dried over anhydrous sodium sulfate, filtered and the filtrate was concentrated under reduced pressure. The residue was purified by silica gel TLC preparative plate (petroleum ether: ethyl acetate = 1: 1) to give **35-a** as a yellow compound (190mg, yield 53%). LC-MS (ESI): m/z =521 [M + H]⁺.

### Synthesis of compound 35

**35-a** (190mg, 0.36mmol) was dissolved in dichloromethane (3mL), trifluoroacetic acid (3mL) was added and the mixture was stirred at room temperature for 3 hours. The reaction mixture was concentrated under reduced pressure and the residue was partitioned between ethyl acetate (50mL) and IN aqueous hydrochloric acid solution (50mL). The aqueous phase was adjusted to pH=10 with saturated aqueous potassium carbonate solution and solid was precipitated out. The solid was filtered out and the filter cake was washed with water (20mL x 3). The solid was dried under vacuum to give **35** as a pale yellow solid (102mg, yield 67%). LC-MS (ESI): m/z =421 [M + H]⁺.

¹H-NMR (400MHz, MeOD) δ: 8.72 (s, 1H), 7.87 (s, 1H), 7.42 (m, 3H), 7.12 (m, 2H), 6.96 (s, 1H), 4.05 (m, 1H), 3.77 (s, 3H), 3.21 (m, 2H), 2.72 (m, 2H), 2.48 (s, 3H), 2.03 (m, 2H), 1.68 (m, 2H) ppm

### Example 36

### 1-[4-(4-{[7-(2,3-dihydro-1-benzofuran-7-yl)-6-methylthieno[3,2-d]pyrimidinyl-2-yl]amino}-1H-pyrazol-1-yl)piperidinyl-1-yl]-2-hydroxyacetamide (Compound 36)

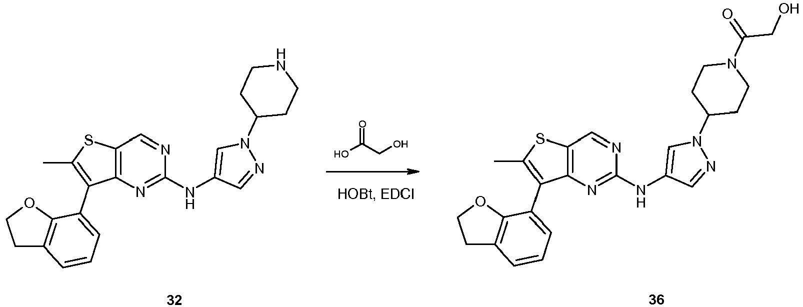

### Synthesis of compound 36

Compound **32** (86mg, 0.2mmoL) was added to dichloromethane (4mL) and diisopropylethylamine (0.4mL), followed by adding 1-hydroxybenzotriazole (54mg, 0.4mmoL). Then 1-ethyl-(3-dimethylaminopropyl)carbodiimide hydrochloride (77mg, 0.4mmoL) and glycolic acid (31mg, 0.4mmoL) were added separately and the mixture was stirred at room temperature for 2 hours. The reaction mixture was concentrated under reduced pressure. The residue was added with 2N aqueous sodium bicarbonate solution (6mL) and extracted with dichloromethane (15mL × 3). The organic phase was washed with 2N aqueous hydrochloric acid solution (15mL × 3), dried over anhydrous sodium sulfate, filtered, and the filtrate was concentrated under reduced pressure to give **36** as a yellow solid (51mg, yield 52%). LC-MS (ESI): m/z =491 [M + H]⁺.

¹H-NMR (400 MHz, CDC13) δ: 8.73 (s, 1H), 7.99 (s, 1H), 7.38 (s, 1H), 7.32 (d, *J* = 8.0 Hz, 1H), 7.27 (d, *J* = 8.0 Hz, 1H), 7.14 (s, 1H), 6.95 (t, *J*=8Hz, 1H), 4.67 (d, *J* = 12 Hz, 1H), 4.57 (t, *J*=8Hz, 2H), 4.27 (m, 3H), 3.76 (t, *J*=4Hz, 2H), 3.61(d, *J* = 12 Hz, 1H), 3.49 (s, 1H), 3.30 (t, *J* = 8 Hz, 2H), 3.15 (t, *J* = 12 Hz, 1H), 2.91 (t, *J* = 12 Hz, 1H), 2.11 (m,2H), 1.77 (m, 2H) ppm

### Example 37

### N-{7-[2-methoxy-4-(trifluoromethyl)phenyl]-6-methylthieno[3,2-d]pyrimidinyl-2-yl]-1-(piperidinyl-4-y1)-1H-pyrazol-4-amine (Compound 37)

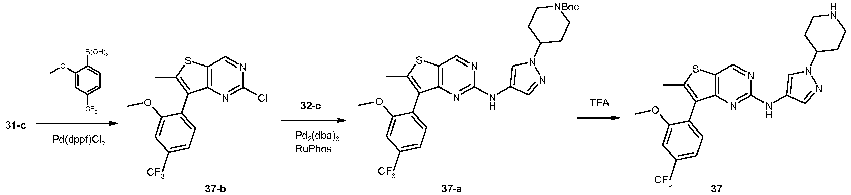

### Synthesis of compound 37-b

Compound **31-c** (640mg, 2.07mmol), 2-methoxy-4-trifluoromethylphenylboronic acid (500mg, 2.27mmol) and sodium carbonate (658mg, 6.21mmol) were suspended in dioxane (5mL) and water (5mL), and [1,1'-bis(diphenylphosphino)ferrocene]dichloropalladium • dichloromethane (171mg, 0.21mmol) was added. The reaction solution was purged with nitrogen gas for three times, heated to 80°C and reacted for 16hours. After cooling to room temperature, the reaction solution was concentrated under reduced pressure, the residue was partitioned between dichloromethane (50mL) and water (50mL). The organic phase was dried over anhydrous sodium sulfate, filtered and the filtrate was concentrated and then purified by silica gel column chromatography (petroleum ether: dichloromethane = 1: 1) to give **37-b** as a white solid (190mg, yield 26%). LC-MS (ESI): m/z =359 [M + H]⁺.

### Synthesis of compound 37-a

Compound **37-b** (200mg, 0.67mmol) and compound **32-c** (113mg, 0.42mmol) were dissolved in N,N-dimethylformamide (3mL), potassium carbonate (173mg, 1.25mmol), 2-dicyclohexylphosphino-2',6'-diisopropoxy-1,1'-biphenyl (98mg, 0.21mmol) and tris(dibenzylideneacetone)dipalladium (58mg, 0.14mmol) were added. Under nitrogen gas atmosphere, the mixture was heated to 110°C to react for 16 hours. After cooling to room temperature, the reaction solution was partitioned between ethyl acetate (100mL) and water (100mL). The organic phase was dried over anhydrous sodium sulfate, filtered and the filtrate was concentrated under reduced pressure. The residue was purified by silica gel TLC preparative plate (petroleum ether: ethyl acetate = 1: 1) to give **37-a** as a yellow compound (98mg, yield 40%). LC-MS (ESI): m/z =589 [M + H]⁺.

### Synthesis of compound 37

**37-a** (98mg, 0.17mmol) was dissolved in dichloromethane (3mL), trifluoroacetic acid (3mL) was added and the mixture was stirred at room temperature for 3 hours. The reaction mixture was concentrated under reduced pressure and the residue was partitioned between ethyl acetate (50mL) and IN aqueous hydrochloric acid solution (50mL). The aqueous phase was adjusted to pH=10 with saturated aqueous potassium carbonate solution and solid was precipitated out. The solid was filtered out and the filter cake was washed with water (20mL x 3). The solid was dried under vacuum to give **37** as a pale yellow solid (70mg, yield 86%). LC-MS (ESI): m/z =489 [M + H]⁺.

¹H-NMR (400MHz, CDCl₃) δ: 8.74 (s, 1H), 7.80 (s, 1H), 7.54 (d, *J*=14Hz, 1H), 7.38 (d, *J*=14Hz, 1H), 7.37 (s, 1H), 6.95 (s, 1H), 4.02 (m, 1H), 3.84 (s, 3H), 3.19 (m, 2H), 2.72 (m, 2H), 2.48 (s, 3H), 2.01 (m, 2H), 1.74 (m, 2H) ppm

### Example 38

### (2- {6-methyl-2-[(1-methyl-1H-pyrazol-4-yl)amino]thieno[3,2-d]pyrimidinyl-7-yl}phenyl)methanol (Compound 38)

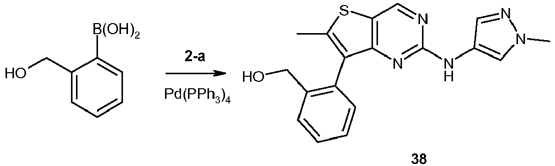

### Synthesis of compound 38

2-Hydroxymethylphenylboronic acid (213mg, 1.395mmol), compound **2-a** (300mg, 0.93mmol), tetrakis(triphenylphosphine)palladium (108mg, 0.093mmol) and potassium carbonate (257mg, 1.86mmol) were dissolved in 1,4-dioxane (8mL) and water (2mL). The reaction was purged with nitrogen gas for three times to remove the oxygen contained in the system, and then stirred at 80°C for 2 hours. The reaction solution was cooled to room temperature and concentrated under reduced pressure. The residue was added with water (20mL) and solid was precipitated out. The solid was filtered out and washed with a mixed solvent (20mL) of petroleum ether and ethyl acetate (1: 1) to give **38** as an off-white solid (325mg, yield 100%). LC-MS (ESI): m/z =352 [M + H]⁺.

¹H NMR (400 MHz, DMSO-*d₆*) δ: 9.42 (s, 1H), 8.98 (s, 1H), 7.69 (d, *J* = 8.0 Hz, 1H), 7.58 (bs, 1H), 7.51 (t, *J* = 7.6 Hz, 1H), 7.40 (t, *J* = 7.6 Hz, 1H), 7.32 (s, 1H), 7.21 (d, *J* = 7.6 Hz, 1H), 5.03 (t, *J* = 5.2 Hz, 1H), 4.39 (dd, *J* = 13.6, 4.8 Hz, 1H), 4.20 (dd, *J* = 14.0, 5.2 Hz, 1H), 3.65 (s, 3H), 2.41 (s, 3H) ppm

### Example 39

### N-[7-(2,3-dihydro-1-benzofuran-7-yl)-6-methylthieno[3,2-d]pyrimidin-2-yl]-1-(tetrahydropyran-4-yl)-1H-pyrazol-4-amine (Compound 39)

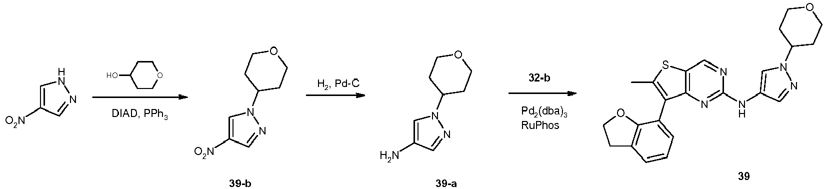

### Synthesis of compound 39-b

4-Nitropyrazole (1.14g, 10mmol), 4-hydroxytetrahydropyran (1.01g, 10mmol), diisopropyl azodicarboxylate (3g, 15mmol) and triphenylphosphine (3.9g, 15mmol) were added to tetrahydrofuran (50mL), and the reaction solution was stirred at room temperature for 6 hours. The reaction solution was concentrated under reduced pressure and the residue was purified by silica gel TLC preparative plate (petroleum ether: ethyl acetate = 1: 1 to 1: 2) to give **39-b** as a yellow solid (1460mg, yield 71%). LC-MS (ESI): m/z =199 [M + H]⁺.

### Synthesis of compound 39-a

Compound **39-b** (1.0g, 5mmol) and palladium-carbon (0.1g) were added to methanol (10mL) under hydrogen gas atmosphere (1atm). The reaction solution was heated to 40 °C and stirred for 3 hours. After cooling to room temperature, the reaction solution was filtered and the filtrate was concentrated under reduced pressure to give **39-a** as a purple solid (830mg, yield 100%), which was used without further purification. LC-MS (ESI): m/z =168 [M + H]⁺.

### Synthesis of compound 39

Compound **39-a** (135mg, 0.5mmol), compound **32-b** (150mg, 0.5mmol), potassium carbonate (138mg, 1mmol), tris(dibenzylidene indenone)dipalladium (14mg, 0.01mmol) and 2-dicyclohexylphosphine-2',6'-diisopropoxy-1,1'-biphenyl (15mg, 0.02mmol) were dissolved in N,N-dimethylformamide (15mL) and the reaction solution was purged with nitrogen gas for three times to remove oxygen contained in the system and then heated at 110°C for 6 hours. The reaction solution was cooled to room temperature, and concentrated under reduced pressure. The residue was purified by silica gel column chromatography (dichloromethane: methanol = 40: 1) to give compound **39** (31mg, yield 14%). LC-MS (ESI): m/z =533 [M + H]⁺.

¹H-NMR (400 MHz, CDC13) δ: 8.73 (s, 1H), 8.00 (s, 1H), 7.40 (s, 1H), 7.32 (d, *J* = 8.0 Hz, 1H), 7.27 (d, *J* = 8.0 Hz, 1H), 6.95 (t, *J*=8Hz, 1H), 4.58 (t, *J*=8Hz, 2H), 4.26 (m, 1H), 4.11 (d, *J* = 8 Hz, 2H), 3.53 (t, *J*=12Hz, 2H), 3.35 (t, *J*=12Hz, 2H), 2.55 (s, 3H), 1.93 (m, 4H) ppm

### Example 40

### N-[7-{2-[(dimethylamino)methyl]phenyl}-6-methylthieno[3,2-d]pyrimidinyl-2-yl]-1-methyl-1H-pyrazol-4-amine (Compound 40)

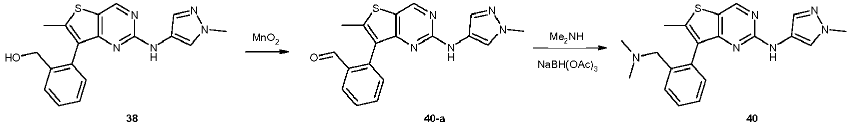

### Synthesis of compound 40-a

Compound **38** (302mg, 0.86mmol) was dissolved in dichloromethane (10mL), manganese dioxide (225mg, 2.58mmol) was added. The reaction mixture was stirred at room temperature for 16 hours, filtered through celite and the filtrate was concentrated under reduced pressure to remove solvent thereby giving **40-a** as a pale yellow solid (227mg, yield 76%). LC-MS (ESI): m/z =350 [M + H]⁺.

### Synthesis of compound 40

Compound **40-a** (107mg, 0.31mmol) and dimethylamine hydrochloride (76mg, 0.93mmol) were dissolved in dichloroethane (10mL) and a drop of acetic acid was added. The reaction was stirred at room temperature for 2 hours, then sodium triacetoxyborohydride (329mg, 1.55mmol) was added and the reaction was further stirred for 16 hours. The reaction was quenched by addition of saturated sodium bicarbonate solution (20mL) and extracted with dichloromethane (20mL × 3). The organic phase was washed with brine (50mL), dried over anhydrous sodium sulfate, filtered and the filtrate was concentrated under reduced pressure, and the residue was purified by silica gel TLC preparative plate (dichloromethane: methanol = 10: 1) to give **40** as a yellow solid (26mg, yield 23%). LC-MS (ESI): m/z =379 [M + H]⁺.

¹H NMR (400 MHz, CDCl₃) δ: 8.75 (s, 1H), 7.68 (d, *J* = 7.6 Hz, 1H), 7.58 (s, 1H), 7.46 (t, *J* = 7.6 Hz, 1H), 7.37 (t, *J* = 7.6 Hz, 1H), 7.32 (s, 2H), 7.21 (d, *J* = 7.6 Hz, 1H), 3.70 (s, 3H), 3.30 (d, *J* = 13.2 Hz, 1H), 3.14 (d, *J* = 13.2 Hz, 1H), 2.45 (s, 3H), 2.02 (s, 6H) ppm

### Example 41

### N-{7-[2-(dimethylamino)phenyl]-6-methylthieno[3,2-d]pyrimidin-2-yl}-1-methyl-1H-pyrazol-4-amine (Compound 41)

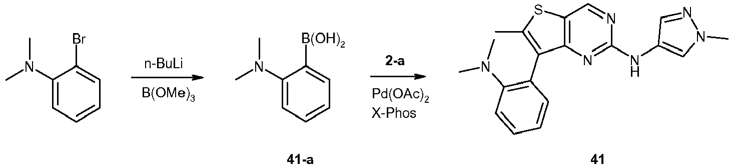

### Synthesis of compound 41-a

### N,N-dimethyl-o-bromoaniline (4g, 20mmol) was added to anhydrous tetrahydrofuran (100mL) at -78°C, and 2.5M n-butyllithium (10mL, 25mmol) was then slowly added dropwise and stirred for 2 hours. Trimethylborate (2.6g, 25mmol) was added to the reaction mixture and stirred for another 2 hours. After warming to room temperature, the reaction was quenched by the addition of 0.1N hydrochloric acid solution (200mL) and the mixture was extracted with dichloromethane (150mL x 3), then washed with water (150mL x 3).The organic phase was concentrated under reduced pressure to give compound 41-a (3.0g, yield 91 %). LC-MS (ESI): m/z =166 [M + H]⁺.

### Synthesis of compound 41

Compound **2-a** (160mg, 0.5mmol), compound **41-a** (125mg, 0.75mmol), palladium acetate (112mg, 0.5mmol) were dissolved in toluene (4mL) and water (1mL), and 2-dicyclohexylphosphine-2,4,6-triisopropylbiphenyl (24mg, 0.05mmol) and potassium phosphate (422mg, 1mmol) were added. The reaction mixture was stirred at 90°C for 8 hours under nitrogen gas atmosphere. After cooling to room temperature, the reaction mixture was concentrated under reduced pressure to remove toluene. The residue was added with ethyl acetate (150mL) and filtered through celite, the filtrate was washed with water (150mL × 3), dried over anhydrous sodium sulfate and filtered, and the filtrate was concentrated under reduced pressure. The residue was purified by silica gel column chromatography (ethyl acetate: petroleum ether = 1: 1) to give compound **41** (80mg, yield 44%). LC-MS (ESI): m/z =382 [M + H]⁺.

### Example 42

### N-[7-(4-(methoxypyridin-3-yl)-6-methylthieno[3,2-d]pyrimidin-2-yl]-1-methyl-1H-pyrazol-4-amine (Compound 42)

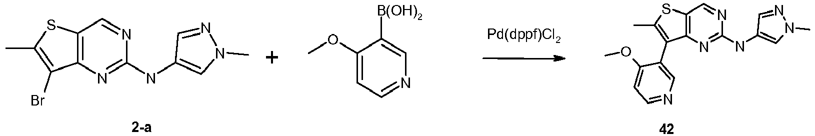

### Synthesis of compound 42

Compound **2-a** (100mg, 0.31mmol), 4-methoxypyridin-3-boronic acid (71mg, 0.46mmol) and sodium carbonate (99mg, 0.93mmol) were suspended in dioxane (0.5mL) and water (0.5mL), and [1,1'-bis(diphenylphosphino)ferrocene]dichloropalladium • dichloromethane (26mg, 0.03mmol) was added. The reaction solution was purged with nitrogen gas for three times and heated to 90°C under microwave to react for 40 minutes. After cooling to room temperature, the reaction solution was concentrated under reduced pressure, and the residue was partitioned between dichloromethane (50mL) and water (50 mL). The organic phase was dried over anhydrous sodium sulfate, filtered and the filtrate was concentrated under reduced pressure. The residue was purified by preparative HPLC (mobile phase: 10mM aqueous ammonium bicarbonate solution: acetonitrile = 30% to 40%) to give **42** as a white solid (15mg, yield 14%). LC-MS (ESI): m/z =353 [M + H]⁺.

¹H-NMR (400MHz, CDCl₃) δ: 8.75 (s, 1H), 8.59 (d, *J*=6Hz, 1H), 8.55 (s, 1H), 7.76 (s, 1H), 7.38 (s, 1H), 7.26 (s, 1H), 6.99 (d, *J*=6Hz, 1H), 3.91 (s, 3H), 3.79 (s, 3H), 2.63 (s, 3H) ppm

### Example 43

### N-[7-(2-(methoxypyridin-3-yl)-6-methylthieno[3,2-d]pyrimidin-2-yl]-1-methyl-1H-pyrazol-4-amine (Compound 43)

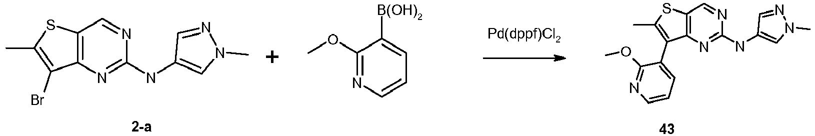

### Synthesis of compound 43

Compound **2-a** (180mg, 0.75mmol), 2-methoxypyridin-3-boronic acid (153mg, 1mmol) and sodium carbonate (106mg, 1mmol) were suspended in dioxane (8mL) and water (2mL), and [1,1'-bis(diphenylphosphino)ferrocene]dichloropalladium • dichloromethane (36mg, 0.05mmol) was added. The reaction solution was purged with nitrogen gas for three times,heated to 90 °C and stirred for 8 hours. After cooling to room temperature, the reaction solution was diluted with ice water (10mL), and extracted with dichloromethane (50mL × 3). The combined organic phase was washed with water (20mL × 3) and brine (20mL) successively, dried over anhydrous sodium sulfate, filtered and the filtrate was concentrated under reduced pressure. The residue was purified by silica gel TLC preparative plate (petroleum ether:ethyl acetate = 10: 1) to give **43** as a yellow solid (61mg, yield 34%). LC-MS (ESI): m/z =366 [M + H]⁺.

¹H-NMR (400MHz, DMSO-d6) δ: 9.43 (s, 1H), 8.97 (s, 1H), 8.31 (s, 1H), 7.81 (s, 1H), 7.66 (s, 1H), 7.37 (s, 1H), 7.22 (s, 1H), 3.84 (s, 3H), 3.70 (s, 3H), 2.50 (s, 3H) ppm

### Example 44

### 8-(2,3-Dihydro-1-benzofuran-7-yl)-N-[1-(4-piperidin)-1H-pyrazol-4-yl]quinazolin-2-amine (Compound 44)

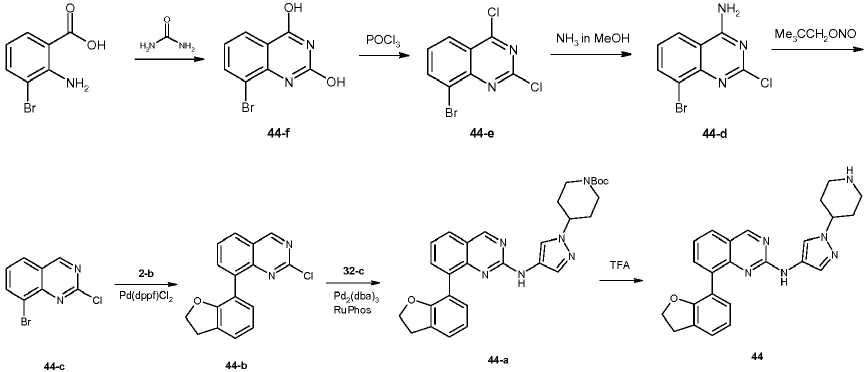

### Synthesis of compound 44-f

2-Amino-3-bromobenzoic acid (5.0g, 23.26mmol) was mixed with urea (7.0g, 116.28mmol) and the mixture was heated at 210°C for 2 hours. The reaction mixture was cooled to 90°C, then water (50mL) was added and the mixture was stirred for 30 minutes. The reaction mixture was cooled to room temperature and filtered. The filter cake was dried under vacuum to give **44-f** as a yellow solid (5.5g, yield 98%) which was used without further purification. LC-MS (ESI): m/z =241 [M + H]⁺.

### Synthesis of compound 44-e

Compound **44-f** (5.5g, 22.9mmol) was dissolved in phosphorus oxychloride (30mL), N,N-dimethylaniline (5mL) was added and the reaction solution was heated at 110°C for 18 hours. The reaction was cooled to room temperature and concentrated under reduced pressure to remove phosphorus oxychloride. The residue was concentrated to dryness and the residue was dissolved in dichloromethane (500mL) and washed with water (500mL). The separated organic phase was dried over anhydrous sodium sulfate, filtered and the filtrate was concentrated under reduced pressure. The residue was purified by silica gel column chromatography (petroleum ether: dichloromethane = 3: 1) to give **44-e** as a pale yellow solid (2.5g, yield 40%). LC-MS (ESI): m/z =277 [M + H]⁺.

### Synthesis of compound 44-d

Compound **44-e** (1.2g, 4.35mmol) was dissolved in dichloromethane (5mL), then 7M ammonia in methanol (50mL) was added and the reaction was stirred at room temperature for 16 hours. The reaction solution was concentrated under reduced pressure. The residue was added with water (50mL) and solid was precipitated out. The solid was filtered out and the filter cake was washed with water (50mL) and dried in vacuo to give **44-d** as a yellow solid (1.5g, yield 100 %), which was used for the next step without further purification.

### Synthesis of compound 44-c

Compound **44-d** (1.5g, 5.84mmol) was dissolved in tetrahydrofuran (20mL) and *tert*-amyl nitrite (2.7g, 23.36mmol) was added. The reaction mixture was heated at 70°C for 18 hours. The reaction mixture was concentrated under reduced pressure and the residue was purified by silica gel column chromatography (petroleum ether: dichloromethane = 3: 1) to give **44-c** as a light yellow solid (0.79g, yield 56%). LC-MS (ESI): m/z =243 [M + H]⁺.

### Synthesis of compound 44-b

Compound **44-c** (1.2g, 5mmol), compound **2-b** (1.25g, 5mmol), [1,1'-bis(diphenylphosphino)ferrocene]dichloropalladium (36mg, 0.05mmol) and sodium carbonate (1.06g, 10mmol) were dissolved in 1,4-dioxane (8mL) and water (2mL). The reaction mixture was purged with nitrogen gas for three times to remove the oxygen contained in the system, and then heated at 90 °C for 8 hours. The reaction was cooled to room temperature, diluted with ice water (10mL) and extracted with dichloromethane (50mL x 3). The combined organic phase was washed with water (20mL x 3) and brine (20mL), dried over anhydrous sodium sulfate, filtered, and the filtrate was concentrated under reduced pressure. The residue was purified by silica gel TLC preparative plate (petroleum ether: ethyl acetate = 10: 1) to give **44-b** as a yellow solid (790mg, yield 56%). LC-MS (ESI): m/z =283 [M + H]⁺.

### Synthesis of compound 44-a

Compound **32-c** (140mg, 0.5mmol), compound **44-b** (140mg, 0.5mmol), potassium carbonate (138mg, 1mmol), tris(dibenzylideneindenone)dipalladium (14mg, 0.01mmol) and 2-dicyclohexylphosphine-2',6'-diisopropyloxy-1,1'-biphenyl (15mg, 0.02mmol) were dissolved in N,N-dimethylformamide (15mL) and the reaction was purged with nitrogen gas for three times to remove oxygen contained in the system and then heated at 110°C for 12 hours. The reaction was cooled to room temperature and concentrated under reduced pressure. The residue was purified by silica gel column chromatography (ethyl acetate: petroleum ether = 10: 1) to give compound **44-a** (100mg, yield 38%). LC-MS (ESI): m/z =513 [M + H]⁺.

### Synthesis of compound 44

Compound **44-a** (100mg, 1.9mmol) was dissolved in dichloromethane (6mL). The reaction was cooled to 0°C, trifluoroacetic acid (2mL) was added and the reaction was stirred at room temperature for 1 hour. The reaction mixture was concentrated under reduced pressure. The residue was adjusted to pH=8-9 with saturated aqueous sodium carbonate solution, and solid was precipitated out. The solid was filtered out and dried in vacuo to give compound **44** (69mg, yield 88%). LC-MS (ESI): m/z =413 [M + H]⁺.

¹H-NMR (400 MHz, DMSO-d6) δ: 9.74 (s, 1H), 9.23 (s, 1H), 7.86 (d, *J* = 8.0 Hz, 1H), 7. 70 (d, *J* = 8.0 Hz, 1H), 7.35 (t, *J*=8Hz, 1H), 7.24 (m, 3H), 7.0 (s, 1H), 4.42 (t, *J*=8Hz, 2H), 3.91 (m, 1H), 3.28 (t, *J* = 8 Hz, 2H), 3.17 (d, *J* = 8 Hz, 2H), 2.55 (t, *J*=8Hz, 2H), 1.71 (m, 2H), 1.55 (m, 2H) ppm

### Example 45

### 8-(2-Methoxyphenyl)-N-[1-(4-piperidinyl)-1H-pyrazol-4-yl]quinazolin-2-amine (Compound 45)

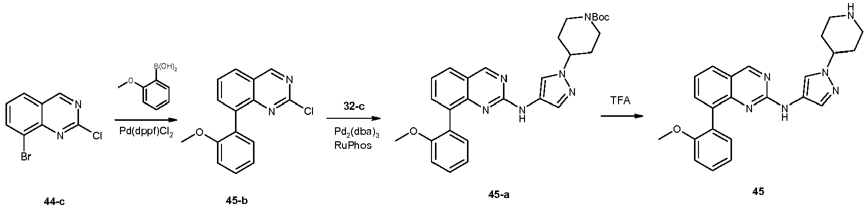

### Synthesis of compound 45-b

Compound **44-c** (600mg, 2.48mmol), pinacol 2-methoxyphenylboronate (415mg, 2.73mmol), [1,1'-bis(diphenylphosphino)ferrocene]dichloropalladium (204mg, 0.25mmol) and sodium carbonate (804mg, 7.44mmol) were dissolved in 1,4-dioxane (5mL) and water (3mL). The reaction solution was purged with nitrogen gas for three times to remove the oxygen contained in the system and then heated at 80 °C for 16 hours. The reaction was cooled to room temperature, diluted with ice water (10mL) and extracted with dichloromethane (50mL x 3). The combined organic phase was washed with water (20mL x 3) and brine (20mL) sequentially, dried over anhydrous sodium sulfate, filtered and the filtrate was concentrated under reduced pressure. The residue was purified by silica gel column chromatography (petroleum ether: dichloromethane = 3: 1) to give **45-b** as a white solid (450mg, yield 67 %). LC-MS (ESI): m/z =271 [M + H]⁺.

### Synthesis of compound 45-a

Compound **32-c** (140mg, 0.5mmol), compound **45-b** (135mg, 0.5mmol), potassium carbonate (138mg, 1mmol), tris(dibenzylidene indenone)dipalladium (14mg, 0.01mmol) and 2-dicyclohexylphosphine-2',6'-diisopropyloxy-1,1'-biphenyl (15mg, 0.02mmol) were dissolved in N,N-dimethylformamide (1mL) and the reaction solution was purged with nitrogen gas for three times to remove oxygen contained in the system and then heated at 110°C for 12 hours. The reaction was cooled to room temperature and concentrated under reduced pressure. The residue was purified by silica gel column chromatography (ethyl acetate: petroleum ether = 10: 1) to give compound **45-a** (110mg, yield 44%). LC-MS (ESI): m/z =541 [M + H]⁺.

### Synthesis of compound 45

Compound **45-a** (110mg, 1.9mmol) was dissolved in dichloromethane (6mL). The reaction was cooled to 0°C,trifluoroacetic acid (2mL) was added and the mixture was stirred at room temperature for 1 hour. The reaction mixture was concentrated under reduced pressure and the residue was adjusted to pH=8-9 with saturated aqueous sodium carbonate solution, and solid was precipitated out. The solid was filtered out and dried in vacuo to give compound **45** (60mg, yield 75%). LC-MS (ESI): m/z =441 [M + H]⁺.

¹H-NMR (400 MHz, CD₃OD) δ: 9.17 (s, 1H), 7.85 (d, *J* = 8.0 Hz, 1H), 7.83 (d, *J* = 8.0 Hz, 1H), 7.81 (s, 1H), 7.69 (t, *J*=8Hz, 1H), 7.37(m, 3H), 7.20 (m, 2H), 4.42 (m, 1H), 3.68 (s, 3H), 3.56(t, *J* = 8 Hz, 2H), 3.25(t, *J* = 8 Hz, 2H), 2.16 (m, 4H) ppm

### Example 46

### 6-fluoro-8-(2-methoxyphenyl)-N-[1-(4-piperidinyl)-1H-pyrazol-4-yl]quinazolin-2-amine (Compound 46)

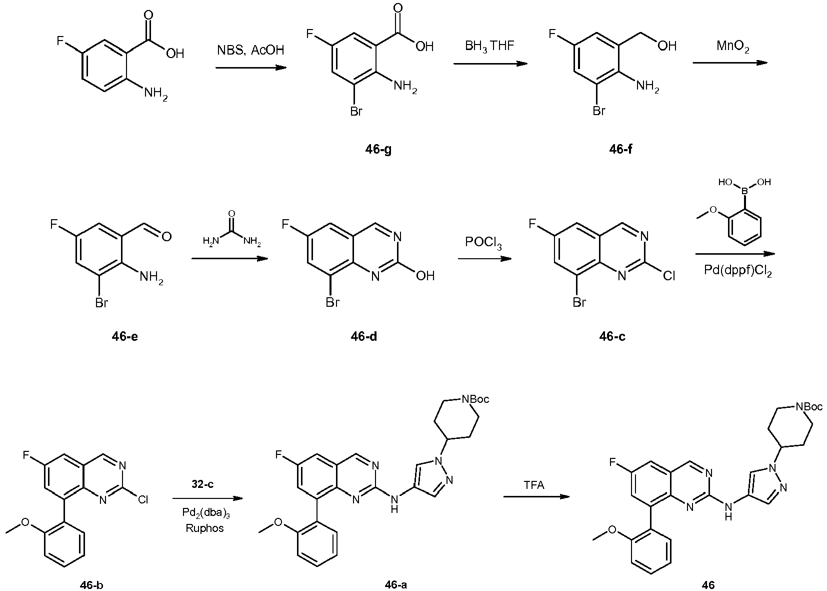

### Synthesis of compound 46-g

At 0°C, 2-amino-5-fluorobenzoic acid (20g, 129mmol) was dissolved in glacial acetic acid (250mL), and N-bromosuccinimide (25g, 140mmol) was added thereto in portions. The mixture was filtered after stirring at room temperature for 16 hours and the filter cake was washed with petroleum ether (100mL × 3). The filter cake was dried in vacuo to give **46-g** as a white solid (18.8g, yield 62%) which was used without further purification. LC-MS (ESI): m/z =234 [M + H]⁺.

### Synthesis of compound 46-f

Borane tetrahydrofuran solution (240mL, 240mmol) was added dropwise to a solution of compound **21-g** (18.8g, 80mmol) in tetrahydrofuran (160mL) at 0°C and the reaction solution was stirred at room temperature for 16 hours. Methanol (10mL) was added to quench the reaction, and the reaction solution was concentrated under reduced pressure to remove the organic solvent. The residue was dissolved in ethyl acetate (200mL). The solution was washed with water (50mL x 3) and brine (50mL) successively, dried over anhydrous sodium sulfate and filtered. The filtrate was concentrated under reduced pressure to give **46-f** as a white solid (17.2g, yield 97%). LC-MS (ESI): m/z =220 [M + H]⁺.

### Synthesis of compound 46-e

Manganese dioxide (34g, 390mmol) was added in portions to a solution of compound **21-f** (17.2g, 78mmol) in chloroform (300mL) at 0°C and the reaction solution was stirred at room temperature for 16 hours. The reaction solution was filtered and the filtrate was concentrated under reduced pressure to give **46-e** as a white solid (16.5g, yield 95%) which was used without further purification. LC-MS (ESI): m/z =218 [M + H]⁺.

### Synthesis of compound 46-d

Compound **46-f** (16.5g, 76mmol) was mixed with urea (64g, 1070mmol), the mixture was heated at 185°C for 30 minutes. The reaction mixture was cooled to room temperature and then water (200mL) was added, the mixture was stirred for 30 minutes. The reaction mixture was filtered and the filter cake was dried in vacuo to give **46-d** as a white solid (18g, yield 97%) which was used without further purification. LC-MS (ESI): m/z =243 [M + H]⁺.

### Synthesis of compound 46-c

Compound **46-d** (18g, 74mmol) was dissolved in phosphorus oxychloride (120mL, 860mmol) at 0°C and the reactionwas heated at 105°C for 16 hours. The reaction solution was cooled to room temperature, concentrated under reduced pressure to remove phosphorus oxychloride, and the residue was added with water (100mL) and stirred. The reaction mixture was filtered and the filter cake was dried under vacuum to give **46-c** as a white solid (5g, yield 26%) which was used without further purification. LC-MS (ESI): m/z =261 [M + H]⁺.

### Synthesis of compound 46-b

Compound **46-c** (1.03g, 3.93mmol), o-methoxyphenylboronic acid (600mg, 3.95mmol), [1,1'-bis(diphenylphosphino)ferrocene]dichloropalladium (150mg, 0.2mmol) and sodium carbonate (1.2g, 11.3mmol) were dissolved in 1,4-dioxane (30mL) and water (10mL). The reaction solution was purged with nitrogen gas for three times to remove the oxygen contained in the system, and then heated at 120°C for 16 hours. The reaction was cooled to room temperature, diluted with ice water (10mL) and extracted with dichloromethane (50mL x 3). The combined organic phase waswashed with water (20mL x 3) and brine (20mL) sequentially, dried over anhydrous sodium sulfate, filtered and the filtrate was concentrated under reduced pressure. The residue was purified by silica gel column chromatography (petroleum ether: ethyl acetate = 5: 1) to give **46-b** as a white solid (0.49g, yield 43%). LC-MS (ESI): m/z =599 [M + H]⁺.

### Synthesis of compound 46-a

Compound **46-b** (140mg, 0.48mmol), compound **32-c** (108mg, 0.41mmol), potassium carbonate (220mg, 1.6mmol), tris(dibenzylidene indenone)dipalladium (20mg, 0.028mmol) and 2-dicyclohexylphosphine-2',6'-diisopropoxy-1,1'-biphenyl (20mg, 0.042 mmol) were dissolved in N,N-dimethylformamide (20mL). The reaction solution was purged with nitrogen gas for three times to remove oxygen contained in the system, and then heated at 130°C for 16 hours. The reaction was cooled to room temperature, diluted with ice water (10mL) and extracted with dichloromethane (50mL x 3). The combined organic phase waswashed with water (20mL x 3) and brine (20mL), dried over anhydrous sodium sulfate, filtered and the filtrate was concentrated under reduced pressure. The residue was purified by silica gel column chromatography (petroleum ether: ethyl acetate = 5: 1) to give **46-a** as a yellow solid (140mg, yield 56%). LC-MS (ESI): m/z =517 [M + H]⁺.

### Synthesis of compound 46

Compound **46-a** (140mg, 0.27mmol) was dissolved in dichloromethane (10mL). The reaction was cooled to 0°C, trifluoroacetic acid (8mL, 70mmol) was added and the mixture was stirred at room temperature for 30 minutes. The reaction mixture was concentrated under reduced pressure. The residue was diluted with water (30mL), adjusted to pH=10 with potassium carbonate solution and the aqueous phase was extracted with dichloromethane (50mL x 3). The combined organic phase was washed with water (20mL x 3) and brine (20mL), dried over anhydrous sodium sulfate, filtered and the filtrate was concentrated under reduced pressure. The residue was purified by silica gel column chromatography (petroleum ether: ethyl acetate = 2: 1) to give **46** a yellow solid (27mg, yield 24%). LC-MS (ESI): m/z =417 [M + H]⁺.

¹H-NMR (400MHz, CD₃OD) δ: 9.11 (s, 1H), 7.64 (s, 1H), 7.56-7.48 (m, 3H), 7.42 (s, 1H), 7.18 (d, *J* = 8.4Hz, 1H), 7.17 (t, *J* = 7.4Hz, 1H), 3.98-4.00 (m, 1H), 3.70 (s, 3H), 3.36-3.27 (m, 2H), 2.89-2.82 (m, 2H), 1.97-1.94 (m, 2H), 1.86-1.82 (m, 2H) ppm

### Example 47

### N-[8-(2-methoxyphenyl)pyridino[4,3-d]pyrimidin-2-yl]-1-(4-piperidin)-1H-pyrazol-4-amine (Compound 47)

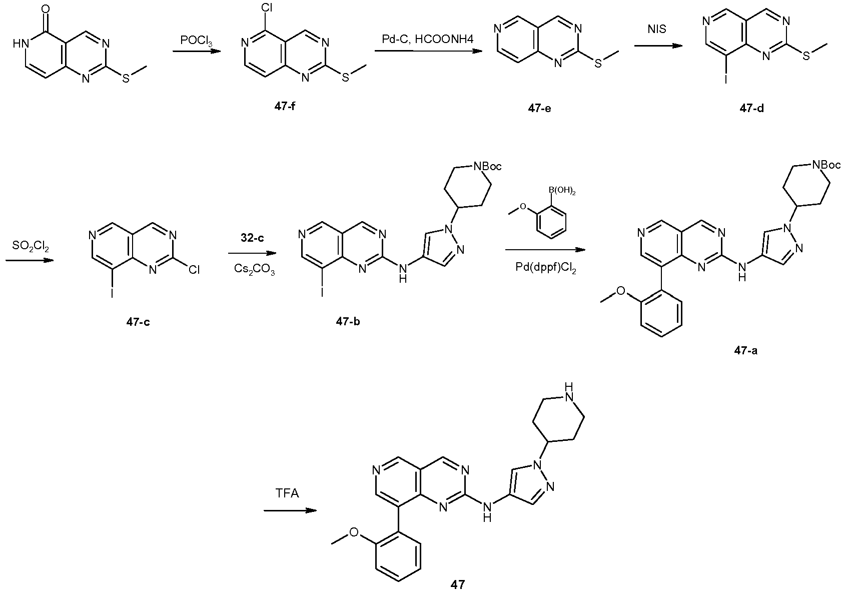

### Synthesis of compound 47-f

Phosphorus oxychloride (150mL) was added to a 500mL three necked flask, and 2-methylthio-5H-6H-pyrido[4,3-d]pyrimidin-5-one (25g, 0.13mol) was added at room temperature. The reaction solution was heated to reflux overnight and most of the phosphorus oxychloride was removed by distillation. After cooling to room temperature, the residue was poured into ice water (3L) and adjusted to pH=7 with solid potassium carbonate. The aqueous phase was extracted with dichloromethane (1L x 2) and the combined organic phase was dried over anhydrous sodium sulphate, filtered and the filtrate was concentrated under reduced pressure to give a yellow solid which was washed with a mixed solvent (150mL) of petroleum ether and ethyl acetate (5: 1) and then dried in vacuo to give compound **47-f** (17g, yield: 63%) which was used without further purification. LC-MS (ESI): m/z =212 [M + H]⁺.

### Synthesis of compound 47-e

Compound **47-f** (10g, 47.4mmol), palladium 10% on carbon (50% aq., 4.5g) and absolute ethanol (100mL) were added to a 250mL three necked flask followed by the addition of solid ammonium formate (6.1g, 94.8mmol). The mixture was heated to reflux for 16 hours. After cooling to room temperature, the reaction mixture was filtered through celite and the filter cake was washed with absolute ethanol (50mL × 2). The combined filtrate was concentrated under reduced pressure and the residue was partitioned between dichloromethane (200mL) and water (200mL). The organic phase was dried over anhydrous sodium sulfate, filtered and the filtrate was concentrated. The residue was washed with a mixed solvent (100mL) of petroleum ether and ethyl acetate (5: 1) and the solid was dried under vacuum to give compound **47-e** (3.3g, yield 39%) which was used without further purification. LC-MS (ESI): m/z =178 [M + H]⁺.

### Synthesis of compound 47-d

Compound **47-e** (1.7g, 9.6mmol) was dissolved in N,N-dimethylformamide (10mL) and trifluoroacetic acid (1.32g, 11.52mmol) and N-iodosuccinimide (2.37g, 10.56mmol) were added, the resulting brown solution was heated to 50 °C and stirred for 16 hours. After cooling to room temperature, the reaction solution was poured into ice water (150mL), extracted with dichloromethane (250mL). The organic phase was washed with saturated sodium thiosulfate solution (100mL), dried over anhydrous sodium sulfate, filtered, and the filtrate was concentrated under reduced pressure. The residue was washed with a mixed solvent (20 mL) of petroleum ether and ethyl acetate (3: 1). The solid was dried in vacuo to give **47-d** as a yellow solid (1.3g, yield 45%), which was used without further purification. LC-MS (ESI): m/z =304 [M + H]⁺.

### Synthesis of compound 47-c

Compound **47-d** (450mg, 1.49mmol) was dissolved in a mixed solvent of acetonitrile (10mL) and dichloromethane (10mL), the reaction solution was cooled to 0°C and sulfonyl chloride (2g, 14.9mmol) was added and the mixture was stirred for further 3 hours. After warming to room temperature, the reaction solution was concentrated under reduced pressure and the residue was washed with a mixed solvent (10mL) of petroleum ether and ethyl acetate (1: 1). The solid was dried in vacuo to give **47-c** as a yellow solid (380mg, yield 78%), which was used without further purification. LC-MS (ESI): m/z =292 [M + H]⁺.

### Synthesis of compound 47-b

Compound **47-c** (380mg, 1.31mmol) and compound **32-c** (278mg, 1.04mmol) were dissolved in N,N-dimethylformamide (3mL), cesium carbonate (426mg, 1.31mmol) was added and the mixture was stirred at room temperature for 16 hours. The reaction mixture was poured into ice-water (50mL) and extracted with ethyl acetate (50mL). The organic phase was dried over anhydrous sodium sulfate, filtered and the filtrate was concentrated under reduced pressure. The residue was purified by silica gel column chromatography (petroleum ether: ethyl acetate = 1: 2) to give **47-b** as a yellow solid (150mg, yield 28%).

### Synthesis of compound 47-a

Compound **47-b** (150mg, 0.29mmol), 2-methoxybenzeneboronic acid (66mg, 0.43mmol) and sodium carbonate (92mg, 0.86mmol) were suspended in dioxane (3mL) and water (3mL), and [1,1'-bis(diphenylphosphino)ferrocene]dichloropalladium-dichloromethane (25mg, 0.03mmol) was added. The reaction solution was purged with nitrogen gas for three times, then heated to 80°C and stirred for 16 hours. After cooling to room temperature, the reaction mixture was concentrated under reduced pressure. The residue was partitioned between dichloromethane (50mL) and water (50mL). The organic phase was dried over anhydrous sodium sulfate, filtered and the filtrate was concentrated under reduced pressure. The residue was purified by silica gel TLC preparative plate (ethyl acetate) to give **47-a** as a yellow solid (60mg, yield 42%). LC-MS (ESI): m/z =502 [M + H]⁺.

### Synthesis of compound 47

Compound **47-a** (60mg, 0.12mmol) was dissolved in dichloromethane (2mL), trifluoroacetic acid (2mL) was added and the mixture was stirred at room temperature for 3 hours. The reaction mixture was concentrated under reduced pressure and the residue was partitioned between ethyl acetate (50mL) and IN aqueous hydrochloric acid solution (50mL). The aqueous phase was adjusted to pH=10 with saturated aqueous potassium carbonate solution and solid was precipitated out. The solid was filtered out, and the filter cake was washed with water (20mL × 3) and dried under vacuum to give **47** as a pale yellow solid (12mg, yield 25%). LC-MS (ESI): m/z =402 [M + H]⁺.

¹H-NMR (400MHz, MeOD) δ: 9.31 (s, 1H), 9.04 (s, 1H), 8.48 (s, 1H), 7.77 (s, 1H), 7.58 (m, 1H), 7.49 (s, 1H), 7.27 (d, *J*=8Hz, 1H), 7.20 (d, *J*=8Hz, 1H), 7.18 (m, 1H), 3.99 (m, 1H), 3.73 (s, 3H), 3.23 (m, 2H), 2.78 (m, 2H), 1.93 (m, 2H), 1.73 (m, 2H) ppm

### Example 48

### N-[7-(4-methylsulfonyl-2-methoxyphenyl)-6-methylthieno[3,2-d]pyrimidin-2-yl]-1-(4-piperidin)-1H-pyrazol-4-amine (Compound 48)

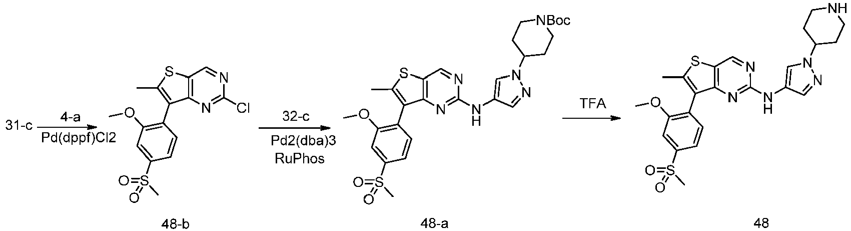

### Synthesis of compound 48-b

Compound **31-c** (598mg, 1.92mmol), compound **4-a** (600mg, 1.92mmol) and sodium carbonate (610mg, 5.76mmol) were suspended in dioxane (5mL) and water (5mL), [1,1'-bis(diphenylphosphino)ferrocene]dichloropalladium·dichloromethane (473mg, 0.58mmol) was added. The reaction solution was purged with nitrogen gas for three times, then heated to 80 °C and reacted overnight. After cooling to room temperature, the reaction mixture was concentrated under reduced pressure. The residue was partitioned betweendichloromethane (50mL) and water (50mL). The organic phase was dried over anhydrous sodium sulfate, filtered and the filtrate was concentrated under reduced pressure. The residue was purified by silica gel column chromatography (petroleum ether: dichloromethane = 1: 1) to give **48-b** as a white solid (250mg, yield 35%). LC-MS (ESI): m/z =369 [M + H]⁺.

### Synthesis of compound 48-a

Compound **48-b** (250mg, 0.68mmol) and compound **32-c** (181mg, 0.68mmol) were dissolved in N,N-dimethylformamide (3mL), and potassium carbonate (281mg, 2.37mmol), 2-dicyclohexylphosphino-2',6'-diisopropoxy-1,1'-biphenyl (58mg, 0.13mmol) and tris(dibenzylideneacetone)dipalladium (136mg, 0.24mmol) were added. The reaction solution was heated to 110°C and stirred for 16 hours under nitrogen gas atmosphere. After cooling to room temperature, the reaction mixture was partitioned between ethyl acetate (100mL) and water (100mL). The organic phase was dried over anhydrous sodium sulfate, filtered and the filtrate was concentrated under reduced pressure. The residue was purified by silica gel TLC preparative plate (petroleum ether: ethyl acetate = 1: 1) to give **48-a** as a pale yellow solid (75mg, yield 18%). LC-MS (ESI): m/z =599 [M + H]⁺.

### Synthesis of compound 48

**48-a** (70mg, 0.12mmol) was dissolved in dichloromethane (3mL), trifluoroacetic acid (3mL) was added, and the mixture was stirred at room temperature for 3 hours. The reaction solution was concentrated under reduced pressure. The residue was partitioned between dichloromethane (100mL) and saturated aqueous potassium carbonate solution (50mL). The organic phase was dried over anhydrous sodium sulfate, filtered and the filtrate was concentrated under reduced pressure. The residue was purified by silica gel TLC preparative plate (dichloromethane: methanol = 10: 1) to give **48** as a white solid (18mg, yield 31%). LC-MS (ESI): m/z =499 [M + H]⁺.

¹H-NMR (400MHz, CDCl₃) δ: 8.75 (s, 1H), 7.75 (s, 1H), 7.70 (dd, *J*=8Hz, *J*=2Hz, 1H), 7.64 (d, *J*=8Hz, 1H), 7.59 (d, *J*=2Hz, 1H), 7.38 (s, 1H), 6.89 (s, 1H), 4.12 (m, 1H), 3.94 (s, 3H), 3.25 (m, 2H), 3.18 (s, 3H), 2.78 (m, 2H), 2.47 (s, 3H), 2.04 (m, 2H), 1.63 (m, 2H) ppm

### Example 49

### 8-(4-methylsulfonyl-2-methoxyphenyl)-N-[1-(4-piperidin)-1H-pyrazol-4-yl]quinazolin-2-amine (Compound 49)

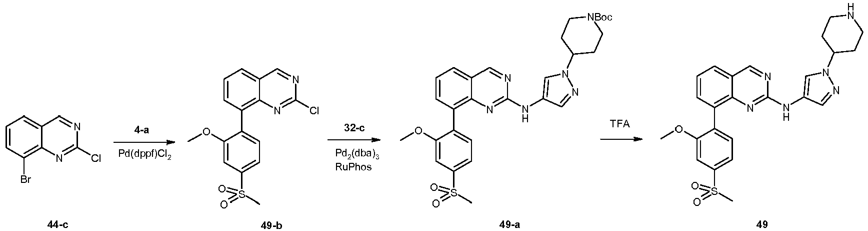

### Synthesis of compound 49-b

Compound **44-c** (930mg, 3.84mmol), compound **4-a** (1.2g, 3.84mmol) and sodium carbonate (1.2g, 11.52mmol) were suspended in dioxane (5mL) and water (5mL), [1,1'-bis(diphenylphosphino)ferrocene]dichloropalladium·dichloromethane (937mg, 1.15mmol) was added. The reaction solution was purged with nitrogen gas for three times, then heated to 80 °C and reacted overnight. After cooling to room temperature, the reaction mixture was concentrated under reduced pressure. The residue was partitioned between dichloromethane (50mL) and water (50mL). The organic phase was dried over anhydrous sodium sulfate, filtered and the filtrate was concentrated under reduced pressure. The residue was purified by silica gel column chromatography (petroleum ether: dichloromethane = 1: 2) to give **49-b** as a white solid (150mg, yield 12%). LC-MS (ESI): m/z =349 [M + H]⁺.

### Synthesis of compound 49-a

Compound **49-b** (150mg, 0.43mmol) and compound **32-c** (114mg, 0.43mmol) were dissolved in N,N-dimethylformamide (3mL), and potassium carbonate (178mg, 1.29mmol), 2-dicyclohexylphosphino-2',6'-diisopropoxy-1,1'-biphenyl (61mg, 0.13mmol) and tris(dibenzylideneacetone)dipalladium (75mg, 0.13 mmol) were added. The reaction solution was heated to 110°C and stirred for 16 hours under nitrogen gas atmosphere. After cooling to room temperature, the reaction mixture was partitioned between ethyl acetate (50mL) and water (50mL). The organic phase was dried over anhydrous sodium sulfate, filtered and the filtrate was concentrated under reduced pressure. The residue was purified by silica gel TLC preparative plate (petroleum ether: ethyl acetate = 1: 1) to give **49-a** as a pale yellow solid (130mg, yield 52%). LC-MS (ESI): m/z =579 [M + H]⁺.

### Synthesis of compound 49

**49-a** (130mg, 0.23mmol) was dissolved in dichloromethane (3mL), trifluoroacetic acid (3mL) was added and the mixture was stirred at room temperature for 3 hours. The reaction mixture was concentrated under reduced pressure and the residue was partitioned between dichloromethane (50mL) and saturated aqueous potassium carbonate solution (50mL). The aqueous phase was adjusted to pH=10 with saturated aqueous potassium carbonate solution and solid was precipitated out. The solid was washed with water (20mL × 3) and dried in vacuo to give **49** as a white solid (85mg, yield 79%). LC-MS (ESI): m/z =479 [M + H]⁺.

¹H-NMR (400MHz, CDCl₃) δ: 9.07 (s, 1H), 7.77 (d, *J*=8Hz, 1H), 7.61-7.71 (m, 4H), 7.49 (s, 1H), 7.38 (m, 2H), 7.09 (s, 1H), 4.01 (m, 1H), 3.78 (s, 3H), 3.22 (m, 2H), 3.19 (s, 3H), 2.80 (m, 2H), 1.94 (m, 2H), 1.71 (m, 2H) ppm

### Example 50

### Ethyl 4-(4-{[7-(4-fluoro-2-methoxyphenyl)-6-methylthieno[3,2-d]pyrimidin-2-yl]amino}-1H-pyrazol-1-yl)piperidin-1-yl)-1-carboxylate (Compound 50)

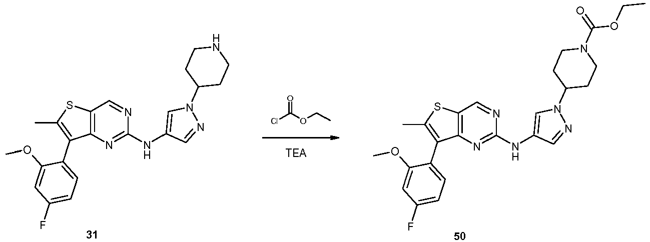

### Synthesis of compound 50

Ethyl chloroformate (163mg, 1.5mmol) was slowly added to a solution of compound **31** (438mg, 1mmol) and triethylamine (304mg, 3mmol) in dichloromethane (10mL) at 0°C, and stirred for 1 hour. After warming to room temperature, the reaction mixture was added with water (20mL) and extracted with dichloromethane (50mL). The organic phase was washed with brine (50mL), dried over anhydrous sodium sulfate, filtered and the filtrate was concentrated under reduced pressure. The residue was purified by preparative HPLC (mobile phase: 10mM aqueous ammonium bicarbonate solution: acetonitrile = 45% to 60%) to give **50** as a yellow solid (275mg, yield 54%). LC-MS (ESI): m/z =511 [M + H]⁺.

¹H-NMR (400 MHz, DMSO-d6) δ: 9.44 (s, 1H), 8.94 (s, 1H), 7.75 (s, 1H), 7.40 - 7.33 (m, 2H), 7.10 (d, *J* = 9.3 Hz, 1H), 6.94 (t, *J* = 8.4 Hz, 1H), 4.15 (s, 1H), 4.08 (dd, *J* = 14.1, 7.0 Hz, 4H), 3.73 (s, 3H), 2.95 (m, 2H), 2.40 (s, 3H), 1.93 (d, *J* = 11.8 Hz, 2H), 1.59 (m, 2H), 1.22 (t, *J* = 7.1 Hz, 3H) ppm

### Example 51

### N- [7-(4-fluoro-2-trideuteromethoxyphenyl)-3 -deutero-6-methylthieno [3,2-d]pyrimidin-2-yl]-1-(piperidin-4-yl)-1H-pyrazol-4-amine (Compound 51)

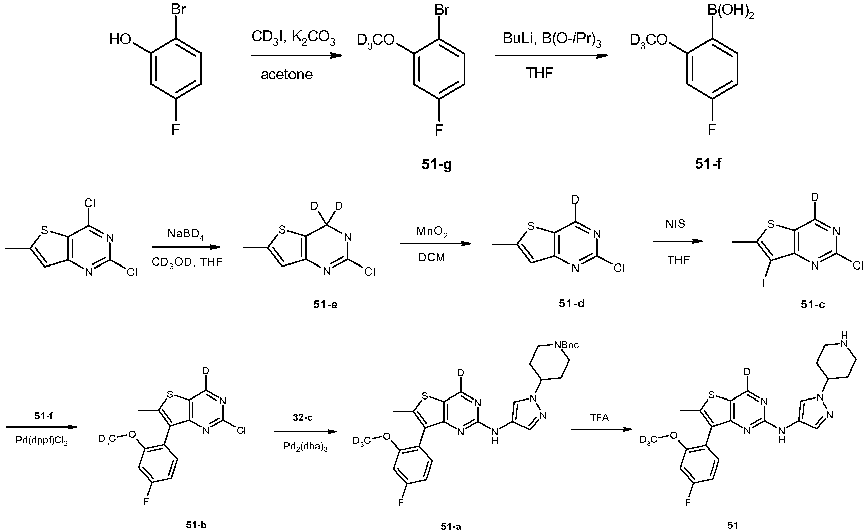

### Synthesis of compound 51-g

The compound 2-bromo-5-fluorophenol (2.56g, 13.4mmol) was dissolved in acetone (80mL), and potassium carbonate (3.70g, 26.8mmol) and deuterated iodomethane (0.83mL, 13.4mmol) were added sequentially to the solution, and the reaction mixture was stirred for 16 hours at room temperature. After completion of the reaction, a 20% aqueous sodium hydroxide solution (80mL) was added to the reaction solution and the mixture was extracted with ethyl acetate (50mL × 2). The organic layers were combined, dried over anhydrous sodium sulfate, filtered and the filtrate was concentrated under reduced pressure. The residue was purified by silica gel column chromatography (ethyl acetate) to give compound **51-g** (1.22g, yield 44%).

### Synthesis of compound 51-f

Compound **51-g** (1.22g, 5.89mmol) was dissolved in tetrahydrofuran (30mL), then the reaction solution was cooled to -78°C, a 2.5M solution of n-butyllithium in tetrahydrofuran (5.9mL, 14.72mmol) was added dropwise slowly, and the mixture was stirred at -78 °C for 1.5 hours, and triisopropylborate (4.1mL, 17.67mmol) was added slowly, then the mixture was stirred at -78 °C for another 1 hour, and then slowly warmed to room temperature, followed by further stirring for 1.5 hours at room temperature. After the reaction was completed, the reaction solution was diluted with 3M hydrochloric acid (60mL) and extracted with ethyl acetate (80mL × 2). The organic layers were combined, dried over anhydrous sodium sulfate and filtered. The filtrate was concentrated under reduced pressure and the residue was purified by silica gel TLC preparative plate (petroleum ether: ethyl acetate = 1: 1) to give compound **51-f** (220mg, yield21.6%).

### Synthesis of compound 51-e

2,4-Dichloro-6-methylthieno[3,2-d]pyrimidine (820mg, 3.76mmol) was dissolved in tetrahydrofuran (20mL) and deuterium methanol (2mL), and the reaction solution was cooled to 0°C, deuterium sodium borohydride(632mg, 15.04mmol) was added in portions. The reaction solution was warmed to room temperature and stirred for another 16 hours. The reaction solution was diluted with saturated ammonium chloride solution (40mL) and the aqueous phase was extracted with ethyl acetate (80mL x 2). The organic layers were combined, dried over anhydrous sodium sulfate, filtered, and the filtrate was concentrated to give compound **51-e** (660mg, yield 93.4%) which was used for the next step without further purification. LC-MS (ESI): m/z =189.1 [M + H]⁺.

### Synthesis of compound 51-d

Compound **51-e** (660mg, 3.5 1mmol) was dissolved in dichloromethane (20mL) at 0°C and active manganese dioxide (3.05g, 35.1mmol) was added, and the reaction solution was allowed to warm to room temperature and further stirred for 16 hours. The reaction solution was filtered through celite and the filter cake was washed with dichloromethane (10mL × 3). The combined filtrate was concentrated under reduced pressure to give **51-d** as a white solid (635mg, yield 97.8%) which was used without further purification. LC-MS (ESI): m/z =186 [M + H]⁺.

### Synthesis of compound 51-c

Compound **51-d** (635mg, 3.43mmol) was dissolved in trifluoroacetic acid (10mL) at 0°C, and N-iodosuccinimide (927mg, 4.12mmol) was added in portions, and the reaction solution was warmed to room temperature and stirred for another 16 hours. The reaction solution was concentrated under reduced pressure, saturated aqueous sodium bicarbonate solution (50mL) was added and the mixture was stirred for 30 minutes. The mixture was filtered and the solid was washed with water (30mL) and dried to give **51-c** as a white solid (320mg, yield 30%) which was used without further purification. LC-MS (ESI): m/z =312 [M + H]⁺.

### Synthesis of compound 51-b

Compound **51-c** (235mg, 0.755mmol), compound **51-f** (220mg, 1.06mmol) and sodium carbonate (240mg, 2.265mmol) were suspended in dioxane (8mL) and water (4mL), [1,1'-bis(diphenylphosphino)ferrocene] dichloropalladium-dichloromethane (55mg, 0.076mmol) was added. The reaction solution was purged with nitrogen gas for three times and heated to 80 °C and reacted for 16 hours. After cooling to room temperature, the reaction mixture was concentrated under reduced pressure. The residue was partitioned between dichloromethane (50mL) and water (50mL). The organic phase was dried over anhydrous sodium sulfate, filtered and the filtrate was concentrated and purified by silica gel column chromatography (petroleum ether: dichloromethane = 1: 1) to give **51-b** as a yellow solid (150mg, yield 63.8%). LC-MS (ESI): m/z =313 [M + H]⁺.

### Synthesis of compound 51-a

Compound **51-b** (150mg, 0.48mmol) and compound **32-c** (128mg, 0.48mmol) were dissolved in N,N-dimethylformamide (15mL), and potassium carbonate (198mg, 1.44mmol), 2-dicyclohexylphosphino-2',6'-diisopropoxy-1,1'-biphenyl (67mg, 0.144mmol) and tris(dibenzylideneacetone)dipalladium (82mg, 0.144mmol) were added. Under nitrogen gas atmosphere, the reaction solution was heated to 110°C and reacted for 16 hours. After cooling to room temperature, the reaction solution was partitioned between dichloromethane (50mL) and water (50mL). The organic phase was dried over anhydrous sodium sulfate, filtered and the filtrate was concentrated under reduced pressure. The residue was purified by silica gel column chromatography (petroleum ether: dichloromethane: ethyl acetate = 1: 1: 2) to give **51-a** as a yellow solid (170mg, yield 65.4%). LC-MS (ESI): m/z =543 [M + H]⁺.

### Synthesis of compound 51

**51-a** (170mg, 0.314mmol) was dissolved in dichloromethane (4mL), trifluoroacetic acid (1mL) was added and the mixture was stirred at room temperature for 1 hour. Saturated sodium bicarbonate solution (30mL) was slowly added to the reaction and the aqueous phase was extracted with dichloromethane (30mL x 2). The organic phases were combined, dried over anhydrous sodium sulfate, filtered and the filtrate was concentrated under reduced pressure. The residue was purified by preparative HPLC (mobile phase: 10mM ammonium bicarbonate + 0.01% aqueous ammonia: acetonitrile = 40% to 70%) to give **51** (45mg, yield 32.5%). LC-MS (ESI): m/z =443 [M + H]⁺.

¹H-NMR (400MHz, CDCl₃) δ: 9.43 (s, 1H), 7.75 (s, 1H), 7.37-7.34 (m, 2H), 7.14 (d, *J*=11.2Hz, 1H), 6.98 (t, *J*=8.0Hz, 1H), 3.96 (s, br., 1H), 3.03 (d, *J*=12.4Hz, 2H), 2.56 (t, *J*=10.8Hz, 2H), 2.40 (s, 3H), 1.83 (d, *J*=11.2Hz, 2H), 1.54-1.49 (m, 2H) ppm

### Effect example 1:IC50 evaluation assayoncytoplasmic tyrosine kinase JAK1,2,3 inhibition

### Experiment Steps

1. The compound was dissolved in 100% DMSO, diluted into solutions with appropriate concentration gradients with water according to experimental requirement, and added to a 384-well plate.
2. JAK2 kinase (Carna, Cat. No. 08-045, Lot. No. 07CBS-1927) and JAK3 kinase (Carna, Cat. No. 08-046, Lot. No. 08CBS- 0371) were diluted to the optimum concentration with the following buffer solution: 50mM HEPES, pH 7.5, 0.0015% Brij-35, 2mM DTT. JAK1 kinase (Carna, Cat. No. 08-144, Lot. No. 11CBS-0144D) was diluted to the optimum concentration with the following buffer solution: 25mM HEPES pH 7.5, 0.01% Brij-35, 2mM DTT, 0.01M Triton. Transfer to the 384-well plate and incubated with the compound for a period of time.
3. The substrates of JAK2,3 were diluted to the optimum concentration with the following buffer: 50mM HEPES, pH 7.5, 0.0015% Brij-35, 10mM MgCl₂, adenosine triphosphate atKm. The substrate of JAK1 was diluted to the optimum concentration with the following buffer: 25mM HEPES, pH 7.5, 0.01% Brij-35, 10mM MgCl₂, 0.01M Triton, adenosine triphosphate atKm. Add to the 384-well plate to initiate the reaction and react for 1 hour at 28°C.
4. 1 Eq.of sulfuric acid solution was added to terminate the reaction, the conversion rate was read with Caliper Reader. The inhibition rate was calculated as the average of two tests.

### Experiment Results

The biological activity of some of the compounds of the present invention was determined by the above assay. The results obtained are shown in Table 1:

**Table 1.IC50 (nM) of some compounds of the present invention inhibiting JAK 1,2,3 kinase**

| Compound | JAK 1 | JAK 2 | JAK 3 |
|---|---|---|---|
| 1 | 6.8 | 1.7 | 1.7 |
| 2 | 14 | 1.8 | 0.99 |
| 4 | 16 | 1.3 | 3.1 |
| 6 | 12 | 1.4 | 1.4 |
| 7 | 13 | 1.2 | 1.8 |
| 10 | 10.6 | 1.1 | 3.6 |
| 11 | 5.1 | 0.94 | 0.73 |
| 12 | 12 | 1.8 | 1.4 |
| 15 | 13 | 1.8 | 2.1 |
| 16 | 34 | 6.1 | 6.4 |
| 17 | 16 | 2.3 | 1.3 |
| 18 | 20 | 4.3 | 4.5 |
| 21 | 1.3 | 0.31 | 0.39 |
| 22 | 43 | 1.7 | 1.5 |
| 23 | 1.1 | 0.37 | 0.64 |
| 24 | 9 | 0.94 | 1.3 |
| 25 | 3.6 | 0.6 | 1.3 |
| 26 | 12 | 1.6 | 1.6 |
| 27 | 10 | 2.3 | 1.6 |
| 28 | 75.8 | 23 | 1.3 |
| 29 | 30.4 | 0.8 | 15.9 |
| 30 | 8.5 | 1.9 | 5.1 |
| 32 | 6.6 | 0.79 | 0.42 |
| 33 | 18.5 | 8.9 | 12.9 |
| 34 | 5.6 | 0.71 | 1.1 |
| 35 | 3.8 | 0.70 | 0.84 |
| 36 | 13 | 0.82 | 0.41 |
| 39 | 18 | 1.6 | 0.71 |
| 41 | 41 | 36 | 33.7 |
| 42 | 47 | 13 | 29 |
| 43 | 7.2 | 1.7 | 1.0 |
| 44 | 54 | 7.7 | 16 |
| 45 | 2.4 | 5.8 | 1.0 |
| 47 | 58 | 9.6 | 3.0 |
| 48 | 4.2 | 0.51 | 1.3 |
| 49 | 58 | 2.1 | 9.8 |

### Effect example 2:IC50 evaluation assay of FGFR1,2,3 kinase inhibition

### Experiment Steps

1. The compound was dissolved in 100% DMSO, diluted into solutions with appropriate concentration gradients with water according to experimental requirement, and added to a 96-well plate.
2. FGFR1 kinase (Carna, Cat. No. 08-133, Lot. No. 09CBS-0989) and FGFR2 kinase (Carna, Cat. No. 08-134, Lot. No. 07CBS-2468), JAK3 kinase (Carna, Cat. No. 08-135, Lot. No. 06CBS- 3177) were diluted to the optimum concentration with the following buffer solution: 50mM HEPES, pH 7.5, 0.0015% Brij-35, 2mM DTT.Transfer to the 96-well plate and incubate with the compound at 28°C for a period of time.
3. The buffer solution (100mM HEPES, pH 7.5,0.0015% Brij-35,0.2% Coating Reagent and 50nM EDTA) was added to terminate the reaction.
4. The conversion rate was read with Caliper Reader. The inhibition rate was calculated as the average of two tests.

### Experiment Results

The biological activity of some of the compounds of the present invention was determined by the above assay. The results obtained are shown in Table 2:

**Table 2. IC50 (nM) of some compounds of the present invention inhibiting FGFR1,2,3 kinase**

| Compound | FGFR 1 | FGFR 2 | FGFR 3 |
|---|---|---|---|
| 31 | 5.1 | 10 | 16 |
| 34 | 3.8 | 8.9 | 15 |

### Effect example 3:IC50 evaluation assay of FLT3, FLT3-ITD, FLT3-D835Y kinase inhibition

### Experiment Steps

1. The compound was dissolved in 100% DMSO, diluted into solutions with appropriate concentration gradients with water according to experimental requirement, and added to a 96-well plate.
2. FLT3 kinase (Carna, Cat. No. 08-154, Lot. No. 07CBS-2350), FLT3-ITDkinase (Invitrogen, Cat. No. PV6191, Lot. No. 1753453) and FLT3-D835Y kinase (Invitrogen, Cat. No. PR7450A, Lot. No. 1629729C) were diluted to the optimum concentration with the following buffer solution: 50mM HEPES, pH 7.5, 0.0015% Brij-35,10mM MgCl₂, 2mM DTT. Transfer to the 96-well plate and incubate with the compound at 28°C for a period of time.
3. The buffer solution (100mM HEPES, pH 7.5,0.0015% Brij-35,0.2% Coating Reagent and 50nM EDTA) was added to terminate the reaction.
4. The conversion rate was read with Caliper Reader. The inhibition rate was calculated as the average of two tests.

### Experiment Results

The biological activity of some of the compounds of the present invention was determined by the above assay. The results obtained are shown in Table 3:

**Table 3. IC50 (nM) of some compounds of the present invention inhibiting FLT3 kinase**

| Compound | FLT3-WT | FLT3-ITD | FLT3-D835Y |
|---|---|---|---|
| 31 | 0.28 | 0.34 | 0.20 |
| 34 | <5 | 0.33 | 0.23 |

### Effect example 4:IC50 evaluation assay of Src family kinase inhibition

### Experiment Steps

1. The compound was dissolved in 100% DMSO, diluted into solutions with appropriate concentration gradients with water according to experimental requirement, and added to a 96-well plate.
2.c-Srckinase (Carna, Cat. No. 08-173, Lot. No. 05CBS-1367),LYNakinase (Carna, Cat. No. 08-171, Lot. No. 06CBS-3296D),FYNkinase (Carna, Cat. No. 08-068, Lot. No. 05CBS-1032),LCKkinase (Carna, Cat. No. 08-170, Lot. No. 07CBS-2482),HCKkinase (BPS, Cat. No. 40440, Lot. No. 1001),FGRkinase (Carna, Cat. No. 08-166, Lot. No. 05CBS-2781),YESkinase (Carna, Cat. No. 08-175, Lot. No. 06CBS-3247) were diluted to the optimum concentration with the following buffer solution: 50mM HEPES, pH 7.5, 0.0015% Brij-35, 10mM MgCl₂, 2mM DTT. Transfer to the 96-well plate and incubate with the compound at 28°Cfor a period of time.
3. The buffer solution (100mM HEPES, pH 7.5,0.0015% Brij-35,0.2% Coating Reagent and 50nM EDTA) was added to terminate the reaction.
4. The conversion rate was read with Caliper Reader. The inhibition rate was calculated as the average of two tests.

### Experiment Results

The biological activity of some of the compounds of the present invention was determined by the above assay. The results obtained are shown in Table 4:

**Table 4. IC50 (nM) of some compounds of the present invention inhibiting Src family kinase**

| Compound | c-Src | LYNα | FYN | LCK | HCK | FGR | YES |
|---|---|---|---|---|---|---|---|
| 31 | 6.0 | 1.3 | 3.7 | 5.5 | 30 | 14 | 5.8 |
| 34 | 6.3 | <5 | <5 | <5 | 27 | 11 | 6.8 |

## Claims

1. A fused ring pyrimidine compound of formula I, a tautomer, an enantiomer, a diastereoisomer or a pharmaceutically acceptable salt thereof; 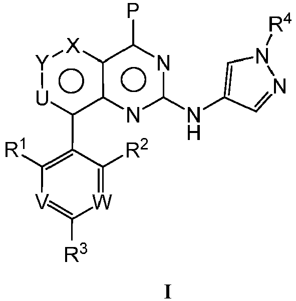
wherein, P is selected from a hydrogen or a deuterium;
X is selected from CH or S;
Y is selected from N or CR⁵;
U is selected from a chemical bond or CH;
V is selected from N or CH;
W is selected from N or CR⁶;
each of R¹, R², R³ and R⁶ is independently selected from the group consisting of a hydrogen, a deuterium, a halogen, a substituted or unsubstituted alkyl, 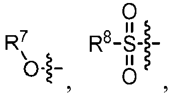 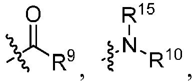 a cycloalkyl or a heterocycloalkyl; each of R⁷, R⁸, R⁹, R¹⁰ and R¹⁵ is independently selected from the group consisting of a hydrogen, a deuterium, a halogen, a hydroxyl, an amino, a substituted or unsubstituted alkyl, an alkoxy, 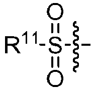 or a heterocycloalkyl; R¹¹ is a hydrogen, a deuterium or an alkyl; or R⁶, R² and the two atoms on the ring to which they are attached form a "substituted or unsubstituted 5- to 7-membered carbon heterocycle"; or, R⁶, R³ and the two atoms on the ring to which they are attached form a "substituted or unsubstituted 5- to 7-membered carbon heterocycle";the heteroatom in "substituted or unsubstituted 5- to 7-membered carbon heterocycle" is selected from the group consisting of nitrogen, oxygen and sulfur;
R⁴ is a hydrogen, a deuterium, a substituted or unsubstituted alkyl, an alkoxy, a cycloalkyl, or a substituted or unsubstituted heterocycloalkyl;
R⁵ is a hydrogen, a deuterium, a halogen, or an alkyl;
in the definitions of R¹, R², R³ and R⁶, the "substituted"in "a substituted or unsubstituted alkyl" means to be substituted with the substituents selected from the group consisting of a halogen, a hydroxyl, an amino, an alkyl, an alkoxy, 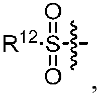 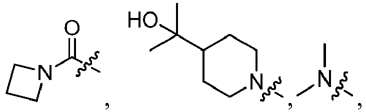 and a heterocycloalkyl, in the case when multiple substituents are present, the substituents are the same or different; R¹² is a hydrogen, a deuterium, or an alkyl;
in the definitions of R⁷, R⁸, R⁹, R¹⁰ and R¹⁵, the "substituted" in "a substituted or unsubstituted alkyl" means to be substituted with the substituents selected from the group consisting of a deuterium, a halogen, a hydroxyl, an amino, an alkyl, an alkoxy, 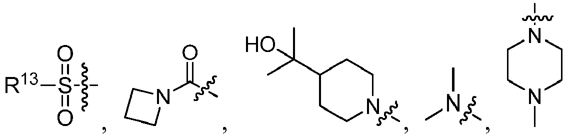 and a heterocycloalkyl, in the case when mulitiple substituents are present, the substituents are the same or different; R¹³ is a hydrogen or an alkyl;
in the definition of R⁴, the "substituted" in "a substituted or unsubstituted alkyl" and "a substituted or unsubstituted heterocycloalkyl" means to be substituted with the substituents selected from the group consisting of a hydroxyl, an alkyl, 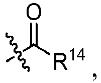 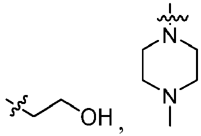 and heterocycloalkyl, in the case when multiple substituents are present, the substituents are the same or different; R¹⁴ is a hydrogen, an alkyl, a hydroxymethyl or an alkoxy;
the "substituted" in "substituted or unsubstituted 5- to 7-membered carbon heterocycle" means to be substituted with one or more than one alkyl.

2. The fused ring pyrimidine compound, the tautomer, the enantiomer, the diastereoisomer or the pharmaceutically acceptable salt thereof according to claim 1, wherein, in the case where each of R¹, R², R³ and R⁶ is independently a halogen, the halogen is fluorine or chlorine;
and/or, in the case where each of R¹, R², R³ and R⁶ is independently "a substituted or unsubstituted alkyl", the alkyl is a C₁₋₄ alkyl;
and/or, in the case where each of R¹, R², R³ and R⁶ is independently a heterocycloalkyl, the heterocycloalkyl is linked to other groups via a carbon atom or a heteroatom thereof;
and/or, in the case where each of R¹, R², R³ and R⁶ is independently a heterocycloalkyl, the heterocycloalkyl is "a heterocycloalkyl with 1-4 heteroatoms and 3-8 carbon atoms in which the heteroatom is oxygen and/or nitrogen";
and/or, in the case where each of R⁷, R⁸, R⁹, R¹⁰ and R¹⁵ is independently a halogen, the halogen is fluorine;
and/or, in the case where each of R⁷, R⁸, R⁹, R¹⁰ and R¹⁵ is independently "a substituted or unsubstituted alkyl", the alkyl is a C₁₋₁₀ alkyl;
and/or, in the case where each of R⁷, R⁸, R⁹, R¹⁰ and R¹⁵ is independently an alkoxy, the alkoxy is a C₁₋₁₀ alkoxy;
and/or, in the case where each of R⁷, R⁸, R⁹, R¹⁰ and R¹⁵ is independently a heterocycloalkyl, the heterocycloalkyl is linked to other groups via a carbon atom or a heteroatom thereof;
and/or, in the case where each of R⁷, R⁸, R⁹, R¹⁰ and R¹⁵ is independently a heterocycloalkyl, the heterocycloalkyl is "a heterocycloalkyl with 1-4 heteroatoms and 3-8 carbon atoms in which the heteroatom is oxygen and/or nitrogen";
and/or, in the case where each of R⁷, R⁸, R⁹, R¹⁰ and R¹⁵ is independently 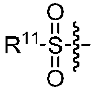 wherein R¹¹ is a C₁₋₄ alkyl;
and/or, in the case where R⁴ is "a substituted or unsubstituted alkyl", the alkyl is a C₁₋₄ alkyl;
and/or, in the case where R⁴ is an alkoxy, the alkoxy is a C₁₋₄ alkoxy;
and/or, in the case where R⁴ is "a substituted or unsubstituted heterocycloalkyl", the heterocycloalkyl is linked to other groups via a carbon atom or a heteroatom thereof;
and/or, in the case where R⁴ is "a substituted or unsubstituted heterocycloalkyl", the heterocycloalkyl is "a heterocycloalkyl with 1-4 heteroatoms and 3-8 carbon atoms in which the heteroatom is oxygen and/or nitrogen";
and/or, in the case where R⁵ is a halogen, the halogen is fluorine;
and/or, in the case where R⁵ is an alkyl, the alkyl is a C₁₋₄ alkyl;
and/or, in the case where the "5- to 7-membered carbon heterocycle" in "a substituted or unsubstituted 5- to 7-membered carbon heterocycle" is "a carbon heterocycle with 1-4 heteroatoms and 2-6 carbon atoms in which the heteroatom is oxygen and/or nitrogen";
and/or, in the case where each of R¹, R², R³ and R⁶ is independently "a substituted or unsubstituted alkyl", the substituent in "a substituted or unsubstituted alkyl" is fluorine;
and/or, in the case where each of R¹, R², R³ and R⁶ is independently "a substituted or unsubstituted alkyl", the substituent in "a substituted or unsubstituted alkyl" is a C₁₋₁₀ alkyl;
and/or, in the case where each of R¹, R², R³ and R⁶ is independently "a substituted or unsubstituted alkyl", the substituent in "a substituted or unsubstituted alkyl" is a C₁₋₁₀ alkoxy;
and/or, in the case where each of R¹, R², R³ and R⁶ is independently "a substituted or unsubstituted alkyl" and the substituent in "a substituted or unsubstituted alkyl" is a heterocycloalkyl, the heterocycloalkyl is linked to other groups via a carbon atom or a heteroatom thereof;
and/or, in the case where each of R¹, R², R³ and R⁶ is independently "a substituted or unsubstituted alkyl" and the substituent in "a substituted or unsubstituted alkyl" is "a heterocycloalkyl with 1-4 heteroatoms and 3-8 carbon atoms in which the heteroatom is oxygen and/or nitrogen";
and/or, in the case where each of R¹, R², R³ and R⁶ is independently "asubstituted or unsubstituted alkyl", the substituent in "a substituted or unsubstituted alkyl" is 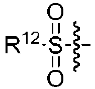 wherein R¹² is a C₁₋₄ alkyl;
and/or, in the case where each of R⁷, R⁸, R⁹, R¹⁰ and R¹⁵ is independently "a substituted or unsubstituted alkyl", the substituent in "a substituted or unsubstituted alkyl" is fluorine;
and/or, in the case where each of R⁷, R⁸, R⁹, R¹⁰ and R¹⁵ is independently "a substituted or unsubstituted alkyl", the substituent in "a substituted or unsubstituted alkyl" is a C₁₋₁₀ alkyl;
and/or, in the case where each of R⁷, R⁸, R⁹, R¹⁰ and R¹⁵ is independently "a substituted or unsubstituted alkyl", the substituent in "a substituted or unsubstituted alkyl" is a C₁₋₁₀alkoxy;
and/or, in the case where each of R⁷, R⁸, R⁹, R¹⁰ and R¹⁵ is independently "a substituted or unsubstituted alkyl", the substituent in "a substituted or unsubstituted alkyl" is a heterocycloalkyl, the heterocycloalkyl is linked to other groups via a carbon atom or a heteroatom thereof;
and/or, in the case where each of R⁷, R⁸, R⁹, R¹⁰ and R¹⁵ is independently "a substituted or unsubstituted alkyl", the substituent in "a substituted or unsubstituted alkyl" is "a heterocycloalkyl with 1-4 heteroatoms and 3-8 carbon atoms in which the heteroatom is oxygen and/or nitrogen";
and/or, in the case where each of R⁷, R⁸, R⁹, R¹⁰ and R¹⁵ is independently "a substituted or unsubstituted alkyl", the substituent in "a substituted or unsubstituted alkyl" is 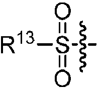 wherein R¹³ is a C₁₋₄ alkyl;
and/or, in the case where R⁴ is "a substituted or unsubstituted alkyl" or "a substituted or unsubstituted heterocycloalkyl", the substituent in "a substituted or unsubstituted alkyl" or "a substituted or unsubstituted heterocycloalkyl" is a C₁₋₄ alkyl;
and/or, in the case where R⁴ is "a substituted or unsubstituted alkyl" or "a substituted or unsubstituted heterocycloalkyl", the substituent in "a substituted or unsubstituted alkyl" or "a substituted or unsubstituted heterocycloalkyl" is a heterocycloalkyl, the heterocycloalkyl is linked to other groups via a carbon atom or a heteroatom thereof;
and/or, in the case where R⁴ is "a substituted or unsubstituted alkyl" or "a substituted or unsubstituted heterocycloalkyl", the substituent in "a substituted or unsubstituted alkyl" or "a substituted or unsubstituted heterocycloalkyl" is "a heterocycloalkyl with 1-4 heteroatoms and 3-6 carbon atoms in which the heteroatom is oxygen and/or nitrogen";
and/or, in the case where R⁴ is "a substituted or unsubstituted alkyl" or "a substituted or unsubstituted heterocycloalkyl", the substituent in "a substituted or
unsubstituted alkyl" or "a substituted or unsubstituted heterocycloalkyl" is 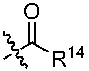 wherein R¹⁴ is a C₁₋₄ alkyl;
and/or, in the case where R⁴ is "a substituted or unsubstituted alkyl" or "a substituted or unsubstituted heterocycloalkyl", the substituent in "a substituted or
unsubstituted alkyl" or "a substituted or unsubstituted heterocycloalkyl" is 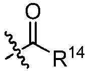 wherein R¹⁴ is a C₁₋₄alkoxy;
and/or, in the case where the substituent in "a substituent and unsubstituent 5- to 7-membered carbon heterocycle" is a C₁₋₄ alkyl.

3. The fused ring pyrimidine compound, the tautomer, the enantiomer, the diastereoisomer or the pharmaceutically acceptable salt thereof according to claim 2, wherein, in the case where each of R¹, R², R³ and R⁶ is independently "a substituted or unsubstituted alkyl", the alkyl is a methyl;
and/or, in the case where each of R¹, R², R³ and R⁶ is independently a heterocycloalkyl, the heterocycloalkyl is "a heterocycloalkyl with 1-4 heteroatoms and 3-6 carbon atoms in which the heteroatom is oxygen and/or nitrogen";
and/or, in the case where each of R⁷, R⁸, R⁹, R¹⁰ and R¹⁵ is independently "a substituted or unsubstituted alkyl", the alkyl is a C₁₋₄ alkyl;
and/or, in the case where each of R⁷, R⁸, R⁹, R¹⁰ and R¹⁵ is independently an alkoxy, the alkoxy is a C₁₋₄ alkoxy;
and/or, in the case where each of R⁷, R⁸, R⁹, R¹⁰ and R¹⁵ is independently a heterocycloalkyl, the heterocycloalkyl is "a heterocycloalkyl with 1-4 heteroatoms and 3-6 carbon atoms in which the heteroatom is oxygen and/or nitrogen";
and/or, in the case where each of R⁷, R⁸, R⁹, R¹⁰ and R¹⁵ is independently 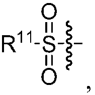 R¹¹ is a methyl; and/or, in the case where R⁴ is "a substituted or unsubstituted alkyl", the alkyl is a methyl, an ethyl, a propyl or an isopropyl;
and/or, in the case where R⁴ is "a substituted or unsubstituted heterocycloalkyl", the heterocycloalkyl is "a heterocycloalkyl with 1-4 heteroatoms and 3-6 carbon atoms in which the heteroatom is oxygen and/or nitrogen";
and/or, in the case where R⁵ is an alkyl, the alkyl is a methyl;
and/or, the "5- to 7-membered carbon heterocycle" in "substituted or unsubstituted 5- to 7-membered carbon heterocycle" is 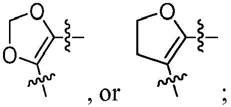 and/or, in the case where each of R¹, R², R³ and R⁶ is independently "a substituted or unsubstituted alkyl", the substituent in "a substituted or unsubstituted alkyl" is a C₁₋₄ alkyl;
and/or, in the case where each of R¹, R², R³ and R⁶ is independently "a substituted or unsubstituted alkyl", the substituent in "a substituted or unsubstituted alkyl" is a C₁₋₄ alkoxy;
and/or, in the case where each of R¹, R², R³ and R⁶ is independently "a substituted or unsubstituted alkyl", the substituent in "a substituted or unsubstituted alkyl" is "a heterocycloalkyl with 1-4 heteroatoms and 3-6 carbon atoms in which the heteroatom is oxygen and/or nitrogen";
and/or, in the case where each of R¹, R², R³ and R⁶ is independently "substituted or unsubstituted alkyl", the substituent in "substituted or unsubstituted alkyl" is 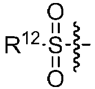 wherein R¹² is a methyl;
and/or, in the case where each of R⁷, R⁸, R⁹, R¹⁰ and R¹⁵ is independently "a substituted or unsubstituted alkyl", the substituent in "a substituted or unsubstituted alkyl" is a C₁₋₄ alkyl;
and/or, in the case where each of R⁷, R⁸, R⁹, R¹⁰ and R¹⁵ is independently "a substituted or unsubstituted alkyl", the substituent in "a substituted or unsubstituted alkyl" is a C₁₋₄ alkoxy;
and/or, in the case where each of R⁷, R⁸, R⁹, R¹⁰ and R¹⁵ is independently "a substituted or unsubstituted alkyl", the substituent in "a substituted or unsubstituted alkyl" is "a heterocycloalkyl with 1-4 heteroatoms and 3-6 carbon atoms in which the heteroatom is oxygen and/or nitrogen";
and/or, in the case where each of R¹, R², R³ and R⁶ is independently "a substituted or unsubstituted alkyl", the substituent in "a substituted or unsubstituted alkyl" is 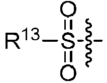 wherein R¹³ is a methyl;
and/or, in the case where R⁴ is "a substituted or unsubstituted alkyl" or "a substituted or unsubstituted heterocycloalkyl", the substituent in "a substituted or unsubstituted alkyl" or "a substituted or unsubstituted heterocycloalkyl" is a methyl;
and/or, in the case where R⁴ is "a substituted or unsubstituted alkyl" or "a substituted or unsubstituted heterocycloalkyl", the substituent in "a substituted or
unsubstituted alkyl" or "a substituted or unsubstituted heterocycloalkyl" is 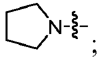 and/or, in the case where R⁴ is "a substituted or unsubstituted alkyl" or "a substituted or unsubstituted heterocycloalkyl", the substituent in "a substituted or unsubstituted alkyl" or "a substituted or unsubstituted heterocycloalkyl" is 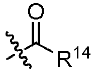 wherein R¹⁴ is a methyl;
and/or, in the case where R⁴ is "a substituted or unsubstituted alkyl" or "a substituted or unsubstituted heterocycloalkyl", the substituent in "a substituted or
unsubstituted alkyl" or "a substituted or unsubstituted heterocycloalkyl" is 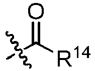 wherein R¹⁴ is a *tert*-butoxy or an ethoxy;
and/or, in the case where the alkyl substituent in "substituted or unsubstituted 5-to 7-membered carbon heterocycle" is a methyl, an ethyl or a propyl.

4. The fused ring pyrimidine compound, the tautomer, the enantiomer, the diastereoisomer or the pharmaceutically acceptable salt thereof according to claim 3, wherein, in the case where each of R¹, R², R³ and R⁶ is independently a heterocycloalkyl, the heterocycloalkyl is 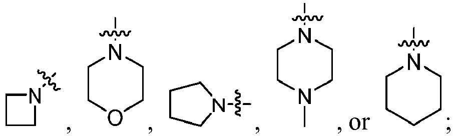 and/or, in the case where each of R⁷, R⁸, R⁹, R¹⁰ and R¹⁵ is independently "a substituted or unsubstituted alkyl", the alkyl is a methyl, a trideuteromethyl, an ethyl, a propyl or an isopropyl;
and/or, in the case where each of R⁷, R⁸, R⁹, R¹⁰ and R¹⁵ is independently an alkoxy, the alkoxy is a methoxy;
and/or, in the case where each of R⁷, R⁸, R⁹, R¹⁰ and R¹⁵ is independently a heterocycloalkyl, the heterocycloalkyl is 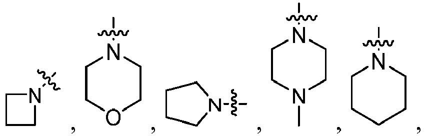 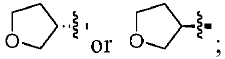 and/or, in the case where R⁴ is "a substituted or unsubstituted heterocycloalkyl",
the heterocycloalkyl in "a substituted or unsubstituted heterocycloalkyl" is 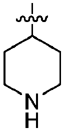 or 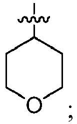 and/or, in the case where each of R¹, R², R³ and R⁶ is independently "a substituted or unsubstituted alkyl", the substituent in "a substituted or unsubstituted alkyl" is a methyl;
and/or, in the case where each of R¹, R², R³ and R⁶ is independently "a substituted or unsubstituted alkyl", the substituent in "a substituted or unsubstituted alkyl" is a methoxy;
and/or, in the case where each of R¹, R², R³ and R⁶ is independently "a substituted or unsubstituted alkyl", the substituent in "a substituted or unsubstituted alkyl" is 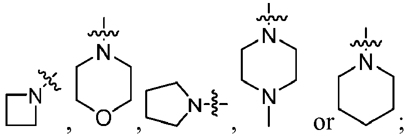 and/or, in the case where each of R⁷, R⁸, R⁹, R¹⁰ and R¹⁵ is independently "a substituted or unsubstituted alkyl", the substituent in "a substituted or unsubstituted alkyl" is a methyl;
and/or, in the case where each of R⁷, R⁸, R⁹, R¹⁰ and R¹⁵ is independently "a substituted or unsubstituted alkyl", the substituent in "a substituted or unsubstituted alkyl" is a methoxy;
and/or, in the case where each of R⁷, R⁸, R⁹, R¹⁰ and R¹⁵ is independently "a substituted or unsubstituted alkyl", the substituent in "a substituted or unsubstituted alkyl" is

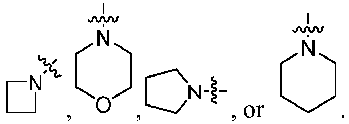

5. The fused ring pyrimidine compound, the tautomer, the enantiomer, the diastereoisomer or the pharmaceutically acceptable salt thereof according to any one of claims 1-4, wherein the compound **I** is shown as formula **I-1** or **I-2**,

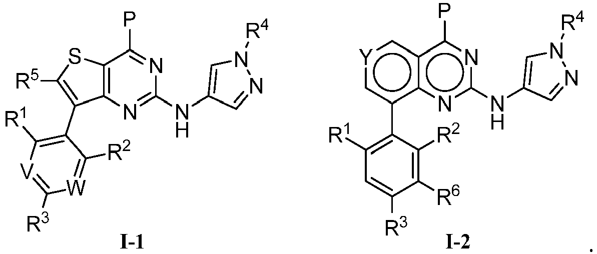

6. The fused ring pyrimidine compound, the tautomer, the enantiomer, the diastereoisomer or the pharmaceutically acceptable salt thereof according to claim 5, wherein, the compound **I-**1 is shown as formula **I-1-1** or **I-1-2**, 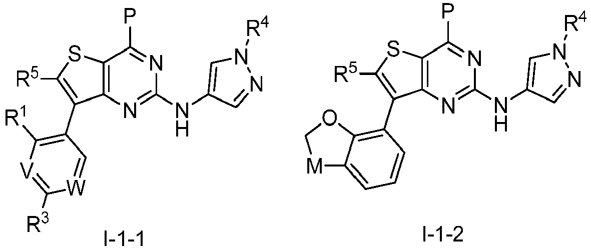 wherein, M is CH₂ or O;
the compound 1-2 is shown as formula 1-2-1 or 1-2-2,

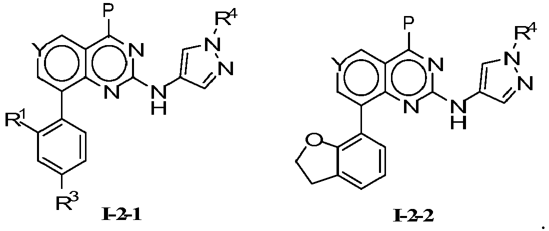

7. The fused ring pyrimidine compound, the tautomer, the enantiomer, the diastereoisomer or the pharmaceutically acceptable salt thereof according to any one of claims 1-4, wherein, in compound **I,** Y is CR⁵;
and/or, in compound **I,** R⁵ is a hydrogen or an alkyl;
and/or, in compound **I,** W is CR⁶;
and/or, in compound **I,** R⁶ is a hydrogen;
and/or, in compound **I,** R⁶ and R² together with two atoms on the ring to which they are attached form "a substituted or unsubstituted 5- to 7-membered carbon heterocycle";
and/or, in compound **I,** each of R¹ and R² is independently a hydrogen or 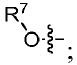 and/or, in compound **I,** R¹ or R² is a hydrogen;
and/or, in compound **I,** R³ is a hydrogen, 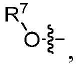 a halogen, or 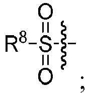 and/or, in compound **I,** R⁴ is "a substituted or unsubstituted alkyl", or "a substituted or unsubstituted heterocycloalkyl".

8. The fused ring pyrimidine compound, the tautomer, the enantiomer, the diastereoisomer or the pharmaceutically acceptable salt thereof according to claim 1, which is selected from the group consisting of

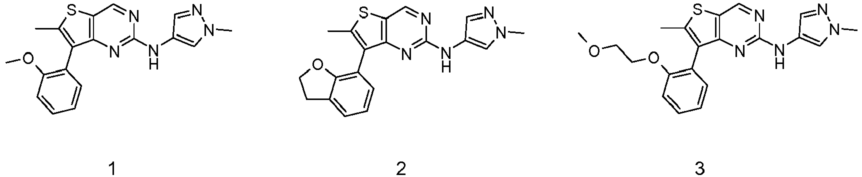

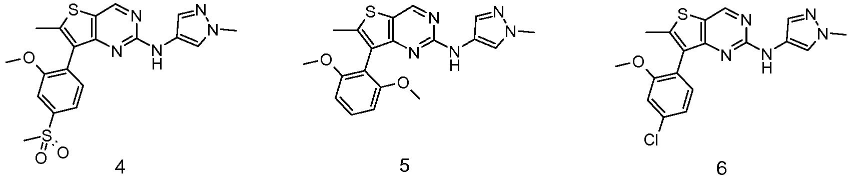

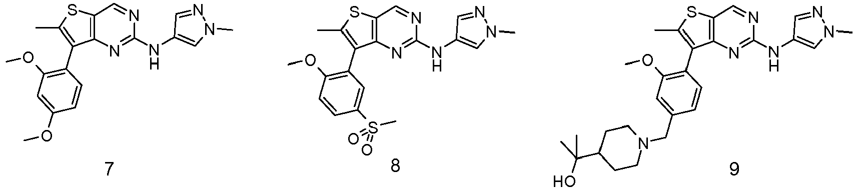

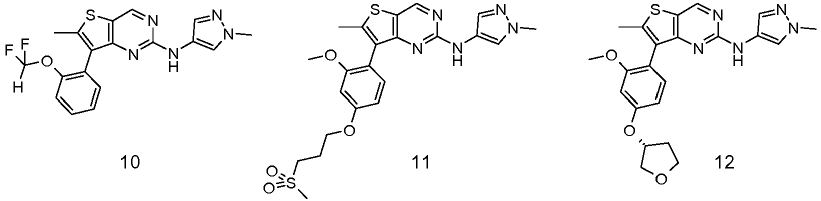

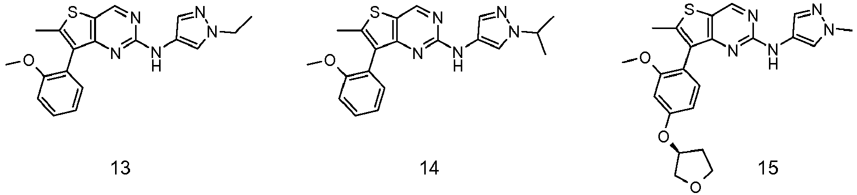

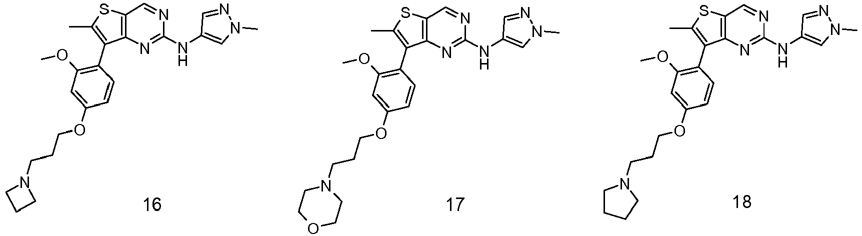

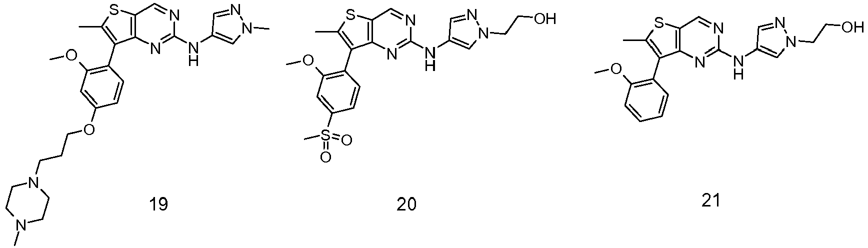

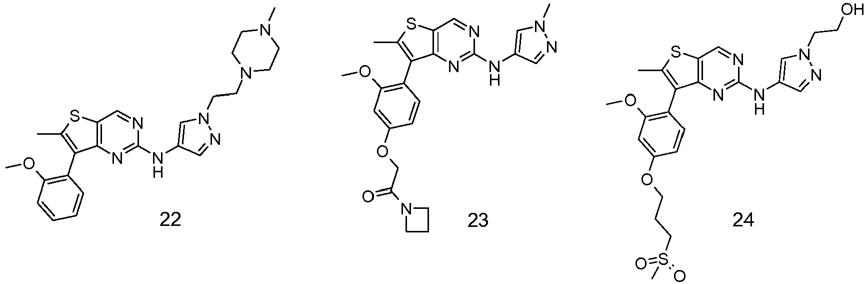

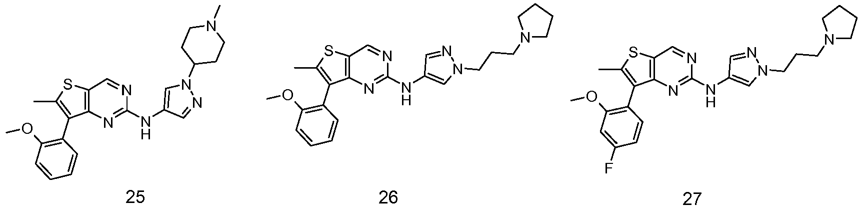

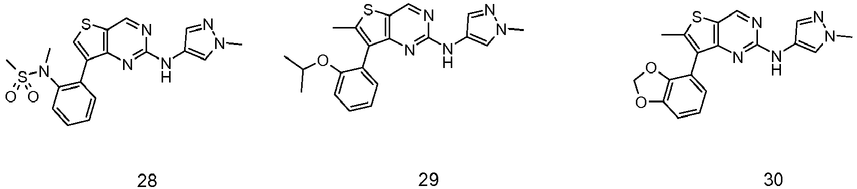

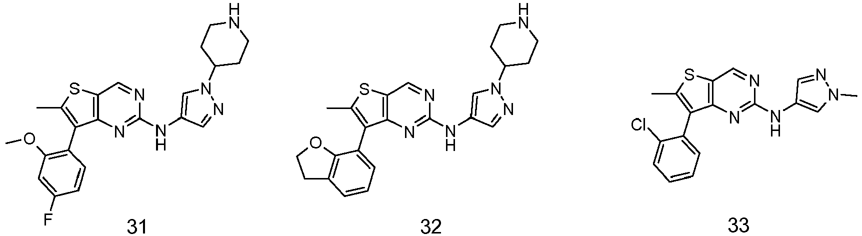

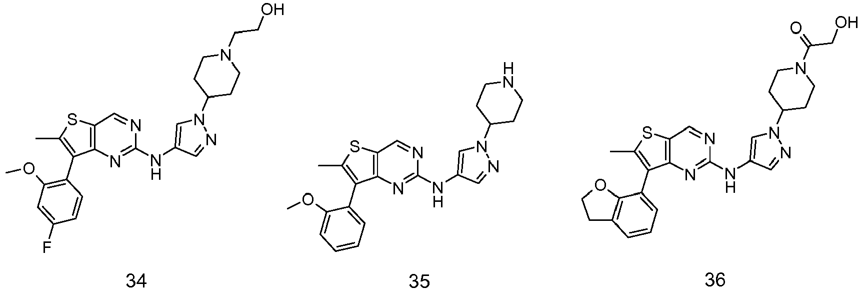

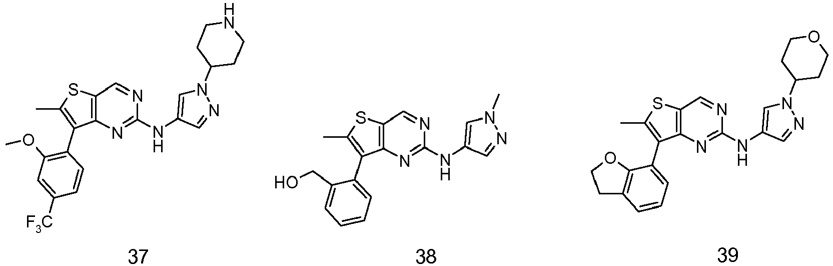

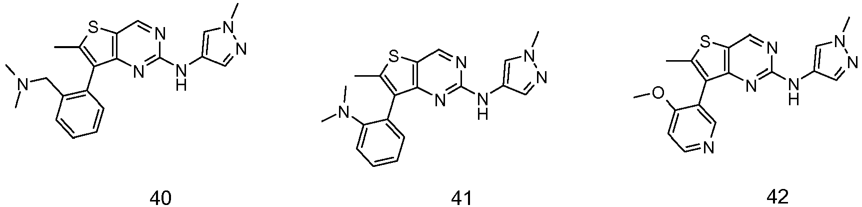

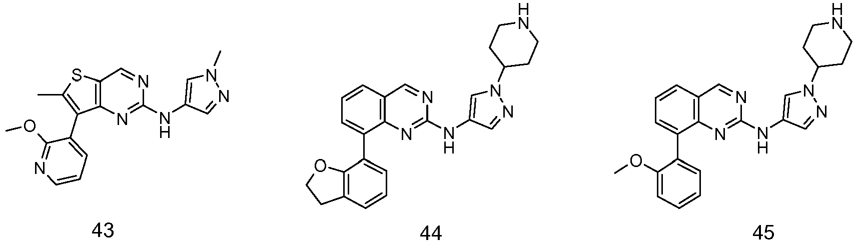

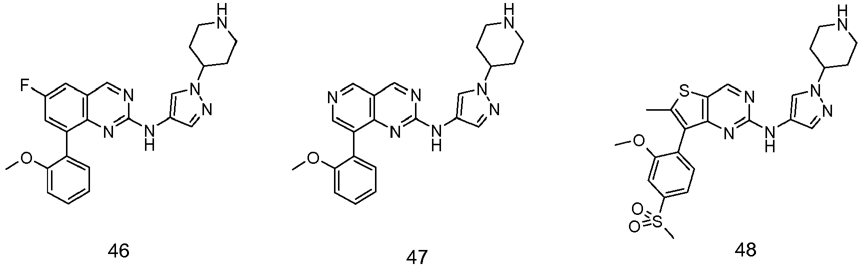

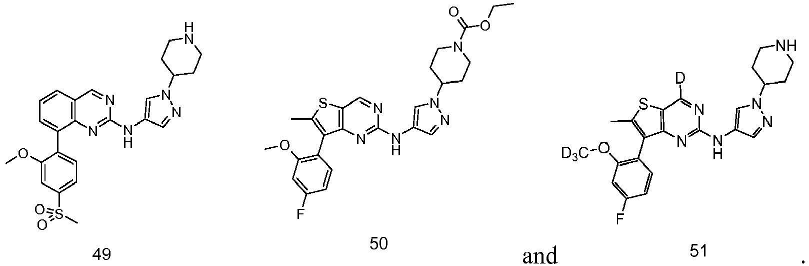

9. A process for preparing the fused ring pyrimidine compound according to any one of claims 1-8, which is any one of processes 1-13,
process 1 comprises carrying out a substitution reaction with compound **1-a** and a methylation reagent in an organic solvent and in the presence of a base to give compound **I**; 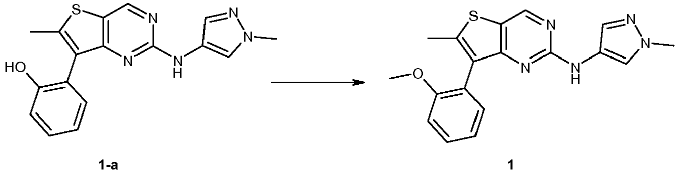
process 2 comprises carrying out a substitution reaction with compound II and compound VI in an organic solvent and in the presence of a catalyst to give compound **I**; 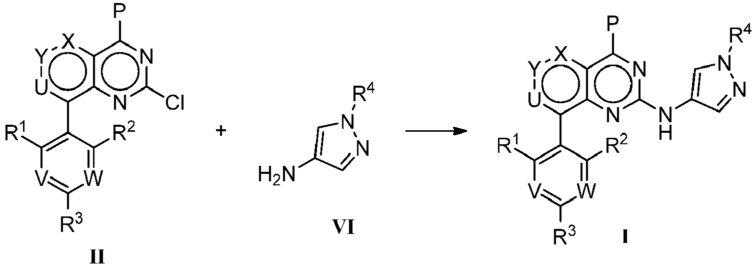
process 3 comprises carrying out a coupling reaction with compound III and compound VII in an organic solvent and water and in the presence of a base and a palladium catalyst to give compound **I**; wherein, A is Br or I; 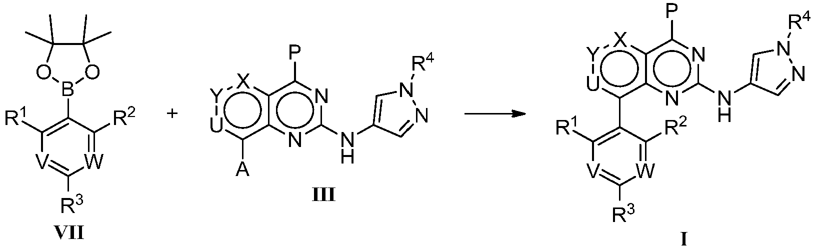
process 4 comprises carrying out a substitution reaction with compound **9-a** and 2-(4-piperidyl)-2-propanol in an organic solvent and in the presence of a base to give compound **9;** 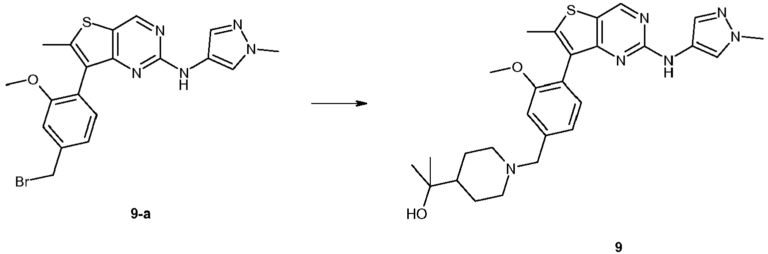
process 5 comprises carrying out a substitution reaction with compound **17-a** and morpholine in an organic solvent and in the presence of a base to give compound **17;** 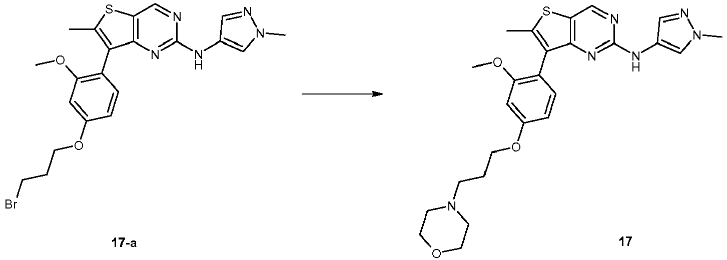
process 6 comprises carrying out a substitution reaction with compound **17-a** and pyrrolidine in an organic solvent and in the presence of a base to give compound **18;** 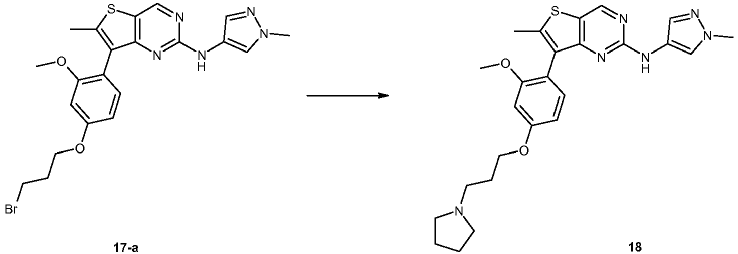
process 7 comprises carrying out a substitution reaction with compound **17-a** and N-methylpiperazine in an organic solvent and in the presence of a base to give compound **19;** 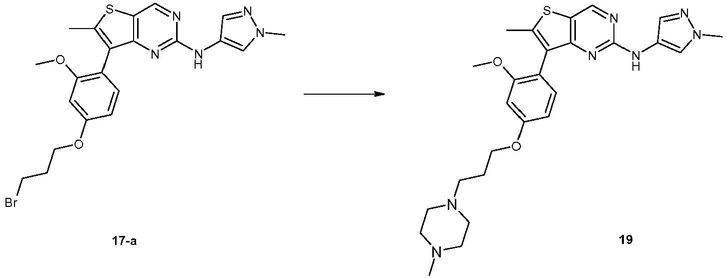
process 8 comprises carrying out a condensation reaction with compound **23-**band azetidine in an organic solvent and in the presence of a base, N-hydroxybenzotriazole and 1-ethyl-(3-dimethylaminopropyl)carbodiimide hydrochloride to give compound **23;** 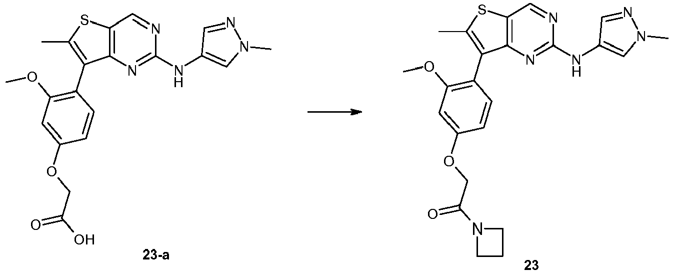
process 9 comprises deprotecting compound IV in an organic solvent and in the presence of an acid to give compound **I**; wherein R⁴ is 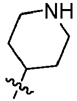 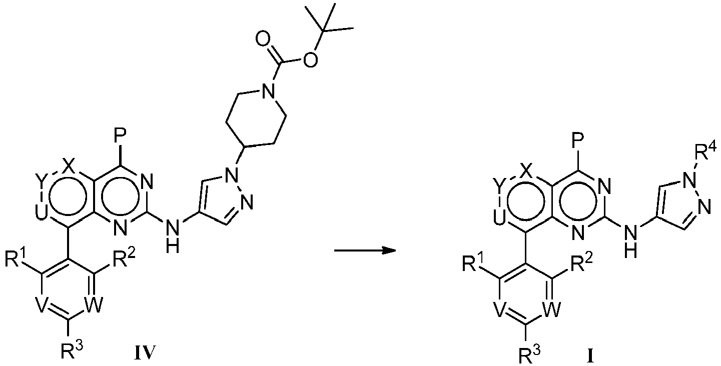
process 10 comprises carrying out a substitution reaction with compound 31 and 2-haloethanol in an organic solvent and in the presence of a base to give compound **34;** 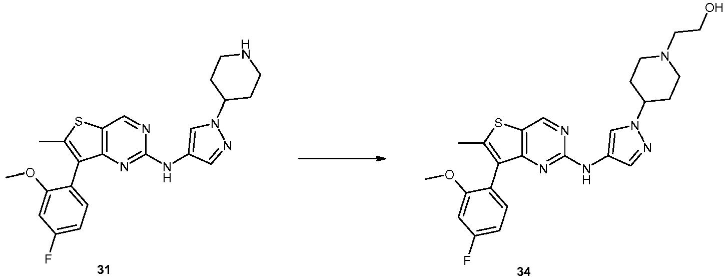
process 11 comprises carrying out a condensation reaction with compound **32** and 2-hydroxyacetic acid in an organic solvent and in the presence of a base, 1-hydroxybenzotriazole and 1-ethyl-(3-dimethylaminopropyl)carbodiimide hydrochloride to give compound **36;** 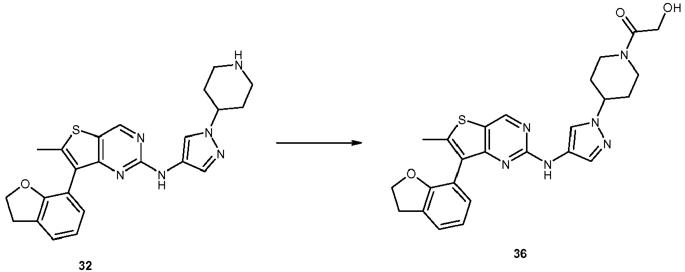
process 12 comprises carrying out a reduction amination reaction with compound **40-a,** dimethylamine and sodium triacetoxyborohydride in an organic solvent and in the presence of an acid to give compound **40;** 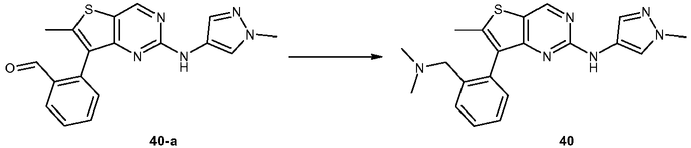
process 13 comprises carrying out a condensation reaction with compound **31** and ethyl chloroformate in an organic solvent and in the presence of a base to give compound **50;**

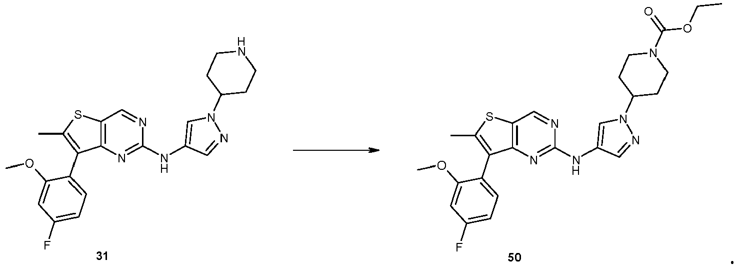

10. A compound having a structure of formula III or formula IV, 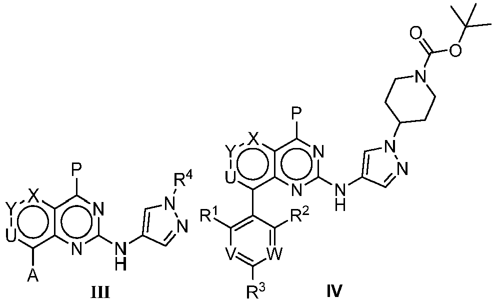
wherein, each of R¹, R², R³, X, Y, U, P, V and W is as defined in any one of claims 1-8;
wherein, A is Br or **I**; R⁴ is as defined in any one of claims 1-8;
or a compound which is selected from the group consisting of

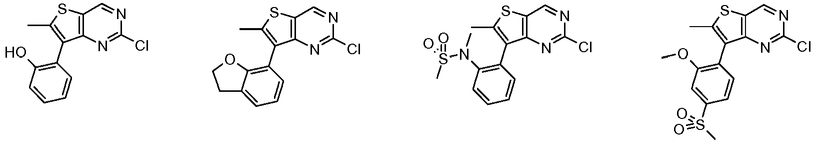

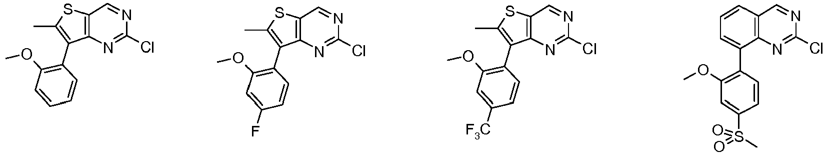

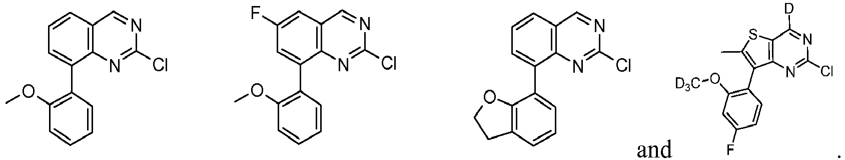

11. The compound according to claim 10, wherein the compound having a structure of formula III is selected from the group consisting of 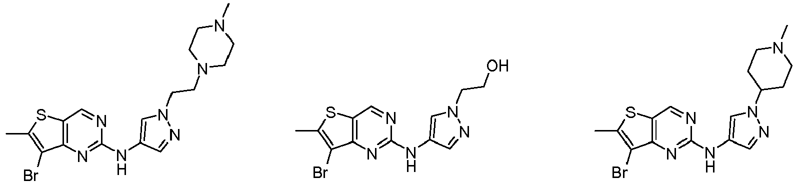 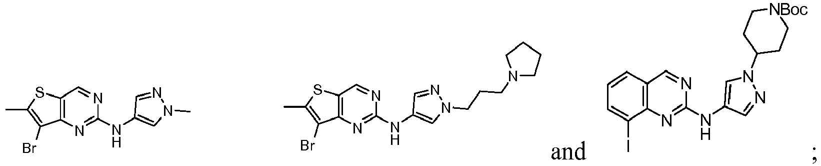 the compound having a structure of formula IV is selected from the group consisting of

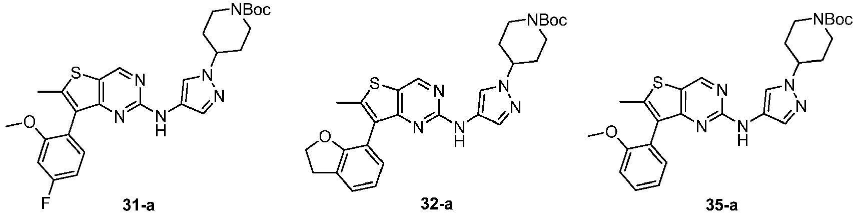

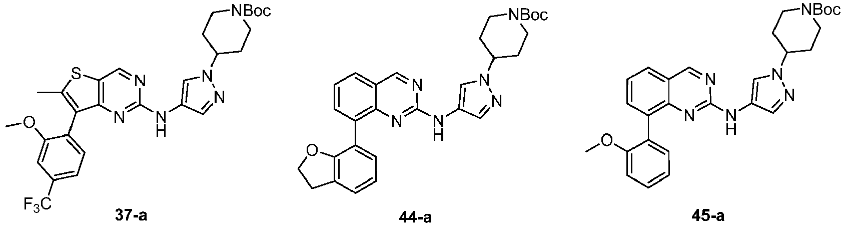

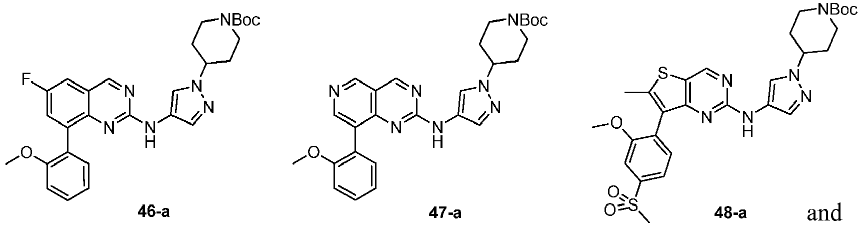

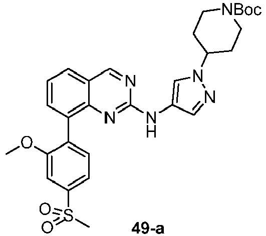

12. A compound V which is selected from the group consisting of

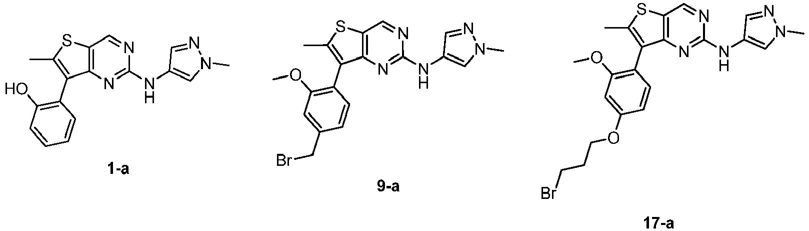

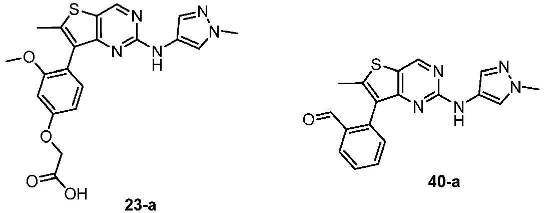

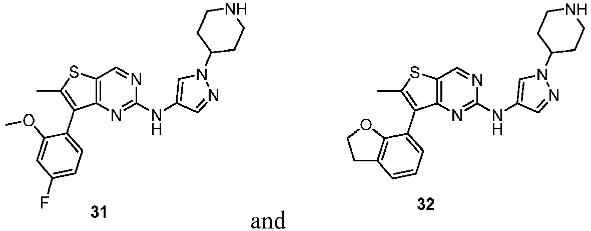

13. The fused ring pyrimidine compound, the tautomer, the enantiomer, the diastereoisomer or the pharmaceutically acceptable salt thereof according to any one of claims 1-8 for use in the prevention, alleviation or treatment of the diseases selected from the group consisting of immune system disease, autoimmune disease, cell proliferative disease, allergic disorder and cardiovascular disease.

14. The fused ring pyrimidine compound, the tautomer, the enantiomer, the diastereoisomer or the pharmaceutically acceptable salt thereof according to claim 13, wherein the immune system disease is organ transplant rejection;
and/or, the autoimmune disease is selected from the group consisting of rheumatoid arthritis, psoriasis, Crohn's disease and multiple sclerosis;
and/or, the cell proliferative disease is selected from the group consisting of myelofibrosis, hematological tumor and solid tumor; wherein the hematological tumor preferably is leukemia and/or lymphoma; the solid tumor is selected from the group consisting of renal cancer, liver cancer, stomach cancer, lung cancer, breast cancer, prostate cancer, pancreatic cancer, thyroid cancer, ovarian cancer, glioblastoma, skin cancer and melanoma;
and/or, the allergic disorder is bronchial asthma;
and/or, the cardiovascular disease is selected from the group consisting of ischemic cardiomyopathy, heart failure and myocardial infarction.

15. A pharmaceutical composition, comprising the fused ring pyrimidine compound, the tautomer, the enantiomer, the diastereoisomer or the pharmaceutically acceptable salt thereof according to any one of claims 1-8, and one or more than one pharmaceutically acceptable carrier(s) and/or diluent(s).

16. The pharmaceutical composition according to claim 15, wherein the dose of the fused ring pyrimidine compound, the tautomer, the enantiomer, the diastereoisomer or the pharmaceutically acceptable salt thereof is a therapeutically effective amount;
and/or, the pharmaceutical composition is used in combination with one or more than one clinically used chemotherapeutic agent, wherein preferably the dosage of which is a liposomal dosage.

## Patentansprüche

1. Pyrimidinverbindung mit anelliertem Ring der Formel I, ein Tautomer, ein Enantiomer, ein Diastereoisomer oder ein pharmazeutisch annehmbares Salz davon; 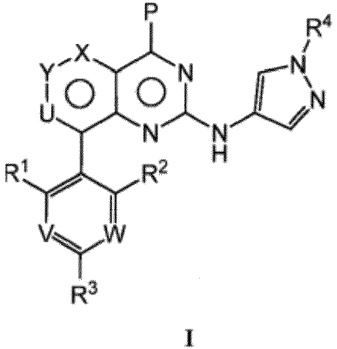
wobei P ausgewählt ist aus einem Wasserstoff oder einem Deuterium;
X aus CH oder S ausgewählt ist;
Y aus N oder CR⁵ ausgewählt ist; U aus einer chemischen Bindung oder CH ausgewählt ist;
V aus N oder CH ausgewählt ist;
W aus N oder CR⁶ ausgewählt ist;
R¹, R², R³ und R⁶ jeweils unabhängig aus der Gruppe bestehend aus einem Wasserstoff, einem Deuterium, einem Halogen, einem substituierten oder unsubstituierten Alkyl, 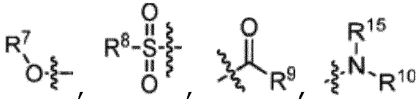 , einem Cycloalkyl oder einem Heterocycloalkyl ausgewählt sind; R⁷, R⁸, R⁹, R¹⁰ und R¹⁵ jeweils unabhängig aus der Gruppe bestehend aus einem Wasserstoff, einem Deuterium, einem Halogen, einem Hydroxyl, einem Amino, einem substituierten oder unsubstituierten Alkyl, einem Alkoxy, 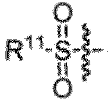 oder einem Heterocycloalkyl ausgewählt sind; R¹¹ ein Wasserstoff, ein Deuterium oder ein Alkyl ist; oder R⁶, R² und die beiden Atome an dem Ring, an den sie gebunden sind, einen "substituierten oder unsubstituierten 5- bis 7-gliedrigen Kohlenstoff-Heterocyclus" bilden; oder R⁶, R³ und die beiden Atome an dem Ring, an den sie gebunden sind, einen "substituierten oder unsubstituierten 5- bis 7-gliedrigen Kohlenstoff-Heterocyclus" bilden; wobei das Heteroatom in dem "substituierten oder unsubstituierten 5- bis 7-gliedrigen Kohlenstoff-Heterocyclus" aus der Gruppe bestehend aus Stickstoff, Sauerstoff und Schwefel ausgewählt ist; R⁴ ein Wasserstoff, ein Deuterium, ein substituiertes oder unsubstituiertes Alkyl, ein Alkoxy, ein Cycloalkyl oder ein substituiertes oder unsubstituiertes Heterocycloalkyl ist;
R⁵ ein Wasserstoff, ein Deuterium, ein Halogen oder ein Alkyl ist;
in den Definitionen von R¹, R², R³ und R⁶ das "substituiert" in "ein substituiertes oder unsubstituiertes Alkyl" durch die Substituenten aus der Gruppe bestehend aus einem Halogen, einem Hydroxyl, einem Amino, einem Alkyl, einem Alkoxy, 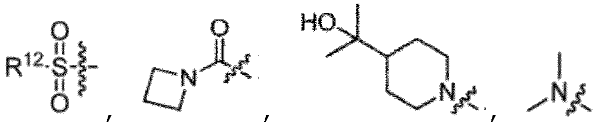 und einem Heterocycloalkyl substituiert bedeutet, in dem Fall, dass mehrere Substituenten vorliegen, die Substituenten gleich oder verschieden sind; R¹² ein Wasserstoff, ein Deuterium oder ein Alkyl ist; in den Definitionen von R⁷, R⁸, R⁹, R¹⁰ und R¹⁵ das "substituiert" in "ein substituiertes oder unsubstituiertes Alkyl" durch die Substituenten aus der Gruppe bestehend aus einem Deuterium, einem Halogen, einem Hydroxyl, einem Amino, einem Alkyl, einem Alkoxy, 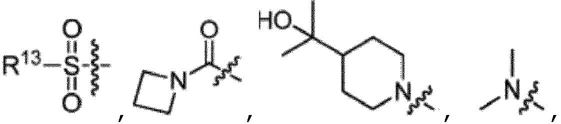 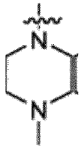 und einem Heterocycloalkyl substituiert bedeutet, in dem Fall, dass mehrere Substituenten vorliegen, die Substituenten gleich oder verschieden sind; R¹³ ist ein Wasserstoff oder ein Alkyl; in der Definition von R⁴ das "substituiert" in "ein substituiertes oder unsubstituiertes Alkyl" und "ein substituiertes oder unsubstituiertes Heterocycloalkyl" durch die Substituenten aus der Gruppe bestehend aus einem Hydroxyl, einem Alkyl, 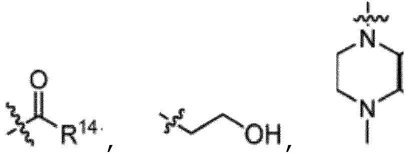 und Heterocycloalkyl substituiert bedeutet, in dem Fall, dass mehrere Substituenten vorliegen, die Substituenten gleich oder verschieden sind; R¹⁴ ein Wasserstoff, ein Alkyl, ein Hydroxylmethyl oder ein Alkoxy ist; das "substituiert" in "substituierte oder unsubstituierter 5- bis 7-gliedriger Kohlenstoff-Heterocyclus" durch ein oder mehr als ein Alkyl substituiert bedeutet.

2. Pyrimidinverbindung mit anelliertem Ring, Tautomer, Enantiomer, Diastereoisomer oder pharmazeutisch annehmbares Salz davon nach Anspruch 1,
wobei in dem Fall, dass R¹, R², R³ und R⁶ jeweils unabhängig für ein Halogen stehen, es sich bei dem Halogen um Fluor oder Chlor handelt;
und/oder in dem Fall, dass R¹, R², R³ und R⁶ jeweils unabhängig für "ein substituiertes oder unsubstituiertes Alkyl" stehen, es sich bei dem Alkyl um ein C₁₋₄-Alkyl handelt;
und/oder in dem Fall, dass R¹, R², R³ und R⁶ jeweils unabhängig für ein Heterocycloalkyl stehen, das Heterocycloalkyl über ein Kohlenstoffatom oder ein Heteroatom davon mit anderen Gruppen verknüpft ist;
und/oder in dem Fall, dass R¹, R², R³ und R⁶ jeweils unabhängig für ein Heterocycloalkyl stehen, es sich bei dem Heterocycloalkyl um ein "Heterocycloalkyl mit 1-4 Heteroatomen und 3-8 Kohlenstoffatomen, wobei es sich bei dem Heteroatom um Sauerstoff und/oder Stickstoff handelt," handelt;
und/oder in dem Fall, dass R⁷, R⁸, R⁹, R¹⁰ und R¹⁵ jeweils unabhängig für ein Halogen stehen, es sich bei dem Halogen um Fluor handelt;
und/oder in dem Fall, dass R⁷, R⁸, R⁹, R¹⁰ und R¹⁵ jeweils unabhängig für "ein substituiertes oder unsubstituiertes Alkyl" stehen, es sich bei dem Alkyl um ein C₁₋₁₀-Alkyl handelt;
und/oder in dem Fall, dass R⁷, R⁸, R⁹, R¹⁰ und R¹⁵ jeweils unabhängig für ein Alkoxy stehen, es sich bei dem Alkoxy um ein C₁₋₁₀-Alkoxy handelt; und/oder in dem Fall, dass R⁷, R⁸, R⁹, R¹⁰ und R¹⁵ jeweils unabhängig für ein Heterocycloalkyl stehen, das Heterocycloalkyl über ein Kohlenstoffatom oder ein Heteroatom davon mit anderen Gruppen verknüpft ist;
und/oder in dem Fall, dass R⁷, R⁸, R⁹, R¹⁰ und R¹⁵ jeweils unabhängig für ein Heterocycloalkyl stehen, es sich bei dem Heterocycloalkyl um ein "Heterocycloalkyl mit 1-4 Heteroatomen und 3-8 Kohlenstoffatomen, wobei es sich bei dem Heteroatom um Sauerstoff und/oder Stickstoff handelt," handelt;
und/oder in dem Fall, dass R⁷, R⁸, R⁹, R¹⁰ und R¹⁵
jeweils unabhängig für 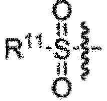 stehen, wobei R¹¹ für ein C₁₋₄-Alkyl steht;
und/oder in dem Fall, dass R⁴ für ein "substituiertes oder unsubstituiertes Alkyl" steht, es sich bei dem Alkyl um ein C₁₋₄-Alkyl handelt;
und/oder in dem Fall, dass R⁴ für ein Alkoxy steht, es sich bei dem Alkoxy um ein C₁₋₄-Alkoxy handelt;
und/oder in dem Fall, dass R⁴ für "ein substituiertes oder unsubstituiertes Heterocycloalkyl" steht, das Heterocycloalkyl über ein Kohlenstoffatom oder ein Heteroatom davon mit anderen Gruppen verknüpft ist; und/oder in dem Fall, dass R⁴ für "ein substituiertes oder unsubstituiertes Heterocycloalkyl" steht, es sich bei dem Heterocycloalkyl um ein "Heterocycloalkyl mit 1-4 Heteroatomen und 3-8 Kohlenstoffatomen, wobei es sich bei dem Heteroatom um Sauerstoff und/oder Stickstoff handelt," handelt; und/oder in dem Fall, dass R⁵ für ein Halogen steht, es sich bei dem Halogen um Fluor handelt;
und/oder in dem Fall, dass R⁵ für ein Alkyl steht, es sich bei dem Alkyl um ein C₁₋₄-Alkyl handelt;
und/oder in dem Fall, dass es sich bei dem "5- bis 7-gliedrigen Kohlenstoff-Heterocyclus" in "einem substituierten oder unsubstituierten 5- bis 7-gliedrigen Kohlenstoff-Heterocyclus" um einen Kohlenstoff-Heterocyclus mit 1-4 Heteroatomen und 2-6 Kohlenstoffatomen, wobei es sich bei dem Heteroatom um Sauerstoff oder Stickstoff handelt," handelt;
und/oder in dem Fall, dass R¹, R², R³ und R⁶ jeweils unabhängig für "ein substituiertes oder unsubstituiertes Alkyl" stehen, es sich bei dem Substituenten in "einem substituierten oder unsubstituierten Alkyl" um Fluor handelt;
und/oder in dem Fall, dass R¹, R², R³ und R⁶ jeweils unabhängig für "ein substituiertes oder unsubstituiertes Alkyl" stehen, es sich bei dem Substituenten in "einem substituierten oder unsubstituierten Alkyl" um C₁₋₁₀-Alkyl handelt;
und/oder in dem Fall, dass R¹, R², R³ und R⁶ jeweils unabhängig für "ein substituiertes oder unsubstituiertes Alkyl" stehen, es sich bei dem Substituenten in "einem substituierten oder unsubstituierten Alkyl" um C₁₋₁₀-Alkoxy handelt;
und/oder in dem Fall, dass R¹, R², R³ und R⁶ jeweils unabhängig für "ein substituiertes oder unsubstituiertes Alkyl" stehen und es sich bei dem Substituenten in "einem substituierten oder unsubstituierten Alkyl" um ein Heterocyclyl handelt, das Heterocycloalkyl über ein Kohlenstoffatom oder ein Heteroatom davon mit anderen Gruppen verknüpft ist;
und/oder in dem Fall, dass R¹, R², R³ und R⁶ jeweils unabhängig für "ein substituiertes oder unsubstituiertes Alkyl" stehen, es sich bei dem Substituenten in "einem substituierten oder unsubstituierten Alkyl" um "ein Heterocycloalkyl mit 1-4 Heteroatomen und 3-8 Kohlenstoffatomen, in denen es sich bei dem Heteroatom um Sauerstoff und/oder Stickstoff handelt," handelt,
und/oder in dem Fall, dass R¹, R², R³ und R⁶ jeweils unabhängig für "ein substituiertes oder unsubstituiertes Alkyl stehen", es sich bei dem Substituenten in "einem substituierten oder
unsubstituierten Alkyl" um 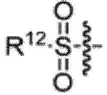 handelt, wobei R¹² für C₁₋₄-Alkyl steht;
und/oder in dem Fall, dass R⁷, R⁸, R⁹, R¹⁰ und R¹⁵ jeweils unabhängig für "ein substituiertes oder unsubstituiertes Alkyl" stehen, es sich bei dem Substituenten in "einem substituierten oder unsubstituierten Alkyl" um Fluor handelt;
und/oder in dem Fall, dass R⁷, R⁸, R⁹, R¹⁰ und R¹⁵ jeweils unabhängig für "ein substituiertes oder unsubstituiertes Alkyl" stehen, es sich bei dem Substituenten in "einem substituierten oder unsubstituierten Alkyl" um ein C₁₋₁₀-Alkyl handelt; und/oder in dem Fall, dass R⁷, R⁸, R⁹, R¹⁰ und R¹⁵ jeweils unabhängig für "ein substituiertes oder unsubstituiertes Alkyl" stehen, es sich bei dem Substituenten in "einem substituierten oder unsubstituierten Alkyl" um C₁₋₁₀-Alkoxy handelt; und/oder in dem Fall, dass R⁷, R⁸, R⁹, R¹⁰ und R¹⁵ jeweils unabhängig für "ein substituiertes oder unsubstituiertes Alkyl" stehen und es sich bei dem Substituenten in "einem substituierten oder unsubstituierten Alkyl" um ein Heterocycloalkyl handelt, das Heterocycloalkyl über ein Kohlenstoffatom oder ein Heteroatom davon mit anderen Gruppen verknüpft ist;
und/oder in dem Fall, dass R⁷, R⁸, R⁹, R¹⁰ und R¹⁵ jeweils unabhängig für "ein substituiertes oder unsubstituiertes Alkyl" stehen, es sich bei dem Substituenten in "einem substituierten oder unsubstituierten Alkyl" um "ein Heterocycloalkyl mit 1-4 Heteroatomen und 3-8 Kohlenstoffatomen, in denen es sich bei dem Heteroatom um Sauerstoff und/oder Stickstoff handelt," handelt,
und/oder in dem Fall, dass R⁷, R⁸, R⁹, R¹⁰ und R¹⁵ jeweils unabhängig für "ein substituiertes oder unsubstituiertes Alkyl stehen", es sich bei dem Substituenten in "einem substituierten oder
unsubstituierten Alkyl" um 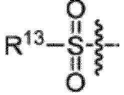 handelt, wobei R¹³ für ein C₁₋₄-Alkyl steht;
und/oder in dem Fall, dass R⁴ für "ein substituiertes oder unsubstituiertes Alkyl" oder "ein substituiertes oder unsubstituiertes Heterocycloalkyl" steht, es sich bei dem Substituenten um "einem substituierten oder unsubstituierten Alkyl" oder "einem substituierten oder unsubstituierten Heterocycloalkyl" um ein C₁₋₄-Alkyl handelt;
und/oder in dem Fall, dass R⁴ für "ein substituiertes oder unsubstituiertes Alkyl" oder "ein substituiertes oder unsubstituiertes Heterocycloalkyl" steht, es sich bei dem Substituenten in "einem substituierten oder unsubstituierten Alkyl" oder "einem substituierten oder unsubstituierten Heterocycloalkyl" um ein Heterocycloalkyl handelt, das Heterocycloalkyl über ein Kohlenstoffatom oder ein Heteroatom davon mit anderen Gruppen verknüpft ist;
und/oder in dem Fall, dass R⁴ für "ein substituiertes oder unsubstituiertes Alkyl" oder "ein substituiertes oder unsubstituiertes Heterocycloalkyl" steht, es sich bei dem Substituenten in "einem substituierten oder unsubstituierten Alkyl" oder "einem substituierten oder unsubstituierten Heterocycloalkyl" um ein "Heterocycloalkyl mit 1-4 Heteroatomen und 3-8 Kohlenstoffatomen, wobei es sich bei dem Heteroatom um Sauerstoff und/oder Stickstoff handelt," handelt;
und/oder in dem Fall, dass R⁴ für "ein substituiertes oder unsubstituiertes Alkyl" oder "ein substituiertes oder unsubstituiertes Heterocycloalkyl" steht, es sich bei dem Substituenten in "einem substituierten oder unsubstituierten Alkyl" oder "einem substituierten oder unsubstituierten
Heterocycloalkyl" um 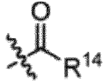 handelt, wobei R¹⁴ für ein C₁₋₄-Alkyl steht;
und/oder in dem Fall, dass R⁴ für "ein substituiertes oder unsubstituiertes Alkyl" oder "ein substituiertes oder unsubstituiertes Heterocycloalkyl" steht, es sich bei dem Substituenten in "einem substituierten oder unsubstituierten Alkyl" oder "einem substituierten oder unsubstituierten
Heterocycloalkyl" um 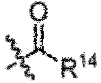 handelt, wobei R¹⁴ für ein C₁₋₄-Alkoxy steht;
und/oder in dem Fall, dass der Substituent in "einem substituierten und unsubstituierten 5- bis 7-gliedrigen Kohlenstoff-Heterocyclus" für ein C₁₋₄-Alkyl steht.

3. Pyrimidinverbindung mit anelliertem Ring, Tautomer, Enantiomer, Diastereoisomer oder pharmazeutisch annehmbares Salz davon nach Anspruch 2,
wobei in dem Fall, dass R¹, R², R³ und R⁶ jeweils für "ein substituiertes oder unsubstituiertes Alkyl" stehen, es sich bei dem Alkyl um ein Methyl handelt; und/oder in dem Fall, dass, R¹, R², R³ und R⁶ jeweils unabhängig für ein Heterocycloalkyl stehen, es sich bei dem Heterocycloalkyl um ein "Heterocycloalkyl mit 1-4 Heteroatomen und 3-6 Kohlenstoffatomen, wobei es sich bei dem Heteroatom um Sauerstoff und/oder Stickstoff handelt," handelt;
und/oder wobei in dem Fall, dass R⁷, R⁸, R⁹, R¹⁰ und R¹⁵ jeweils unabhängig für "ein substituiertes oder unsubstituiertes Alkyl" stehen, es sich bei dem Alkyl um ein C₁₋₄-Alkyl handelt;
und/oder in dem Fall, dass R⁷, R⁸, R⁹, R¹⁰ und R¹⁵ jeweils unabhängig für ein Alkoxy stehen, es sich bei dem Alkoxy um ein C₁₋₄-Alkoxy handelt;
und/oder in dem Fall, dass R⁷, R⁸, R⁹, R¹⁰ und R¹⁵ jeweils unabhängig für ein Heterocycloalkyl stehen, es sich bei dem Heterocycloalkyl um ein "Heterocycloalkyl mit 1-4 Heteroatomen und 3-6 Kohlenstoffatomen, wobei es sich bei dem Heteroatom um Sauerstoff und/oder Stickstoff handelt," handelt;
und/oder in dem Fall, dass R⁷, R⁸, R⁹, R¹⁰ und R¹⁵
jeweils unabhängig für 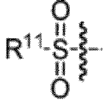 stehen, R¹¹ für ein Methyl steht;
und/oder in dem Fall, dass R⁴ für "ein substituiertes oder unsubstituiertes Alkyl" steht, es sich bei dem Alkyl um ein Methyl, ein Ethyl, ein Propyl oder ein Isopropyl handelt;
und/oder in dem Fall, dass R⁴ für "ein substituiertes oder unsubstituiertes Heterocycloalkyl" steht, es sich bei dem Heterocycloalkyl um ein "Heterocycloalkyl mit 1-4 Heteroatomen und 3-6 Kohlenstoffatomen, wobei es sich bei dem Heteroatom um Sauerstoff und/oder Stickstoff handelt," handelt;
und/oder in dem Fall, dass R⁵ für Alkyl steht, es sich bei dem Alkyl um ein Methyl handelt;
und/oder es sich bei dem "5- bis 7-gliedrigen Kohlenstoff-Heterocyclus" in "substituierter oder unsubstituierter 5- bis 7-gliedriger Kohlenstoff-Heterocyclus" um 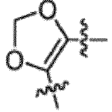 oder 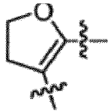 handelt;
und/oder in dem Fall, dass R¹, R², R³ und R⁶ jeweils unabhängig für "ein substituiertes oder unsubstituiertes Alkyl" stehen, es sich bei dem Substituenten in "einem substituierten oder unsubstituierten Alkyl" um ein C₁₋₄-Alkyl handelt; und/oder in dem Fall, dass R¹, R², R³ und R⁶ jeweils unabhängig für "ein substituiertes oder unsubstituiertes Alkyl" stehen, es sich bei dem Substituenten in "einem substituierten oder unsubstituierten Alkyl" um ein C₁₋₄-Alkoxy handelt; und/oder in dem Fall, dass R¹, R², R³ und R⁶ jeweils unabhängig für "ein substituiertes oder unsubstituiertes Alkyl" stehen, es sich bei dem Substituenten in "einem substituierten oder unsubstituierten Alkyl" um ein "Heterocycloalkyl mit 1-4 Heteroatomen und 3-6 Kohlenstoffatomen, wobei es sich bei dem Heteroatom um Sauerstoff und/oder Stickstoff handelt," handelt;
und/oder in dem Fall, dass R¹, R², R³ und R⁶ jeweils unabhängig für "substituiertes oder unsubstituiertes Alkyl" stehen, es sich bei dem Substituenten in "substituiertem oder
unsubstituiertem Alkyl" um 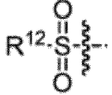 handelt, wobei R¹² für ein Methyl steht;
und/oder in dem Fall, dass R⁷, R⁸, R⁹, R¹⁰ und R¹⁵ jeweils unabhängig für "ein substituiertes oder unsubstituiertes Alkyl" stehen, es sich bei dem Substituenten in "einem substituierten oder unsubstituierten Alkyl" um ein C₁₋₄-Alkyl handelt;
und/oder in dem Fall, dass R⁷, R⁸, R⁹, R¹⁰ und R¹⁵ jeweils unabhängig für "ein substituiertes oder unsubstituiertes Alkyl" stehen, es sich bei dem Substituenten in "einem substituierten oder unsubstituierten Alkyl" um ein C₁₋₄-Alkoxy handelt; und/oder in dem Fall, dass R⁷, R⁸, R⁹, R¹⁰ und R¹⁵ jeweils unabhängig für "ein substituiertes oder unsubstituiertes Alkyl" stehen, es sich bei dem Substituenten in "einem substituierten oder unsubstituierten Alkyl" um ein "Heterocycloalkyl mit 1-4 Heteroatomen und 3-6 Kohlenstoffatomen, wobei es sich bei dem Heteroatom um Sauerstoff und/oder Stickstoff handelt," handelt;
und/oder in dem Fall, dass R¹, R², R³ und R⁶ jeweils unabhängig für "ein substituiertes oder unsubstituiertes Alkyl" stehen, es sich die dem Substituenten in "einem substituierten oder
unsubstituierten Alkyl" um 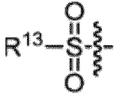 handelt, wobei R¹³ für ein Methyl steht;
und/oder in dem Fall, dass R⁴ für "ein substituiertes oder unsubstituiertes Alkyl" oder "ein substituiertes oder unsubstituiertes Heterocycloalkyl" steht, es sich bei dem Substituenten in "einem substituierten oder unsubstituierten Alkyl" oder "einem substituierten oder unsubstituierten Heterocycloalkyl" um ein Methyl handelt;
und/oder in dem Fall, dass R⁴ für "ein substituiertes oder unsubstituiertes Alkyl" oder "ein substituiertes oder unsubstituiertes Heterocycloalkyl" steht, es sich bei dem Substituenten in "einem substituierten oder unsubstituierten Alkyl" oder "einem substituierten oder unsubstituierten
Heterocycloalkyl" um 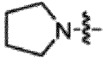 handelt;
und/oder in dem Fall, dass R⁴ für "ein substituiertes oder unsubstituiertes Alkyl" oder "ein substituiertes oder unsubstituiertes Heterocycloalkyl" steht, es sich bei dem Substituenten in "einem substituierten oder unsubstituierten Alkyl" oder "einem substituierten oder unsubstituierten
Heterocycloalkyl" um 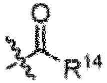 handelt, wobei R¹⁴ für ein Methyl steht;
und/oder in dem Fall, dass R⁴ für "ein substituiertes oder unsubstituiertes Alkyl" oder "ein substituiertes oder unsubstituiertes Heterocycloalkyl" steht, es sich bei dem Substituenten in "einem substituierten oder unsubstituierten Alkyl" oder "einem substituierten oder unsubstituierten
Heterocycloalkyl" um 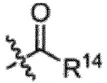 handelt, wobei R¹⁴ für ein *tert*-Butoxy oder ein Ethoxy steht;
und/oder in dem Fall, dass der Alkylsubstituent in "substituierter oder unsubstituierter 5- bis 7-gliedriger Kohlenstoff-Heterocyclus" für ein Methyl, ein Ethyl oder ein Propyl steht.

4. Pyrimidinverbindung mit anelliertem Ring, Tautomer, Enantiomer, Diastereoisomer oder pharmazeutisch annehmbares Salz davon nach Anspruch 3,
wobei in dem Fall, dass R¹, R², R³ und R⁶ jeweils für ein Heterocycloalkyl stehen, es sich bei dem Heterocycloalkyl um 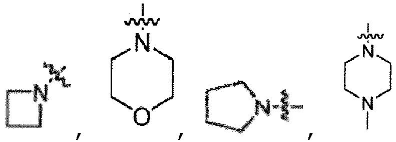 oder 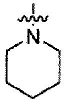 handelt;
und/oder in dem Fall, dass R⁷, R⁸, R⁹, R¹⁰ und R¹⁵ jeweils unabhängig für "unsubstituiertes oder unsubstituiertes Alkyl" stehen, es sich bei dem Alkyl um ein Methyl, ein Trideuteromethyl, ein Ethyl, ein Propyl oder ein Isopropyl handelt;
und/oder in dem Fall, dass R⁷, R⁸, R⁹, R¹⁰ und R¹⁵ jeweils unabhängig für ein Alkoxy stehen, es sich bei dem Alkoxy um ein Methoxy handelt;
und/oder in dem Fall, dass R⁷, R⁸, R⁹, R¹⁰ und R¹⁵ jeweils unabhängig für ein Heterocycloalkyl stehen, es sich bei dem Heterocycloalkyl um 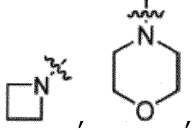 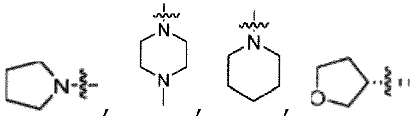 oder 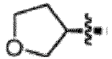 handelt;
und/oder in dem Fall, dass R⁴ für "ein substituiertes oder unsubstituiertes Heterocycloalkyl" steht, es sich bei dem Heterocycloalkyl in "einem substituierten oder unsubstituierten Heterocycloalkyl" um 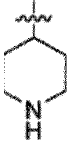 oder 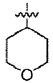 handelt;
und/oder in dem Fall, dass R¹, R², R³ und R⁶ jeweils unabhängig für "ein substituiertes oder unsubstituiertes Alkyl" stehen, es sich bei dem Substituenten in "einem substituierten oder unsubstituierten Alkyl" um ein Methyl handelt; und/oder in dem Fall, dass R¹, R², R³ und R⁶ jeweils unabhängig für "ein substituiertes oder unsubstituiertes Alkyl" stehen, es sich bei dem Substituenten in "einem substituierten oder unsubstituierten Alkyl" um ein Methoxy handelt; und/oder in dem Fall, dass R¹, R², R³ und R⁶ jeweils unabhängig für "ein substituiertes oder unsubstituiertes Alkyl" stehen, es sich bei dem Substituenten in "einem substituierten oder unsubstituierten Alkyl" um 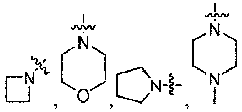 oder 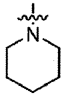 handelt;
und/oder in dem Fall, dass R⁷, R⁸, R⁹, R¹⁰ und R¹⁵ jeweils unabhängig für "ein substituiertes oder unsubstituiertes Alkyl" stehen, es sich bei dem Substituenten in "einem substituierten oder unsubstituierten Alkyl" um ein Methyl handelt; und/oder in dem Fall, dass R⁷, R⁸, R⁹, R¹⁰ und R¹⁵ jeweils unabhängig für "ein substituiertes oder unsubstituiertes Alkyl" stehen, es sich bei dem Substituenten in "einem substituierten oder unsubstituierten Alkyl" um ein Methoxy handelt; und/oder in dem Fall, dass R⁷, R⁸, R⁹, R¹⁰ und R¹⁵ jeweils unabhängig für "ein substituiertes oder unsubstituiertes Alkyl" stehen, es sich bei dem Substituenten in "einem substituierten oder unsubstituierten Alkyl" um 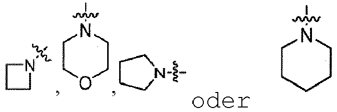 handelt.

5. Pyrimidinverbindung mit anelliertem Ring, Tautomer, Enantiomer, Diastereoisomer oder pharmazeutisch annehmbares Salz davon nach einem der Ansprüche 1-4, wobei die Verbindung **I** als Formel **I-1** oder **I-2** gezeigt ist,

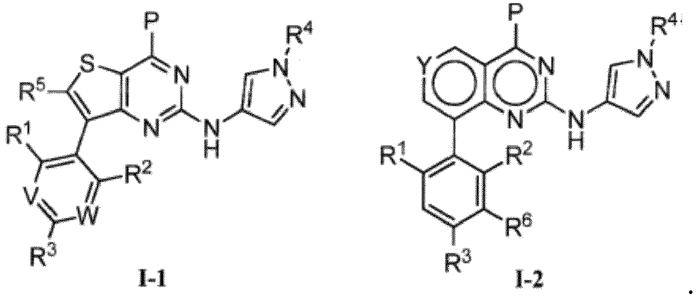

6. Pyrimidinverbindung mit anelliertem Ring, Tautomer, Enantiomer, Diastereoisomer oder pharmazeutisch annehmbares Salz davon nach Anspruch 5, wobei die Verbindung **I-1** als Formel **I-1-1** oder **I-1-2** gezeigt ist, 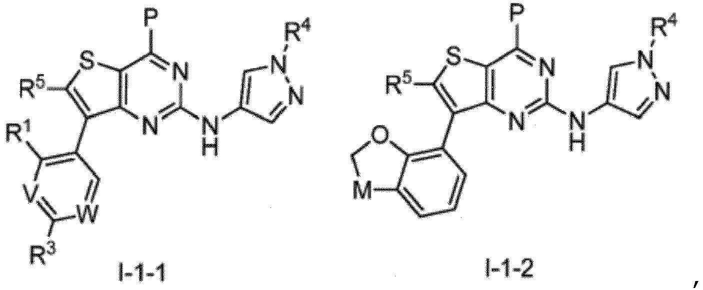 wobei M für CH₂ oder O steht;
die Verbindung **I-2** als Formel **I-2-1** oder **I-2-2** gezeigt ist,

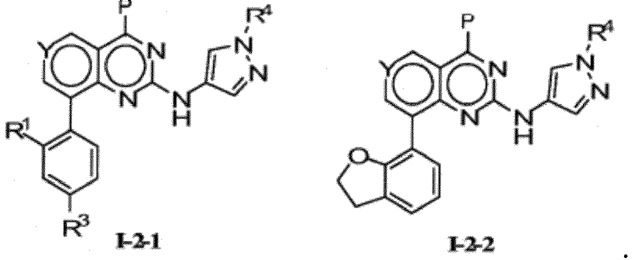

7. Pyrimidinverbindung mit anelliertem Ring, Tautomer, Enantiomer, Diastereoisomer oder pharmazeutisch annehmbares Salz davon nach einem der Ansprüche 1-4, wobei in Verbindung **I** Y für CR⁵ steht;
und/oder in Verbindung **I** R⁵ für einen Wasserstoff oder ein Alkyl steht;
und/oder in Verbindung **I** W für CR⁶ steht;
und/oder in Verbindung **I** R⁶ für einen Wasserstoff steht;
und/oder in Verbindung **I** R⁶ und R² zusammen mit zwei Atomen an dem Ring, an den sie gebunden sind, "einen substituierten oder unsubstituierten 5- bis 7-gliedrigen Kohlenstoff-Heterocyclus" bilden;
und/oder in Verbindung **I** R¹ und R² jeweils unabhängig für einen Wasserstoff oder 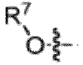 stehen;
und/oder in Verbindung **I** R¹ oder R² für einen Wasserstoff stehen;
und/oder in Verbindung **I** R³ für einen Wasserstoff, 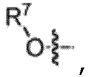 ein Halogen oder 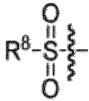 steht;
und/oder in Verbindung **I** R⁴ für "ein substituiertes oder unsubstituiertes Alkyl" oder "ein substituiertes oder unsubstituiertes Heterocycloalkyl" steht.

8. Pyrimidinverbindung mit anelliertem Ring, Tautomer, Enantiomer, Diastereoisomer oder pharmazeutisch annehmbares Salz davon nach Anspruch 1, die ausgewählt ist aus der Gruppe bestehend aus

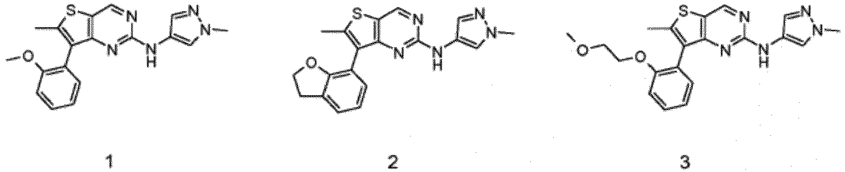

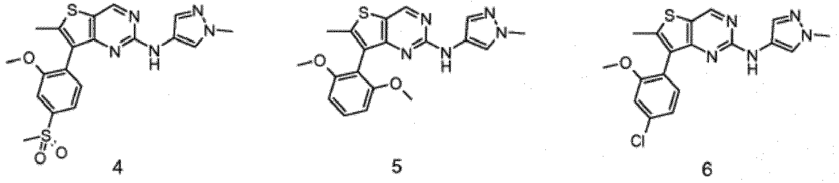

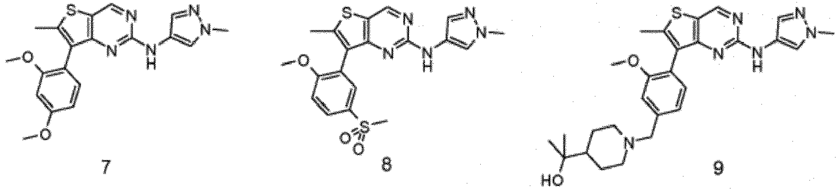

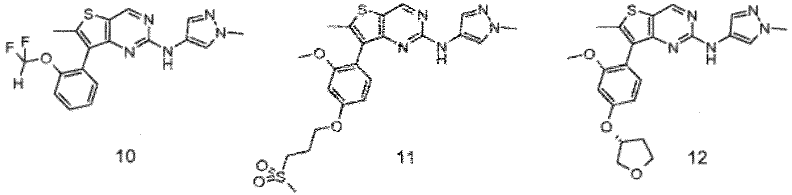

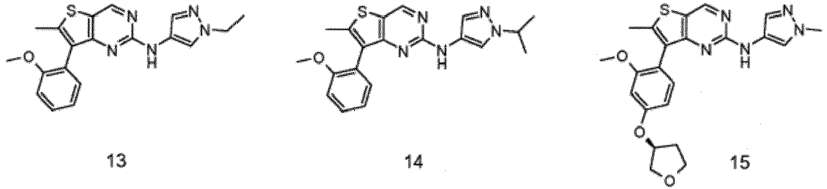

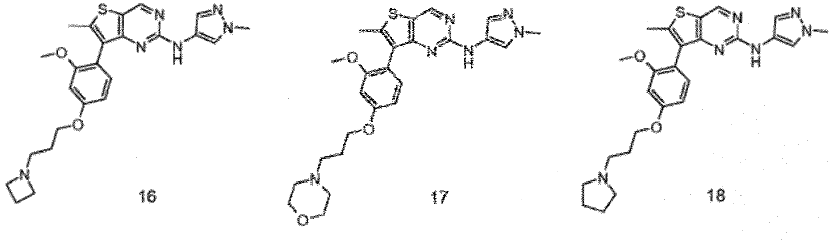

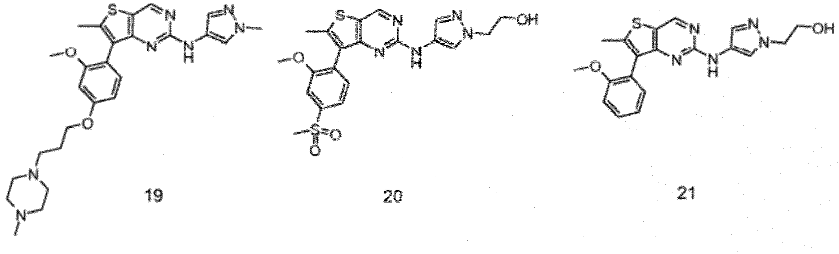

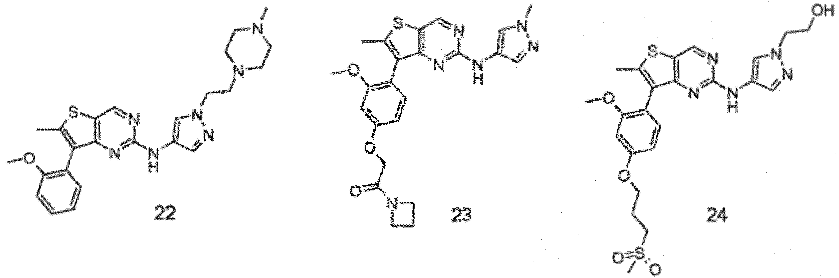

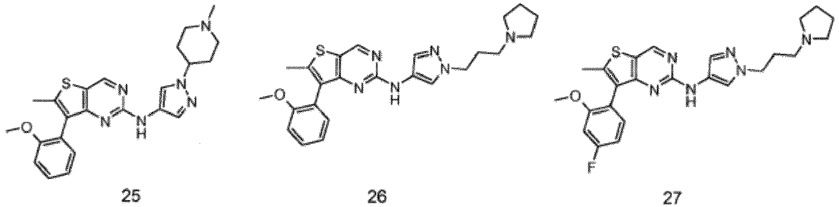

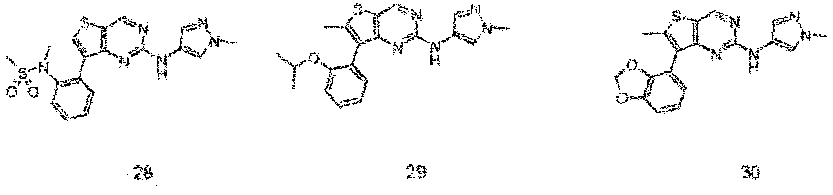

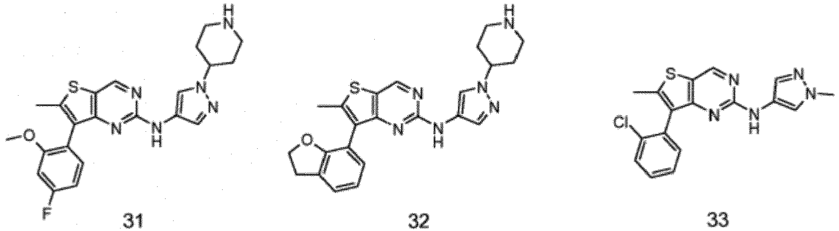

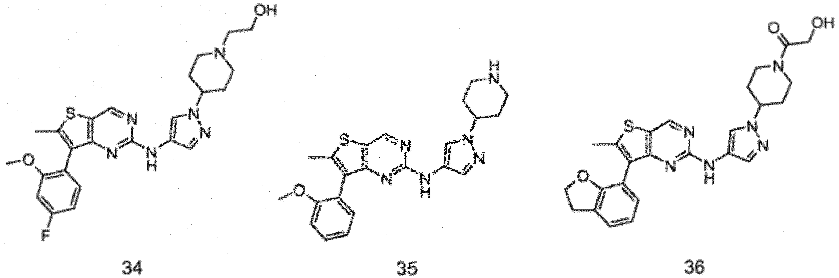

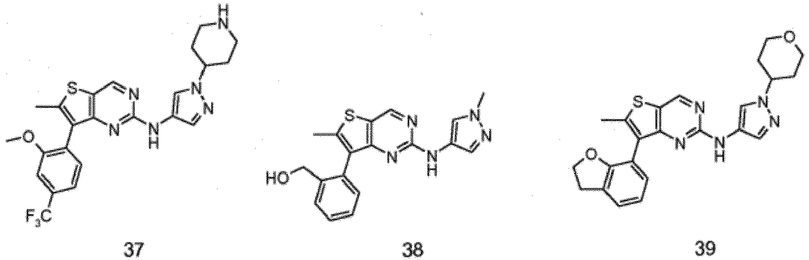

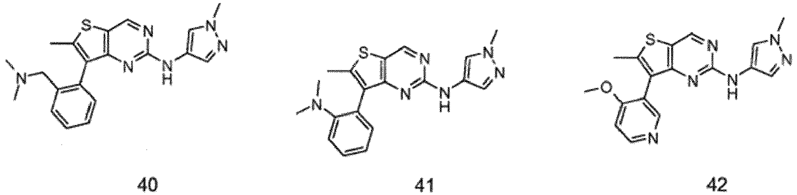

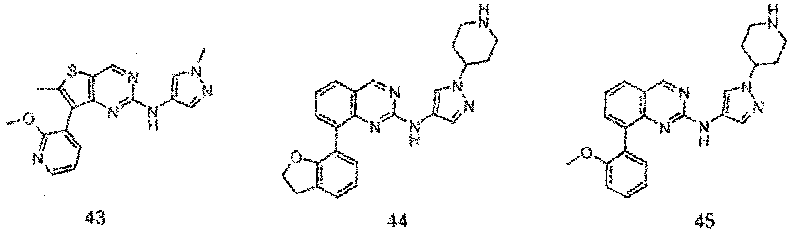

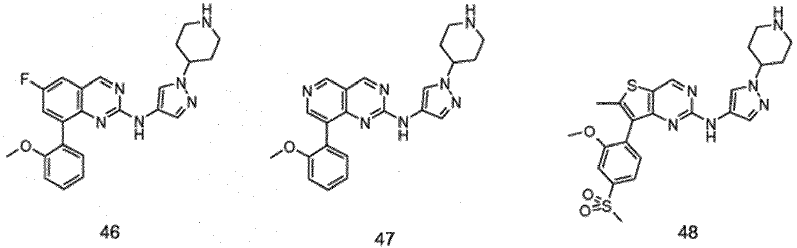

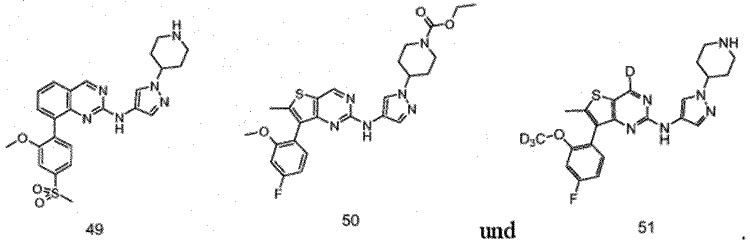

9. Verfahren zur Herstellung der Pyrimidinverbindung mit anelliertem Ring nach einem der Ansprüche 1-8, wobei es sich um eines der Verfahren 1-13 handelt,
Verfahren 1 die Durchführung einer Substitutionsreaktion mit Verbindung **1-a** und einem Methylierungsreagenz in einem organischen Lösungsmittel und in Gegenwart einer Base zum Erhalt von Verbindung **I** umfasst; 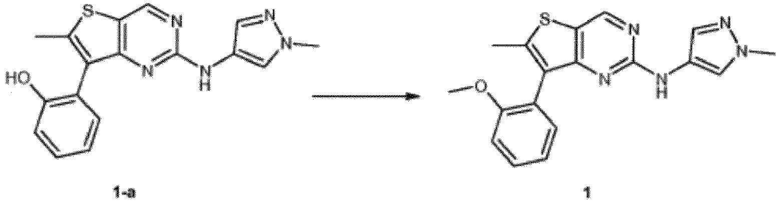
Verfahren 2 die Durchführung einer Substitutionsreaktion mit Verbindung II und Verbindung VI in einem organischen Lösungsmittel und in Gegenwart eines Katalysators zum Erhalt von Verbindung **I** umfasst; 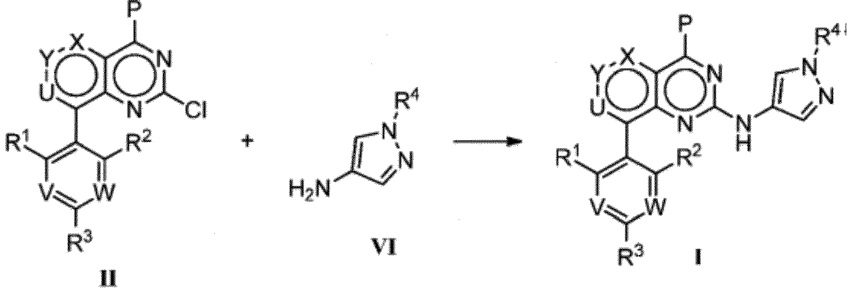
Verfahren 3 die Durchführung einer Kupplungsreaktion mit Verbindung III und Verbindung VII in einem organischen Lösungsmittel und Wasser und in Gegenwart einer Base und eines Palladiumkatalysators zum Erhalt von Verbindung **I** umfasst; wobei A für Br oder I steht; 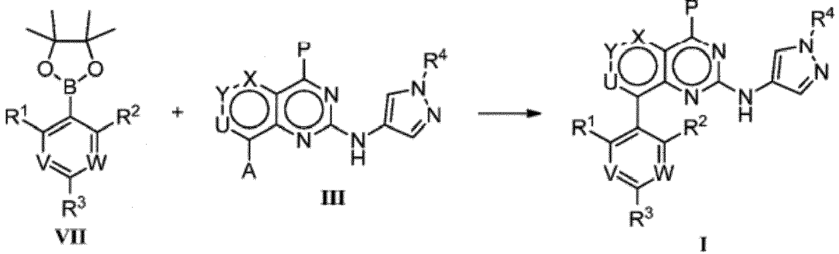
Verfahren 4 die Durchführung einer Substitutionsreaktion mit Verbindung **9-a** und 2-(4-Piperidyl)-2-propanol in einem organischen Lösungsmittel und in Gegenwart einer Base zum Erhalt von Verbindung **9** umfasst; 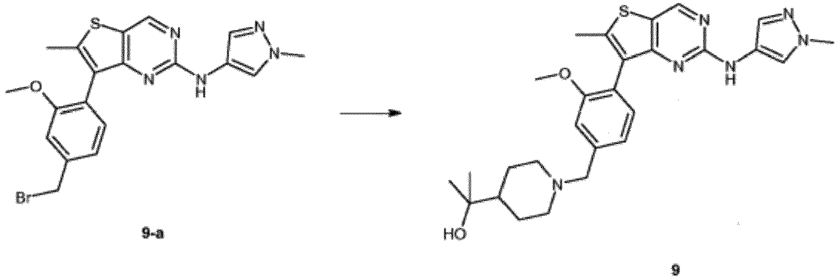
Verfahren 5 die Durchführung einer Substitutionsreaktion mit Verbindung **17-a** und Morpholin in einem organischen Lösungsmittel und in Gegenwart einer Base zum Erhalt von Verbindung **17** umfasst; 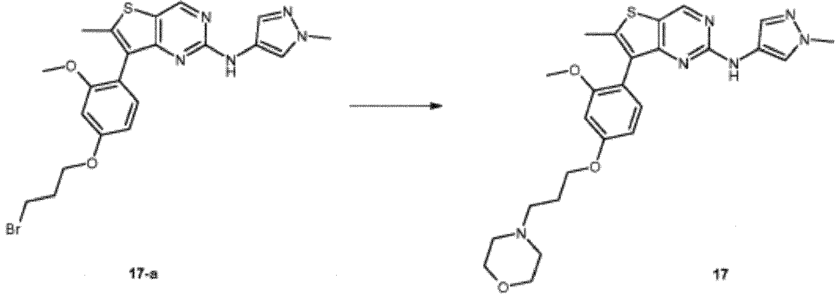
Verfahren 6 die Durchführung einer Substitutionsreaktion mit Verbindung **17-a** und Pyrrolidin in einem organischen Lösungsmittel und in Gegenwart einer Base zum Erhalt von Verbindung **18** umfasst; 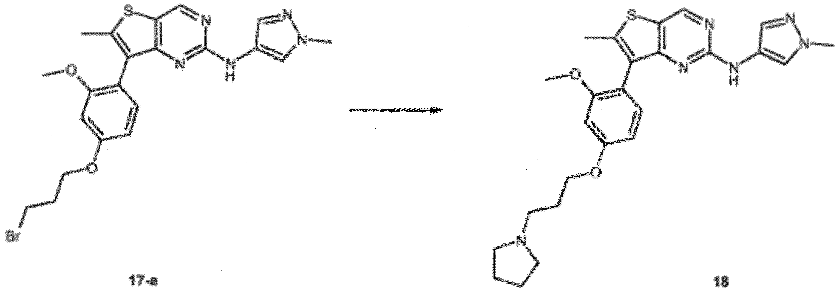
Verfahren 7 die Durchführung einer Substitutionsreaktion mit Verbindung **17-a** und N-Methylpiperazin in einem organischen Lösungsmittel und in Gegenwart einer Base zum Erhalt von Verbindung **19** umfasst; 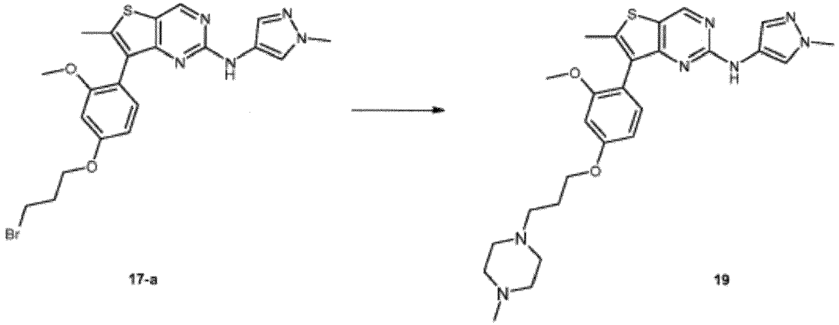
Verfahren 8 die Durchführung einer Kondensationsreaktion mit Verbindung **23-b** und Azetidin in einem organischen Lösungsmittel und in Gegenwart einer Base und in Gegenwart von N-Hydroxybenzotriazol und 1-Ethyl-(3-dimethylaminopropyl)carbodiimidhydrochlorid zum Erhalt von Verbindung **23** umfasst; 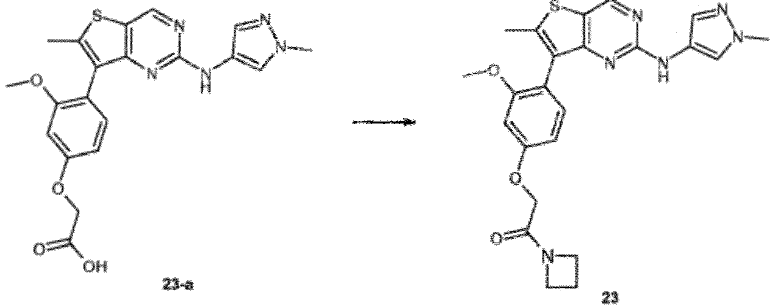
Verfahren 9 die Entschützung von Verbindung **IV** in einem organischen Lösungsmittel und in Gegenwart einer Säure zum Erhalt von Verbindung **I** umfasst; wobei R⁴ für 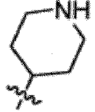 steht; 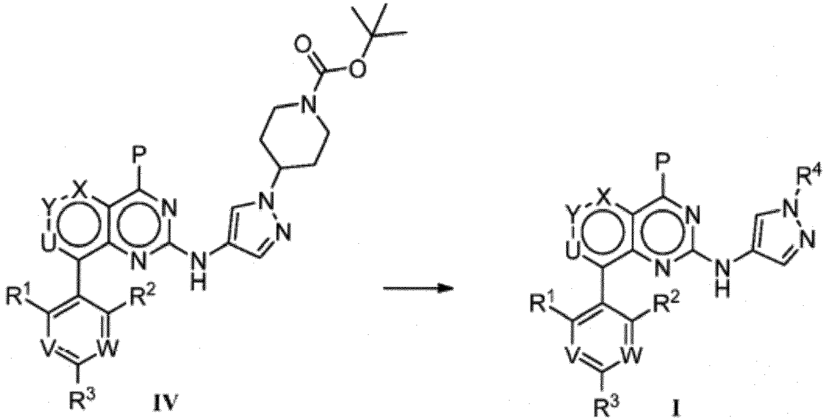
Verfahren 10 die Durchführung einer Substitutionsreaktion mit Verbindung **31** und 2-Halogenethanol in einem organischen Lösungsmittel und in Gegenwart einer Base zum Erhalt von Verbindung **34** umfasst; 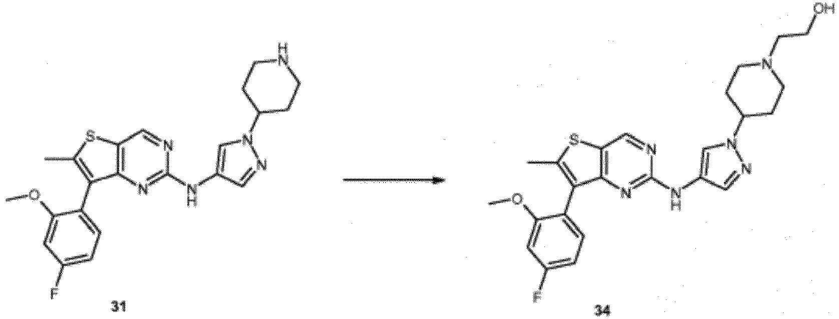
Verfahren 11 die Durchführung einer Kondensationsreaktion mit Verbindung **32** und 2-Hydroxyessigsäure in einem organischen Lösungsmittel und in Gegenwart einer Base und in Gegenwart von 1-Hydroxybenzotriazol und 1-Ethyl-(3-dimethylaminopropyl)carbodiimidhydrochlorid zum Erhalt von Verbindung **36** umfasst; 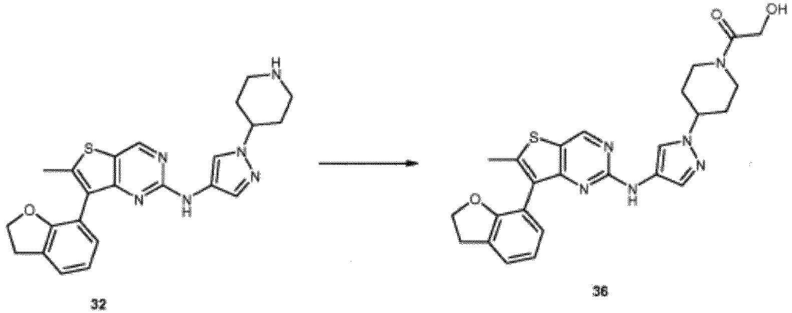
Verfahren 12 die Durchführung einer Reduktions-Aminierungs-Reaktion mit Verbindung **40-a,** Dimethylamin und Natriumtriacetoxyborhydrid in einem organischen Lösungsmittel und in Gegenwart einer Säure zum Erhalt von Verbindung **40** umfasst; 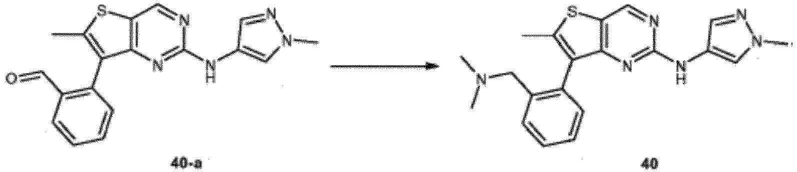
Verfahren 13 die Durchführung einer Kondensationsreaktion mit Verbindung **31** und Chlorameisensäureethylester in einem organischen Lösungsmittel und in Gegenwart einer Base zum Erhalt von Verbindung **50** umfasst;

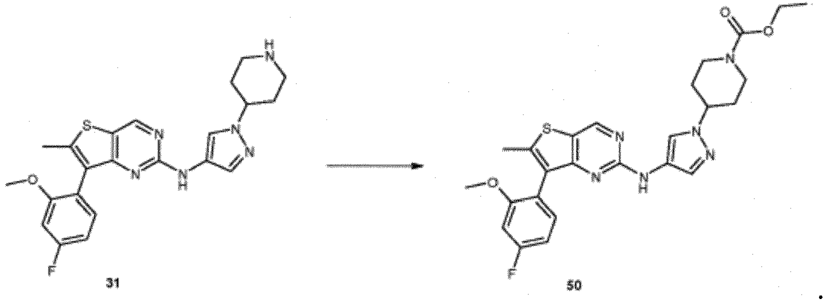

10. Verbindung mit einer Struktur der Formel III oder der Formel IV, 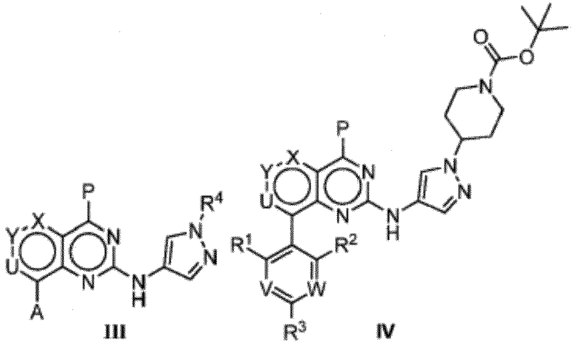
wobei R¹, R², R³, X, Y, U, P, V und W jeweils wie in einem der Ansprüche 1-8 definiert sind;
wobei A für Br oder I steht; R⁴ wie in einem der Ansprüche 1-8 definiert ist;
oder Verbindung, die ausgewählt ist aus der Gruppe bestehend aus

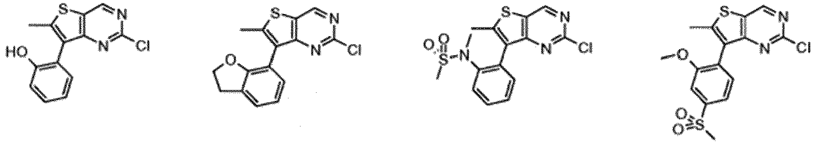

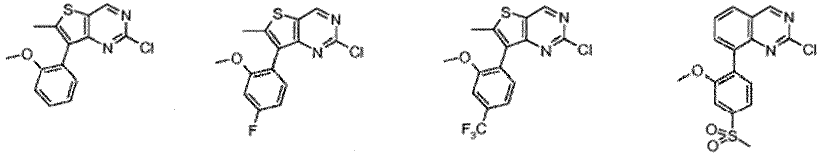

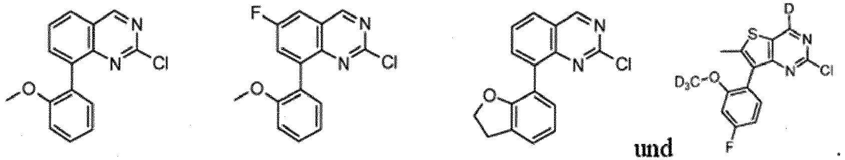

11. Verbindung nach Anspruch 10, wobei die Verbindung mit einer Struktur der Formel III ausgewählt ist aus der Gruppe bestehend aus 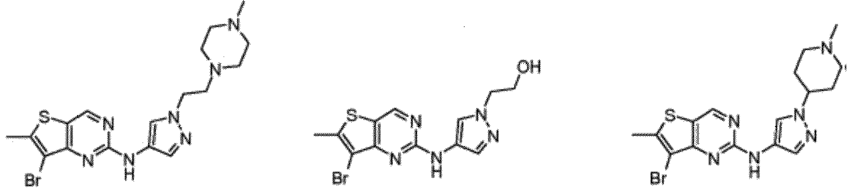 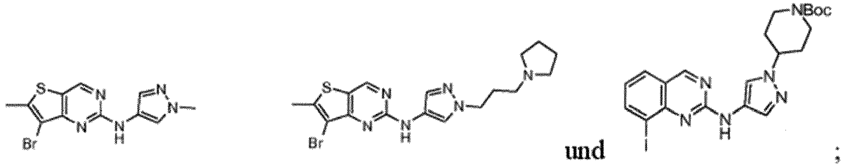 die Verbindung mit einer Struktur der Formel IV ausgewählt ist aus der Gruppe bestehend aus

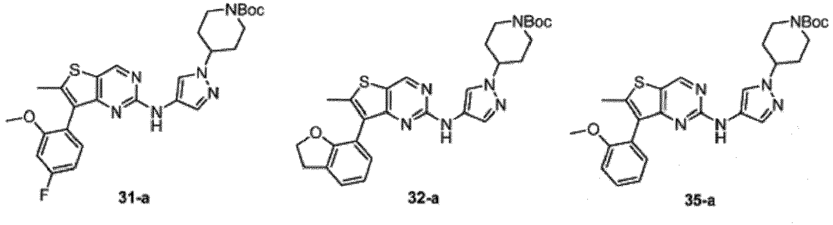

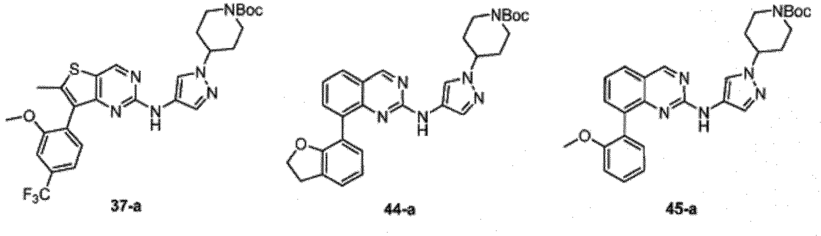

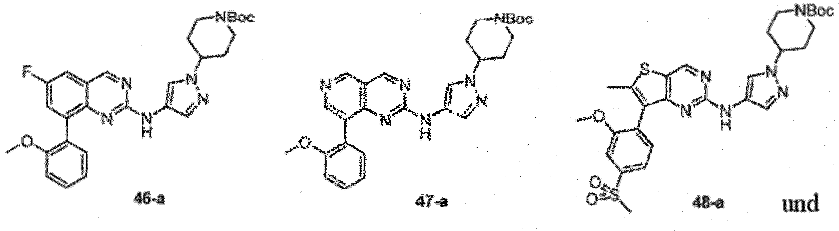

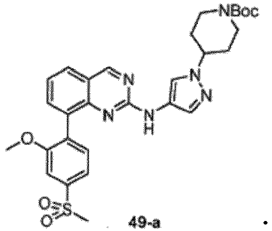

12. Verbindung **V,** die ausgewählt ist aus der Gruppe bestehend aus

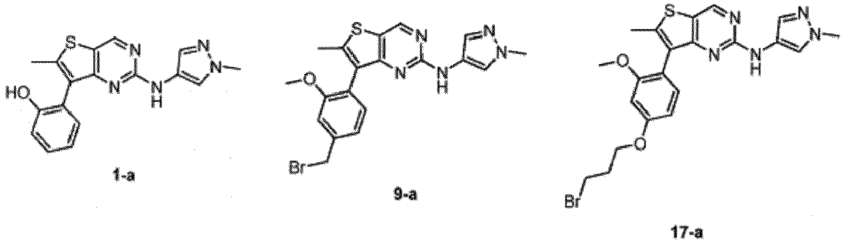

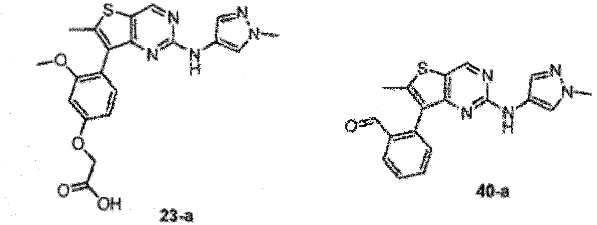

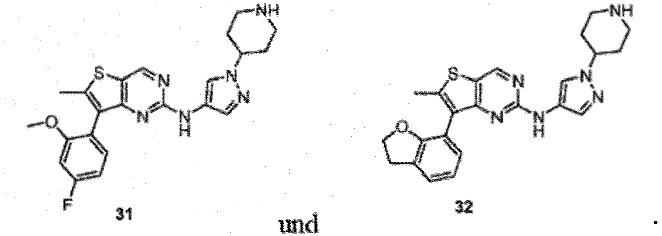

13. Pyrimidinverbindung mit anelliertem Ring, Tautomer, Enantiomer, Diastereoisomer oder pharmazeutisch annehmbares Salz davon nach einem der Ansprüche 1-8 zur Verwendung bei der Prävention, Linderung oder Behandlung der Erkrankungen aus der Gruppe bestehend aus Immunsystemerkrankung, Autoimmunerkrankung, Zellproliferationserkrankung, allergischer Störung und Herz-Kreislauf-Erkrankung.

14. Pyrimidinverbindung mit anelliertem Ring, Tautomer, Enantiomer, Diastereoisomer oder pharmazeutisch annehmbares Salz davon nach Anspruch 13, wobei es sich bei der Immunsystemerkrankung um Organtransplantatabstoßung handelt;
und/oder die Autoimmunerkrankung aus der Gruppe bestehend aus rheumatoider Arthritis, Psoriasis, Morbus Crohn und multipler Sklerose ausgewählt ist; und/oder die Zellproliferationserkrankung aus der Gruppe bestehend aus Myelofibrose, hämatologischem Tumor und solidem Tumor ausgewählt ist; wobei es sich bei dem hämatologischen Tumor vorzugsweise um Leukämie und/oder Lymphom handelt; der solide Tumor aus der Gruppe bestehend aus Nierenkrebs, Leberkrebs, Magenkrebs, Lungenkrebs, Brustkrebs, Prostatakrebs, Bauchspeicheldrüsenkrebs, Schilddrüsenkrebs, Eierstockkrebs, Glioblastom, Hautkrebs und Melanom ausgewählt ist;
und/oder es sich bei der allergischen Störung um Bronchialasthma handelt;
und/oder die Herz-Kreislauf-Erkrankung aus der Gruppe bestehend aus ischämischer Kardiomyopathie, Herzversagen und Myokardinfarkt ausgewählt ist.

15. Pharmazeutische Zusammensetzung, umfassend die Pyrimidinverbindung mit anelliertem Ring, das Tautomer, das Enantiomer, das Diastereoisomer oder das pharmazeutisch annehmbare Salz davon nach einem der Ansprüche 1-8 und einen oder mehr als einen pharmazeutisch annehmbaren Träger und/oder Verdünnungsmittel.

16. Pharmazeutische Zusammensetzung nach Anspruch 15, wobei es sich bei der Dosis der Pyrimidinverbindung mit anelliertem Ring, des Tautomers, des Enantiomers, des Diastereoisomers oder des pharmazeutisch annehmbaren Salzes davon um eine therapeutisch wirksame Menge handelt;
und/oder die pharmazeutische Zusammensetzung in Kombination mit einem oder mehr als einem klinisch verwendeten Chemotherapeutikum verwendet wird, wobei es sich bei der Dosierung vorzugsweise um eine liposomale Dosierung handelt.

## Revendications

1. Composé de pyrimidine à cycles condensés de formule I, tautomère, énantiomère, diastéréoisomère ou sel pharmaceutiquement acceptable de celui-ci ; 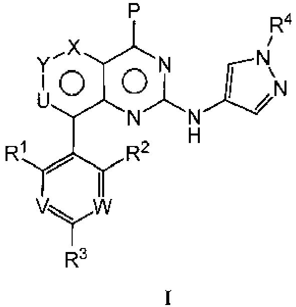
dans lequel, P est choisi parmi un hydrogène ou un deutérium ;
X est choisi parmi CH ou S ;
Y est choisi parmi N ou CR⁵ ;
U est choisi parmi une liaison chimique ou CH ;
V est choisi parmi N ou CH ;
W est choisi parmi N ou CR⁶ ;
chacun de R¹, R², R³ et R⁶ est indépendamment choisi dans le groupe constitué d'un hydrogène, un deutérium, un halogène, un alkyle substitué ou non substitué, 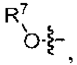 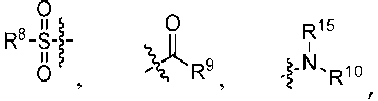 un cycloalkyle ou un hétérocycloalkyle ; chacun de R⁷, R⁸, R⁹, R¹⁰ et R¹⁵ est indépendamment choisi dans le groupe constitué d'un hydrogène, un deutérium, un halogène, un hydroxyle, un amino, un alkyle substitué ou non substitué, un alcoxy, 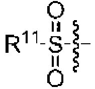 ou un hétérocycloalkyle ; R¹¹ est un hydrogène, un deutérium ou un alkyle ; ou R⁶, R² et les deux atomes sur le cycle auxquels ils sont liés forment un « hétérocycle de 5 à 7 chaînons de carbone substitué ou non substitué » ; ou, R⁶, R³ et les deux atomes sur le cycle auxquels ils sont liés forment un « hétérocycle de 5 à 7 chaînons de carbone substitué ou non substitué » ; l'hétéroatome dans un « hétérocycle de 5 à 7 chaînons de carbone substitué ou non substitué » est choisi dans le groupe constitué d'azote, oxygène et soufre ; R⁴ est un hydrogène, un deutérium, un alkyle substitué ou non substitué, un alcoxy, un cycloalkyle, ou un hétérocycloalkyle substitué ou non substitué ; R⁵ est un hydrogène, un deutérium, un halogène, ou un alkyle ;
dans les définitions de R¹, R², R³ et R⁶, le « substitué » dans « un alkyle substitué ou non substitué » signifie être substitué par les substituants choisis dans le groupe constitué d'un halogène, un hydroxyle, un amino, un alkyle, un alcoxy, 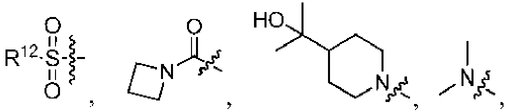 et un hétérocycloalkyle, dans le cas où des substituants multiples sont présents, les substituants sont identiques ou différents ; R¹² est un hydrogène, un deutérium ou un alkyle ;
dans les définitions de R⁷, R⁸, R⁹, R¹⁰ et R¹⁵, le « substitué » dans « un alkyle substitué ou non substitué » signifie être substitué par les substituants choisis dans le groupe constitué d'un deutérium, un halogène, un hydroxyle, un amino, un alkyle, un alcoxy, 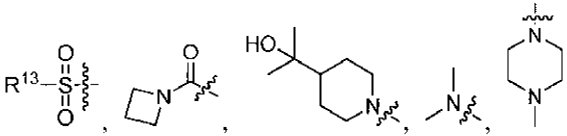 et un hétérocycloalkyle, dans le cas où des substituants multiples sont présents, les substituants sont identiques ou différents ; R¹³ est un hydrogène ou un alkyle ; dans la définition de R⁴, le « substitué » dans « un alkyle substitué ou non substitué » « un hétérocycloalkyle substitué ou non substitué » signifie être substitué par les substituants choisis dans le groupe constitué d'un hydroxyle, un alkyle, 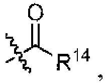 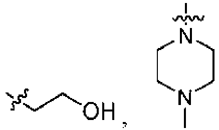 et un hétérocycloalkyle, dans le cas où des substituants multiples sont présents, les substituants sont identiques ou différents ; R¹⁴ est un hydrogène, un alkyle, un hydroxyméthyle ou un alcoxy ; le « substitué » dans « hétérocycle de 5 à 7 chaînons de carbone substitué ou non substitué » signifie être substitué par un ou plusieurs alkyles.

2. Composé de pyrimidine à cycles condensés, tautomère, énantiomère, diastéréoisomère ou sel pharmaceutiquement acceptable de celui-ci selon la revendication 1,
dans lequel, dans le cas où chacun de R¹, R², R³ et R⁶ est indépendamment un halogène, l'halogène est fluor ou chlore ;
et/ou, dans le cas où chacun de R¹, R², R³ et R⁶ est indépendamment « un alkyle substitué ou non substitué », l'alkyle est un alkyle en C₁₋₄ ;
et/ou, dans le cas où chacun de R¹, R², R³ et R⁶ est indépendamment un hétérocycloalkyle, l'hétérocycloalkyle est lié à d'autres groupes par l'intermédiaire d'un atome de carbone ou d'un hétéroatome de celui-ci ;
et/ou, dans le cas où chacun de R¹, R², R³ et R⁶ est indépendamment un hétérocycloalkyle, l'hétérocycloalkyle est « un hétérocycloalkyle avec 1 à 4 hétéroatomes et 3 à 8 atomes de carbone dans lequel l'hétéroatome est oxygène et/ou azote » ;
et/ou, dans le cas où chacun de R⁷, R⁸, R⁹, R¹⁰ et R¹⁵ est indépendamment un halogène, l'halogène est fluor ; et/ou, dans le cas où chacun de R⁷, R⁸, R⁹, R¹⁰ et R¹⁵ est indépendamment « un alkyle substitué ou non substitué », l'alkyle est un alkyle en C₁₋₁₀ ;
et/ou, dans le cas où chacun de R⁷, R⁸, R⁹, R¹⁰ et R¹⁵ est indépendamment un alcoxy, l'alcoxy est un alcoxy en C₁₋₁₀ ;
et/ou, dans le cas où chacun de R⁷, R⁸, R⁹, R¹⁰ et R¹⁵ est indépendamment un hétérocycloalkyle, l'hétérocycloalkyle est lié à d'autres groupes par l'intermédiaire d'un atome de carbone ou d'un hétéroatome de celui-ci ;
et/ou, dans le cas où chacun de R⁷, R⁸, R⁹, R¹⁰ et R¹⁵ est indépendamment un hétérocycloalkyle, l'hétérocycloalkyle est « un hétérocycloalkyle avec 1 à 4 hétéroatomes et 3 à 8 atomes de carbone dans lequel l'hétéroatome est oxygène et/ou azote » ;
et/ou, dans le cas où chacun de R⁷, R⁸, R⁹, R¹⁰ et R¹⁵ est indépendamment 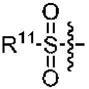 dans lequel R¹¹ est un alkyle en C₁₋₄ ;
et/ou, dans le cas où R⁴ est « un alkyle substitué ou non substitué », l'alkyle est un alkyle en C₁₋₄ ;
et/ou, dans le cas où R⁴ est un alcoxy, l'alcoxy est un alcoxy en C₁₋₄ ;
et/ou, dans le cas où R⁴ est « un hétérocycloalkyle substitué ou non substitué », l'hétérocycloalkyle est lié à d'autres groupes par l'intermédiaire d'un atome de carbone ou d'un hétéroatome de celui-ci ;
et/ou, dans le cas où R⁴ est « un hétérocycloalkyle substitué ou non substitué », l'hétérocycloalkyle est « un hétérocycloalkyle avec 1 à 4 hétéroatomes et 3 à 8 atomes de carbone dans lequel l'hétéroatome est oxygène et/ou azote » ;
et/ou, dans le cas où R⁵ est un halogène, l'halogène est fluor ;
et/ou, dans le cas où R⁵ est un alkyle, l'alkyle est un alkyle en C₁₋₄ ;
et/ou, dans le cas où « l'hétérocycle carboné de 5 à 7 chaînons » dans « un hétérocycle carboné de 5 à 7 chaînons substitué ou non substitué » est « un hétérocycle carboné avec 1 à 4 hétéroatomes et 2 à 6 atomes de carbone dans lequel l'hétéroatome est oxygène et/ou azote » ;
et/ou, dans le cas où chacun de R¹, R², R³ et R⁶ est indépendamment « un alkyle substitué ou non substitué », le substituant dans « un alkyle substitué ou non substitué » est fluor ;
et/ou, dans le cas où chacun de R¹, R², R³ et R⁶ est indépendamment « un alkyle substitué ou non substitué », le substituant dans « un alkyle substitué ou non substitué » est un alkyle en C₁₋₁₀ ;
et/ou, dans le cas où chacun de R¹, R², R³ et R⁶ est indépendamment « un alkyle substitué ou non substitué », le substituant dans « un alkyle substitué ou non substitué » est un alcoxy en C₁₋₁₀ ;
et/ou, dans le cas où chacun de R¹, R², R³ et R⁶ est indépendamment « un alkyle substitué ou non substitué » et le substituant dans « un alkyle substitué ou non substitué » est un hétérocycloalkyle, l'hétérocycloalkyle est lié à d'autres groupes par l'intermédiaire d'un atome de carbone ou d'un hétéroatome de celui-ci ;
et/ou, dans le cas où chacun de R¹, R², R³ et R⁶ est indépendamment « un alkyle substitué ou non substitué » et le substituant dans « un alkyle substitué ou non substitué » est « un hétérocycloalkyle avec 1 à 4 hétéroatomes et 3 à 8 atomes de carbone dans lequel l'hétéroatome est oxygène et/ou azote » ;
et/ou, dans le cas où chacun de R¹, R², R³ et R⁶ est indépendamment « un alkyle substitué ou non substitué », le substituant dans « un alkyle substitué ou non substitué » est 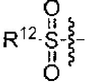 dans lequel R¹² est un alkyle en C₁₋₄ ;
et/ou, dans le cas où chacun de R⁷, R⁸, R⁹, R¹⁰ et R¹⁵ est indépendamment « un alkyle substitué ou non substitué », le substituant dans « un alkyle substitué ou non substitué » est fluor ;
et/ou, dans le cas où chacun de R⁷, R⁸, R⁹, R¹⁰ et R¹⁵ est indépendamment « un alkyle substitué ou non substitué », le substituant dans « un alkyle substitué ou non substitué » est un alkyle en C₁₋₁₀ ;
et/ou, dans le cas où chacun de R⁷, R⁸, R⁹, R¹⁰ et R¹⁵ est indépendamment « un alkyle substitué ou non substitué », le substituant dans « un alkyle substitué ou non substitué » est un alcoxy en C₁₋₁₀ ;
et/ou, dans le cas où chacun de R⁷, R⁸, R⁹, R¹⁰ et R¹⁵ est indépendamment « un alkyle substitué ou non substitué », le substituant dans « un alkyle substitué ou non substitué » est un hétérocycloalkyle, l'hétérocycloalkyle est lié à d'autres groupes par l'intermédiaire d'un atome de carbone ou d'un hétéroatome de celui-ci ;
et/ou, dans le cas où chacun de R⁷, R⁸, R⁹, R¹⁰ et R¹⁵ est indépendamment « un alkyle substitué ou non substitué », le substituant dans « un alkyle substitué ou non substitué » est « un hétérocycloalkyle avec 1 à 4 hétéroatomes et 3 à 8 atomes de carbone dans lequel l'hétéroatome est oxygène et/ou azote » ;
et/ou, dans le cas où chacun de R⁷, R⁸, R⁹, R¹⁰ et R¹⁵ est indépendamment « un alkyle substitué ou non substitué », le substituant dans « un alkyle substitué ou non substitué » est 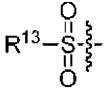 dans lequel R¹³ est un alkyle en C₁₋₄ ;
et/ou, dans le cas où R⁴ est « un alkyle substitué ou non substitué » ou « un hétérocycloalkyle substitué ou non substitué », le substituant dans « un alkyle substitué ou non substitué » ou « un hétérocycloalkyle substitué ou non substitué » est un alkyle en C₁₋₄ ;
et/ou, dans le cas où R⁴ est « un alkyle substitué ou non substitué » ou « un hétérocycloalkyle substitué ou non substitué », le substituant dans « un alkyle substitué ou non substitué » ou « un hétérocycloalkyle substitué ou non substitué » est un hétérocycloalkyle, l'hétérocycloalkyle est lié à d'autres groupes par l'intermédiaire d'un atome de carbone ou d'un hétéroatome de celui-ci ;
et/ou, dans le cas où R⁴ est « un alkyle substitué ou non substitué » ou « un hétérocycloalkyle substitué ou non substitué », le substituant dans « un alkyle substitué ou non substitué » ou « un hétérocycloalkyle substitué ou non substitué » est « un hétérocycloalkyle avec 1 à 4 hétéroatomes et 3 à 6 atomes de carbone dans lequel l'hétéroatome est oxygène et/ou azote » ;
et/ou, dans le cas où R⁴ est « un alkyle substitué ou non substitué » ou « un hétérocycloalkyle substitué ou non substitué », le substituant dans « un alkyle substitué ou non substitué » ou « un hétérocycloalkyle substitué ou non substitué » est 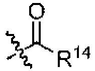 dans lequel R¹⁴ est un alkyle en C₁₋₄ ;
et/ou, dans le cas où R⁴ est « un alkyle substitué ou non substitué » ou « un hétérocycloalkyle substitué ou non substitué », le substituant dans « un alkyle substitué ou non substitué » ou « un hétérocycloalkyle substitué ou non substitué » est 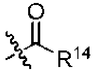 dans lequel R¹⁴ est un alcoxy en C₁₋₄ ;
et/ou, dans le cas où le substituant dans « un hétérocycle carboné de 5 à 7 chaînons substitué et non substitué » est un alkyle en C₁₋₄.

3. Composé de pyrimidine à cycles condensés, tautomère, énantiomère, diastéréoisomère ou sel pharmaceutiquement acceptable de celui-ci selon la revendication 2, dans lequel, dans le cas où chacun de R¹, R², R³ et R⁶ est indépendamment « un alkyle substitué ou non substitué », l'alkyle est un méthyle ;
et/ou, dans le cas où chacun de R¹, R², R³ et R⁶ est indépendamment un hétérocycloalkyle, l'hétérocycloalkyle est « un hétérocycloalkyle avec 1 à 4 hétéroatomes et 3 à 6 atomes de carbone dans lequel l'hétéroatome est oxygène et/ou azote » ;
et/ou, dans le cas où chacun de R⁷, R⁸, R⁹, R¹⁰ et R¹⁵ est indépendamment « un alkyle substitué ou non substitué », l'alkyle est un alkyle en C₁₋₄ ;
et/ou, dans le cas où chacun de R⁷, R⁸, R⁹, R¹⁰ et R¹⁵ est indépendamment un alcoxy, l'alcoxy est un alcoxy en C₁₋₄ ;
et/ou, dans le cas où chacun de R⁷, R⁸, R⁹, R¹⁰ et R¹⁵ est indépendamment un hétérocycloalkyle, l'hétérocycloalkyle est « un hétérocycloalkyle avec 1 à 4 hétéroatomes et 3 à 6 atomes de carbone dans lequel l'hétéroatome est oxygène et/ou azote » ;
et/ou, dans le cas où chacun de R⁷, R⁸, R⁹, R¹⁰ et R¹⁵ est indépendamment 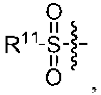 R¹¹ est un méthyle ;
et/ou, dans le cas où R⁴ est « un alkyle substitué ou non substitué », l'alkyle est un méthyle, un éthyle, un propyle ou un isopropyle ;
et/ou, dans le cas où R⁴ est « un hétérocycloalkyle substitué ou non substitué », l'hétérocycloalkyle est « un hétérocycloalkyle avec 1 à 4 hétéroatomes et 3 à 6 atomes de carbone dans lequel l'hétéroatome est oxygène et/ou azote » ;
et/ou, dans le cas où R⁵ est un alkyle, l'alkyle est un méthyle ;
et/ou, l'« hétérocycle carboné de 5 à 7 chaînons » dans un « hétérocycle carboné de 5 à 7 chaînons substitué ou
non substitué » est 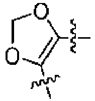 ou 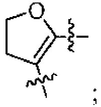 et/ou, dans le cas où chacun de R¹, R², R³ et R⁶ est indépendamment « un alkyle substitué ou non substitué », le substituant dans « un alkyle substitué ou non substitué » est un alkyle en C₁₋₄ ;
et/ou, dans le cas où chacun de R¹, R², R³ et R⁶ est indépendamment « un alkyle substitué ou non substitué », le substituant dans « un alkyle substitué ou non substitué » est un alcoxy en C₁₋₄ ;
et/ou, dans le cas où chacun de R¹, R², R³ et R⁶ est indépendamment « un alkyle substitué ou non substitué », le substituant dans « un alkyle substitué ou non substitué » est « un hétérocycloalkyle avec 1 à 4 hétéroatomes et 3 à 6 atomes de carbone dans lequel l'hétéroatome est oxygène et/ou azote » ;
et/ou, dans le cas où chacun de R¹, R², R³ et R⁶ est indépendamment « alkyle substitué ou non substitué », le substituant dans « alkyle substitué ou non substitué » est 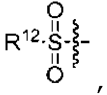 dans lequel R¹² est un méthyle ;
et/ou, dans le cas où chacun de R⁷, R⁸, R⁹, R¹⁰ et R¹⁵ est indépendamment « un alkyle substitué ou non substitué », le substituant dans « un alkyle substitué ou non substitué » est un alkyle en C₁₋₄ ;
et/ou, dans le cas où chacun de R⁷, R⁸, R⁹, R¹⁰ et R¹⁵ est indépendamment « un alkyle substitué ou non substitué », le substituant dans « un alkyle substitué ou non substitué » est un alcoxy en C₁₋₄ ;
et/ou, dans le cas où chacun de R⁷, R⁸, R⁹, R¹⁰ et R¹⁵ est indépendamment « un alkyle substitué ou non substitué », le substituant dans « un alkyle substitué ou non substitué » est « un hétérocycloalkyle avec 1 à 4 hétéroatomes et 3 à 6 atomes de carbone dans lequel l'hétéroatome est oxygène et/ou azote » ;
et/ou, dans le cas où chacun de R¹, R², R³ et R⁶ est indépendamment « un alkyle substitué ou non substitué », le substituant dans « un alkyle substitué ou non substitué » est 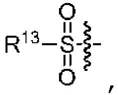 dans lequel R¹³ est un méthyle ;
et/ou, dans le cas où R⁴ est « un alkyle substitué ou non substitué » ou « un hétérocycloalkyle substitué ou non substitué », le substituant dans « un alkyle substitué ou non substitué » ou « un hétérocycloalkyle substitué ou non substitué » est un méthyle ;
et/ou, dans le cas où R⁴ est « un alkyle substitué ou non substitué » ou « un hétérocycloalkyle substitué ou non substitué », le substituant dans « un alkyle substitué ou non substitué » ou « un hétérocycloalkyle substitué
ou non substitué » est 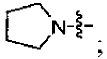 et/ou, dans le cas où R⁴ est « un alkyle substitué ou non substitué » ou « un hétérocycloalkyle substitué ou non substitué », le substituant dans « un alkyle substitué ou non substitué » ou « un hétérocycloalkyle substitué ou non substitué » est 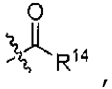 dans lequel R¹⁴ est un méthyle ;
et/ou, dans le cas où R⁴ est « un alkyle substitué ou non substitué » ou « un hétérocycloalkyle substitué ou non substitué », le substituant dans « un alkyle substitué ou non substitué » ou « un hétérocycloalkyle substitué ou non substitué » est 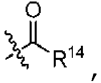 dans lequel R¹⁴ est un tert-butoxy ou un éthoxy ;
et/ou, dans le cas où le substituant alkyle dans « hétérocycle carboné de 5 à 7 chaînons substitué ou non substitué » est un méthyle, un éthyle ou un propyle.

4. Composé de pyrimidine à cycles condensés, tautomère, énantiomère, diastéréoisomère ou sel pharmaceutiquement acceptable de celui-ci selon la revendication 3, dans lequel, dans le cas où chacun de R¹, R², R³ et R⁶ est indépendamment un hétérocycloalkyle, l'hétérocycloalkyle est 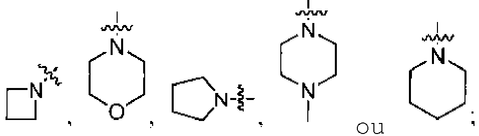 et/ou, dans le cas où chacun de R⁷, R⁸, R⁹, R¹⁰ et R¹⁵ est indépendamment « un alkyle substitué ou non substitué », l'alkyle est un méthyle, un trideutérométhyle, un éthyle, un propyle ou un isopropyle ;
et/ou, dans le cas où chacun de R⁷, R⁸, R⁹, R¹⁰ et R¹⁵ est indépendamment un alcoxy, l'alcoxy est un méthoxy ; et/ou, dans le cas où chacun de R⁷, R⁸, R⁹, R¹⁰ et R¹⁵ est indépendamment un hétérocycloalkyle, l'hétérocycloalkyle est 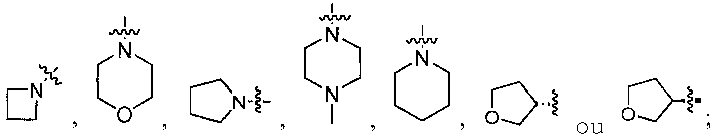 et/ou, dans le cas où R⁴ est « un hétérocycloalkyle substitué ou non substitué »,
l'hétérocycloalkyle dans « un hétérocycloalkyle substitué ou non substitué » est 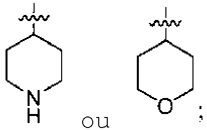 et/ou, dans le cas où chacun de R¹, R², R³ et R⁶ est indépendamment « un alkyle substitué ou non substitué », le substituant dans « un alkyle substitué ou non substitué » est un méthyle ;
et/ou, dans le cas où chacun de R¹, R², R³ et R⁶ est indépendamment « un alkyle substitué ou non substitué », le substituant dans « un alkyle substitué ou non substitué » est un méthoxy ;
et/ou, dans le cas où chacun de R¹, R², R³ et R⁶ est indépendamment « un alkyle substitué ou non substitué », le substituant dans « un alkyle substitué ou non substitué » est 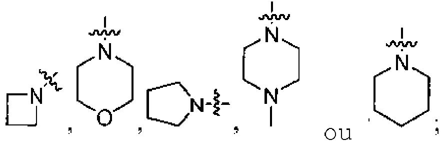 et/ou, dans le cas où chacun de R⁷, R⁸, R⁹, R¹⁰ et R¹⁵ est indépendamment « un alkyle substitué ou non substitué », le substituant dans « un alkyle substitué ou non substitué » est un méthyle ;
et/ou, dans le cas où chacun de R⁷, R⁸, R⁹, R¹⁰ et R¹⁵ est indépendamment « un alkyle substitué ou non substitué », le substituant dans « un alkyle substitué ou non substitué » est un méthoxy ;
et/ou, dans le cas où chacun de R⁷, R⁸, R⁹, R¹⁰ et R¹⁵ est indépendamment « un alkyle substitué ou non substitué », le substituant dans « un alkyle substitué ou non substitué » est

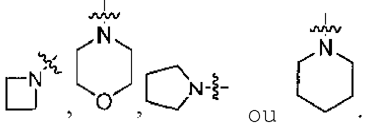

5. Composé de pyrimidine à cycles condensés, tautomère, énantiomère, diastéréoisomère ou sel pharmaceutiquement acceptable de celui-ci selon l'une quelconque des revendications 1 à 4, le composé I étant décrit dans la formule I-1 ou I-2,

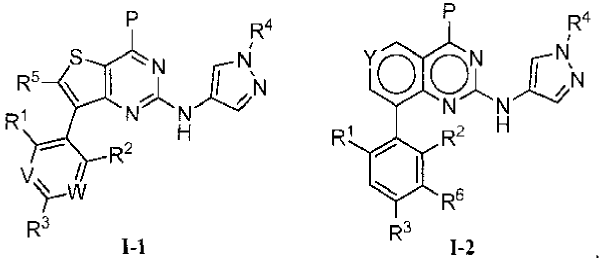

6. Composé de pyrimidine à cycles condensés, tautomère, énantiomère, diastéréoisomère ou sel pharmaceutiquement acceptable de celui-ci selon la revendication 5, dans lequel, le composé I-1 est décrit dans la formule I-1-1 ou I-1-2, 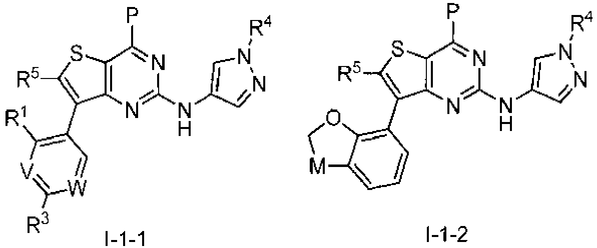 dans lequel, M est CH₂ ou O ;
le composé I-2 est décrit dans la formule I-2-1 ou I-2-2,

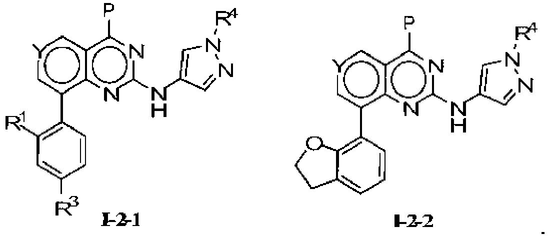

7. Composé de pyrimidine à cycles condensés, tautomère, énantiomère, diastéréoisomère ou sel pharmaceutiquement acceptable de celui-ci selon l'une quelconque des revendications 1 à 4, dans lequel, dans le composé I, Y est CR⁵ ;
et/ou, dans le composé I, R⁵ est un hydrogène ou un alkyle ;
et/ou, dans le composé I, W est CR⁶ ;
et/ou, dans le composé I, R⁶ est un hydrogène ;
et/ou, dans le composé I, R⁶ et R², conjointement avec deux atomes sur le cycle auquel ils sont liés forment « un hétérocycle carboné de 5 à 7 chaînons substitué ou non substitué » ;
et/ou, dans le composé I, chacun de R¹ et R² est indépendamment un hydrogène ou 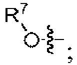 et/ou, dans le composé I, R¹ ou R² est un hydrogène ;
et/ou, dans le composé I, R³ est un hydrogène, 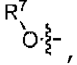 un halogène, ou 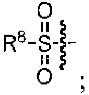 et/ou, dans le composé I, R⁴ est « un alkyle substitué ou non substitué », ou « un hétérocycloalkyle substitué ou non substitué ».

8. Composé de pyrimidine à cycles condensés, tautomère, énantiomère, diastéréoisomère ou sel pharmaceutiquement acceptable de celui-ci selon la revendication 1, qui est choisi dans le groupe constitué de

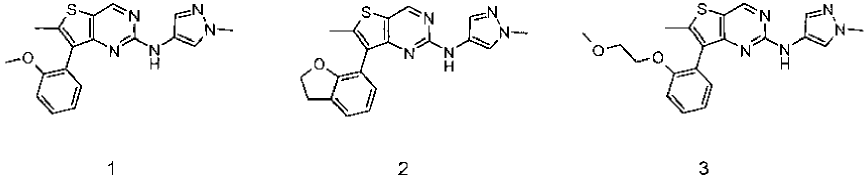

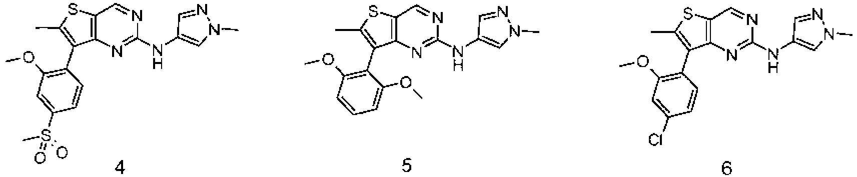

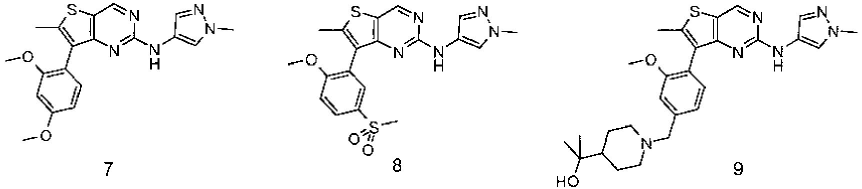

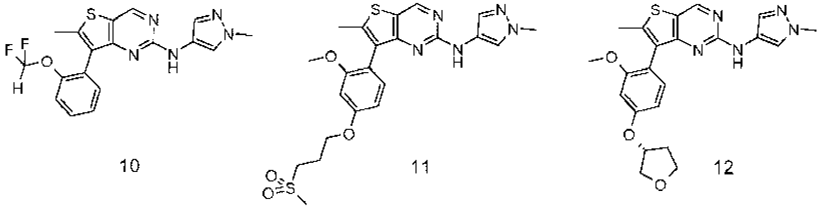

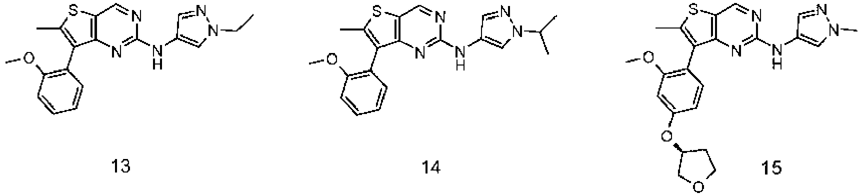

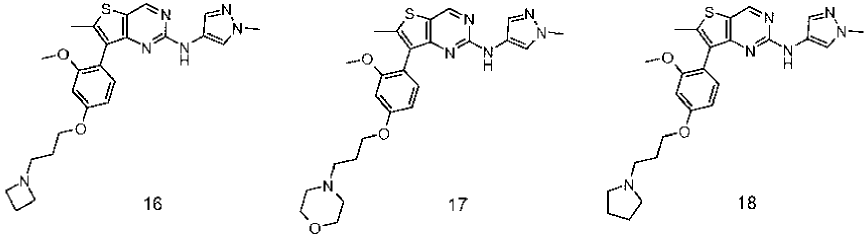

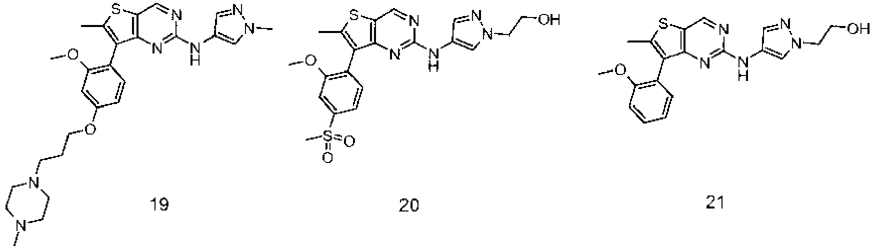

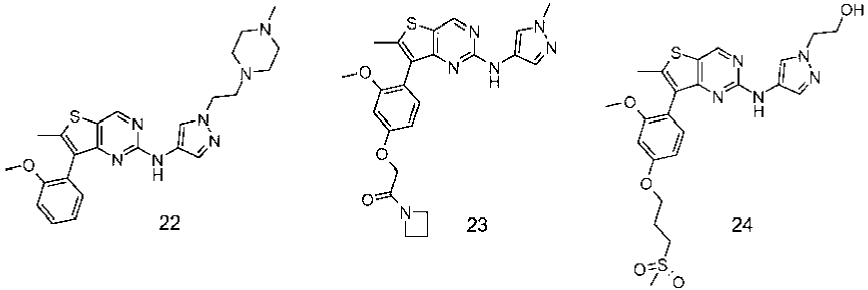

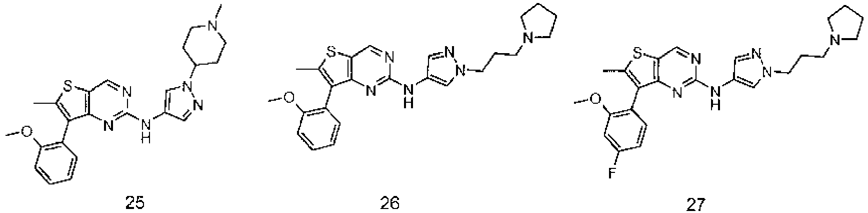

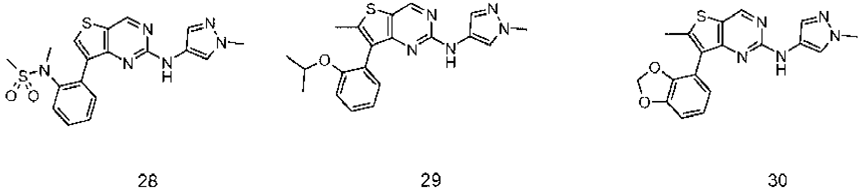

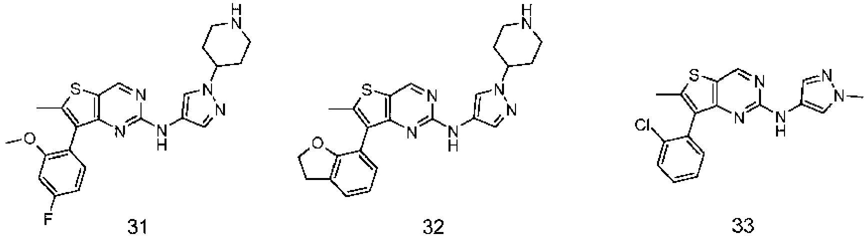

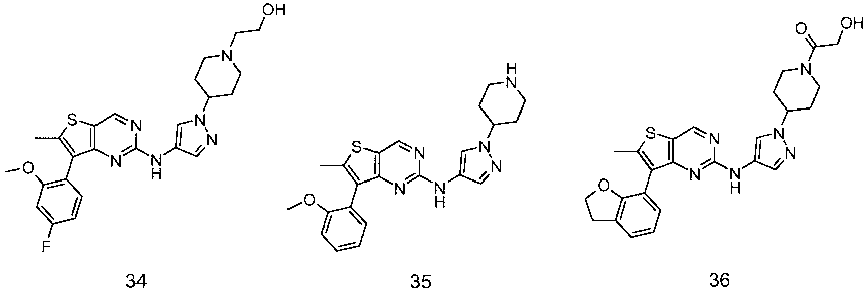

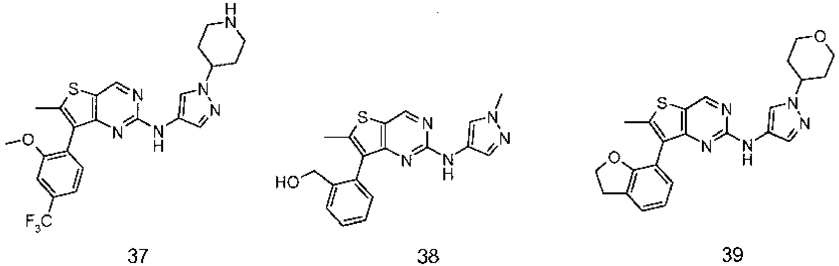

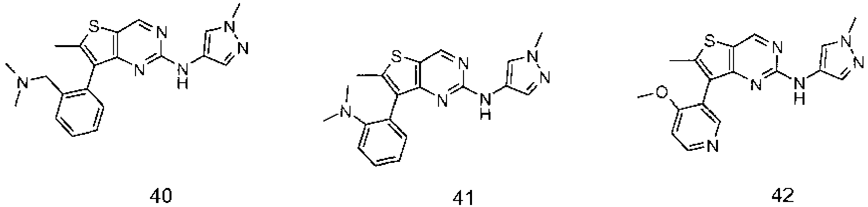

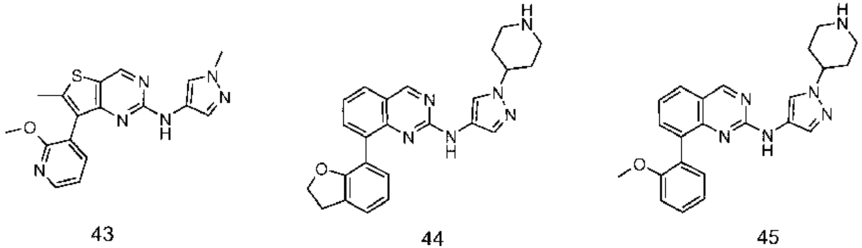

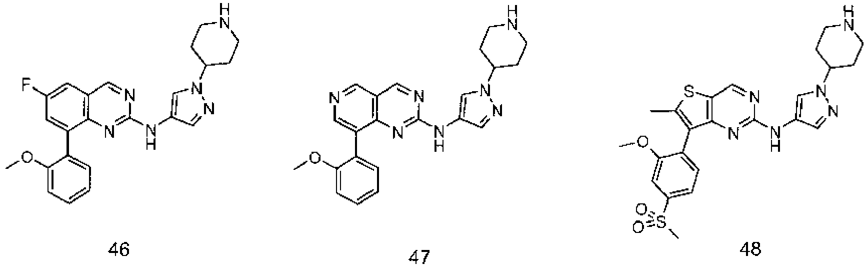

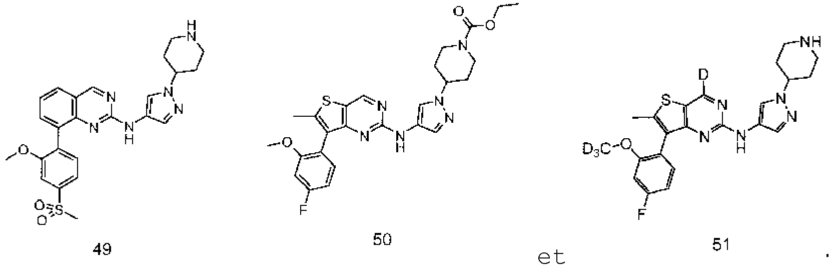

9. Procédé de préparation du composé de pyrimidine à cycles condensés selon l'une quelconque des revendications 1 à 8, qui est l'un quelconque des procédés 1 à 13,
le procédé 1 comprend la conduite d'une réaction de substitution avec le composé 1-a et un réactif de méthylation dans un solvant organique et en présence d'une base pour obtenir le composé I ; 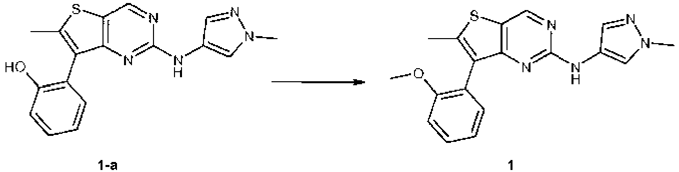
le procédé 2 comprend la conduite d'une réaction de substitution avec le composé II et le composé VI dans un solvant organique et en présence d'un catalyseur pour obtenir le composé I ; 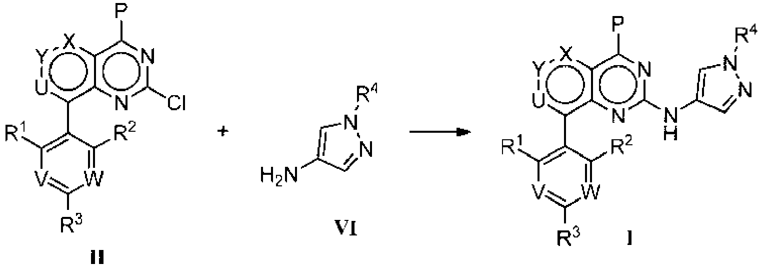
le procédé 3 comprend la conduite d'une réaction de couplage avec le composé III et le composé VII dans un solvant organique et de l'eau et en présence d'une base et d'un catalyseur de palladium pour obtenir le composé I ; dans lequel, A est Br ou I ; 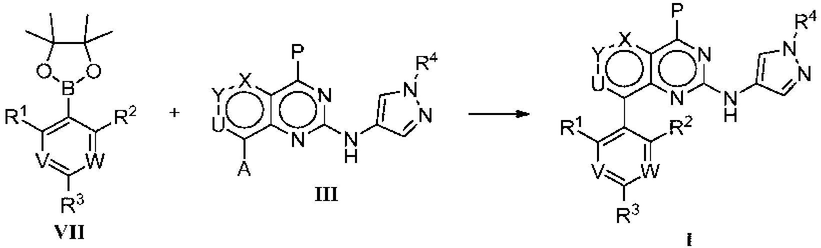
le procédé 4 comprend la conduite d'une réaction de substitution avec le composé 9-a et du 2-(4-pipéridyl)-2-propanol dans un solvant organique et en présence d'une base pour obtenir le composé 9 ; 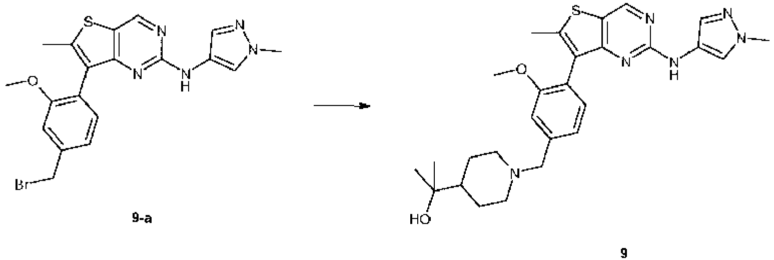
le procédé 5 comprend la conduite d'une réaction de substitution avec le composé 17-a et de la morpholine dans un solvant organique et en présence d'une base pour obtenir le composé 17 ; 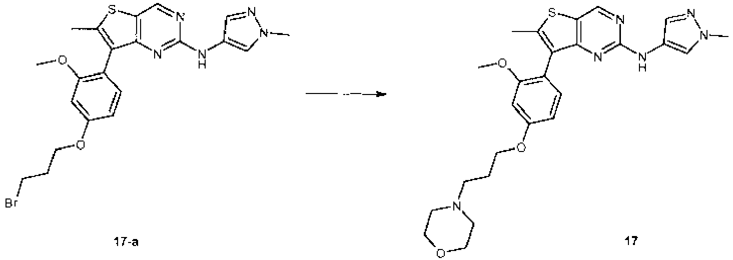
le procédé 6 comprend la conduite d'une réaction de substitution avec le composé 17-a et de la pyrrolidine dans un solvant organique et en présence d'une base pour obtenir le composé 18 ; 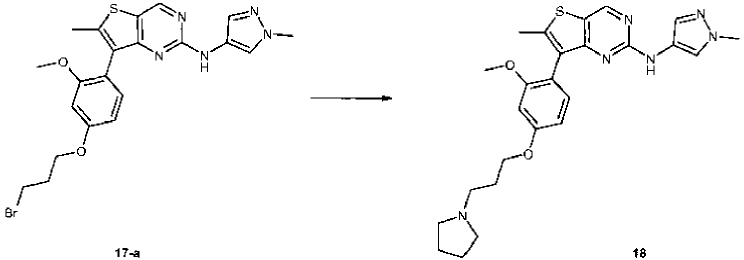
le procédé 7 comprend la conduite d'une réaction de substitution avec le composé 17-a et de la N-méthylpipérazine dans un solvant organique et en présence d'une base pour obtenir le composé 19 ; 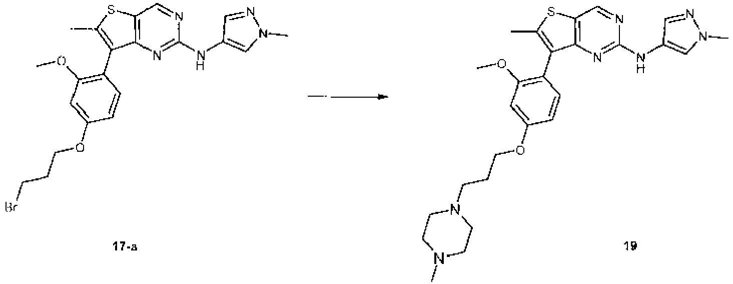
le procédé 8 comprend la conduite d'une réaction de condensation avec le composé 23-b et de l'azétidine dans un solvant organique et en présence d'une base, de N-hydroxybenzotriazole et de chlorhydrate de 1-éthyl-(3-diméthylaminopropyl)carbodiimide pour obtenir le composé 23 ; 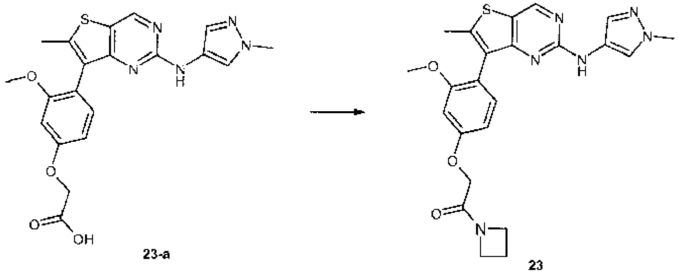
le procédé 9 comprend la déprotection du composé IV dans un solvant organique et en présence d'un acide pour obtenir le composé I ; dans lequel R⁴ est 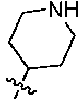 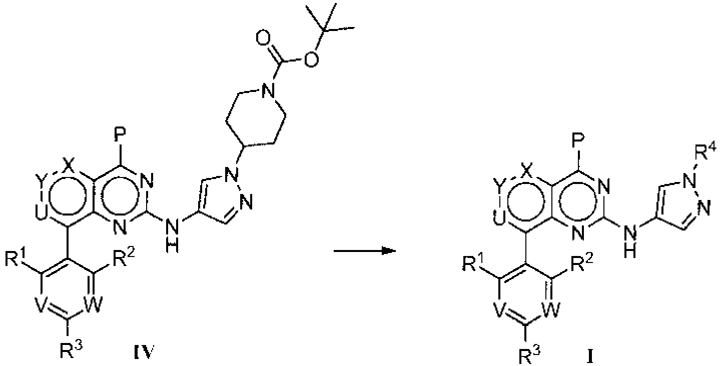
le procédé 10 comprend la conduite d'une réaction de substitution avec le composé 31 et du 2-halogénoéthanol dans un solvant organique et en présence d'une base pour obtenir le composé 34 ; 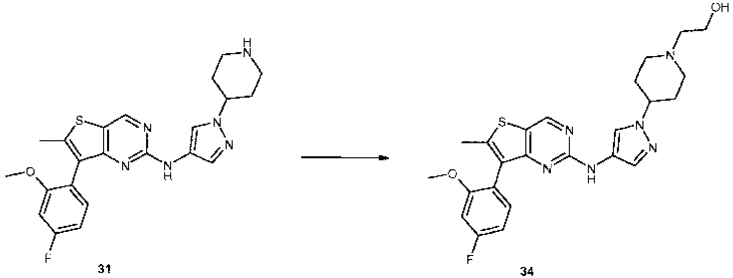
le procédé 11 comprend la conduite d'une réaction de condensation avec le composé 32 et de l'acide 2-hydroxyacétique dans un solvant organique et en présence d'une base, de 1-hydroxybenzotriazole et de chlorhydrate de 1-éthyl-(3-diméthylaminopropyl)carbodiimide pour obtenir le composé 36 ; 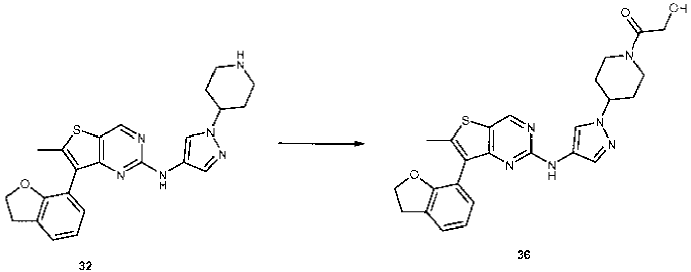
le procédé 12 comprend la conduite d'une réaction d'amination réductrice avec le composé 40-a, de la diméthylamine et du triacétoxyborohydrure de sodium dans un solvant organique et en présence d'un acide pour obtenir le composé 40 ; 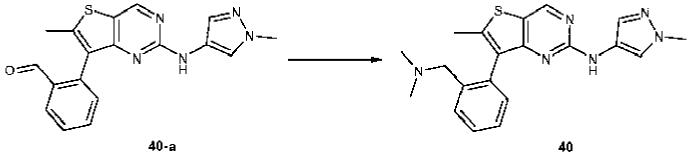
le procédé 13 comprend la conduite d'une réaction de condensation avec le composé 31 et du chloroformate d'éthyle dans un solvant organique et en présence d'une base pour obtenir le composé 50 ;

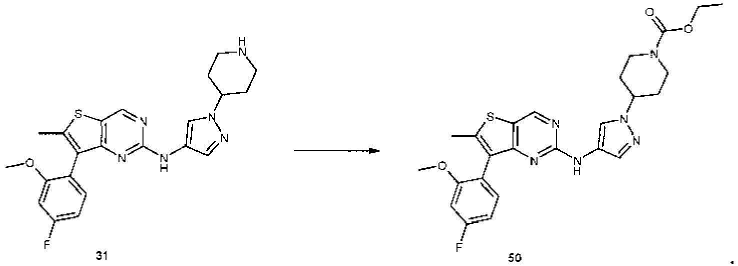

10. Composé ayant une structure de formule III ou de formule IV, 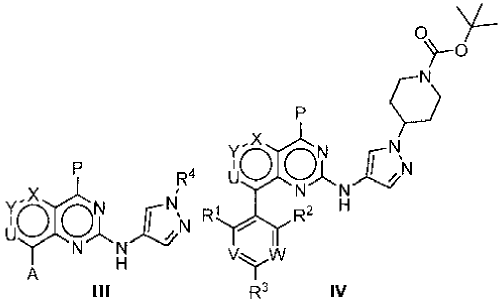
dans lequel, chacun de R¹, R², R³, X, Y, U, P, V et W est tel que défini dans l'une quelconque des revendications 1 à 8 ;
dans lequel, A est Br ou I ; R⁴ est tel que défini dans l'une quelconque des revendications 1 à 8 ;
ou un composé qui est choisi dans le groupe constitué de

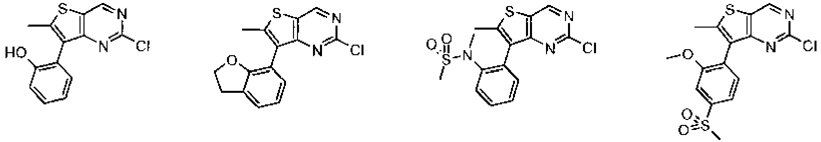

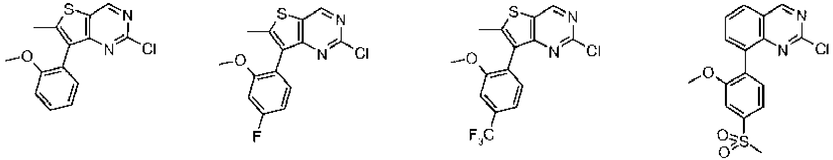

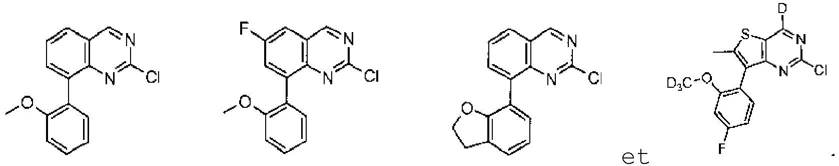

11. Composé selon la revendication 10, le composé ayant une structure de formule III étant choisi dans le groupe constitué de 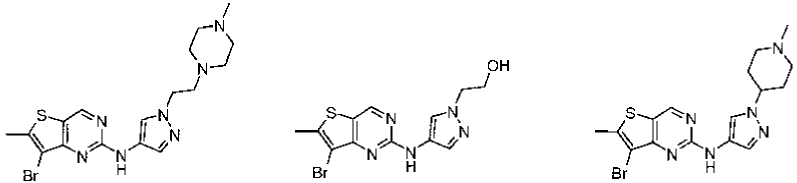 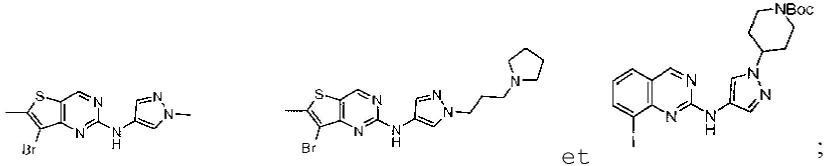 le composé ayant une structure de formule IV étant choisi dans le groupe constitué de 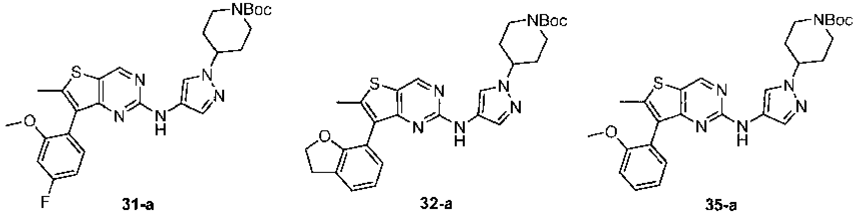 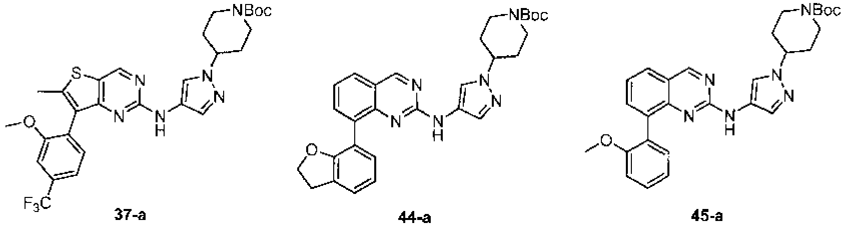 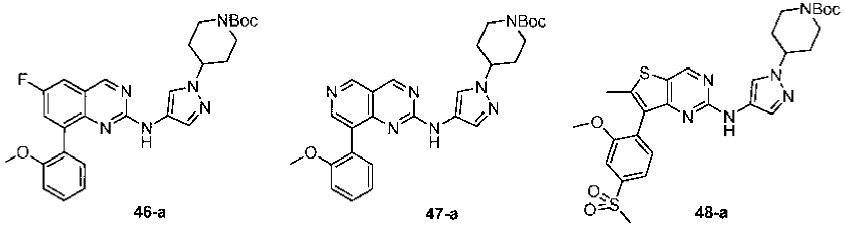 et

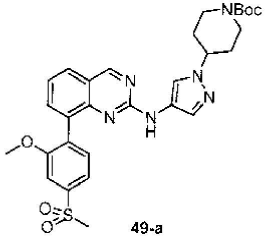

12. Composé V qui est choisi dans le groupe constitué de

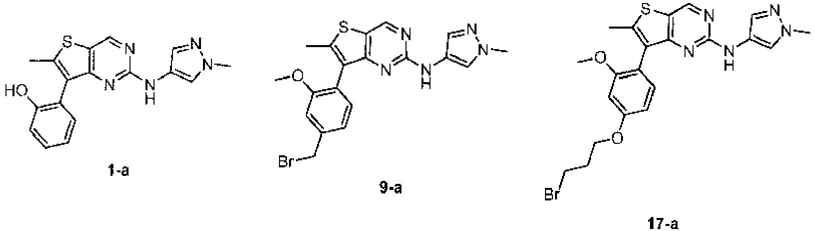

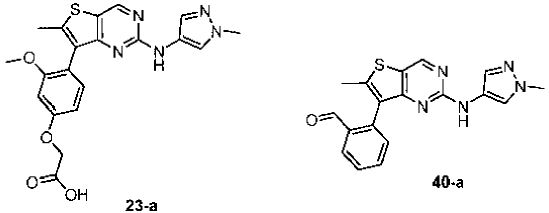

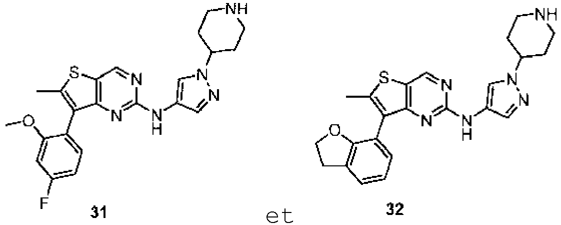

13. Composé de pyrimidine à cycles condensés, tautomère, énantiomère, diastéréoisomère ou sel pharmaceutiquement acceptable de celui-ci selon l'une quelconque des revendications 1 à 8 pour utilisation dans la prévention, le soulagement ou le traitement des maladies choisies dans le groupe constitué d'une maladie du système immunitaire, d'une maladie auto-immune, d'une maladie de prolifération cellulaire, d'un trouble allergique et d'une maladie cardiovasculaire.

14. Composé de pyrimidine à cycles condensés, tautomère, énantiomère, diastéréoisomère ou sel pharmaceutiquement acceptable de celui-ci selon la revendication 13, la maladie du système immunitaire étant un rejet de greffe d'organe ;
et/ou, la maladie auto-immune étant choisie dans le groupe constitué de la polyarthrite rhumatoïde, du psoriasis, de la maladie de Crohn et de la sclérose en plaques ;
et/ou, la maladie de prolifération cellulaire étant choisie dans le groupe constitué de la myélofibrose, d'une tumeur hématologique et d'une tumeur solide ; la tumeur hématologique étant de préférence une leucémie et/ou un lymphome ; la tumeur solide étant choisie dans le groupe constitué d'un cancer rénal, d'un cancer du foie, d'un cancer de l'estomac, d'un cancer du poumon, d'un cancer du sein, d'un cancer de la prostate, d'un cancer du pancréas, d'un cancer de la thyroïde, d'un cancer des ovaires, d'un glioblastome, d'un cancer de la peau et d'un mélanome ;
et/ou, le trouble allergique étant l'asthme bronchique ; et/ou, la maladie cardiovasculaire étant choisie dans le groupe constitué d'une cardiomyopathie ischémique, d'une insuffisance cardiaque et d'un infarctus du myocarde.

15. Composition pharmaceutique, comprenant le composé de pyrimidine à cycles condensés, le tautomère, l'énantiomère, le diastéréoisomère ou le sel pharmaceutiquement acceptable de celui-ci selon l'une quelconque des revendications 1 à 8, et un ou plusieurs véhicule(s) et/ou diluant(s) pharmaceutiquement acceptable(s).

16. Composition pharmaceutique selon la revendication 15, dans laquelle la dose du composé de pyrimidine à cycles condensés, du tautomère, de l'énantiomère, du diastéréoisomère ou du sel pharmaceutiquement acceptable de celui-ci est une quantité thérapeutiquement efficace ;
et/ou, la composition pharmaceutique est utilisée en combinaison avec un ou plusieurs agents chimiothérapeutiques cliniquement utilisés, dont la forme pharmaceutique est, de préférence, une forme pharmaceutique liposomale.
